# EUROPEAN PATENT APPLICATION

(11) **EP 4 012 795 A1**
(43) Date of publication of application: **15.06.2022**
(21) Application number: 21212225.3
(22) Date of filing: 03.12.2021
(51) Int. Cl.: H01L 51/54

(54) **LUMINESCENCE DEVICE AND AMINE COMPOUND FOR ORGANIC ELECTROLUMINESCENCE DEVICE**

(30) Priority: 09.12.2020 KR 20200171688
(71) Applicant: Samsung Display Co., Ltd., Gyeonggi-Do (KR)
(72) Inventor: IMAIZUMI, Taku, Yokohama (JP); UNO, Takuya, Yokohama (JP)
(74) Representative: Shearman, James Ward

(57) **Abstract**

An organic electroluminescence device includes a first electrode, a hole transport region disposed on the first electrode, an emission layer disposed on the hole transport region, an electron transport region disposed on the emission layer, and a second electrode disposed on the electron transport region; the hole transport region includes at least one layer selected from a hole injection layer, a hole transport layer, a buffer layer, and an electron blocking layer; and the at least one layer includes an amine compound represented by Formula 1, thereby exhibiting high luminous efficiency and a long service life:

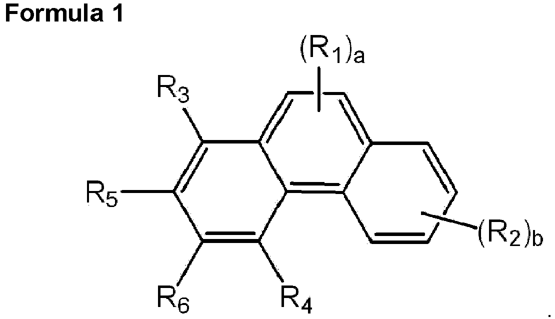

## Description

### BACKGROUND

### 1. Field

One or more aspects of embodiments of the present disclosure relate to an organic electroluminescence device and an amine compound for the organic electroluminescence device.

### 2. Description of Related Art

Organic electroluminescence displays are being actively developed as image displays. Unlike liquid crystal display apparatuses and/or the like, an organic electroluminescence display is a so-called self-luminescent display apparatus, in which holes and electrons injected from a first electrode and a second electrode recombine in an emission layer, and a luminescent material including an organic compound in the emission layer emits light to implement display (e.g., display of images).

In the application of an organic electroluminescence device to a display apparatus, there is a desire for an organic electroluminescence device having a low driving voltage, high luminous efficiency, and/or a long service life (e.g., lifespan), and new materials capable of stably attaining such characteristics for an organic electroluminescence device are desired.

### SUMMARY

One or more aspects of embodiments of the present disclosure are directed toward a luminescence device and an amine compound for an organic electroluminescence device, and for example, also provides a luminescence device having high efficiency and an amine compound included in a hole transport region of the luminescence device.

One or more embodiments of the present disclosure provide an amine compound represented by Formula 1:

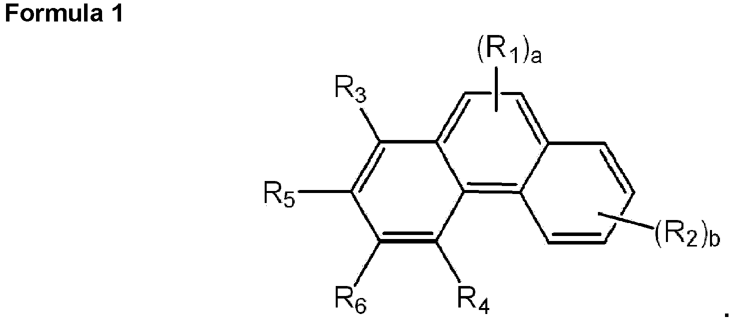

In Formula 1, R₁ to R₄ are each independently a hydrogen atom, a deuterium atom, a halogen atom, or a substituted or unsubstituted alkyl group having 1 to 20 carbon atoms, a is an integer of 0 to 2, b is an integer of 0 to 4, and one of R₅ or R₆ is represented by Formula 2, and the other is a hydrogen atom, a deuterium atom, a halogen atom, or a substituted or unsubstituted alkyl group having 1 to 20 carbon atoms.

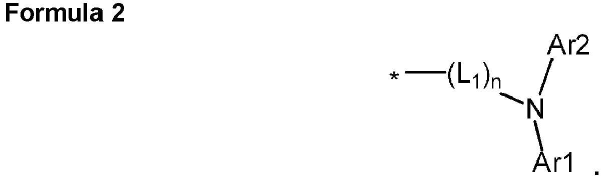

In Formula 2 above, L₁ is a substituted or unsubstituted arylene group having 6 to 30 ring-forming carbon atoms, or a substituted or unsubstituted heteroarylene group having 2 to 30 ring-forming carbon atoms, but does not include a fluorenylene group, a carbazolene group, or an anthracenylene group, n is 1 or 2, Ar1 is represented by any one among Formula 3-1 to Formula 3-3, and Ar2 is a substituted or unsubstituted aryl group having 6 to 30 ring-forming carbon atoms, or a substituted or unsubstituted heteroaryl group having 2 to 30 ring-forming carbon atoms, but does not include a phenanthrene group, a triphenylene group, or a 2-fluorenyl group, with the proviso that when Ar1 is represented by Formula 3-3, Ar2 is a substituted or unsubstituted aryl group having 16 to 30 ring-forming carbon atoms, or a substituted or unsubstituted heteroaryl group having 2 to 30 ring-forming carbon atoms.

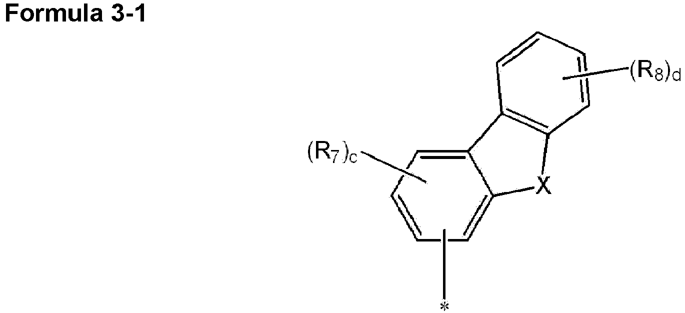

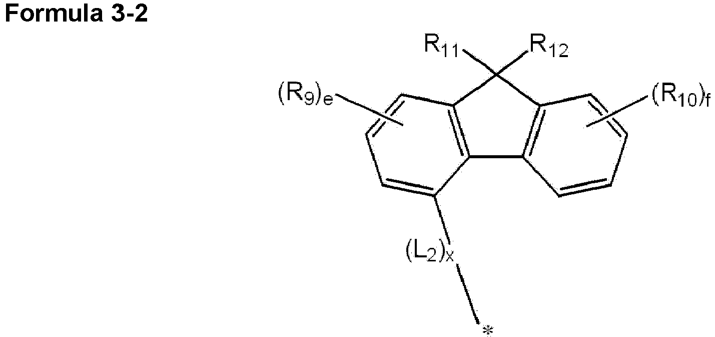

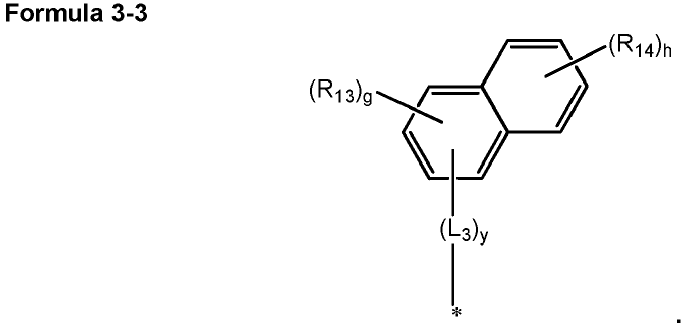

In Formula 3-1 to Formula 3-3, X is O or S, R₇ to R₁₂ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, , or are bonded to an adjacent group to form a ring, but do not include an amine group, R₁₃ and R₁₄ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, but do not include an amine group, L₂ and L₃ are each independently a substituted or unsubstituted arylene group having 6 to 30 ring-forming carbon atoms, or a substituted or unsubstituted heteroarylene group having 2 to 30 ring-forming carbon atoms, c, e, and g are each independently an integer of 0 to 3, d, f, and h are each independently an integer of 0 to 4, and x and y are each independently an integer of 0 to 3.

In an embodiment, the amine compound represented by Formula 1 may be represented by Formula 4 or Formula 5:

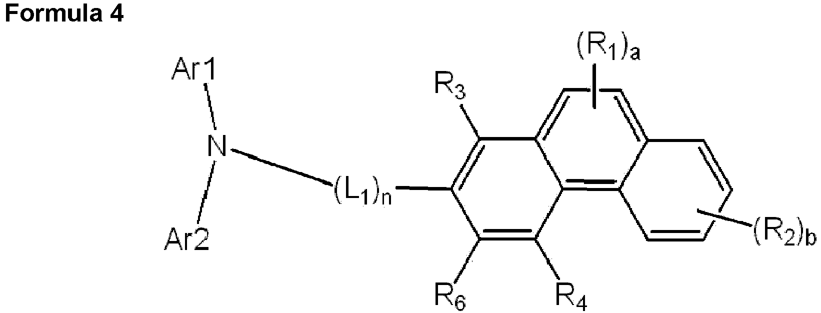

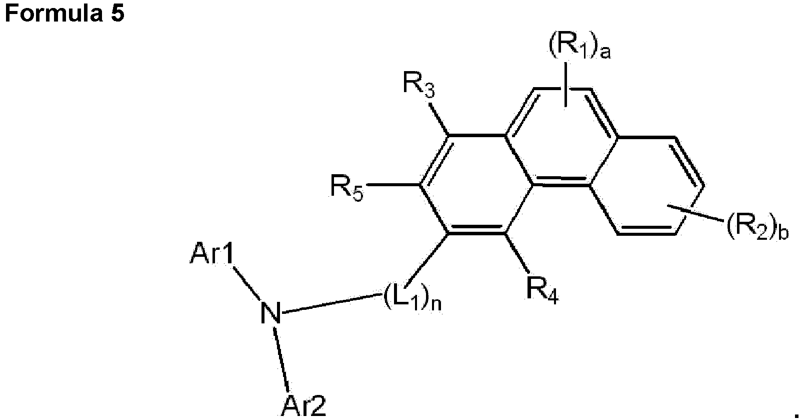

In Formula 4 and Formula 5, R₁ to R₆, Ar1, Ar2, L₁, n, a, and b are each independently the same as defined in Formula 1 and Formula 2.

In an embodiment, the amine compound represented by Formula 1 may be represented by Formula 6-1:

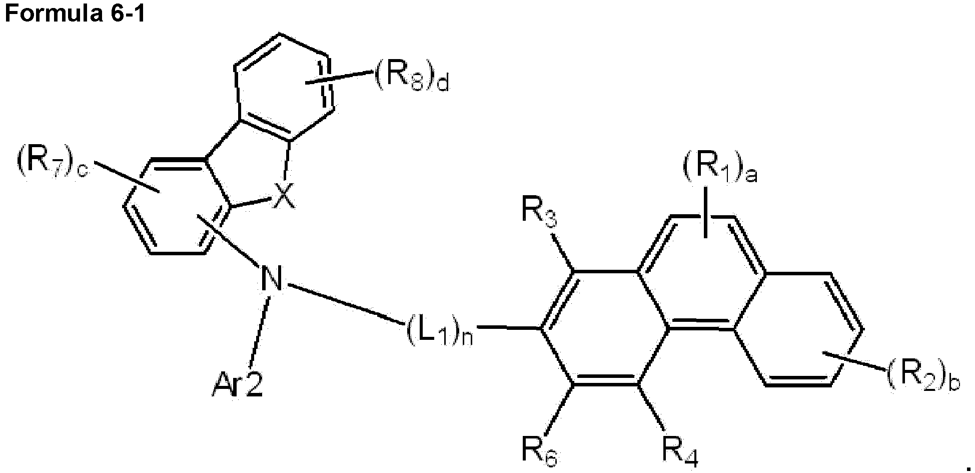

In Formula 6-1, Ar2, R₁ to R₄, R₆ to R₈, L₁, n, X, and a to d are each independently the same as defined in Formula 1 to Formula 3-1.

In an embodiment, the amine compound represented by Formula 1 may be represented by Formula 6-2:

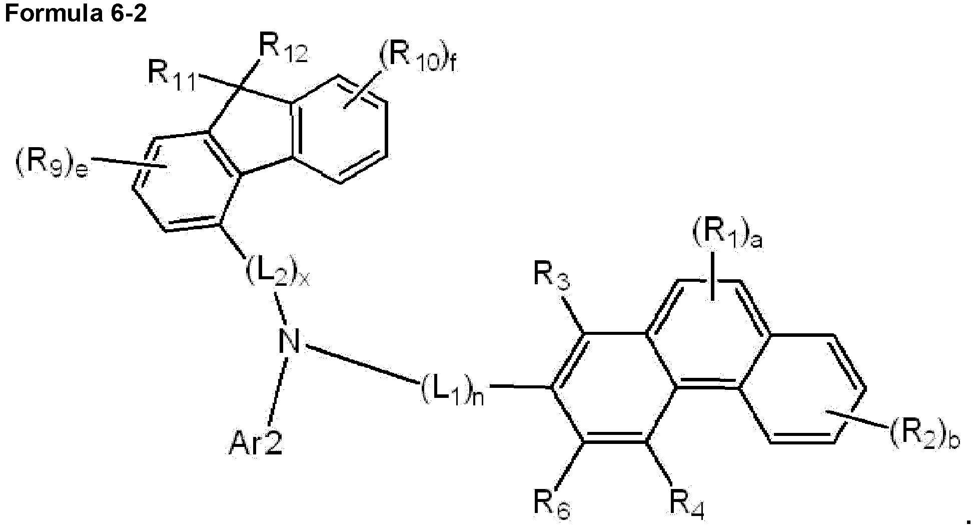

In Formula 6-2, Ar2, R₁ to R₄, R₆, R₉ to R₁₂, L₁, L₂, n, x, a, b, e, and f are each independently the same as defined in Formula 1, Formula 2, and Formula 3-2.

In an embodiment, the amine compound represented by Formula 1 may be represented by Formula 6-3:

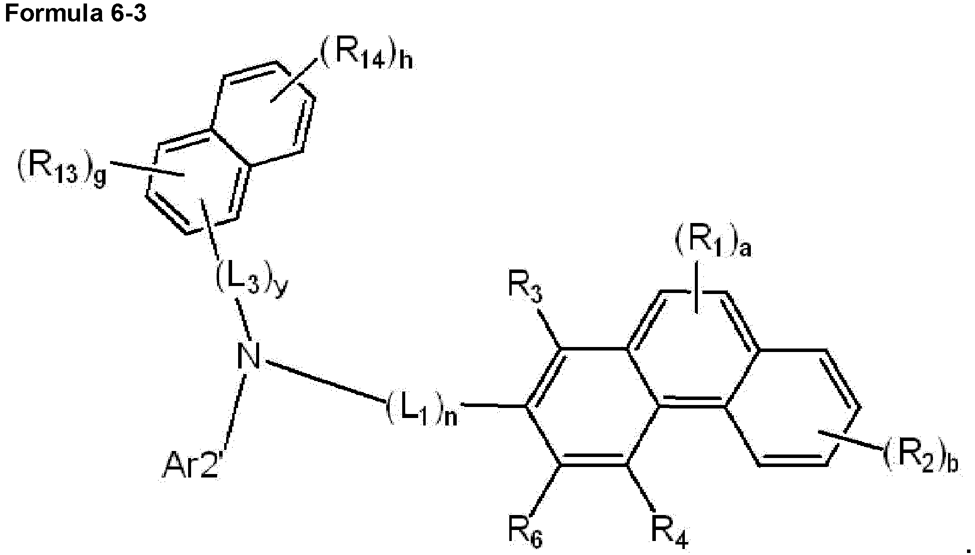

In Formula 6-3, Ar2' is a substituted or unsubstituted aryl group having 16 to 30 ring-forming carbon atoms, or a substituted or unsubstituted heteroaryl group having 2 to 30 ring-forming carbon atoms, but does not include a phenanthrene group, a triphenylene group, or a 2-fluorenyl group, and R₁ to R₄, R₆, R₁₃, R₁₄, L₁, L₃, a, b, n, y, g, and h are each independently the same as defined in Formula 1, Formula 2, and Formula 3-3.

In an embodiment, the amine compound represented by Formula 1 may be represented by Formula 7-1:

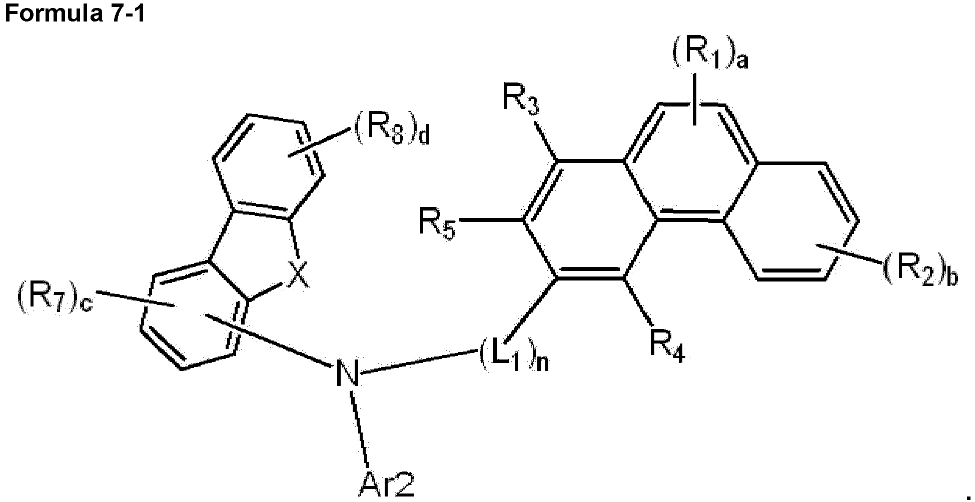

In Formula 7-1, Ar2, R₁ to R₅, R₇, R₈, L₁, n, X, and a to d are each independently the same as defined in Formula 1 to Formula 3-1.

In an embodiment, the amine compound represented by Formula 1 may be represented by Formula 7-2:

### Formula 7-2

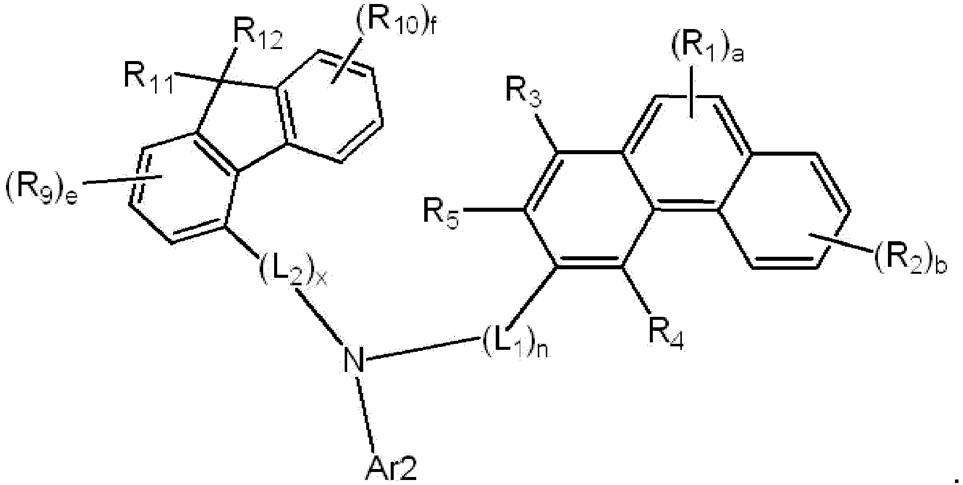

In Formula 7-2, Ar2, R₁ to R₅, R₉ to R₁₂, L₁, L₂, n, x, a, b, e, and f are each independently the same as defined in Formula 1, Formula 2, and Formula 3-2.

In an embodiment, the amine compound represented by Formula 1 may be represented by Formula 7-3:

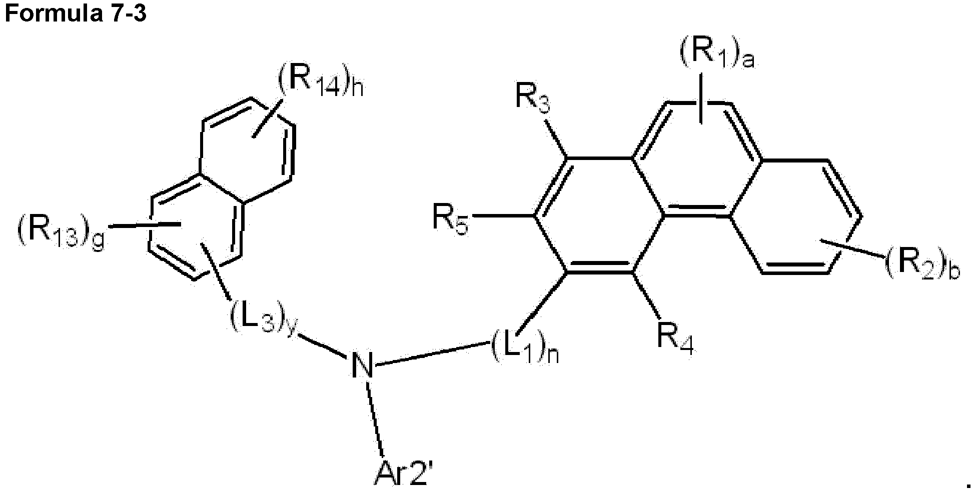

In Formula 7-3, Ar2' is a substituted or unsubstituted aryl group having 16 to 30 ring-forming carbon atoms, or a substituted or unsubstituted heteroaryl group having 2 to 30 ring-forming carbon atoms, but does not include a phenanthrene group, a triphenylene group, or a 2-fluorenyl group, and R₁ to R₅, R₁₃, R₁₄, L₁, L₃, a, b, n, y, g, and h are each independently the same as defined in Formula 1, Formula 2, and Formula 3-3.

In an embodiment, the amine compound represented by Formula 1 may be any one selected from the compounds represented by Compound Group 1 and Compound Group 2.

In an embodiment of the present disclosure, a luminescence device (e.g. an organic electroluminescence device) includes a first electrode, a hole transport region provided on the first electrode, an emission layer provided on the hole transport region, an electron transport region provided on the emission layer, and a second electrode provided on the electron transport region, wherein the hole transport region includes at least one layer selected from a hole injection layer, a hole transport layer, a buffer layer, and an electron blocking layer, and the at least one layer includes the amine compound represented by Formula 1.

In an embodiment, the hole transport region may include the hole injection layer disposed on the first electrode and the hole transport layer disposed on the hole injection layer, and at least one of the hole transport layer or the hole injection layer may include the amine compound represented by Formula 1.

In an embodiment, the hole transport region may include the hole transport layer disposed on the first electrode and the electron blocking layer disposed on the hole transport layer, and the electron blocking layer may include the amine compound represented by Formula 1.

In an embodiment, the hole transport layer may include a compound represented by Formula H-1:

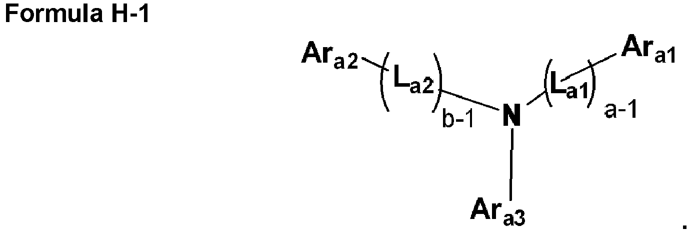

In Formula H-1, Lₐ₁ and Lₐ₂ are each independently a direct linkage, a substituted or unsubstituted arylene group having 6 to 30 ring-forming carbon atoms, or a substituted or unsubstituted heteroarylene group having 2 to 30 ring-forming carbon atoms, a-1 and b-1 are each independently an integer of 0 to 10, and Arₐ₁ to Arₐ₃ are each independently a substituted or unsubstituted aryl group having 6 to 30 ring-forming carbon atoms, or a substituted or unsubstituted heteroaryl group having 2 to 30 ring-forming carbon atoms.

In an embodiment, the emission layer may include a polycyclic compound represented by Formula E-1:

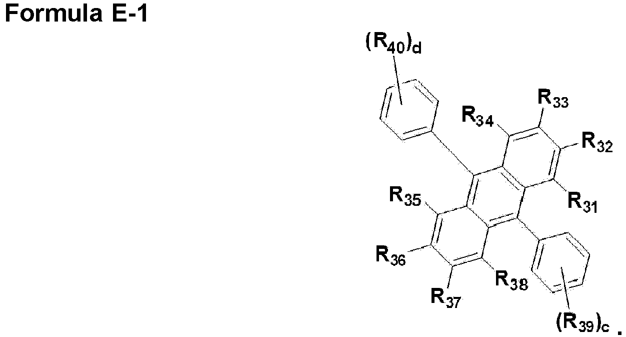

In Formula E-1, R₃₁ to R₄₀ are each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, or are bonded to an adjacent group to form a ring, and c and d are each independently an integer of 0 to 5.

At least some of the above and other features of the invention are set out in the claims.

### BRIEF DESCRIPTION OF THE DRAWINGS

The accompanying drawings are included to provide a further understanding of the present disclosure, and are incorporated in and constitute a part of this specification. The drawings illustrate embodiments of the present disclosure and, together with the description, serve to explain principles of the present disclosure. In the drawings:
FIG. 1 is a plan view of a display apparatus according to an embodiment of the present disclosure;
FIG. 2 is a cross-sectional view of a display apparatus according to an embodiment of the present disclosure;
FIG. 3 is a cross-sectional view schematically illustrating an organic electroluminescence device according to an embodiment of the present disclosure;
FIG. 4 is a cross-sectional view schematically illustrating an organic electroluminescence device according to an embodiment of the present disclosure;
FIG. 5 is a cross-sectional view schematically illustrating an organic electroluminescence device according to an embodiment of the present disclosure;
FIG. 6 is a cross-sectional view schematically illustrating an organic electroluminescence device according to an embodiment of the present disclosure;
FIG. 7 is a cross-sectional view of a display apparatus according to an embodiment of the present disclosure; and
FIG. 8 is a cross-sectional view of a display apparatus according to an embodiment of the present disclosure.

### DETAILED DESCRIPTION

The present disclosure may be modified in many alternate forms, and thus selected embodiments will be illustrated in the drawings and described in more detail. It should be understood, however, that it is not intended to limit the present disclosure to the particular forms disclosed, but rather, is intended to cover all modifications, equivalents, and alternatives falling within the scope of the present disclosure.

When explaining each of the drawings, like reference numbers are used for referring to like elements, and duplicative descriptions thereof may not be provided. In the accompanying drawings, thicknesses and dimensions may be exaggerated for clarity of the present disclosure. It will be understood that, although the terms "first," "second," etc. may be used herein to describe various elements, these elements should not be limited by these terms. These terms are only used to distinguish one element from another. For example, a first element may be referred to as a second element, and, similarly, the second element may be referred to as the first element, without departing from the scope of the present disclosure. Singular forms include plural forms and vice versa unless the context clearly indicates otherwise.

In the present application, it will be understood that the terms "comprise," "include," "have" etc., specify the presence of a feature, a fixed number, a step, an operation, an element, a component, or a combination thereof disclosed in the specification, but do not exclude the possibility of presence or addition of one or more other features, fixed numbers, steps, operations, elements, components, or combination thereof.

In the present application, when a part such as a layer, a film, a region, or a plate is referred to as being "on" or "above" another part, it can be directly on the other part, or an intervening part may also be present. When a part such as a layer, a film, a region, or a plate is referred to as being "under" or "below" another part, it can be directly under the other part, or an intervening part may also be present. When an element is referred to as being "directly on," or "directly below" another element, there are no intervening elements present. It will be understood that when a part is referred to as being "on" another part, it can be disposed on the other part, or disposed under the other part as well.

As used herein, the terms "use," "using," and "used" may be considered synonymous with the terms "utilize," "utilizing," and "utilized," respectively. Further, the use of "may" when describing embodiments of the present disclosure refers to "one or more embodiments of the present disclosure".

Hereinafter, embodiments of the present disclosure will be described with reference to the accompanying drawings.

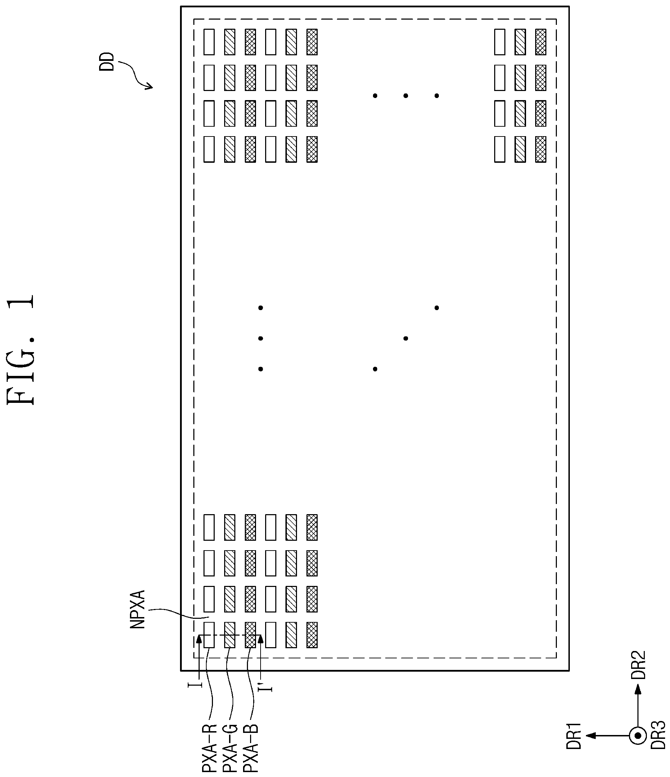

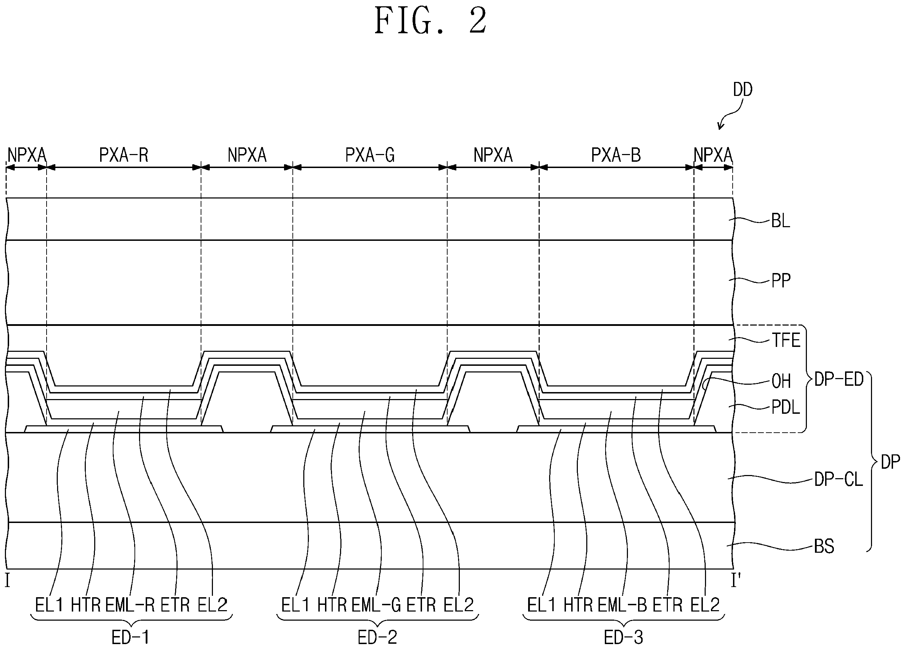

FIG. 1 is a plan view illustrating an embodiment of a display apparatus DD. FIG. 2 is a cross-sectional view of the display apparatus DD of the embodiment. FIG. 2 is a cross-sectional view along line l-l' of FIG. 1.

The display apparatus DD may include a display panel DP and an optical layer PP disposed on the display panel DP. The display panel DP includes organic electroluminescence devices ED-1, ED-2, and ED-3. The display apparatus DD may include a plurality of organic electroluminescence devices ED-1, ED-2, and ED-3. The optical layer PP may be disposed on the display panel DP and may control or reduce reflection of external light in the display panel DP. The optical layer PP may include, for example, a polarization layer and/or a color filter layer. In some embodiments, the optical layer PP may be omitted from the display apparatus DD of an embodiment.

The display panel DP may include a base layer BS, a circuit layer DP-CL provided on the base layer BS, and a display device layer DP-ED. The display device layer DP-ED may include a pixel defining film PDL, the organic electroluminescence devices ED-1, ED-2, and ED-3 disposed between portions of the pixel defining film PDL, and an encapsulation layer TFE disposed on the organic electroluminescence devices ED-1, ED-2, and ED-3.

The base layer BS may provide a base surface on which the display device layer DP-ED is disposed. The base layer BS may be a glass substrate, a metal substrate, a plastic substrate, etc. However, embodiments of the present disclosure are not limited thereto, and the base layer BS may be an inorganic layer, an organic layer, or a composite material layer.

In an embodiment, the circuit layer DP-CL is disposed on the base layer BS, and the circuit layer DP-CL may include a plurality of transistors. Each of the transistors may include a control electrode, an input electrode, and an output electrode. For example, the circuit layer DP-CL may include a switching transistor and/or a driving transistor in order to drive the organic electroluminescence devices ED-1, ED-2, and ED-3 of the display device layer DP-ED.

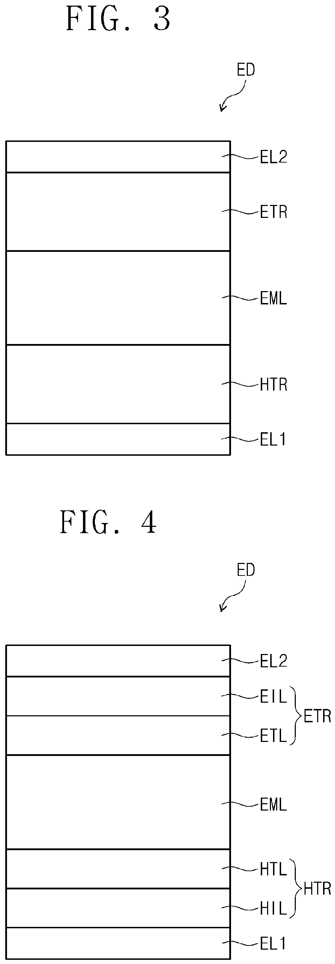

Each of the organic electroluminescence devices ED-1, ED-2, and ED-3 may have a structure of an organic electroluminescence device ED of an embodiment according to FIGS. 3 to 6, which will be described later. Each of the organic electroluminescence devices ED-1, ED-2 and ED-3 may include a first electrode EL1, a hole transport region HTR, emission layers EML-R, EML-G and EML-B, an electron transport region ETR, and a second electrode EL2.

FIG. 2 illustrates an embodiment in which the emission layers EML-R, EML-G, and EML-B of the organic electroluminescence devices ED-1, ED-2, and ED-3 are disposed in the openings OH defined in the pixel defining film PDL, and the hole transport region HTR, the electron transport region ETR, and the second electrode EL2 are provided as a common layer in the entire organic electroluminescence devices ED-1, ED-2, and ED-3. However, embodiments of the present disclosure are not limited thereto, and for example, the hole transport region HTR and the electron transport region ETR in an embodiment may be provided by being patterned inside the opening hole OH defined in the pixel defining film PDL. For example, the hole transport region HTR, the emission layers EML-R, EML-G, and EML-B, and the electron transport region ETR of the organic electroluminescence devices ED-1, ED-2, and ED-3 in an embodiment may be provided by being patterned in an inkjet printing method.

The encapsulation layer TFE may cover the organic electroluminescence devices ED-1, ED-2 and ED-3. The encapsulation layer TFE may seal the display device layer DP-ED. The encapsulation layer TFE may be a thin film encapsulation layer. The encapsulation layer TFE may be formed by laminating one layer or a plurality of layers. The encapsulation layer TFE includes at least one insulation layer. The encapsulation layer TFE according to an embodiment may include at least one inorganic film (hereinafter, an encapsulation-inorganic film). The encapsulation layer TFE according to an embodiment may also include at least one organic film (hereinafter, an encapsulation-organic film) and at least one encapsulation-inorganic film.

The encapsulation-inorganic film may protect the display device layer DP-ED from moisture/oxygen, and the encapsulation-organic film may protect the display device layer DP-ED from foreign substances (such as dust particles). The encapsulation-inorganic film may include silicon nitride, silicon oxynitride, silicon oxide, titanium oxide, aluminium oxide, and/or the like, but embodiments of the present disclosure are not limited thereto. The encapsulation-organic film may include an acrylic-based compound, an epoxy-based compound, and/or the like. The encapsulation-organic film may include a photopolymerizable organic material, but embodiments of the present disclosure are not particularly limited thereto.

The encapsulation layer TFE may be disposed on the second electrode EL2 and may be disposed filling the opening hole OH.

Referring to FIGS. 1 and 2, the display apparatus DD may include a non-light emitting region NPXA and light emitting regions PXA-R, PXA-G and PXA-B. The light emitting regions PXA-R, PXA-G and PXA-B may each be to emit light generated from the luminescence devices ED-1, ED-2 and ED-3, respectively. The light emitting regions PXA-R, PXA-G, and PXA-B may be spaced apart from each other in a plane.

Each of the light emitting regions PXA-R, PXA-G, and PXA-B may be a region divided (e.g., separated) by the pixel defining film PDL. The non-light emitting regions NPXA may be between the adjacent light emitting regions PXA-R, PXA-G, and PXA-B, and may correspond to portions of the pixel defining film PDL. Each of the light emitting regions PXA-R, PXA-G, and PXA-B may correspond to a pixel. The pixel defining film PDL may separate the organic electroluminescence devices ED-1, ED-2 and ED-3. The emission layers EML-R, EML-G and EML-B of the organic electroluminescence devices ED-1, ED-2 and ED-3 may be disposed in openings OH defined by the pixel defining film PDL and separated from each other.

The light emitting regions PXA-R, PXA-G and PXA-B may be divided into a plurality of groups according to the color of light generated from the organic electroluminescence devices ED-1, ED-2 and ED-3. In the display apparatus DD of an embodiment shown in FIGS. 1 and 2, three light emitting regions PXA-R, PXA-G, and PXA-B (which emit red light, green light, and blue light, respectively) are illustrated. For example, the display apparatus DD of an embodiment may include the red light emitting region PXA-R, the green light emitting region PXA-G, and the blue light emitting region PXA-B, which are separated from one another.

In the display apparatus DD according to an embodiment, the plurality of organic electroluminescence devices ED-1, ED-2 and ED-3 may be to emit light beams having wavelengths different from one another. For example, in an embodiment, the display apparatus DD may include a first organic electroluminescence device ED-1 to emit red light, a second organic electroluminescence device ED-2 to emit green light, and a third organic electroluminescence device ED-3 to emit blue light. For example, the red light emitting region PXA-R, the green light emitting region PXA-G, and the blue light emitting region PXA-B of the display apparatus DD may correspond to the first organic electroluminescence device ED-1, the second organic electroluminescence device ED-2, and the third organic electroluminescence device ED-3, respectively.

However, embodiments of the present disclosure are not limited thereto, and the first to third organic electroluminescence devices ED-1, ED-2, and ED-3 may be to emit light beams in substantially the same wavelength range, or at least one organic electroluminescence device may be to emit a light beam in a wavelength range different from the others. For example, the first to third organic electroluminescence devices ED-1, ED-2, and ED-3 may all be to emit blue light.

The light emitting regions PXA-R, PXA-G, and PXA-B in the display apparatus DD according to an embodiment may be arranged in a stripe form. Referring to FIG. 1, the plurality of red light emitting regions PXA-R may be arranged with each other along a second directional axis DR2, the plurality of green light emitting regions PXA-G may be arranged with each other along the second directional axis DR2, and the plurality of blue light emitting regions PXA-B may be arranged with each along the second directional axis DR2. In some embodiments, a red light emitting region PXA-R, a green light emitting region PXA-G, and a blue light emitting region PXA-B may be alternatingly arranged with each other in this order along a first directional axis DR1.

FIGS. 1 and 2 illustrate that all the light emitting regions PXA-R, PXA-G, and PXA-B have similar areas, but embodiments of the present disclosure are not limited thereto, and the light emitting regions PXA-R, PXA-G, and PXA-B may have different areas from each other according to a wavelength range of the emitted light. In this case, the areas of the light emitting regions PXA-R, PXA-G, and PXA-B may refer to areas when viewed in a plane defined by the first directional axis DR1 and the second directional axis DR2 (e.g., in a plan view).

The arrangement form of the light emitting regions PXA-R, PXA-G, and PXA-B is not limited to the feature illustrated in FIG. 1, and the order in which the red light emitting region PXA-R, the green light emitting region PXA-G, and the blue light emitting region PXA-B are arranged may be variously combined and provided according to characteristics of a display quality required in the display apparatus DD. For example, the arrangement form of the light emitting regions PXA-R, PXA-G, and PXA-B may be a PENTILE^{®} arrangement form or a diamond arrangement form.

In some embodiments, the areas of the light emitting regions PXA-R, PXA-G, and PXA-B may be different from each other. For example, in an embodiment, the area of the green light emitting region PXA-G may be smaller than that of the blue light emitting region PXA-B, but embodiments of the present disclosure are not limited thereto.

Hereinafter, FIGS. 3 to 6 are cross-sectional views schematically illustrating organic electroluminescence devices according to embodiments. Each of the organic electroluminescence devices ED according to embodiments may include a first electrode EL1, a hole transport region HTR, an emission layer EML, an electron transport region ETR, and a second electrode EL2 that are sequentially stacked.

The organic electroluminescence device ED of an embodiment includes an amine compound of an embodiment, which will be described later, in the hole transport region HTR disposed between the first electrode EL1 and the second electrode EL2.

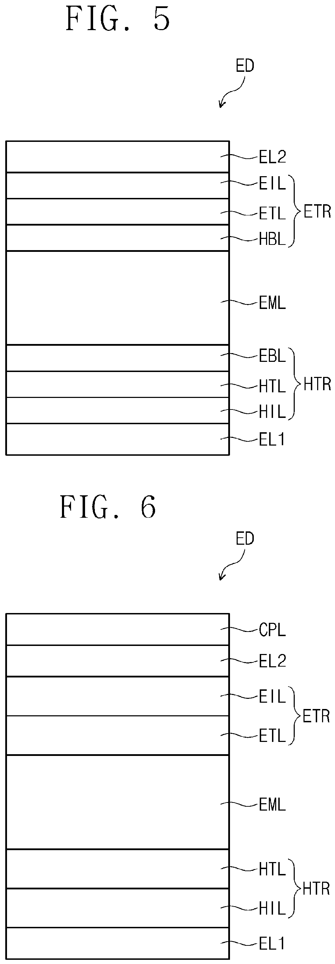

Compared to FIG. 3, FIG. 4 illustrates a cross-sectional view of an organic electroluminescence device ED of an embodiment, in which a hole transport region HTR includes a hole injection layer HIL and a hole transport layer HTL, and an electron transport region ETR includes an electron injection layer EIL and an electron transport layer ETL. Compared to FIG. 3, FIG. 5 illustrates a cross-sectional view of an organic electroluminescence device ED of an embodiment, in which a hole transport region HTR includes a hole injection layer HIL, a hole transport layer HTL, and an electron blocking layer EBL, and an electron transport region ETR includes an electron injection layer EIL, an electron transport layer ETL, and a hole blocking layer HBL. Compared to FIG. 4, FIG. 6 illustrates a cross-sectional view of an organic electroluminescence device ED of an embodiment including a capping layer CPL disposed on a second electrode EL2.

The first electrode EL1 has conductivity. The first electrode EL1 may be formed of a metal alloy or a conductive compound. The first electrode EL1 may be an anode or a cathode. However, embodiments of the present disclosure are not limited thereto. In some embodiments, the first electrode EL1 may be a pixel electrode. The first electrode EL1 may be a transmissive electrode, a transflective electrode, or a reflective electrode. When the first electrode EL1 is a transmissive electrode, the first electrode EL1 may be formed utilizing a transparent metal oxide (such as indium tin oxide (ITO), indium zinc oxide (IZO), zinc oxide (ZnO), and/or indium tin zinc oxide (ITZO)). When the first electrode EL1 is the transflective electrode or the reflective electrode, the first electrode EL1 may include silver (Ag), magnesium (Mg), copper (Cu), aluminium (Al), platinum (Pt), palladium (Pd), gold (Au), nickel (Ni), neodymium (Nd), iridium (Ir), chromium (Cr), lithium (Li), calcium (Ca), LiF/calcium (Ca), LiF/aluminium (Al), molybdenum (Mo), titanium (Ti), a compound thereof, or a mixture thereof (for example, a mixture of Ag and Mg). In some embodiments, the first electrode EL1 may have a multilayer structure including a reflective layer or a transflective layer formed of the above-described materials, and a transparent conductive layer formed of ITO, IZO, ZnO, ITZO, etc. For example, the first electrode EL1 may have a three-layer structure of ITO/Ag/ITO, but embodiments of the present disclosure are not limited thereto. The thickness of the first electrode EL1 may be about 700 Å to about 10,000 Å. For example, the thickness of the first electrode EL1 may be about 1,000 Å to about 3,000 Å.

The hole transport region HTR is provided on the first electrode EL1. The hole transport region HTR may include at least one of a hole injection layer HIL, a hole transport layer HTL, a hole buffer layer, or an electron blocking layer EBL. The thickness of the hole transport region HTR may be, for example, about 50 Å to about 15,000 Å.

The hole transport region HTR may have a single layer formed of a single material, a single layer formed of a plurality of different materials, or a multilayer structure including a plurality of layers formed of a plurality of different materials.

For example, the hole transport region HTR may have a single layer structure of the hole injection layer HIL or the hole transport layer HTL, or may have a single layer structure formed of a hole injection material and a hole transport material. In some embodiments, the hole transport region HTR may have a single layer structure formed of a plurality of different materials, or a structure in which a hole injection layer HIL/hole transport layer HTL, a hole injection layer HIL/hole transport layer HTL/hole buffer layer, a hole injection layer HIL/hole buffer layer, a hole transport layer HTL/hole buffer layer, or a hole injection layer HIL/hole transport layer HTL/electron blocking layer EBL are stacked in order from the first electrode EL1, but embodiments of the present disclosure are not limited thereto.

The hole transport region HTR in the luminescence device ED of an embodiment includes an amine compound according to an embodiment of the present disclosure. For example, the hole transport region HTR may include at least one layer selected from a hole injection layer HIL, a hole transport layer HTL, a buffer layer or an emission-auxiliary layer, and an electron blocking layer EBL, and the at least one layer included in the hole transport region HTR may include an amine compound according to an embodiment of the present disclosure.

The term "substituted or unsubstituted" herein may refer to being unsubstituted, or substituted with at least one substituent selected from the group consisting of a deuterium atom, a halogen atom, a cyano group, a nitro group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, an alkoxy group, a hydrocarbon ring group, an aryl group, and a heterocyclic group. Each of the listed substituents may be further substituted or unsubstituted. For example, a biphenyl group may be interpreted as an aryl group or as a phenyl group substituted with a phenyl group.

In the specification, examples of the halogen atom may include a fluorine atom, a chlorine atom, a bromine atom, and an iodine atom.

In the specification, the phrase "bonded to an adjacent group to form a ring" may refer to being bonded to an adjacent group to form a substituted or unsubstituted hydrocarbon ring, or a substituted or unsubstituted heterocycle. The term "hydrocarbon ring" includes an aliphatic hydrocarbon ring and an aromatic hydrocarbon ring. The term "heterocycle" includes an aliphatic heterocycle and an aromatic heterocycle. The hydrocarbon ring and the heterocycle may be monocyclic or polycyclic. In some embodiments, the rings formed by being bonded to each other may be connected to another ring to form a spiro structure.

In the specification, the term "adjacent group" may refer to a substituent on the same atom or point, a substituent on an atom that is directly connected to the base atom or point, or a substituent sterically positioned (e.g., within intramolecular bonding distance) to the corresponding substituent. For example, two methyl groups in 1,2-dimethylbenzene may be interpreted as "adjacent groups" to each other and two ethyl groups in 1,1-diethylcyclopentane may be interpreted as "adjacent groups" to each other. Two methyl groups in 4,5-dimethylphenanthrene may be interpreted as "adjacent groups" to each other.

In the specification, the alkyl group may be a linear, branched or cyclic alkyl. The number of carbon atoms in the alkyl group is 1 to 50, 1 to 30, 1 to 20, 1 to 10, or 1 to 6. Examples of the alkyl group may include a methyl group, an ethyl group, an n-propyl group, an isopropyl group, an n-butyl group, an s-butyl group, a t-butyl group, an i-butyl group, a 2-ethylbutyl group, a 3,3-dimethylbutyl group, an n-pentyl group, an i-pentyl group, a neopentyl group, a t-pentyl group, a cyclopentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, a cyclohexyl group, a 4-methylcyclohexyl group, a 4-t-butylcyclohexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, a cyclooctyl group, an n-nonyl group, an n-decyl group, an adamantyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-eicosyl group, a 2-ethyleicosyl group, a 2-butyleicosyl group, a 2-hexyleicosyl group, a 2-octyleicosyl group, an n-heneicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, etc., but embodiments of the present disclosure are not limited thereto.

In the specification, the term "alkenyl group" refers to a hydrocarbon group including at least one carbon double bond in the middle or terminal end of an alkyl group having 2 or more carbon atoms. The alkenyl group may be a linear chain or a branched chain. The carbon number is not limited, but may be 2 to 30, 2 to 20 or 2 to 10. Examples of the alkenyl group may include a vinyl group, a 1-butenyl group, a 1-pentenyl group, a 1,3-butadienyl aryl group, a styrenyl group, a styrylvinyl group, etc., without limitation.

In the specification, the term "alkynyl group" refers to a hydrocarbon group including at least one carbon triple bond in the middle or terminal end of an alkyl group having 2 or more carbon atoms. The alkynyl group may be a linear chain or a branched chain. The carbon number is not limited, but may be 2 to 30, 2 to 20 or 2 to 10. Examples of the alkynyl group include an ethynyl group, a propynyl group, etc., without limitation.

In the specification, a hydrocarbon ring group may be any functional group or substituent derived from an aliphatic hydrocarbon ring, or any functional group or substituent derived from an aromatic hydrocarbon ring. The number of ring-forming carbon atoms in the hydrocarbon ring group may be 5 to 60, 5 to 30, or 5 to 20.

The term "aryl group" herein refers to any functional group or substituent derived from an aromatic hydrocarbon ring. The aryl group may be a monocyclic aryl group or a polycyclic aryl group. The number of ring-forming carbon atoms in the aryl group may be 6 to 36, 6 to 30, 6 to 20, or 6 to 15. Examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl group, etc., but embodiments of the present disclosure are not limited thereto.

In the description, the fluorenyl group may be substituted, and two substituents may be combined with each other to form a spiro structure. Examples of cases where the fluorenyl group is substituted are as follows. However, embodiments of the present disclosure are not limited thereto.

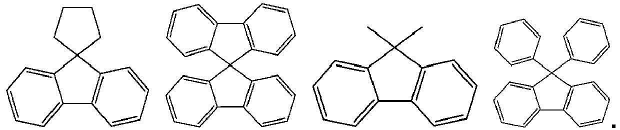

In the specification, the term "heterocyclic group" refers to any functional group or substituent derived from a ring containing at least one of boron (B), oxygen (O), nitrogen (N), phosphorus (P), silicon (Si), or sulfur (S) as a heteroatom. The heterocyclic group includes an aliphatic heterocyclic group and an aromatic heterocyclic group. The aromatic heterocyclic group may be a heteroaryl group. The aliphatic heterocycle and the aromatic heterocycle may be monocyclic or polycyclic.

In the specification, the heterocyclic group may include at least one of B, O, N, P, Si or S as a heteroatom. When the heterocyclic group includes two or more heteroatoms, the two or more heteroatoms may be the same as or different from each other. The heterocyclic group may be a monocyclic heterocyclic group or a polycyclic heterocyclic group and has the concept including a heteroaryl group. The number of ring-forming carbon atoms in the heterocyclic group may be 2 to 30, 2 to 20, or 2 to 10.

In the specification, the aliphatic heterocyclic group may include at least one of B, O, N, P, Si, or S as a heteroatom. The number of ring-forming carbon atoms in the aliphatic heterocyclic group may be 2 to 30, 2 to 20, or 2 to 10. Examples of the aliphatic heterocyclic group include an oxirane group, a thiirane group, a pyrrolidine group, a piperidine group, a tetrahydrofuran group, a tetrahydrothiophene group, a thiane group, a tetrahydropyran group, a 1,4-dioxane group, etc., but embodiments of the present disclosure are not limited thereto.

In the specification, the heteroaryl group may include at least one of B, O, N, P, Si, or S as a heteroatom. When the heteroaryl group contains two or more hetero atoms, the two or more hetero atoms may be the same as or different from each other. The heteroaryl group may be a monocyclic heteroaryl group or polycyclic heteroaryl group. The number of ring-forming carbon atoms in the heteroaryl group may be 2 to 30, 2 to 20, or 2 to 10. Examples of the heteroaryl group may include a thiophene group, a furan group, a pyrrole group, an imidazole group, a triazole group, a pyridine group, a bipyridine group, a pyrimidine group, a triazine group, a triazole group, an acridyl group, a pyridazine group, a pyrazinyl group, a quinoline group, a quinazoline group, a quinoxaline group, a phenoxazine group, a phthalazine group, a pyrido pyrimidine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzimidazole group, a benzothiazole group, a benzocarbazole group, a benzothiophene group, a dibenzothiophene group, a thienothiophene group, a benzofuran group, a phenanthroline group, a thiazole group, an isoxazole group, an oxazole group, an oxadiazole group, a thiadiazole group, a phenothiazine group, a dibenzosilole group, a dibenzofuran group, etc., but embodiments of the present disclosure are not limited thereto.

In the specification, the number of carbon atoms in an amine group is not limited, but may be 1 to 30. The amine group may include an alkyl amine group, an aryl amine group or a heteroaryl amine group. Examples of the amine group may include a methylamine group, a dimethylamine group, a phenylamine group, a diphenylamine group, a naphthylamine group, a 9-methyl-anthracenylamine group, a triphenylamine group, etc., but embodiments of the present disclosure are not limited thereto.

In the specification, the above description with respect to the aryl group may be applied to an arylene group, except that the arylene group is a divalent group.

In the specification, the above description with respect to the heteroaryl group may be applied to a heteroarylene group except that the heteroarylene group is a divalent group.

In the specification, the term "silyl group" refers to an alkyl silyl group or an aryl silyl group. Non-limiting examples of the silyl group include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, etc.

In the specification, the term "boron group" refers to an alkyl boryl group or an aryl boryl group. Non-limiting examples of the boryl group include a trimethylboryl group, a triethylboryl group, a t-butyldimethylboryl group, a triphenylboryl group, a diphenylboryl group, a phenylboryl group, etc.

In the specification, the term "oxy group" may refer to an alkoxy group or an aryl oxy group. The alkoxy group may include a linear, branched or cyclic alkyl chain. The number of carbons in the alkoxy group is not specifically limited, but may be, for example, 1 to 20 or 1 to 10. Non-limiting examples of the oxy group include methoxy, ethoxy, n-propoxy, isopropoxy, butoxy, pentyloxy, hexyloxy, octyloxy, nonyloxy, decyloxy, benzyloxy, etc.

In the specification, the phosphine oxide group may be substituted with, for example, at least one of alkyl or aryl.

In the specification, the phosphine sulfide group may be substituted with, for example, at least one of alkyl or aryl.

In some embodiments, "—∗" herein refers to a position to be connected.

The amine compound according to an embodiment of the present disclosure is represented by Formula 1:

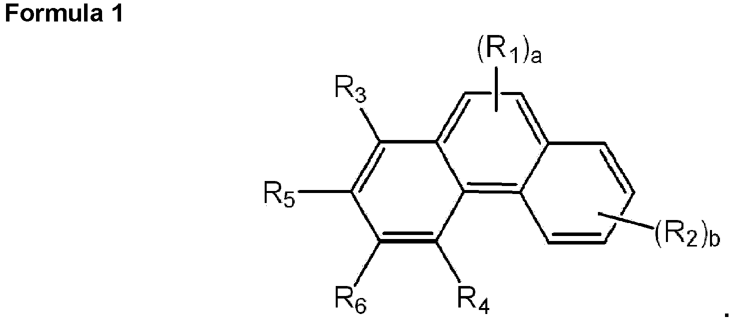

In Formula 1, R₁ to R₄ are each independently a hydrogen atom, a deuterium atom, a halogen atom, or a substituted or unsubstituted alkyl group having 1 to 20 carbon atoms. For example, R₁ to R₄ may each independently be a hydrogen atom, a deuterium atom, a halogen atom, or a substituted or unsubstituted alkyl group having 1 to 10 (or 1 to 6) carbon atoms.

In Formula 1, a is an integer of 0 to 2, and b is an integer of 0 to 4. When a is 2, a plurality of R₁'s may be the same as or different from each other, and when b is 2 or more, a plurality of R₂'s may be the same as or different from each other.

In Formula 1, one of R₅ or R₆ is represented by Formula 2, and the other is a hydrogen atom, a deuterium atom, a halogen atom, or a substituted or unsubstituted alkyl group having 1 to 20 carbon atoms. For example, in Formula 1, one of R₅ or R₆ may be represented by Formula 2, and the other is a hydrogen atom, a deuterium atom, a halogen atom, or a substituted or unsubstituted alkyl group having 1 to 10 (or 1 to 6) carbon atoms.

In Formula 1, R₅ may be represented by Formula 2, and R₆ may be a hydrogen atom, a deuterium atom, a halogen atom, or a substituted or unsubstituted alkyl group having 1 to 20 carbon atoms. For example, in Formula 1, R₅ may be represented by Formula 2, and R₆ may be a hydrogen atom, a deuterium atom, a halogen atom, or a substituted or unsubstituted alkyl group having 1 to 10 (or 1 to 6) carbon atoms.

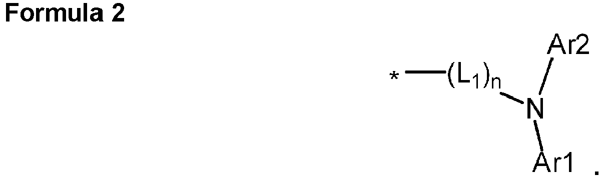

In Formula 2, L₁ is a substituted or unsubstituted arylene group having 6 to 30 ring-forming carbon atoms, or a substituted or unsubstituted heteroarylene group having 2 to 30 ring-forming carbon atoms. For example, in Formula 2, L₁ may be a substituted or unsubstituted arylene group having 6 to 20 ring-forming carbon atoms, or a substituted or unsubstituted heteroarylene group having 2 to 20 ring-forming carbon atoms. However, L₁ does not include a fluorenylene group, a carbazolene group, or an anthracenylene group. For example, L₁ is not a fluorenylene group, a carbazolene group, or an anthracenylene group, and does not include a fluorenylene group, a carbazolene group, or an anthracenylene group as a substituent, either.

In Formula 2, n is 1 or 2, and when n is 2, a plurality of L₁'s may be the same as or different from each other.

In Formula 2, Ar1 is represented by any one among Formula 3-1 to Formula 3-3:

In Formula 2, Ar2 is a substituted or unsubstituted aryl group having 6 to 30 ring-forming carbon atoms, or a substituted or unsubstituted heteroaryl group having 2 to 30 ring-forming carbon atoms, but does not include a phenanthrene group, a triphenylene group, or a 2-fluorenyl group. For example, in Formula 2, Ar2 may be a substituted or unsubstituted aryl group having 6 to 20 ring-forming carbon atoms, or a substituted or unsubstituted heteroaryl group having 2 to 20 ring-forming carbon atoms, but does not include a phenanthrene group, a triphenylene group, or a 2-fluorenyl group. For example, Ar2 is not a phenanthrene group, a triphenylene group, or a 2-fluorenyl group, and does not include a phenanthrene group, a triphenylene group, or a 2-fluorenyl group as a substituent, either.

However, when Ar1 of Formula 2 is represented by Formula 3-3, Ar2 is a substituted or unsubstituted aryl group having 16 to 30 ring-forming carbon atoms, or a substituted or unsubstituted heteroaryl group having 2 to 30 ring-forming carbon atoms. For example, when Ar1 of Formula 2 is represented by Formula 3-3, Ar2 may be a substituted or unsubstituted aryl group having 16 to 30 ring-forming carbon atoms, or a substituted or unsubstituted heteroaryl group having 2 to 20 ring-forming carbon atoms. In these cases, Ar2 does not include a phenanthrene group, a triphenylene group, or a 2-fluorenyl group as a substituent, either.

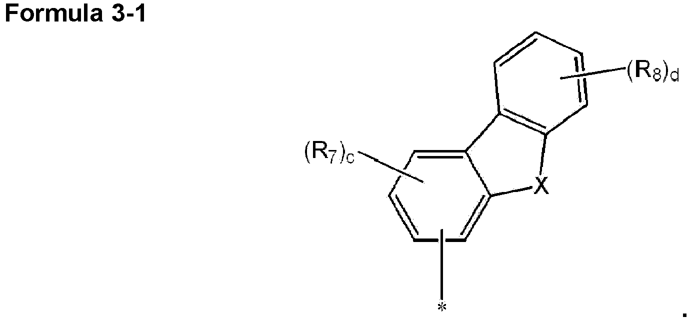

In Formula 3-1, X is O or S.

In Formula 3-1, R₇ and R₈ are each independently a hydrogen atom, a deuterium atom, a halogen atom, a substituted or unsubstituted alkyl group having 1 to 20 (e.g. 1 to 10 or 1 to 6) carbon atoms, a substituted or unsubstituted aryl group having 6 to 30 (e.g. 6 to 20) ring-forming carbon atoms, or a substituted or unsubstituted heteroaryl group having 2 to 30 (e.g. 2 to 20) ring-forming carbon atoms, and/or are bonded to an adjacent group to form a ring. For example, when c is 2 or more and a plurality of R₇'s are adjacent, the adjacent plurality of R₇'s may be bonded to each other to form a ring. In some embodiments, when d is 2 or more and a plurality of R₈'s are adjacent, the adjacent plurality of R₈'s may be bonded to each other to form a ring.

However, R₇ and R₈ do not include an amine group. Specifically R₇ and R₈ are not amine groups and do not include an amine group as a substituent, either.

In Formula 3-1, c is an integer of 0 to 3, meanwhile, when c is 2 or more, a plurality of R₇'s may be the same as or different from each other.

In Formula 3-1, d is an integer of 0 to 4, meanwhile, when d is 2 or more, a plurality of R₈'s may be the same as or different from each other.

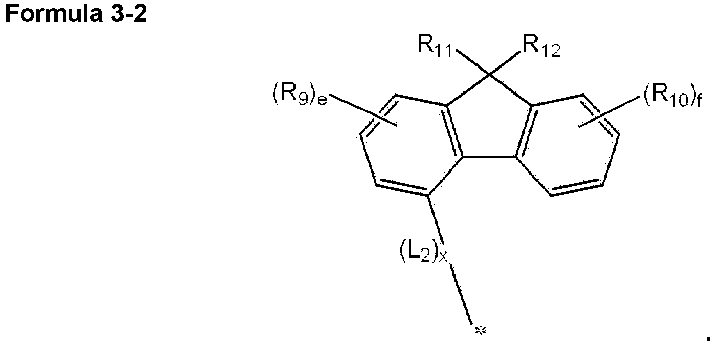

In Formula 3-1, R₉ to R₁₂ are each independently a hydrogen atom, a deuterium atom, a halogen atom, a substituted or unsubstituted alkyl group having 1 to 20 (e.g. 1 to 10 or 1 to 6) carbon atoms, a substituted or unsubstituted aryl group having 6 to 30 (*e.g*. 6 to 20) ring-forming carbon atoms, or a substituted or unsubstituted heteroaryl group having 2 to 30 (*e.g*. 2 to 20) ring-forming carbon atoms, and/or are bonded to an adjacent group to form a ring. For example, when e is 2 or more, and a plurality of R₉'s are adjacent, the adjacent plurality of R₉'s may be bonded to each other to form a ring. In some embodiments, when f is 2 or more, and a plurality of R₁₀'s are adjacent, the adjacent plurality of R₁₀'s may be bonded to each other to form a ring. However, R₉ to R₁₂ do not include an amine group. For example, R₉ to R₁₂ are not amine groups and do not include an amine group as a substituent, either.

In Formula 3-2, e is an integer of 0 to 3, meanwhile, when e is 2 or more, a plurality of Rg's may be the same as or different from each other.

In Formula 3-2, f is an integer of 0 to 4, meanwhile, when f is 2 or more, a plurality of R₁₀'s may be the same as or different from each other.

In Formula 3-2, L₂ is a substituted or unsubstituted arylene group having 6 to 30 ring-forming carbon atoms, or a substituted or unsubstituted heteroarylene group having 2 to 30 ring-forming carbon atoms. For example, in Formula 3-2, L₂ may be a substituted or unsubstituted arylene group having 6 to 20 ring-forming carbon atoms, or a substituted or unsubstituted heteroarylene group having 2 to 20 ring-forming carbon atoms.

In Formula 3-2, x is an integer of 0 to 3, meanwhile, when x is 2 or more, a plurality of L₂'s may be the same as or different from each other.

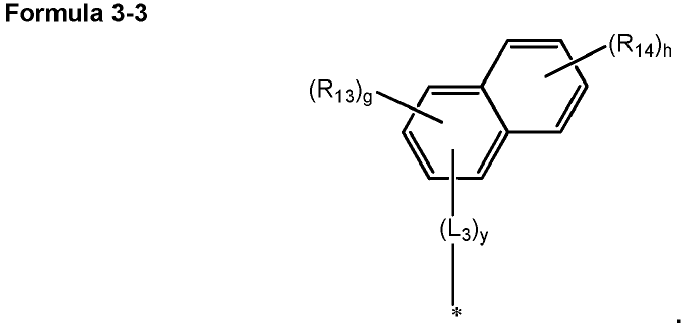

In Formula 3-3, R₁₃ and R₁₄ are each independently a hydrogen atom, a deuterium atom, a halogen atom, a substituted or unsubstituted alkyl group having 1 to 20 (e.g. 1 to 10 or 1 to 6) carbon atoms, a substituted or unsubstituted aryl group having 6 to 30 (e.g. 6 to 20) ring-forming carbon atoms, or a substituted or unsubstituted heteroaryl group having 2 to 30 (*e.g.* 2 to 20) ring-forming carbon atoms. However, R₁₃ and R₁₄ do not include an amine group. For example, R₁₃ and R₁₄ are not amine groups and do not include an amine group as a substituent, either.

In Formula 3-3, g is an integer of 0 to 3, meanwhile, when g is 2 or more, a plurality of R₁₃'s may be the same as or different from each other.

In Formula 3-3, h is an integer of 0 to 4, meanwhile, when h is 2 or more, a plurality of R₁₄'s may be the same as or different from each other.

In Formula 3-3, L₃ is a substituted or unsubstituted arylene group having 6 to 30 ring-forming carbon atoms, or a substituted or unsubstituted heteroarylene group having 2 to 30 ring-forming carbon atoms. For example, in Formula 3-3, L₃ may be a substituted or unsubstituted arylene group having 6 to 20 ring-forming carbon atoms, or a substituted or unsubstituted heteroarylene group having 2 to 20 ring-forming carbon atoms.

In Formula 3-3, y is an integer of 0 to 3, meanwhile, when y is 2 or more, a plurality of L₃'s may be the same as or different from each other.

In an embodiment, R₆ of Formula 1 may be represented by Formula 2, and R₅ may be a hydrogen atom, a deuterium atom, a halogen atom, or a substituted or unsubstituted alkyl group having 1 to 20 carbon atoms. For example, in Formula 1, R₆ may be represented by Formula 2, and R₅ may be a hydrogen atom, a deuterium atom, a halogen atom, or a substituted or unsubstituted alkyl group having 1 to 10 (or 1 to 6) carbon atoms.

In an embodiment, the amine compound represented by Formula 1 may not include an amine group other than the amine group represented by Formula 2. For example, the amine compound represented by Formula 1 may be a monoamine compound.

In an embodiment, the amine compound represented by Formula 1 may not include a heteroaryl group containing N.

In an embodiment, a phenanthrene moiety included in the amine compound according to the present disclosure includes only one phenanthrene moiety included in Formula 1.

In an embodiment, in Formula 2, L₁ may be represented by any one among L₁-1 to L₁-3:

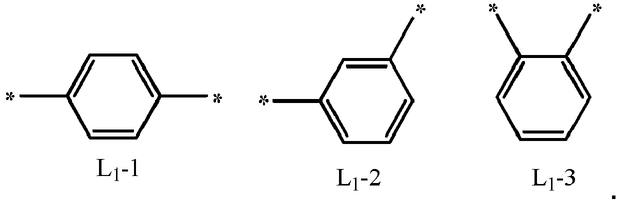

In an embodiment, in Formula 3-2, L₂ may be a direct linkage (*i.e*. x may be 0).

In an embodiment, R₁₁ and R₁₂ in Formula 3-2 may each independently be a substituted or unsubstituted aryl group having 6 to 30 (*e.g*. 6 to 20) ring-forming carbon atoms, or a substituted or unsubstituted heteroaryl group having 2 to 30 (*e.g.* 2 to 20) ring-forming carbon atoms, and/or may be bonded to an adjacent group to form a ring.

In an embodiment, Formula 3-2 may be represented by Formula 8-1 or Formula 8-2:

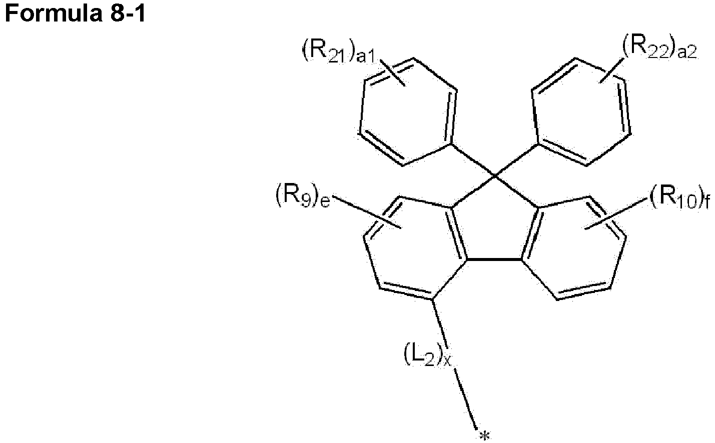

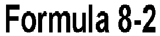

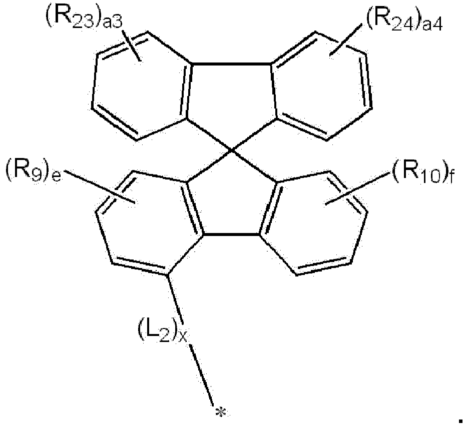

In Formula 8-1 and Formula 8-2, R₂₁ to R₂₄ are each independently a hydrogen atom, a deuterium atom, a halogen atom, a substituted or unsubstituted alkyl group having 1 to 20 (*e.g.* 1 to 10 or 1 to 6) carbon atoms, a substituted or unsubstituted aryl group having 6 to 30 (*e.g*. 6 to 20) ring-forming carbon atoms, or a substituted or unsubstituted heteroaryl group having 2 to 30 (*e.g*. 2 to 20) ring-forming carbon atoms.

In Formula 8-1, a1 and a2 are each independently an integer of 0 to 5, meanwhile, when a1 is 2 or more, a plurality of R₂₁'s may be the same as or different from each other, and when a2 is 2 or more, a plurality of R₂₂'s may be the same as or different from each other.

In Formula 8-2, a3 and a4 are each independently an integer of 0 to 4, meanwhile, when a3 is 2 or more, a plurality of R₂₃'s may be the same as or different from each other, and when a4 is 2 or more, a plurality of R₂₄'s may be the same as or different from each other.

In Formula 8-1 and Formula 8-2, R₉, R₁₀, L₂, x, e and f are each independently the same as defined in Formula 3-2 above.

In an embodiment, L₃ in Formula 3-3 may be a direct linkage (*i.e.* y may be 0), a substituted or unsubstituted phenylene group, a substituted or unsubstituted biphenylene group, a substituted or unsubstituted naphthyl group, a substituted or unsubstituted dibenzofuranyl group, or a substituted or unsubstituted dibenzothiophene group.

In an embodiment, R₅ in Formula 1 may be represented by Formula 2. For example, Formula 1 may be represented by Formula 4:

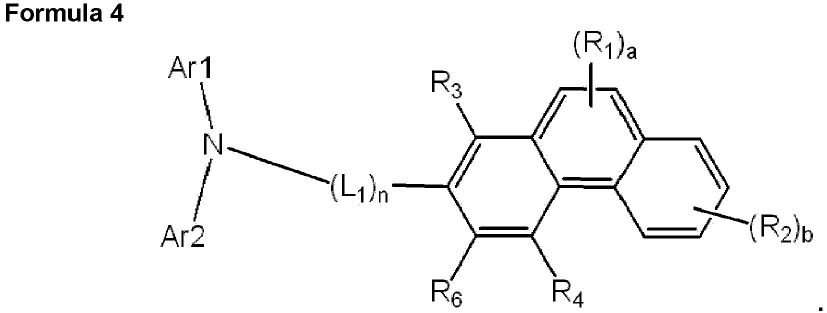

In Formula 4, R₁ to R₄, R₆, Ar1, Ar2, L₁, n, a, and b are each independently the same as defined in Formula 1 and Formula 2.

In an embodiment, Ar1 in Formula 4 may be represented by Formula 3-1 or Formula 3-2. For example, Formula 4 may be represented by Formula 6-1 or Formula 6-2:

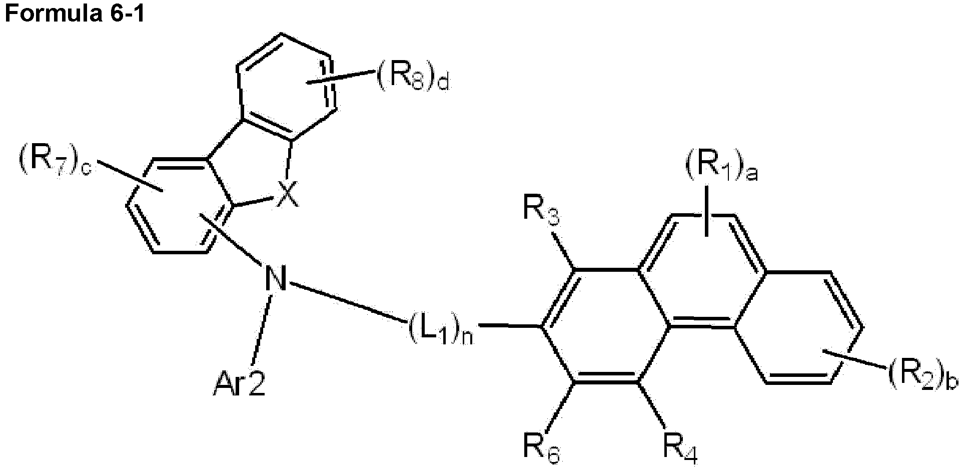

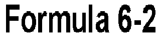

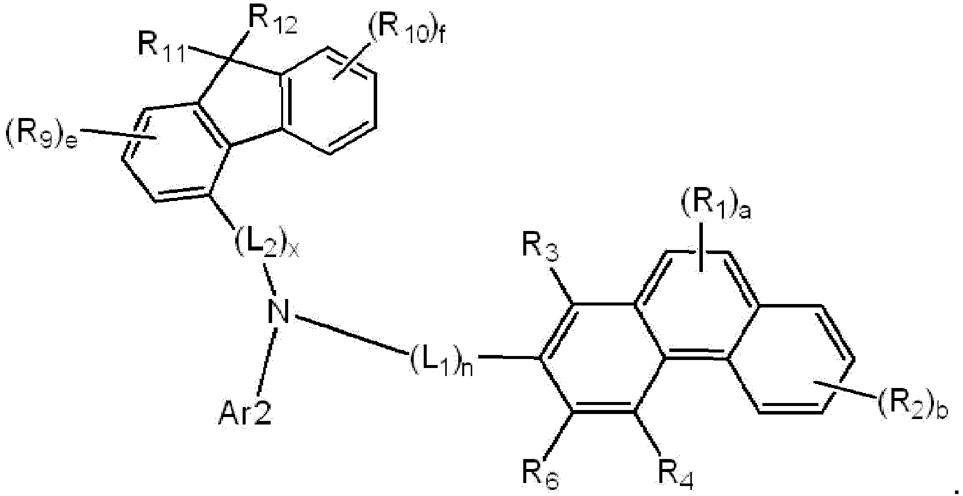

In Formula 6-1 and Formula 6-2, Ar2 is a substituted or unsubstituted aryl group having 6 to 30 (*e.g*. 6 to 20) ring-forming carbon atoms, or a substituted or unsubstituted heteroaryl group having 2 to 30 (*e.g.* 2 to 20) ring-forming carbon atoms, but does not include a phenanthrene group, a triphenylene group, or a 2-fluorenyl group.

In Formula 6-1 and Formula 6-2, R₁ to R₄, R₆ to R₁₂, L₁, L₂, n, x, and a to f are each independently the same as defined in Formula 1 to Formula 3-2.

In an embodiment, Ar1 in Formula 4 may be represented by Formula 3-3. For example, Formula 4 may be represented by Formula 6-3:

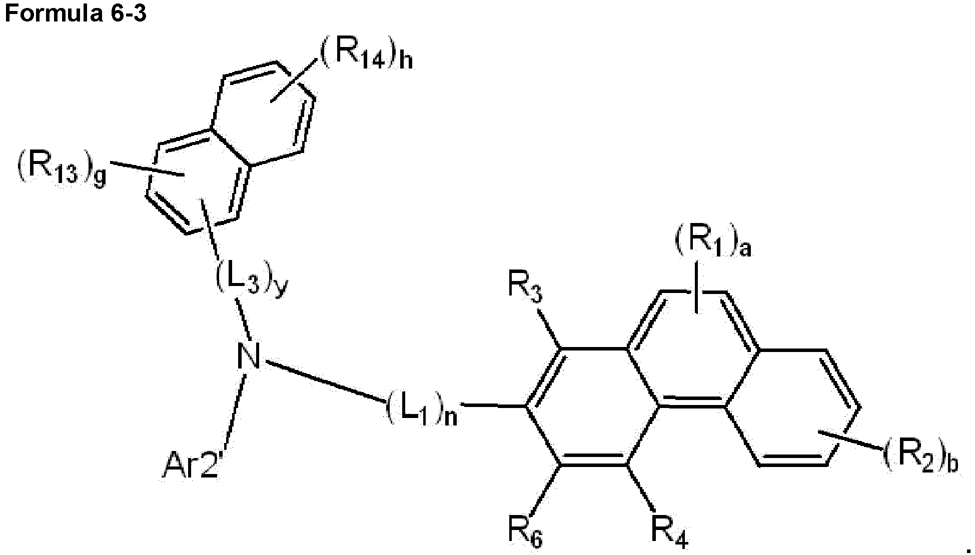

In Formula 6-3, Ar2' is a substituted or unsubstituted aryl group having 16 to 30 ring-forming carbon atoms, or a substituted or unsubstituted heteroaryl group having 2 to 30 (*e.g.* 2 to 20) ring-forming carbon atoms, but does not include a phenanthrene group, a triphenylene group, or a 2-fluorenyl group.

In Formula 6-3, R₁ to R₄, R₆, R₁₃, R₁₄, L₁, L₃, a, b, n, y, g and h are each independently the same as defined in Formula 1, Formula 2, and Formula 3-3.

In an embodiment, R₆ in Formula 1 may be represented by Formula 2. For example, Formula 1 may be represented by Formula 5:

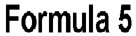

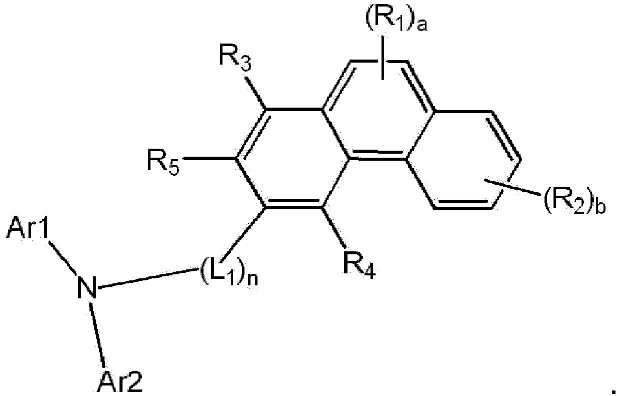

In Formula 5, R₁ to R₅, Ar1, Ar2, L₁, n, a, and b are each independently the same as defined in Formula 1 and Formula 2.

In an embodiment, Ar1 in Formula 5 may be represented by Formula 3-1 or Formula 3-2. For example, Formula 5 may be represented by Formula 7-1 or Formula 7-2:

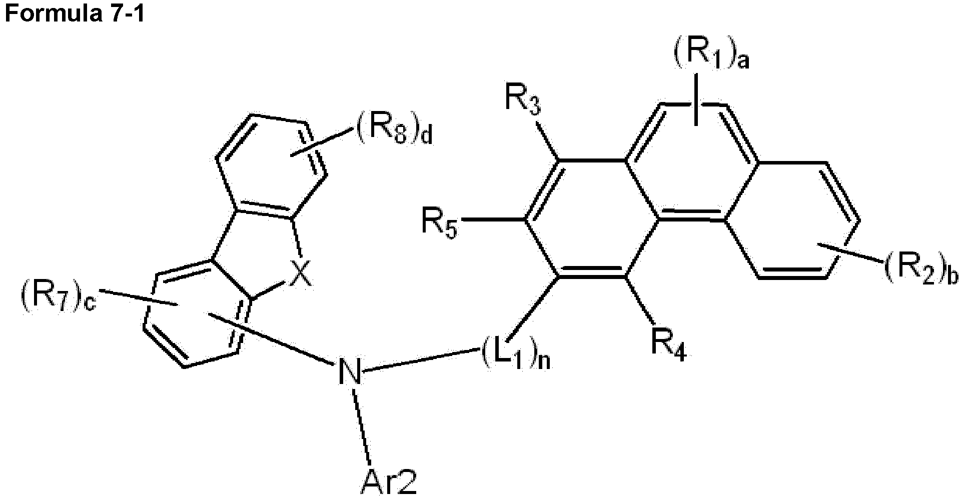

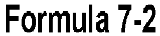

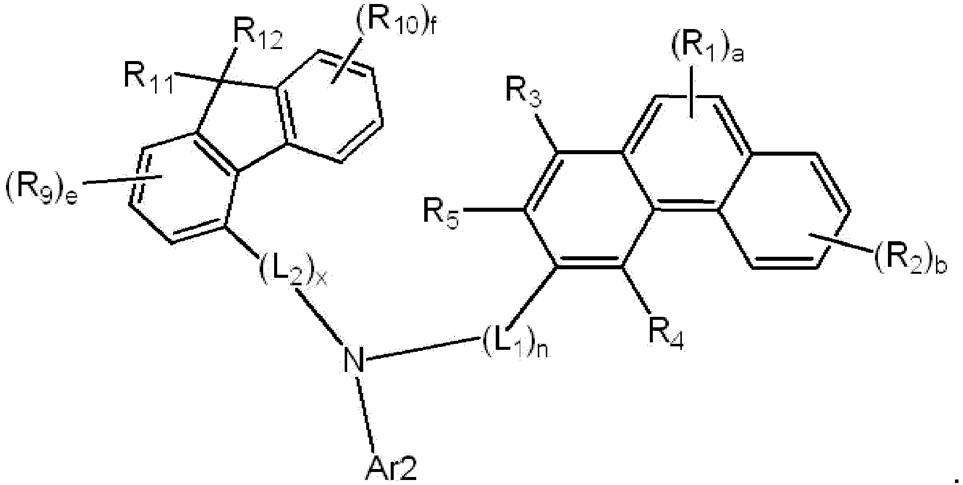

In Formula 7-1 and Formula 7-2, Ar2, R₁ to R₅, R₇ to R₁₂, L₁, L₂, n, x, and a to f are each independently the same as defined in Formula 1 to Formula 3-2.

In an embodiment, Ar1 in Formula 5 may be represented by Formula 3-3. For example, Formula 5 may be represented by Formula 7-3:

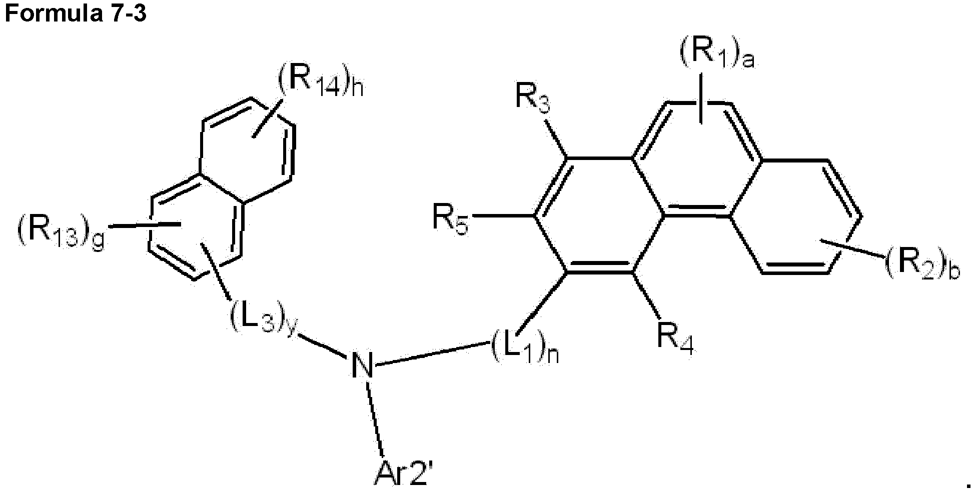

In Formula 7-3, Ar2' is a substituted or unsubstituted aryl group having 16 to 30 ring-forming carbon atoms, or a substituted or unsubstituted heteroaryl group having 2 to 30 (*e.g*. 2 to 20) ring-forming carbon atoms, but does not include a phenanthrene group, a triphenylene group, or a 2-fluorenyl group.

In Formula 7-3, R₁ to R₅, R₁₃, R₁₄, L₁, L₃, a, b, n, y, g and h may each independently be the same as defined in Formula 1, Formula 2, and Formula 3-3.

The amine compound represented by Formula 1 according to an embodiment may be any one selected from the compounds represented by Compound Groups 1 and Compound Group 2. However, embodiments of the present disclosure are not limited thereto.

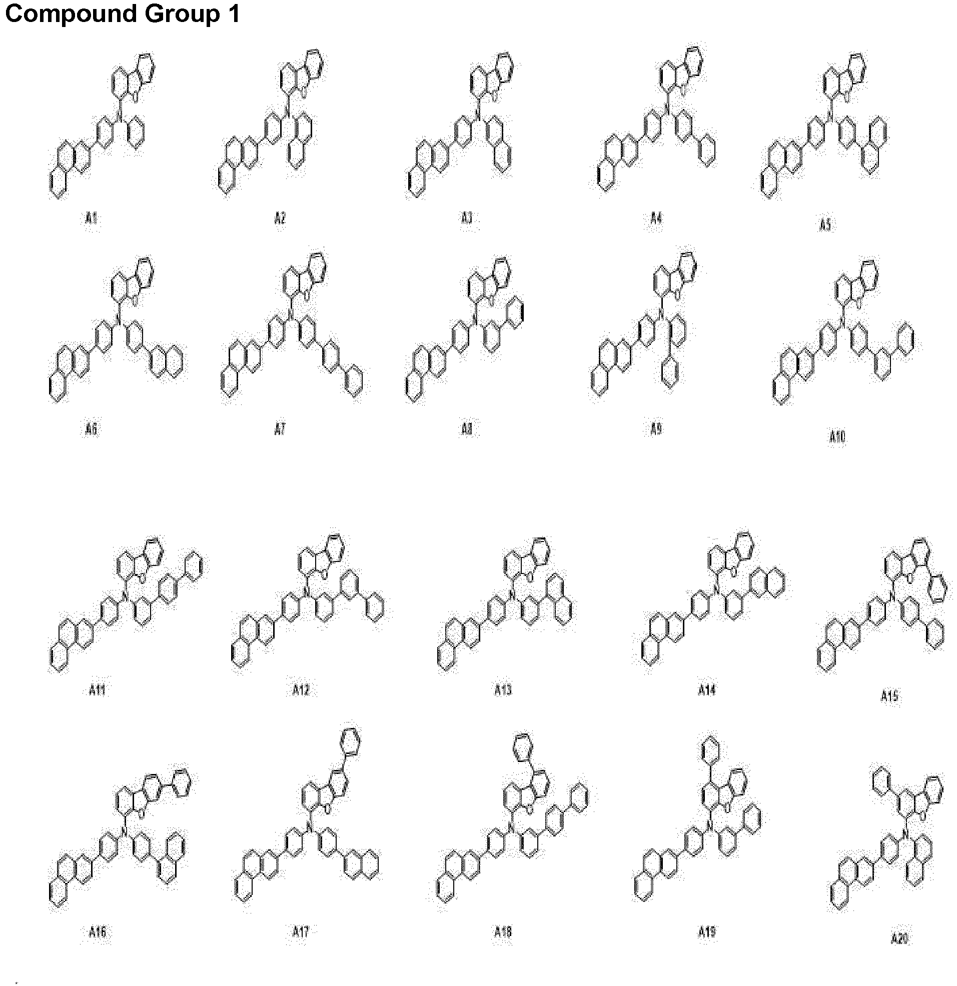

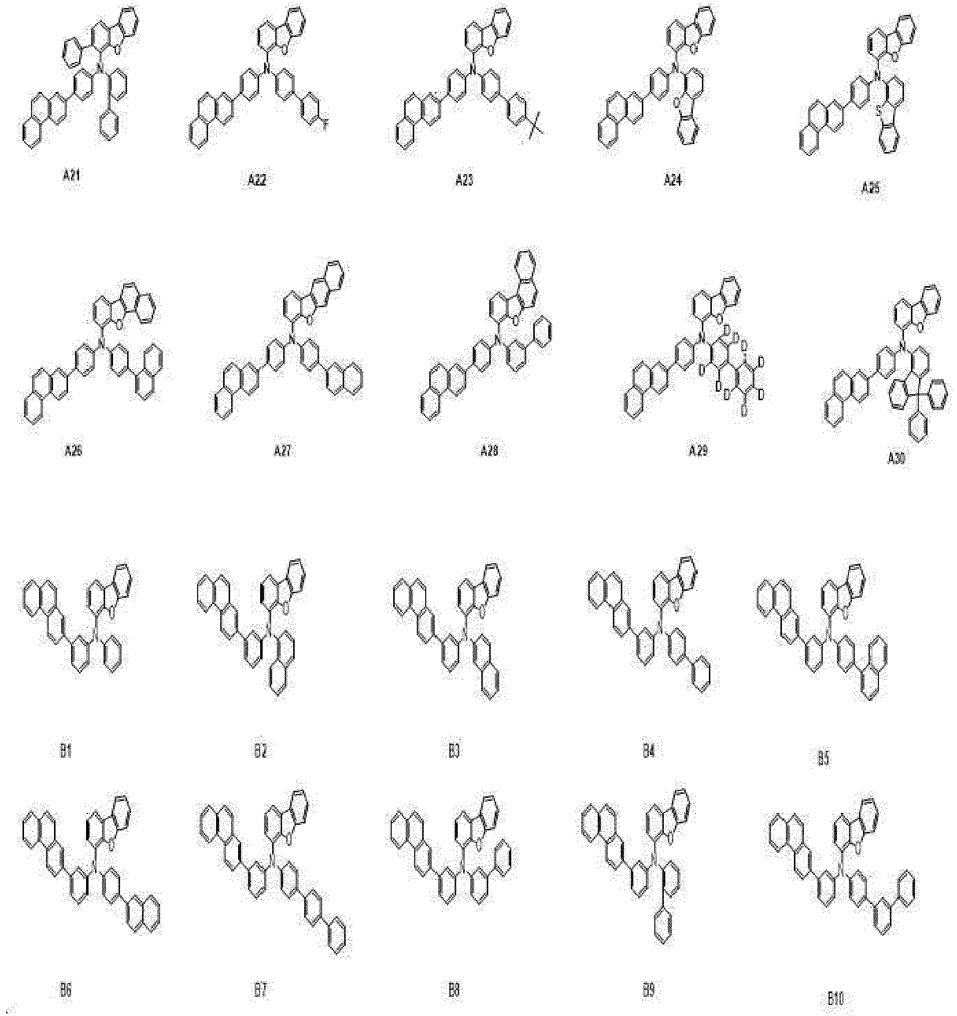

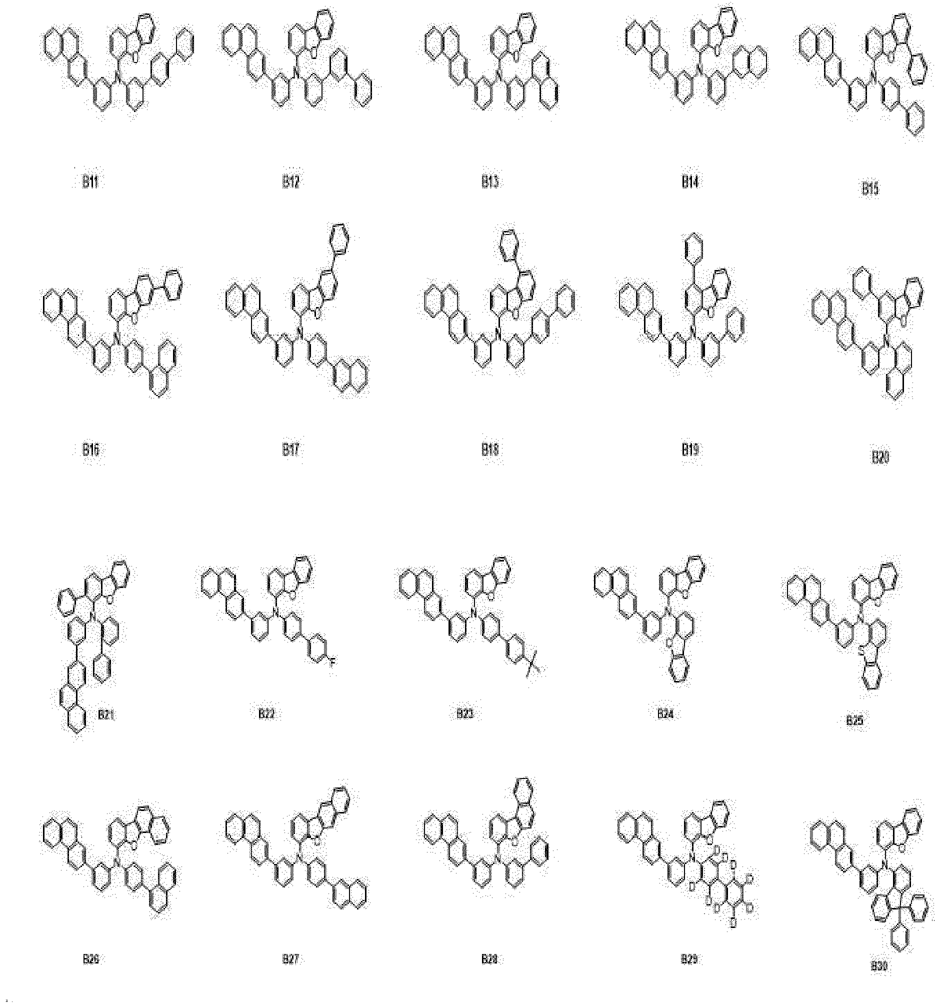

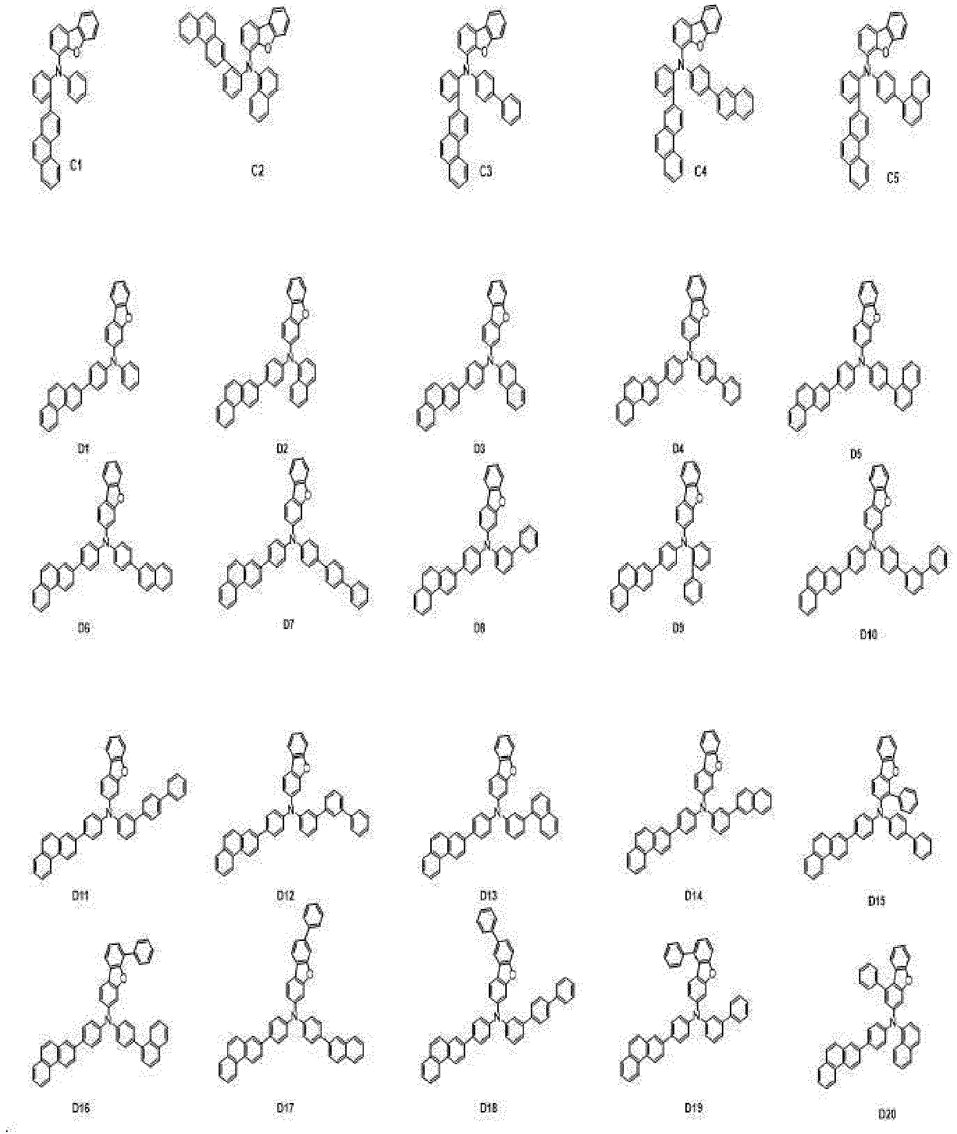

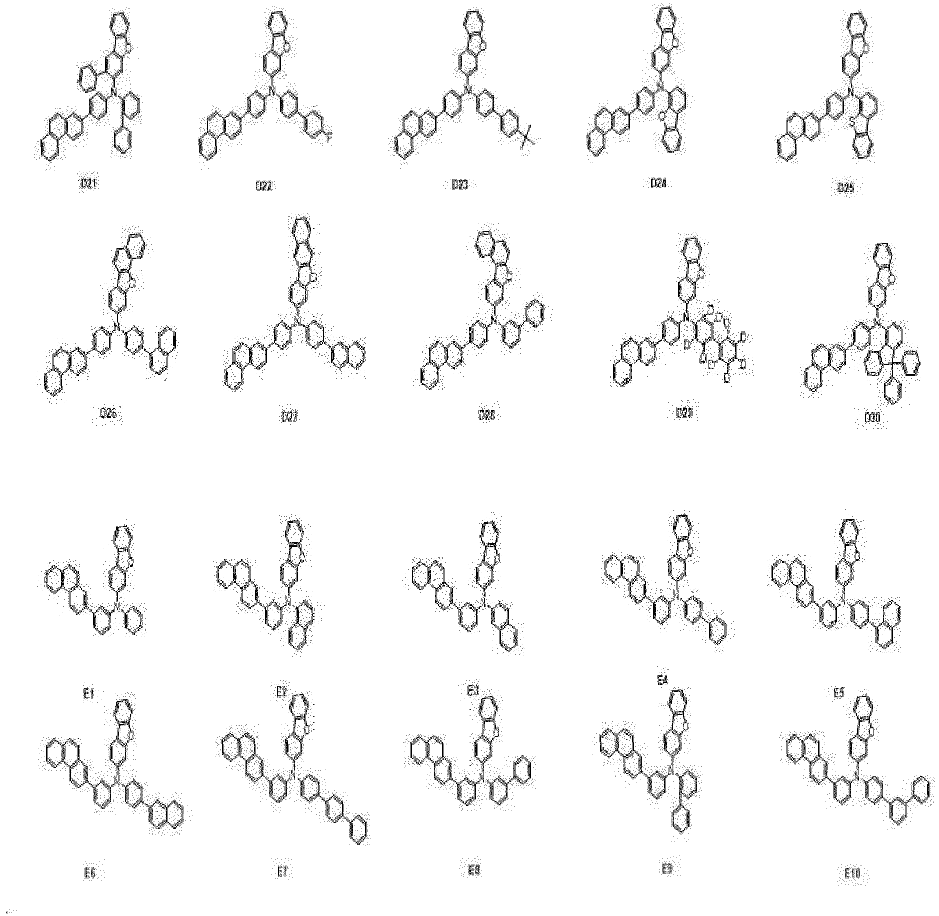

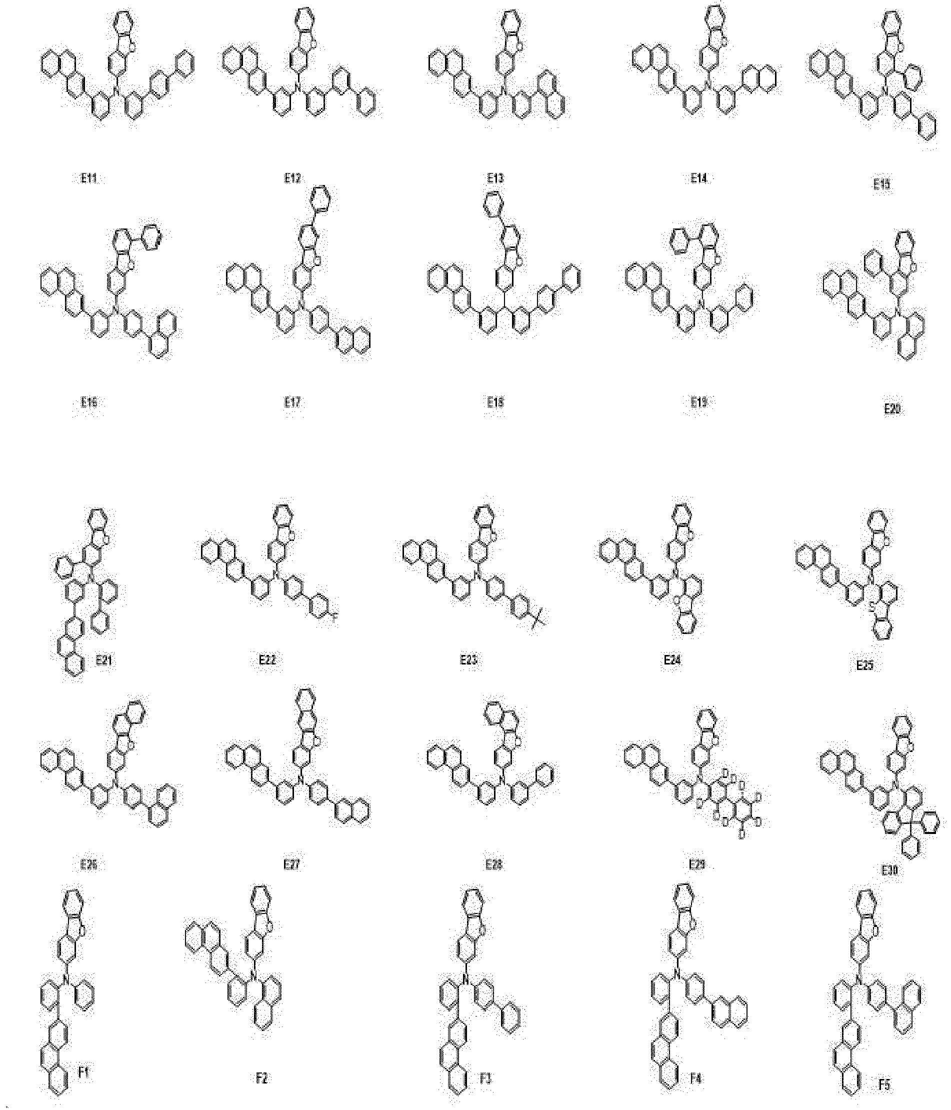

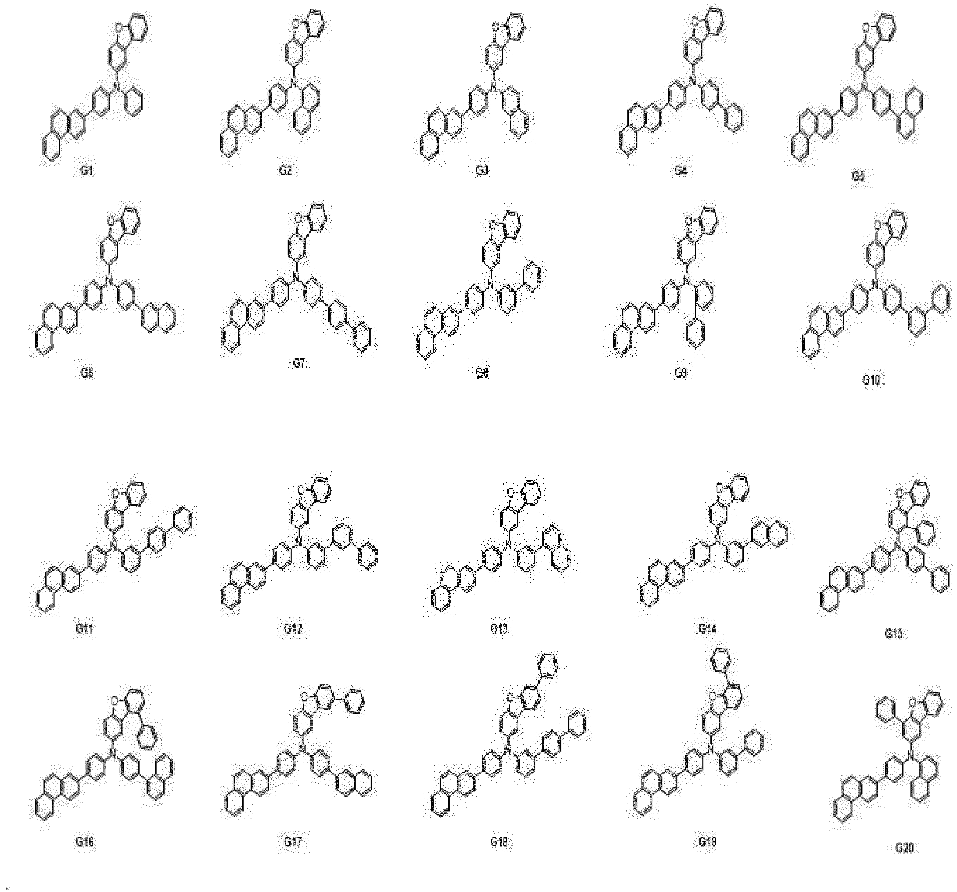

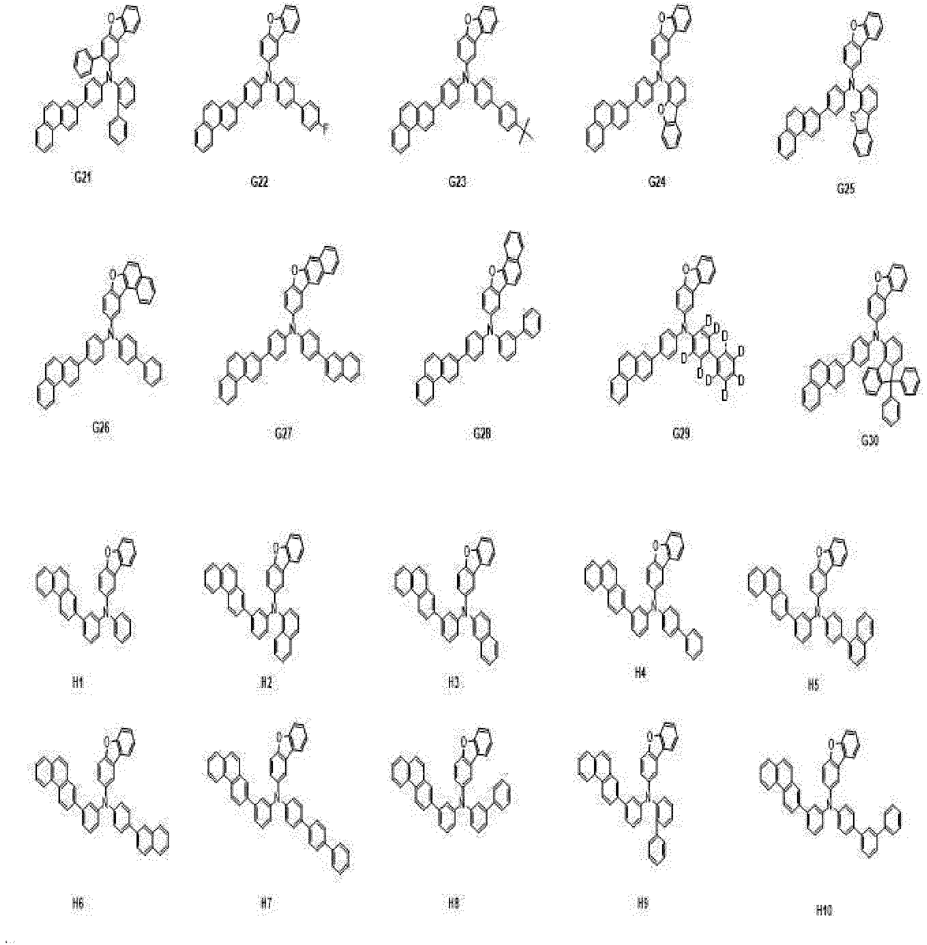

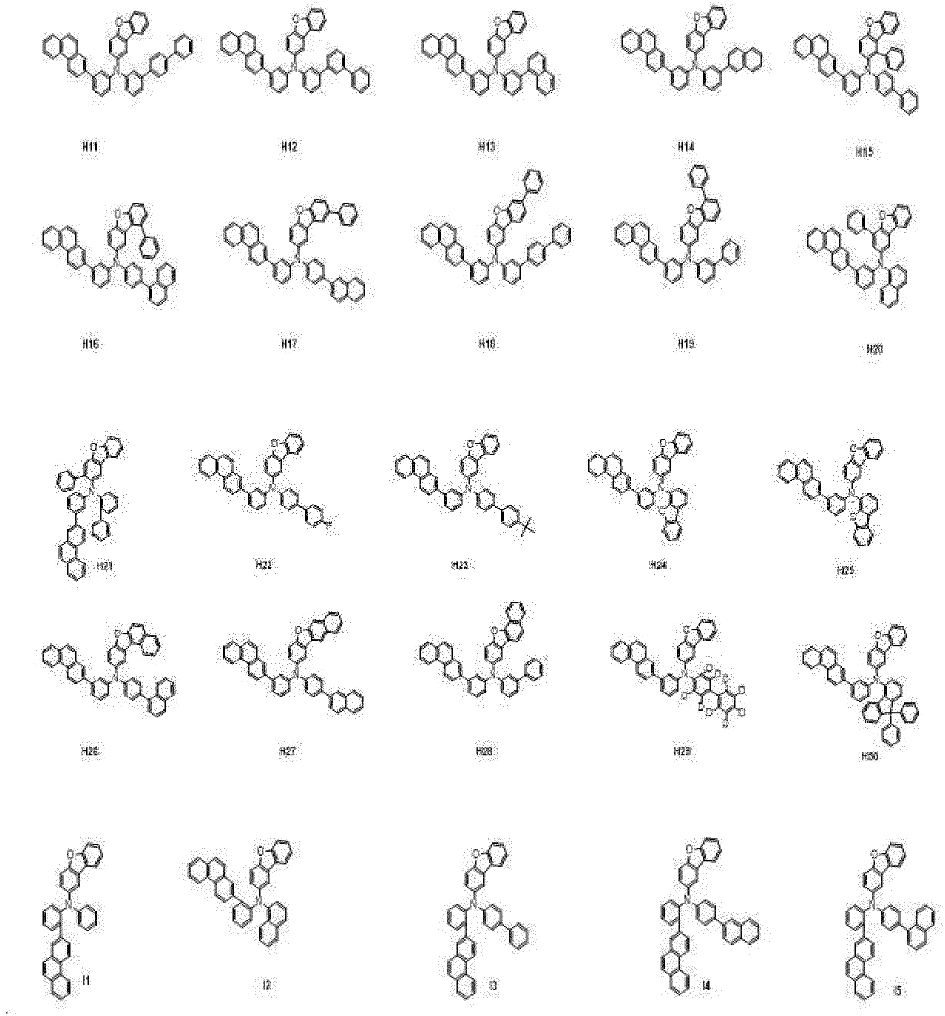

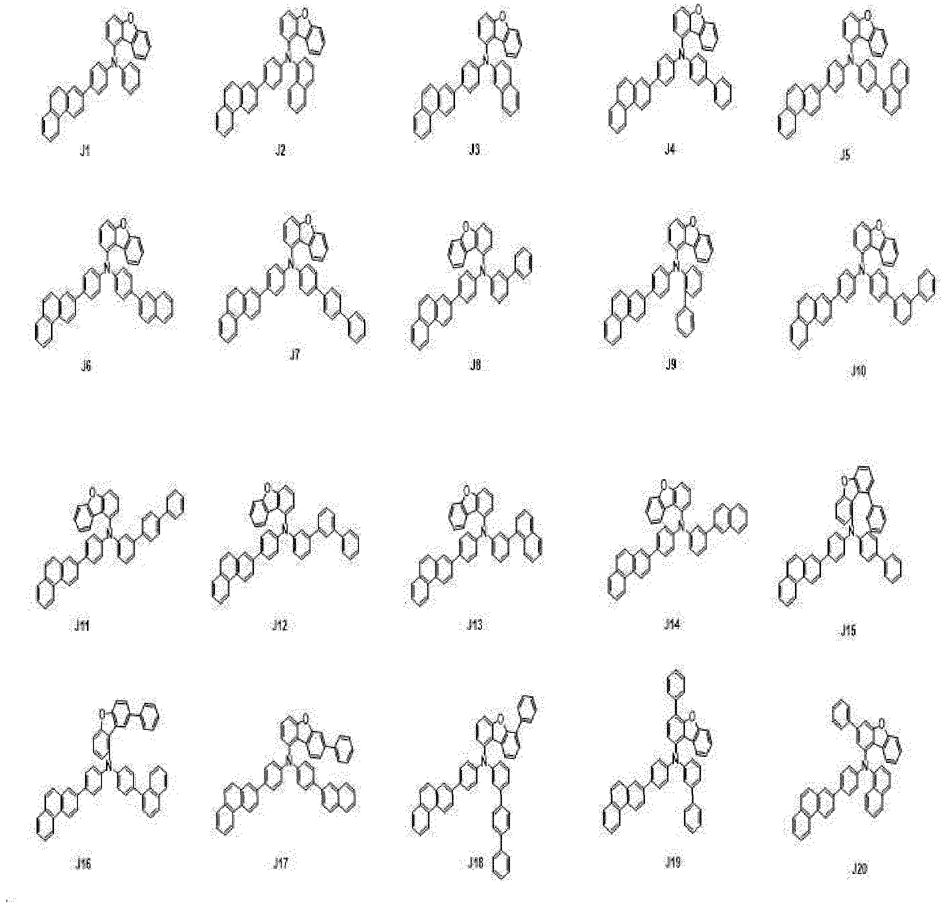

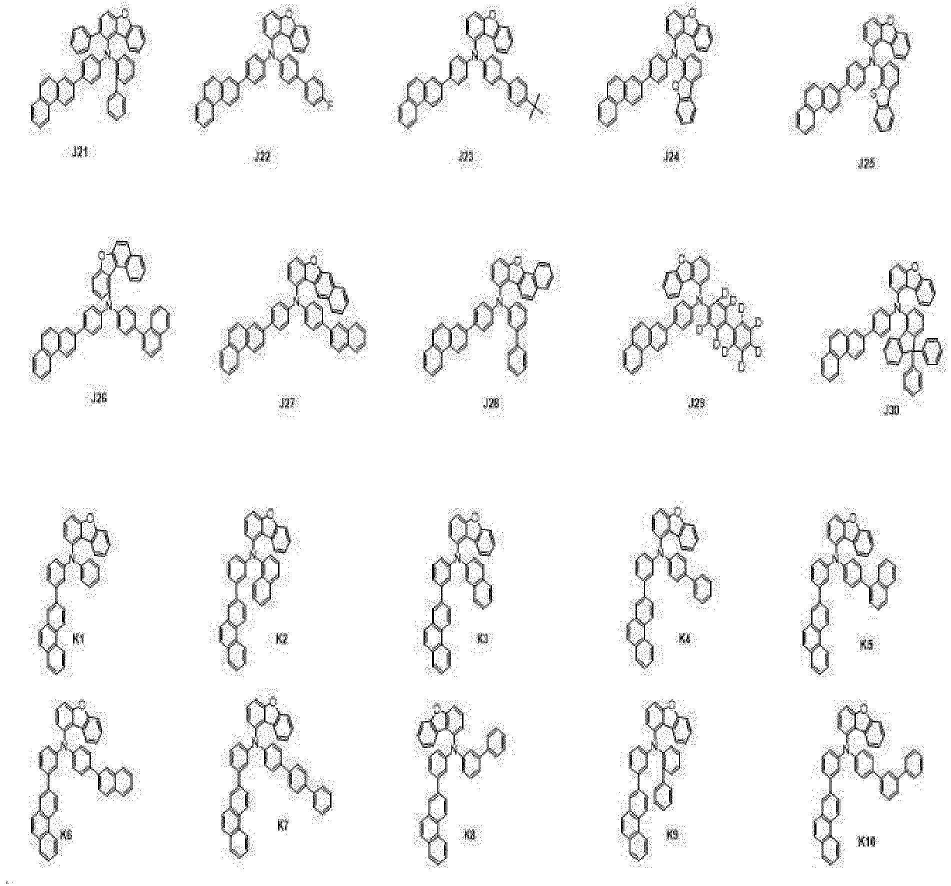

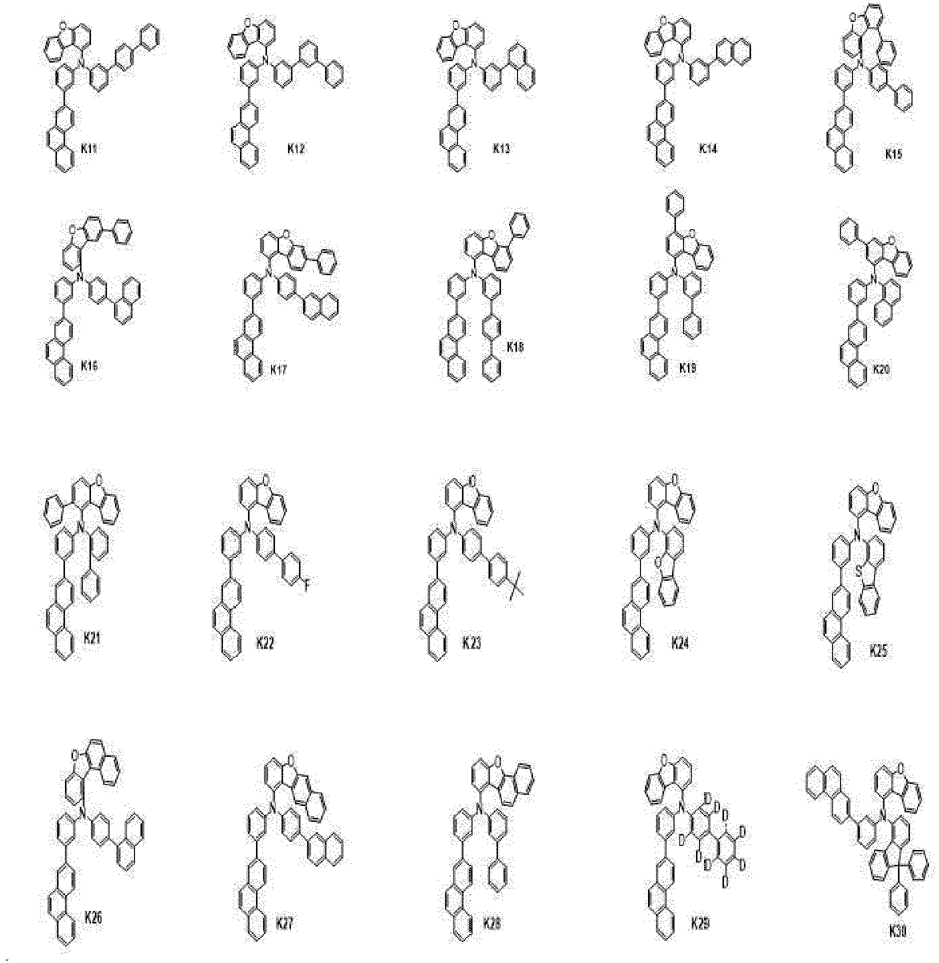

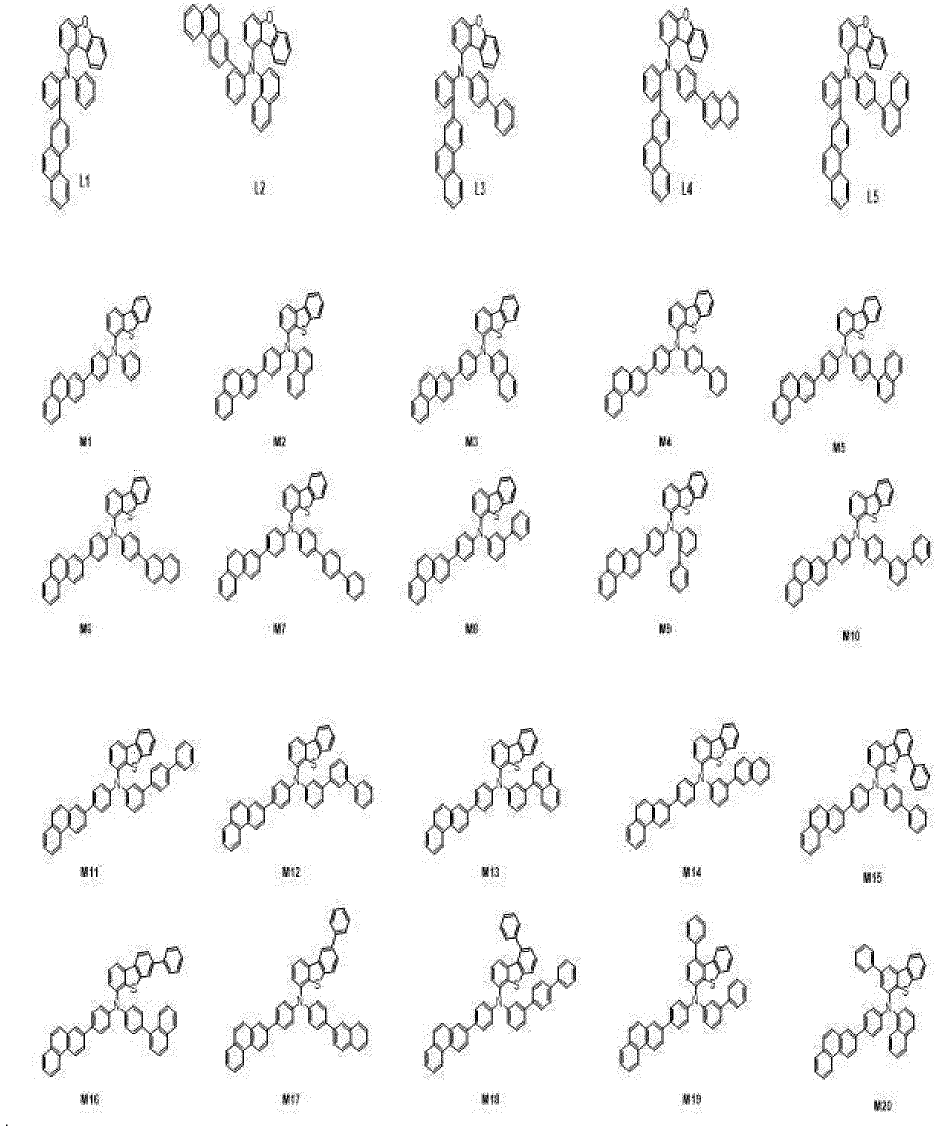

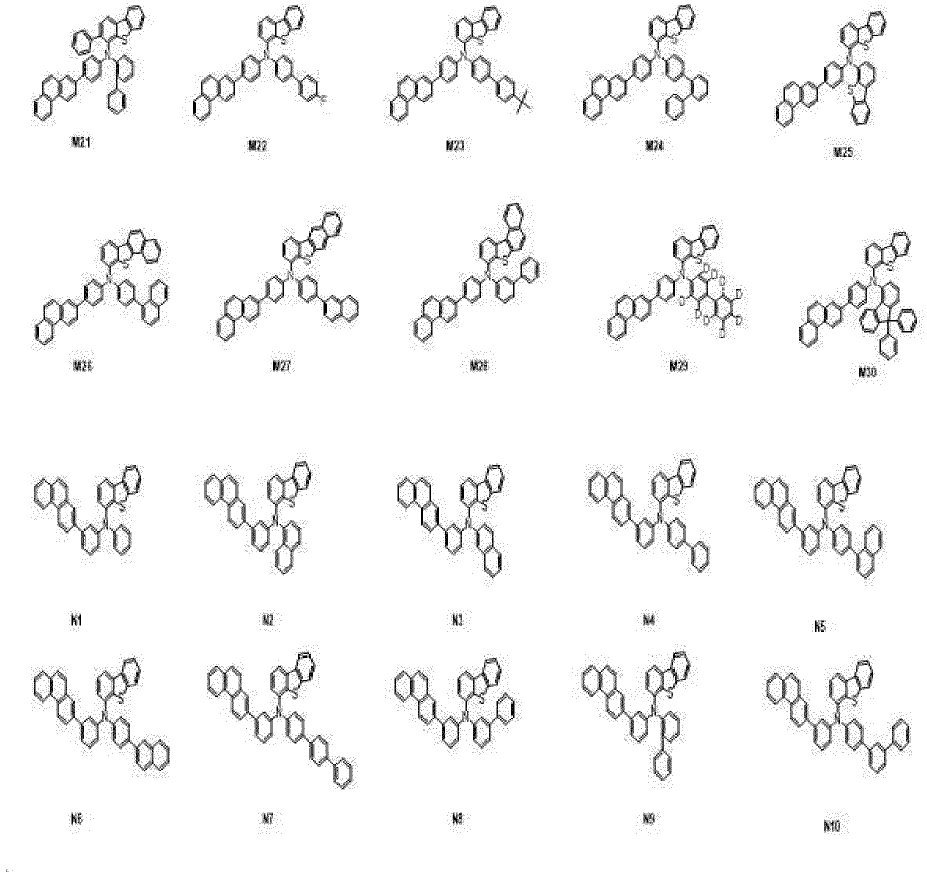

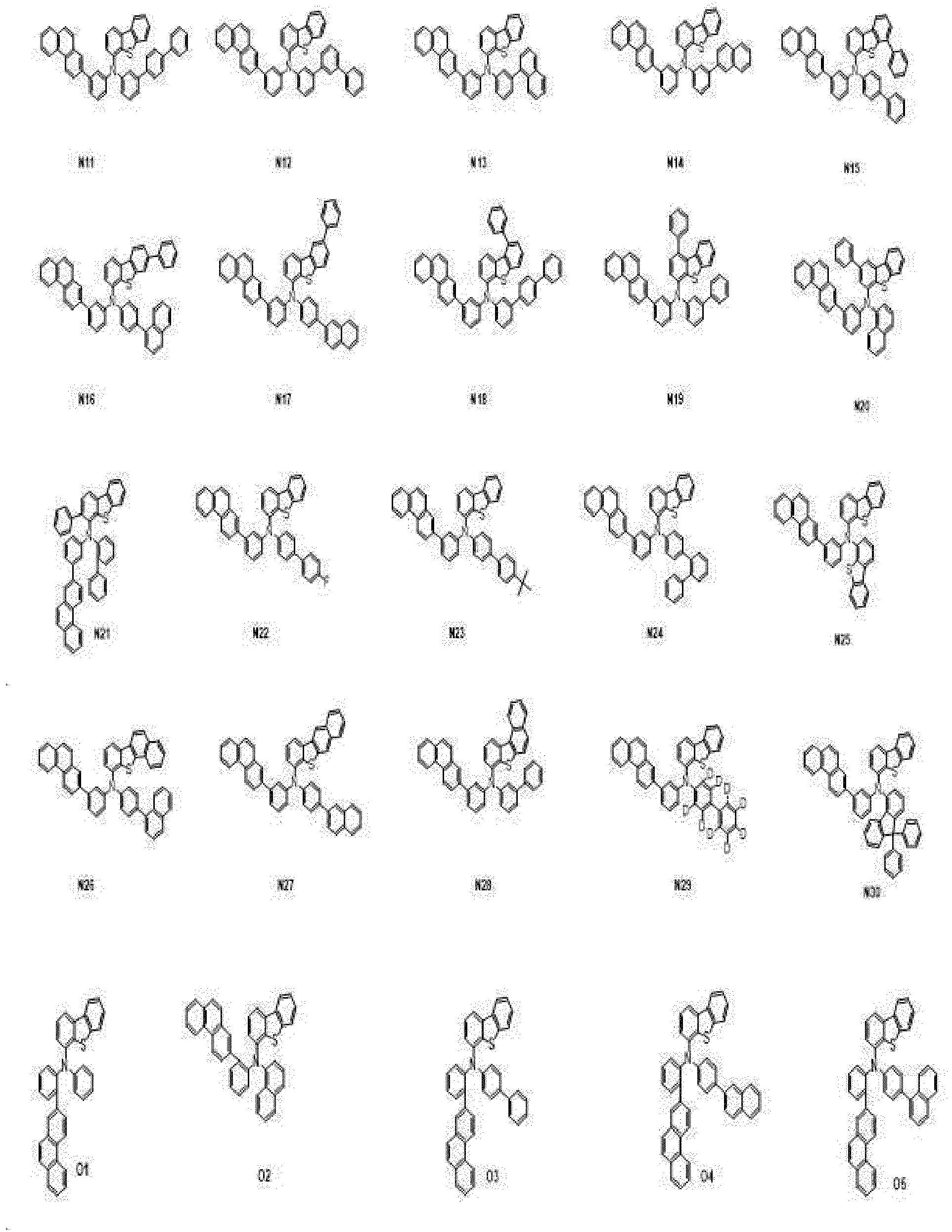

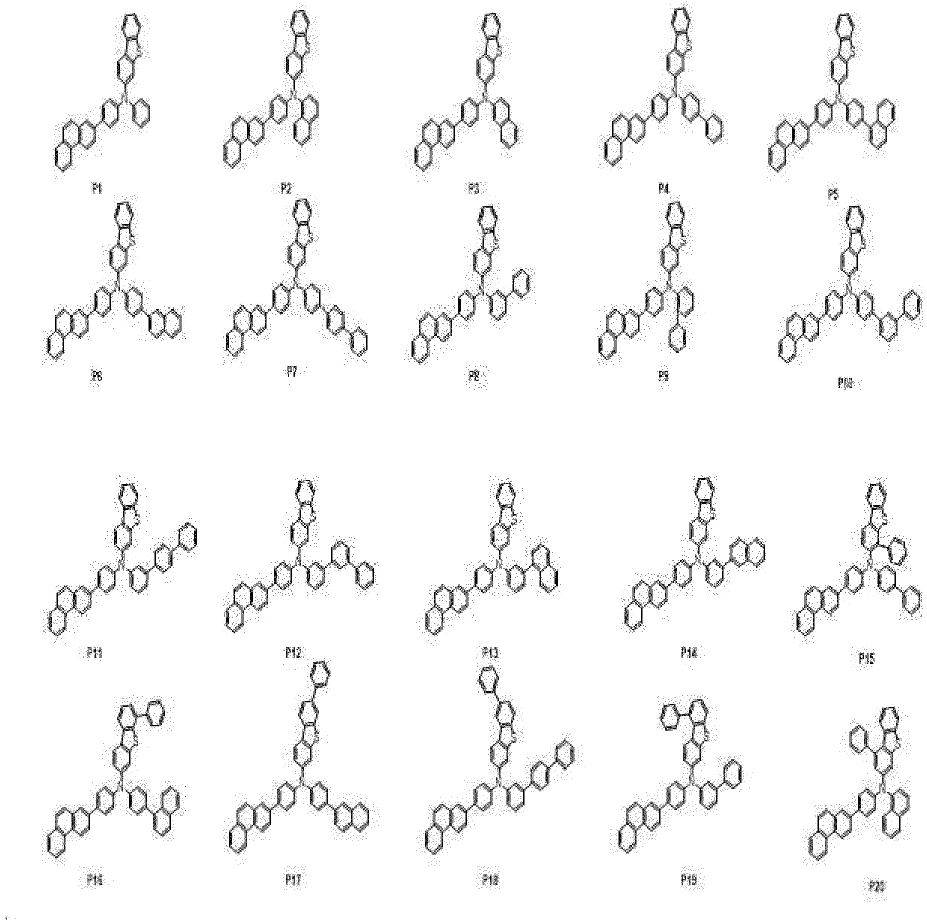

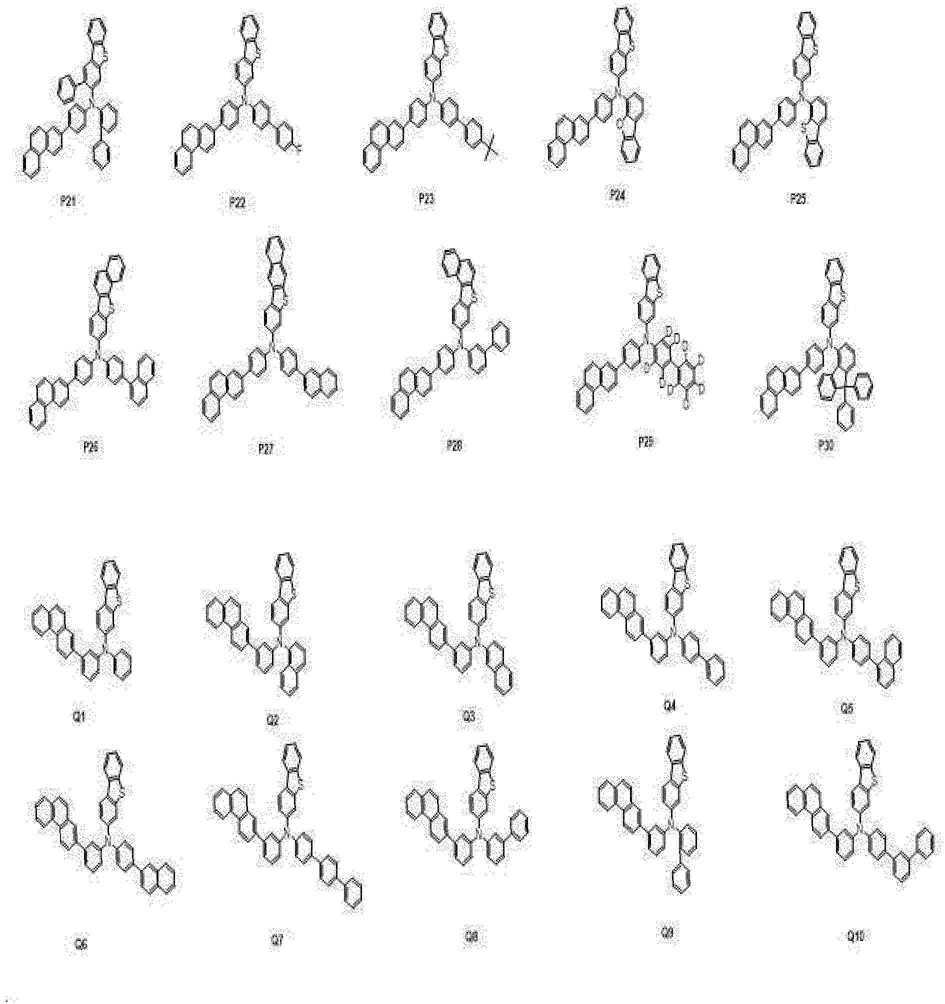

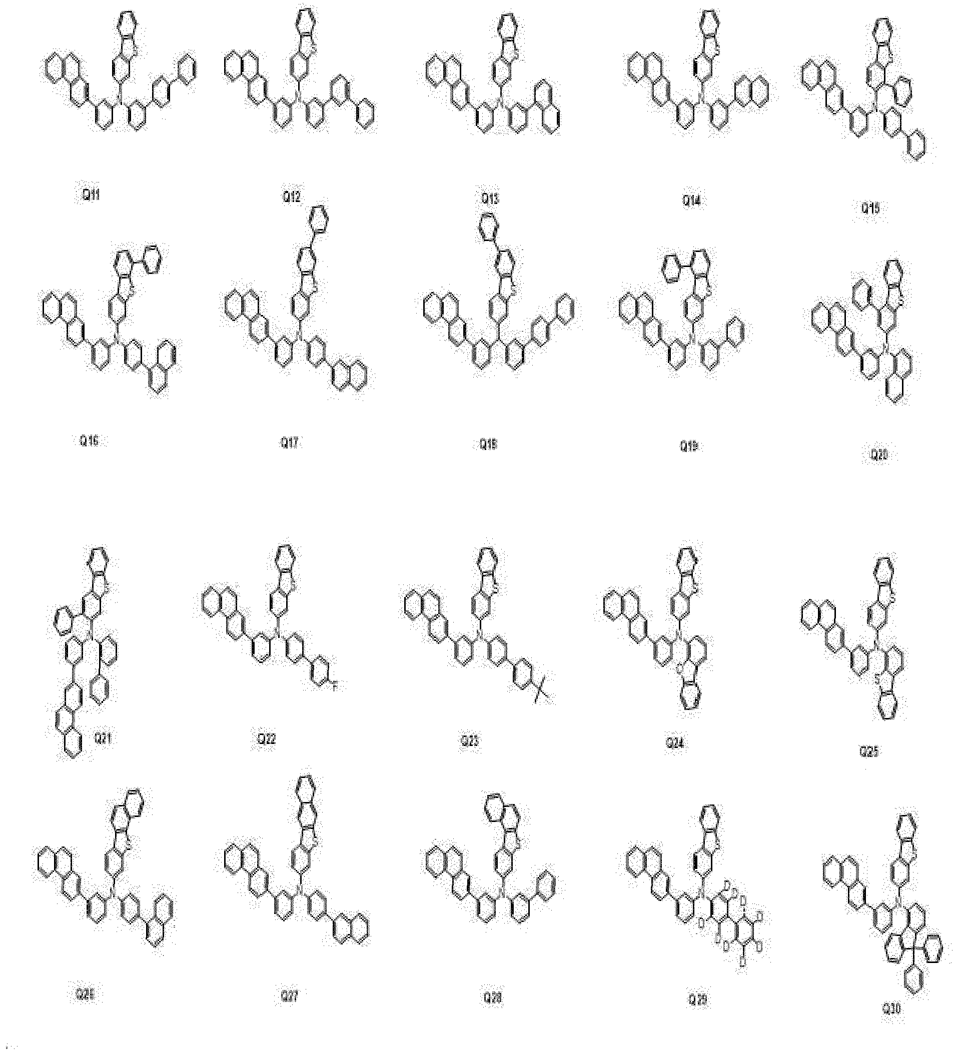

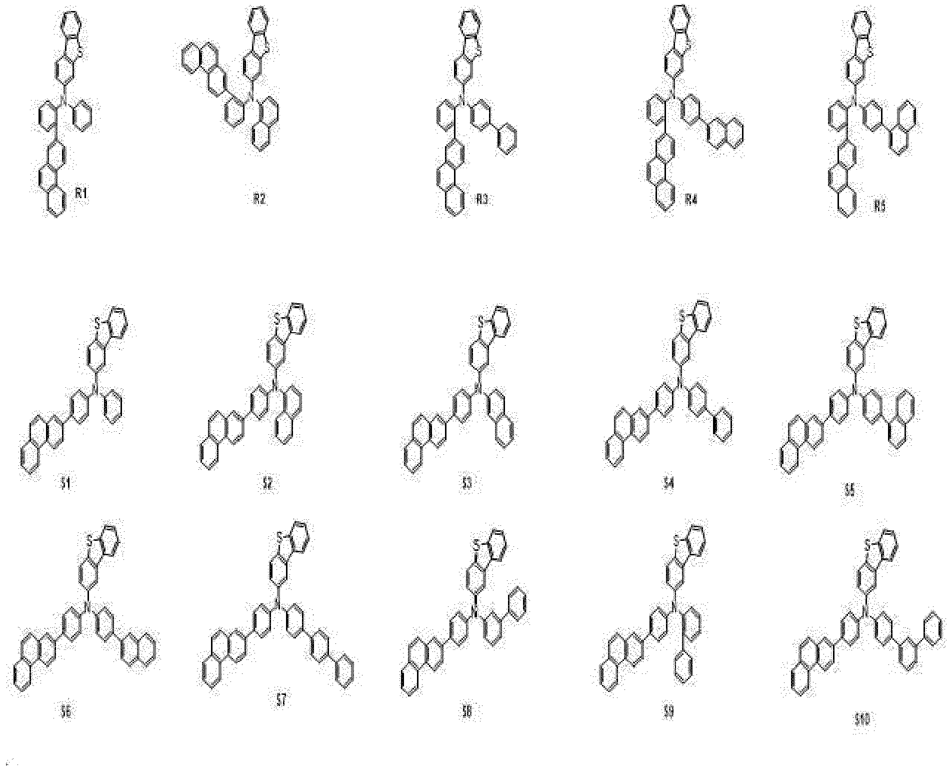

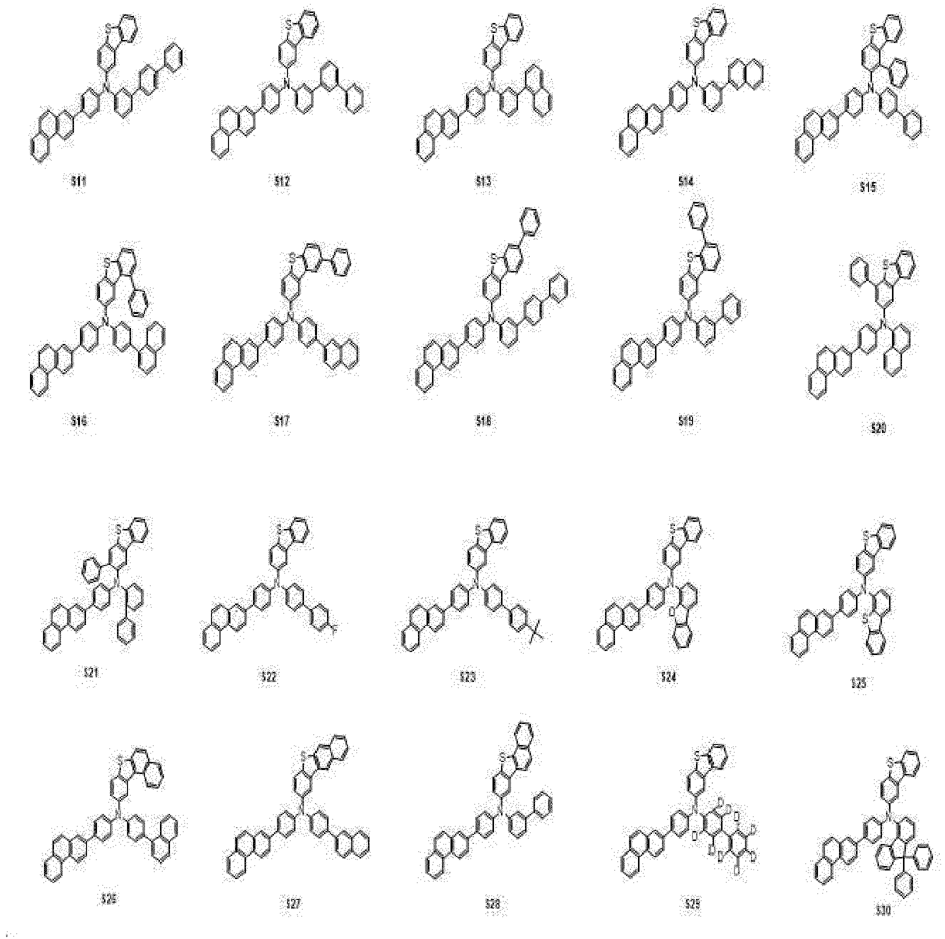

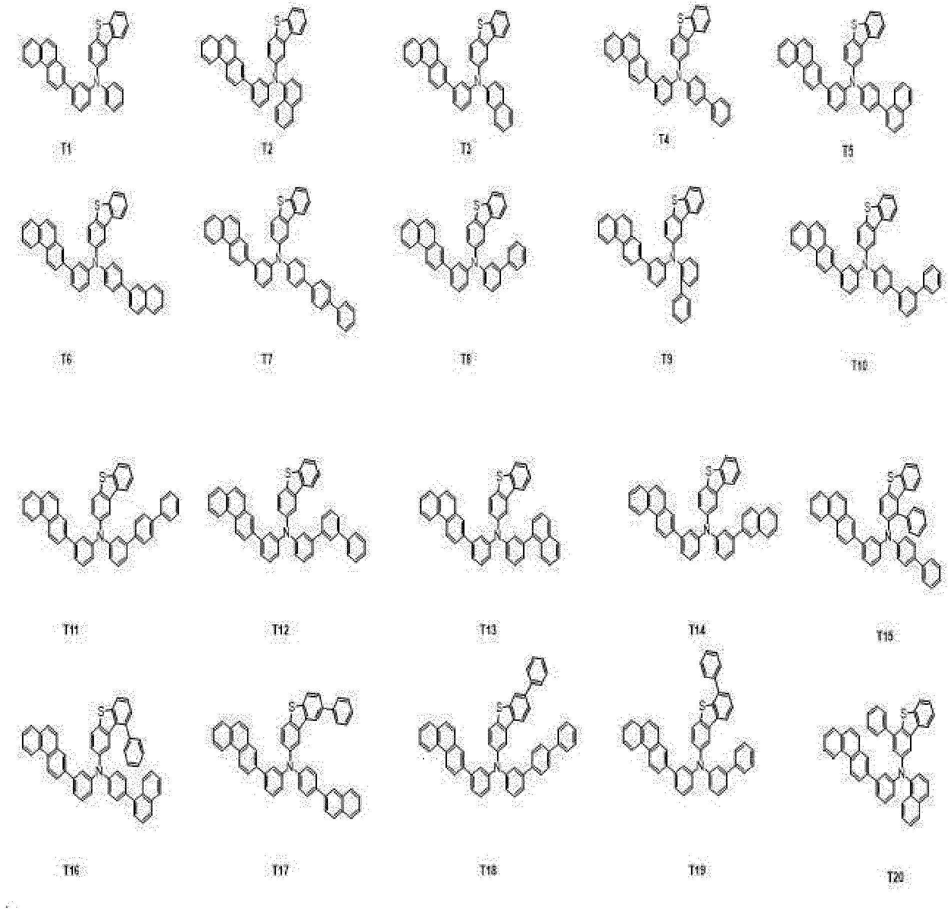

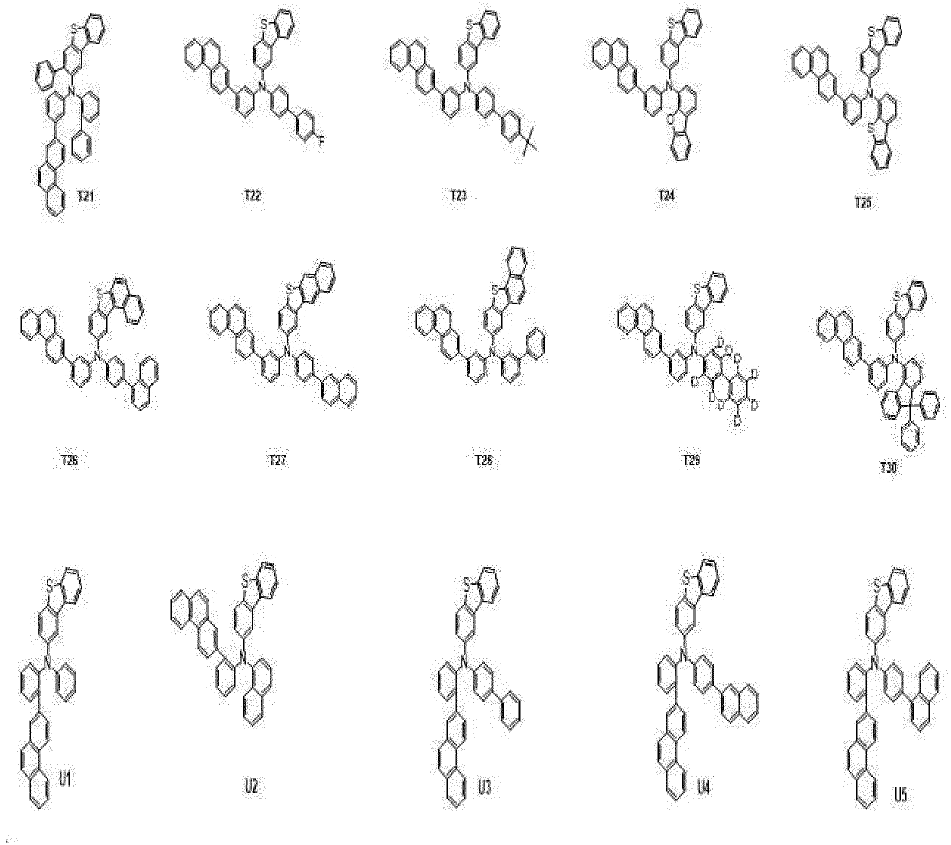

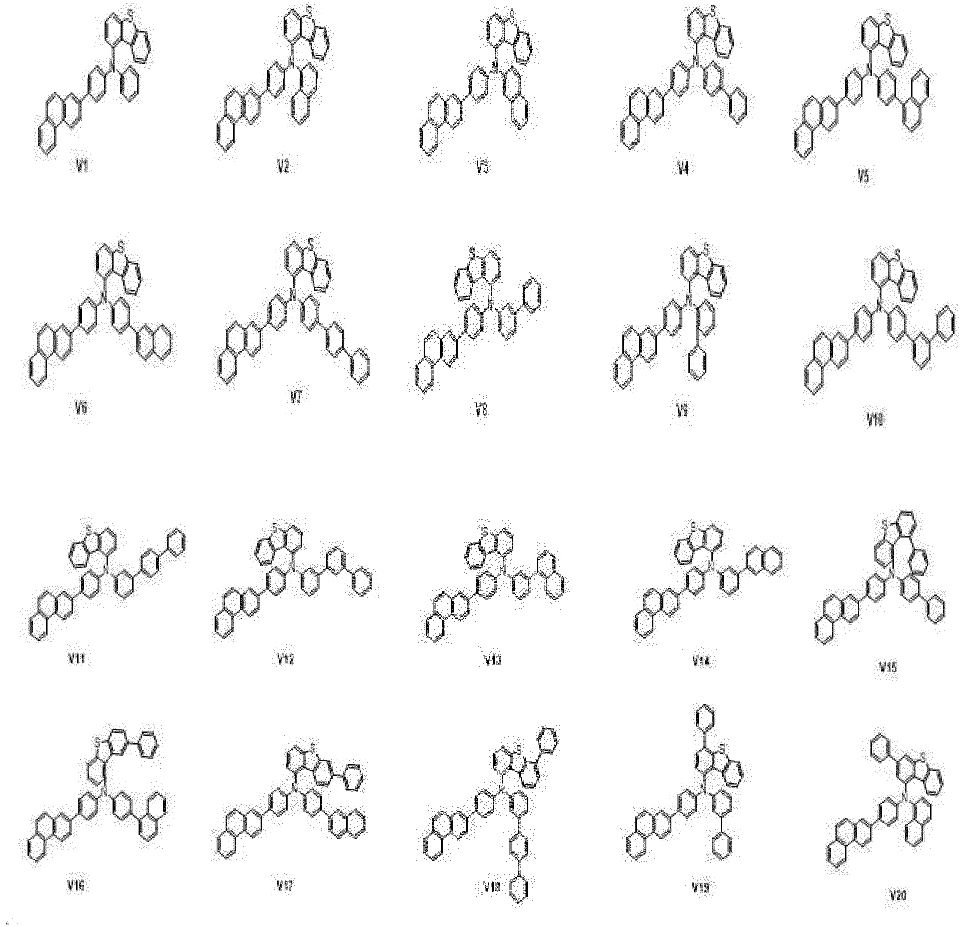

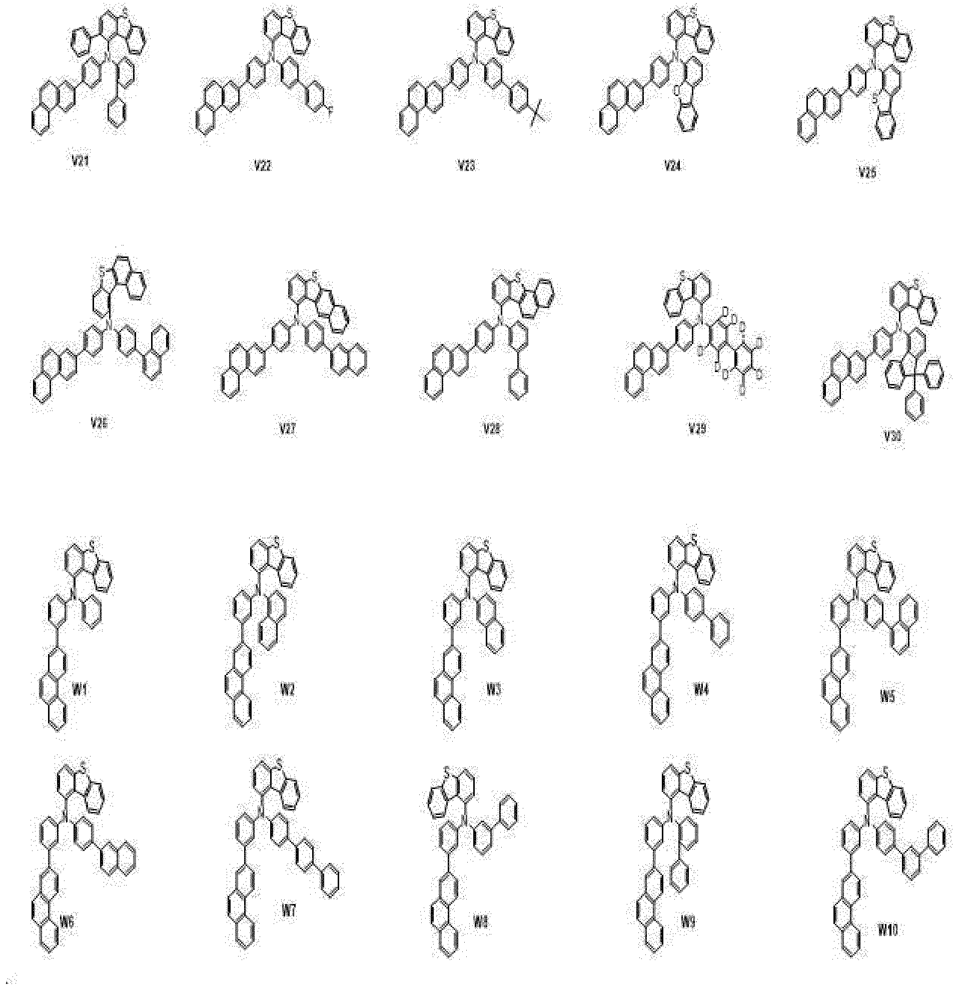

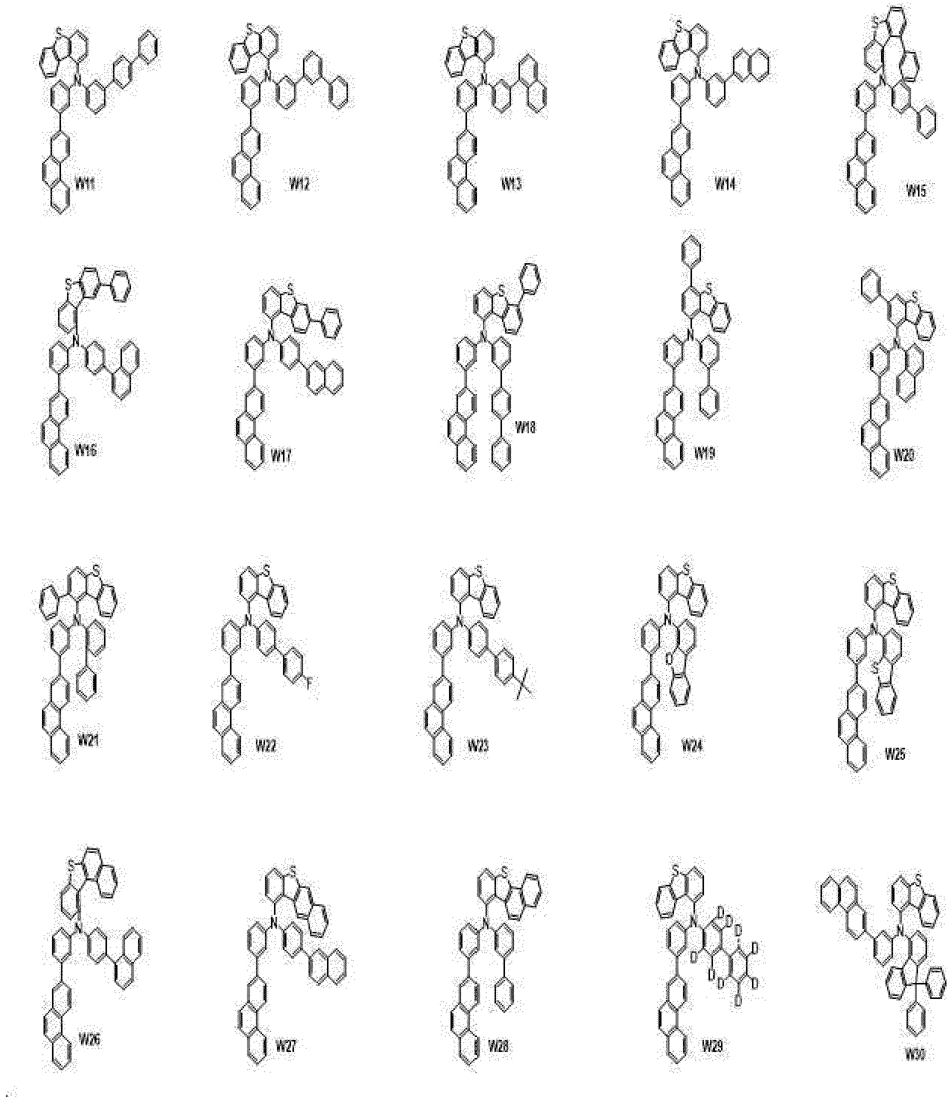

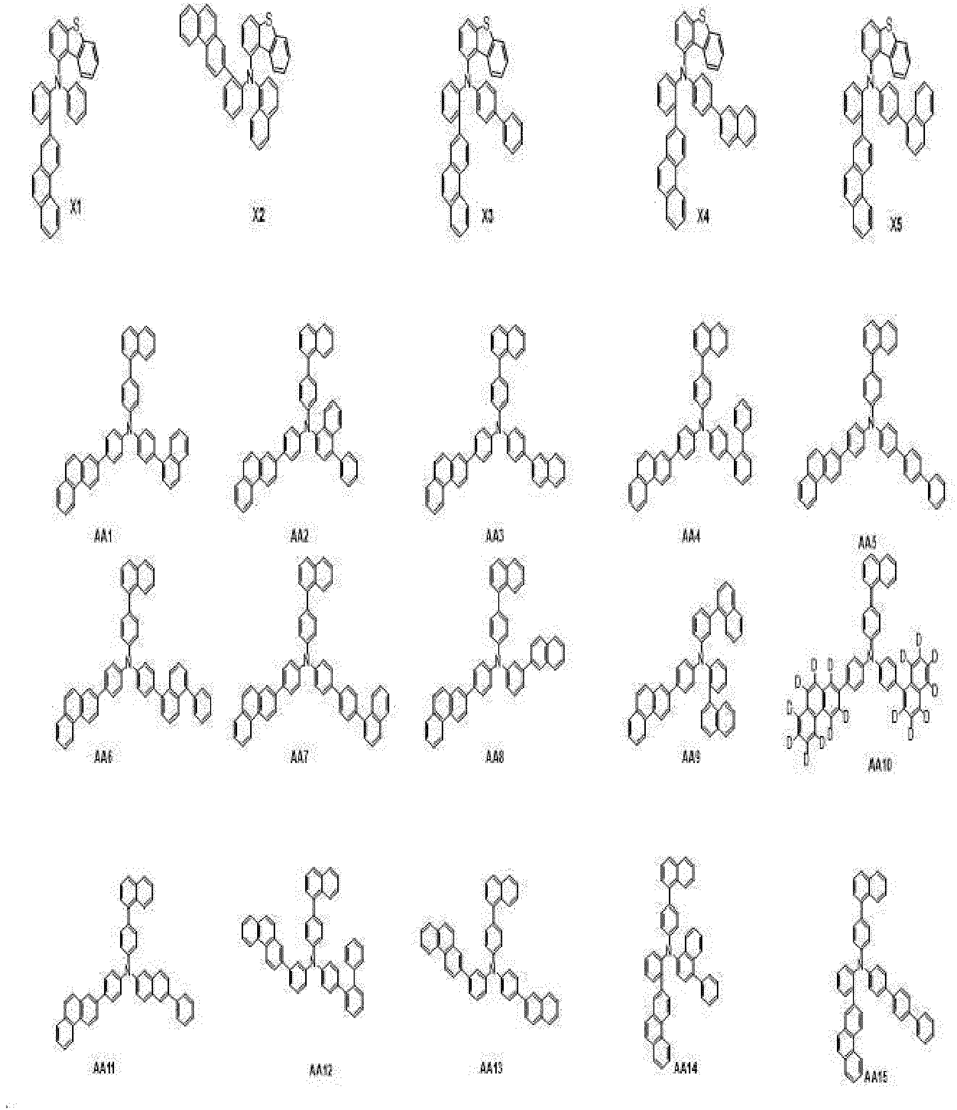

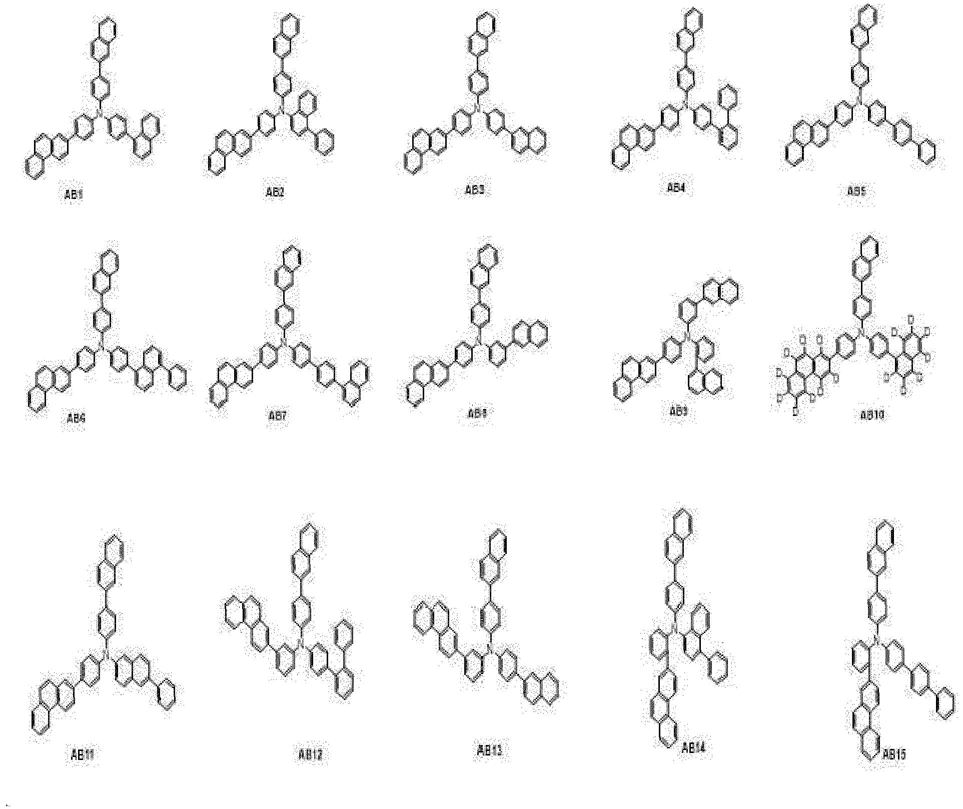

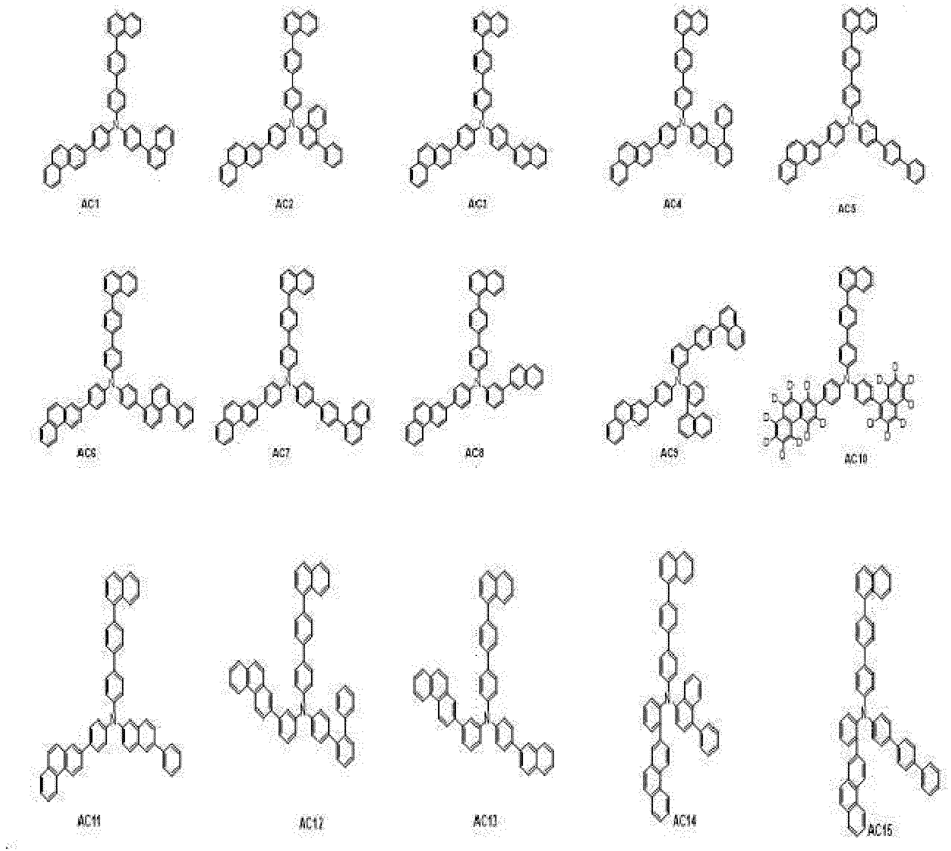

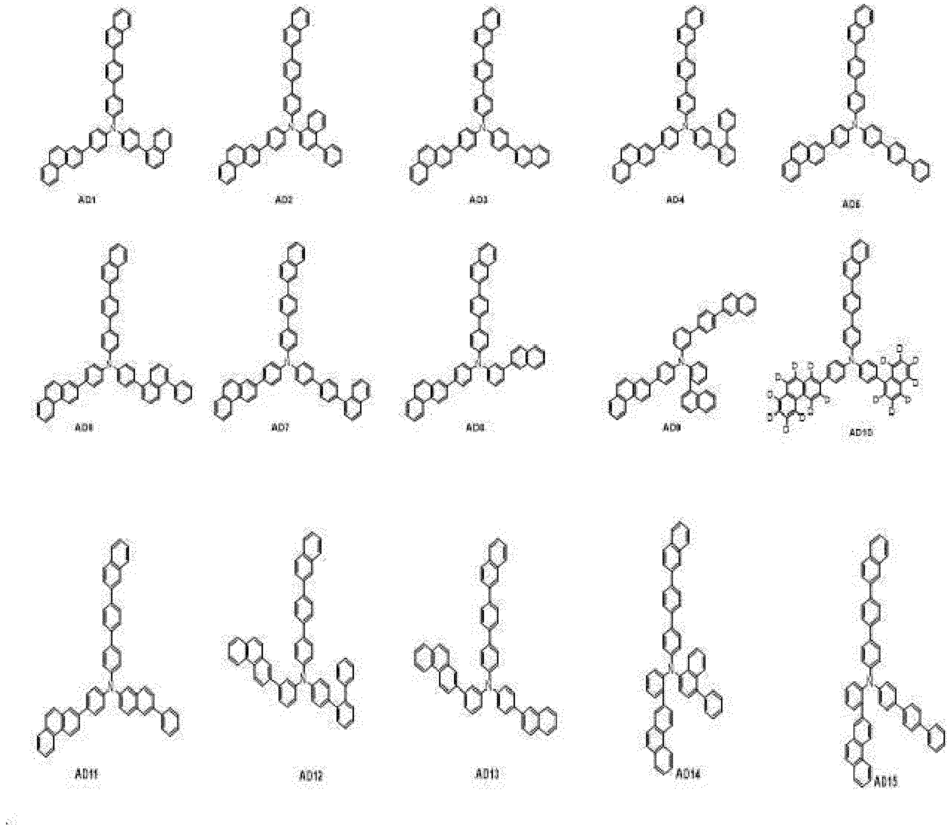

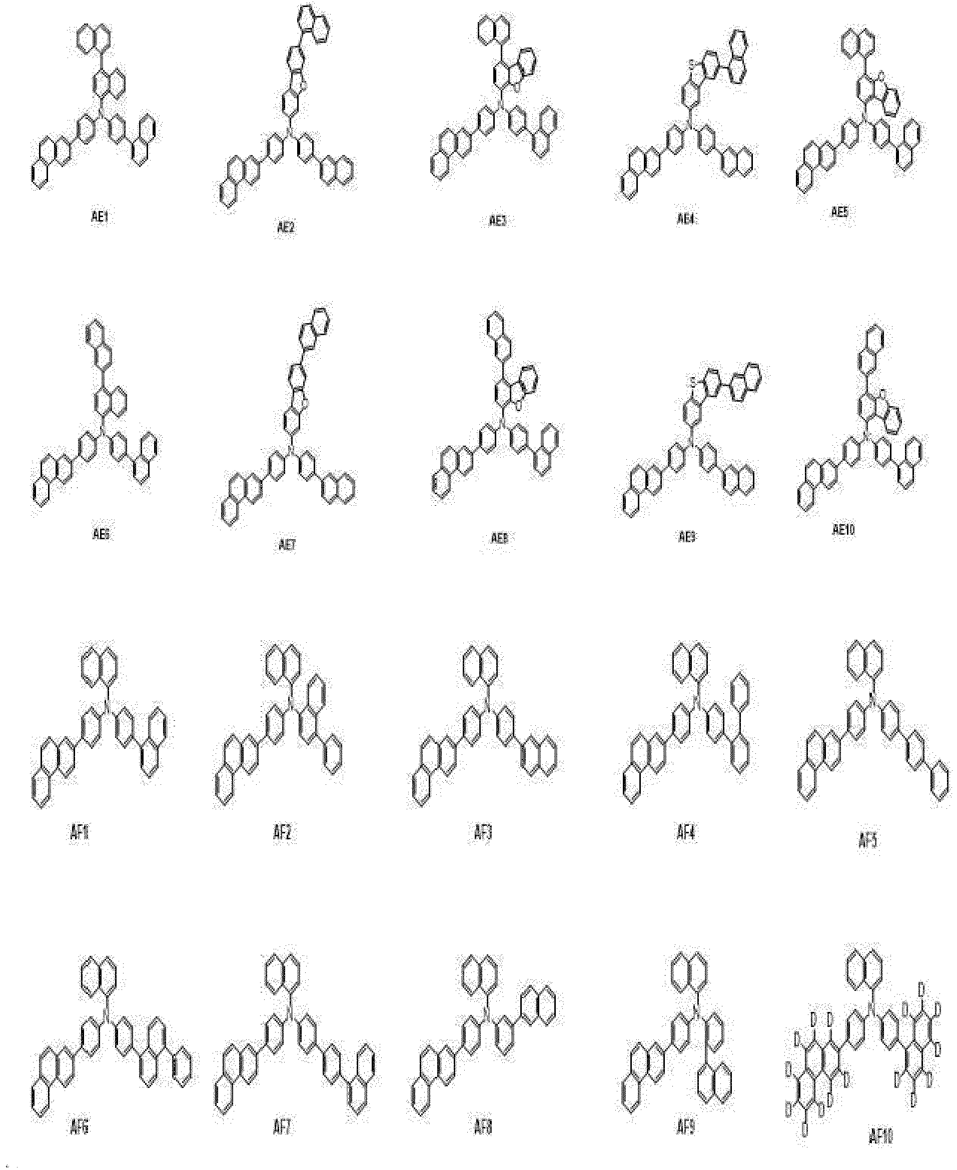

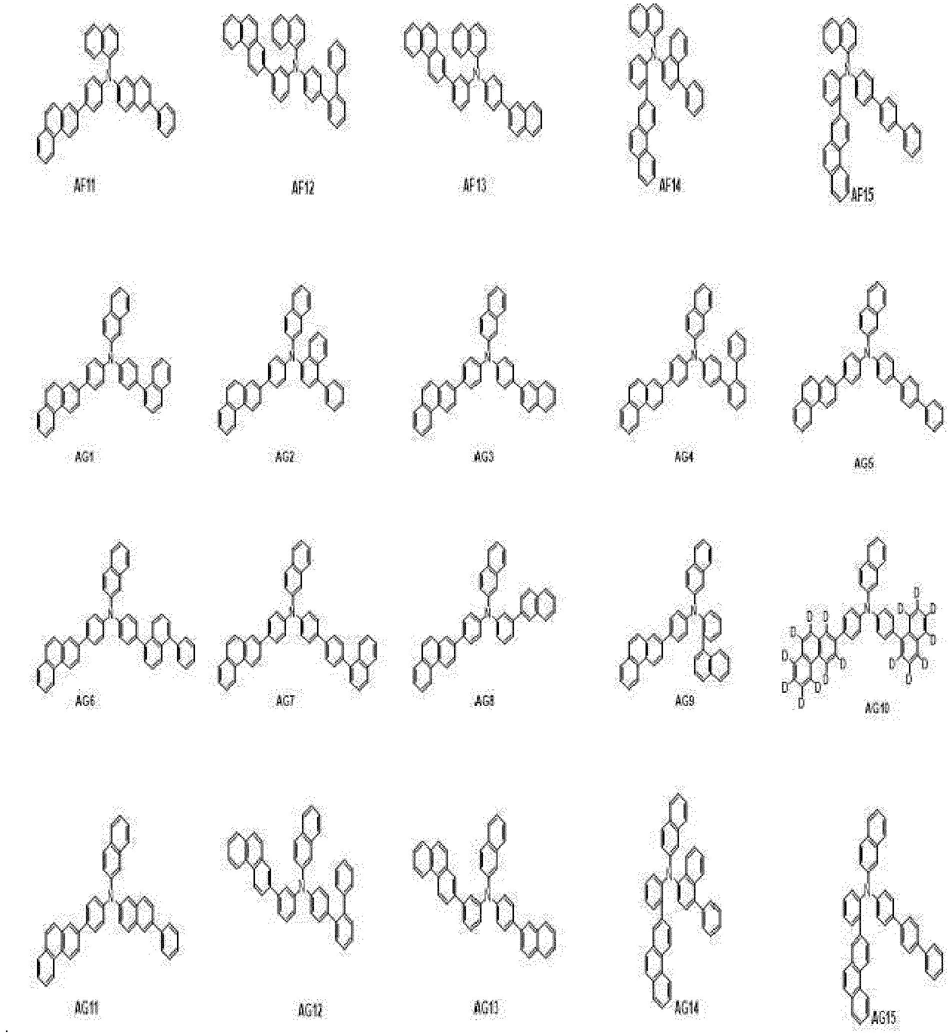

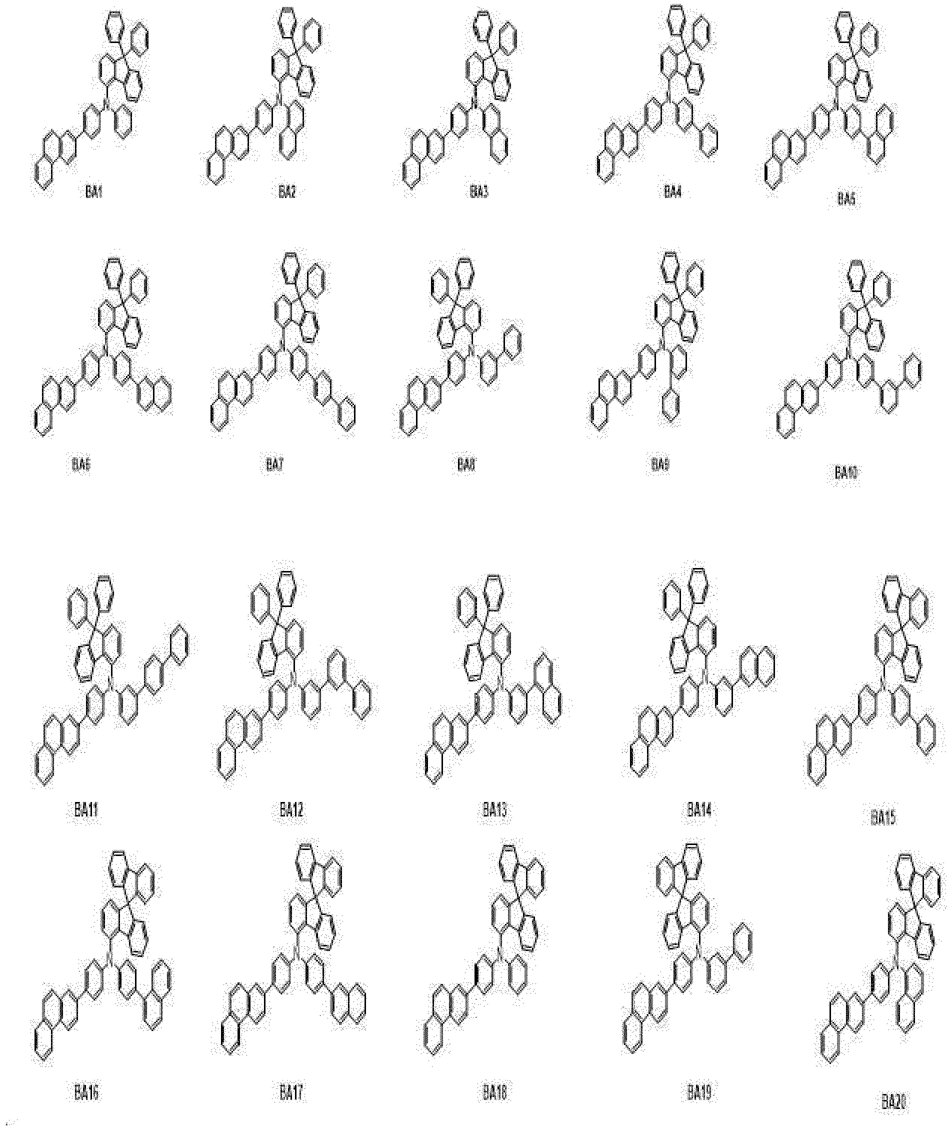

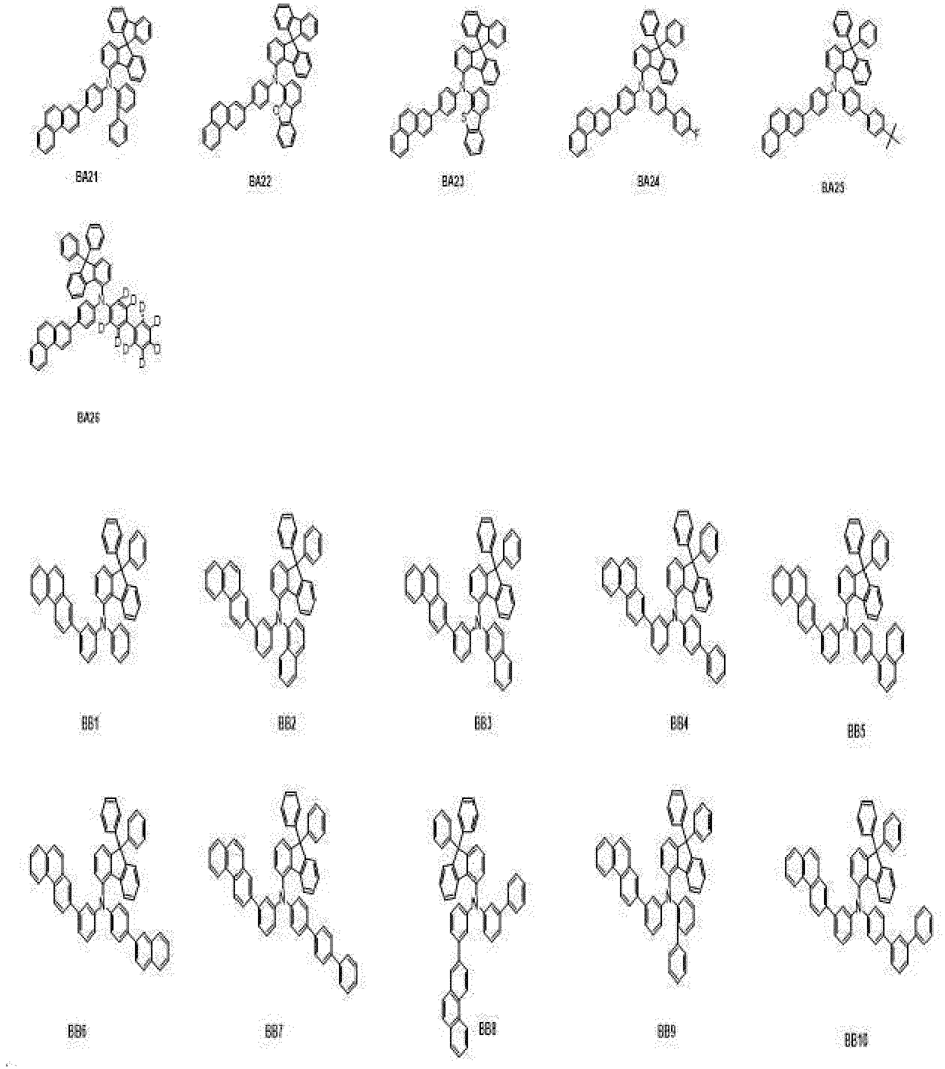

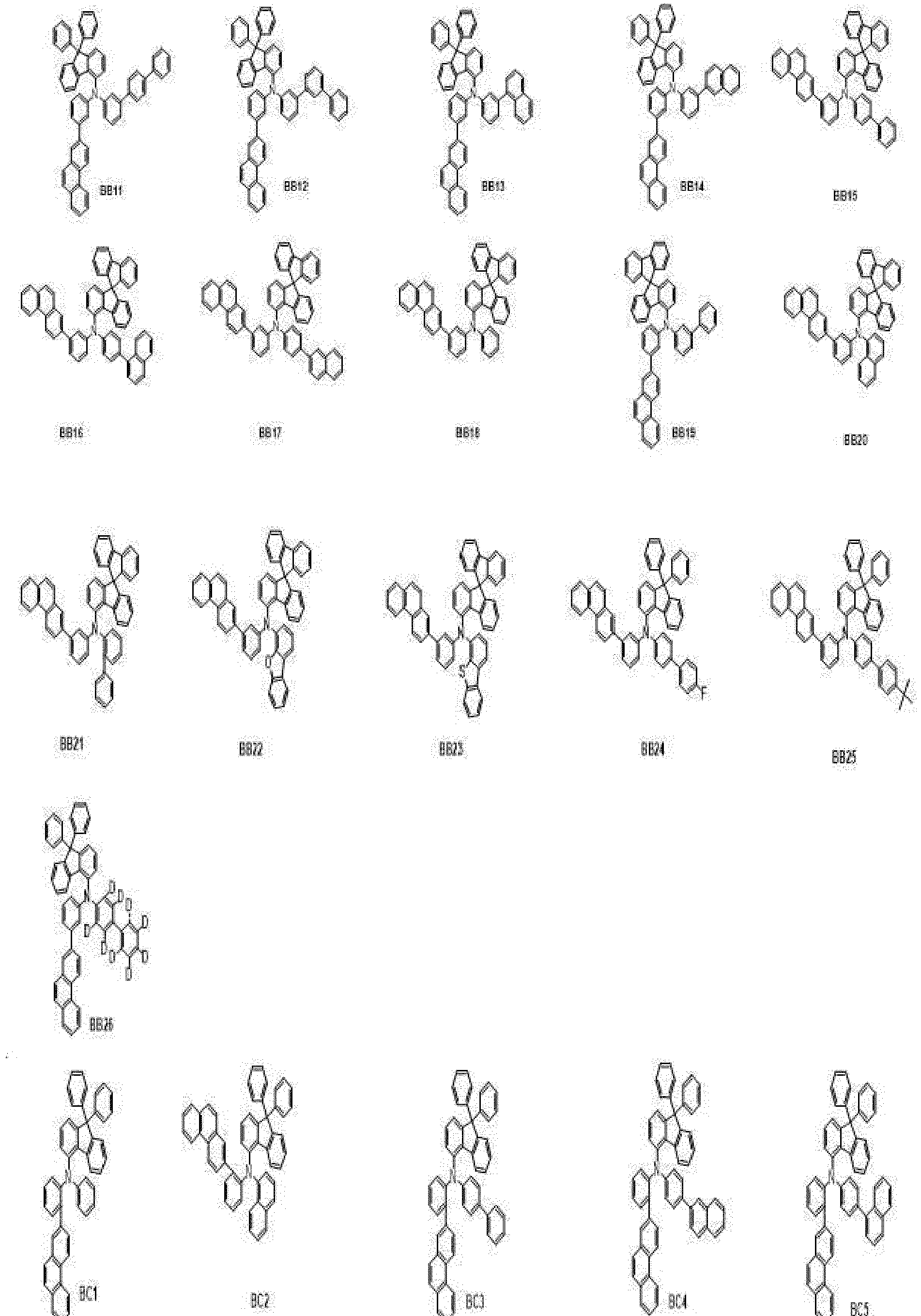

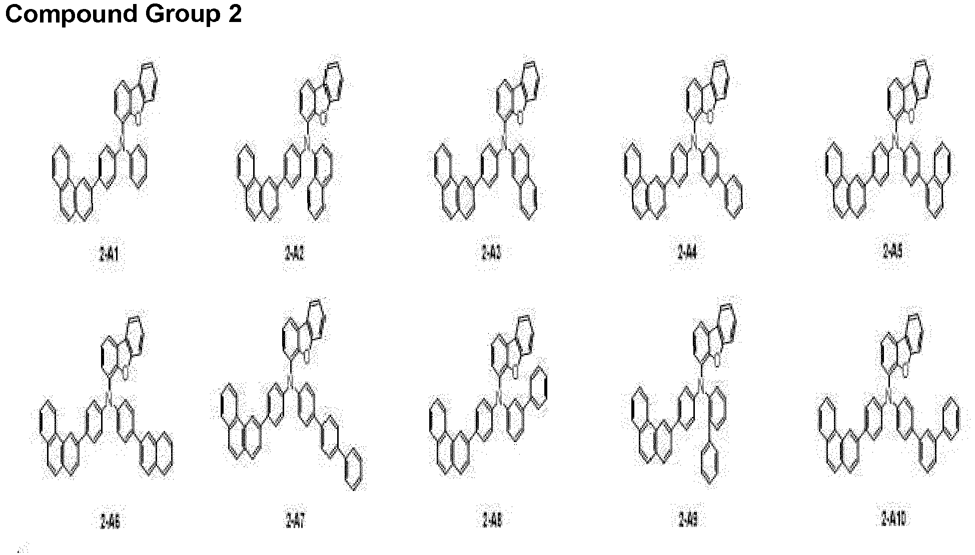

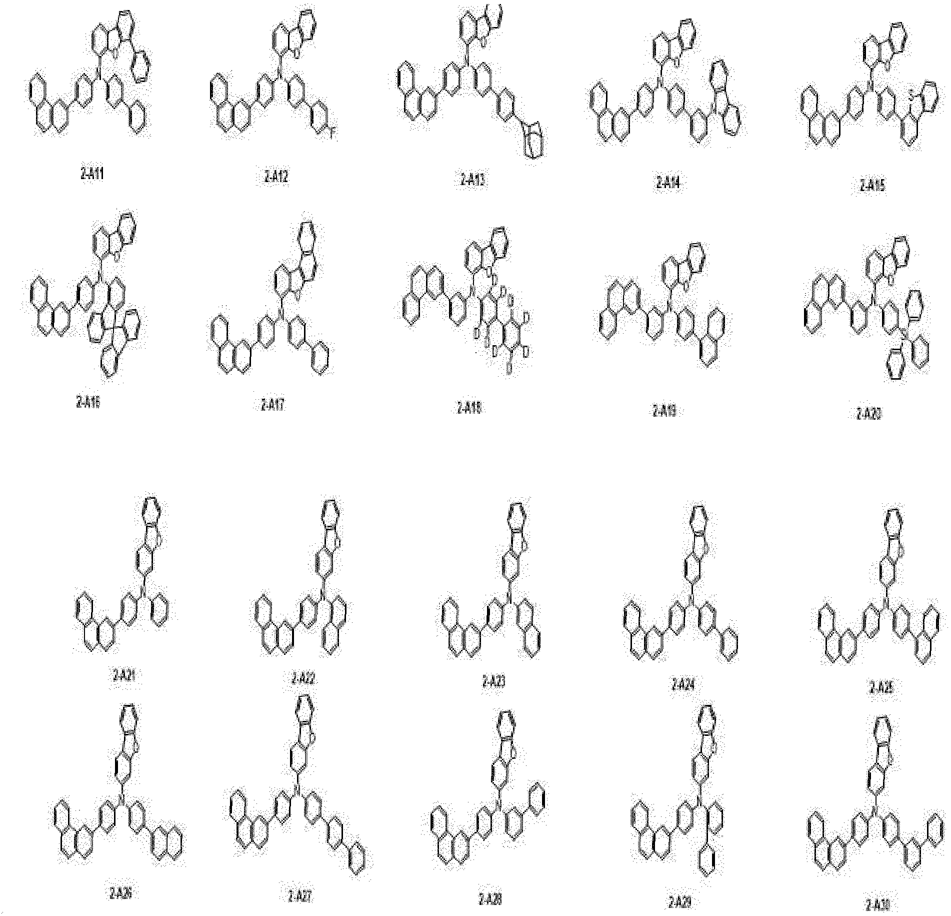

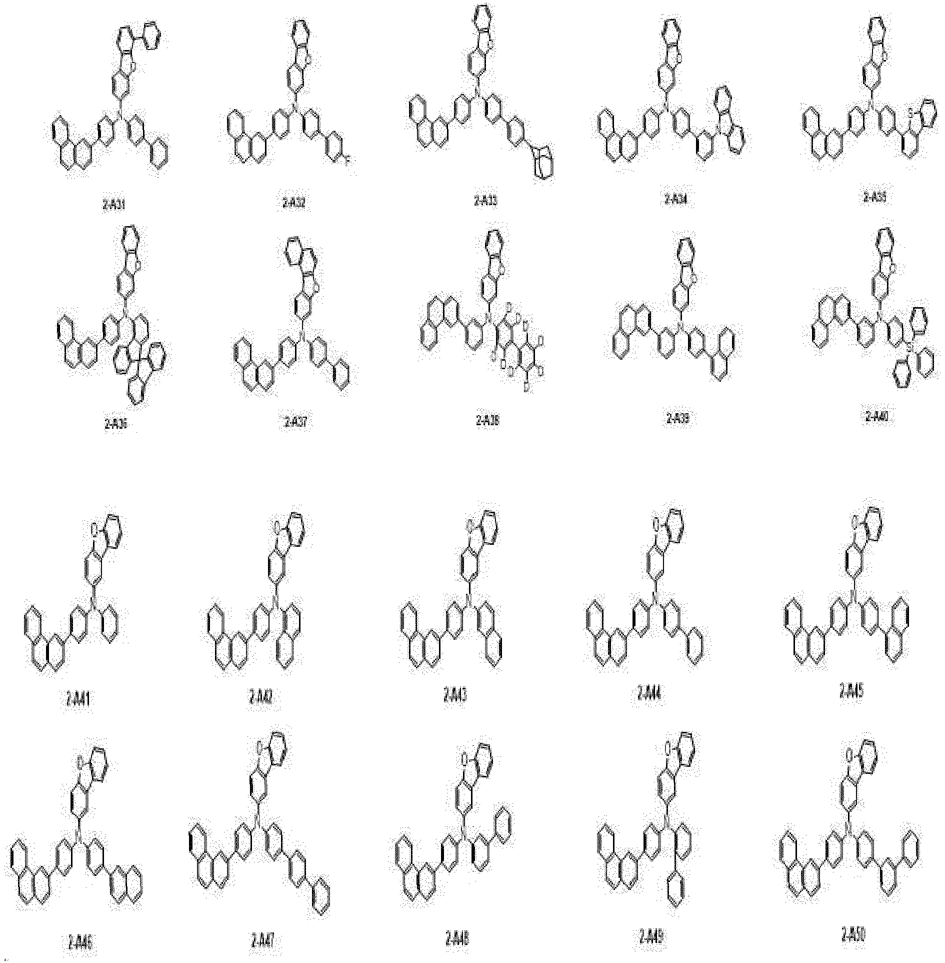

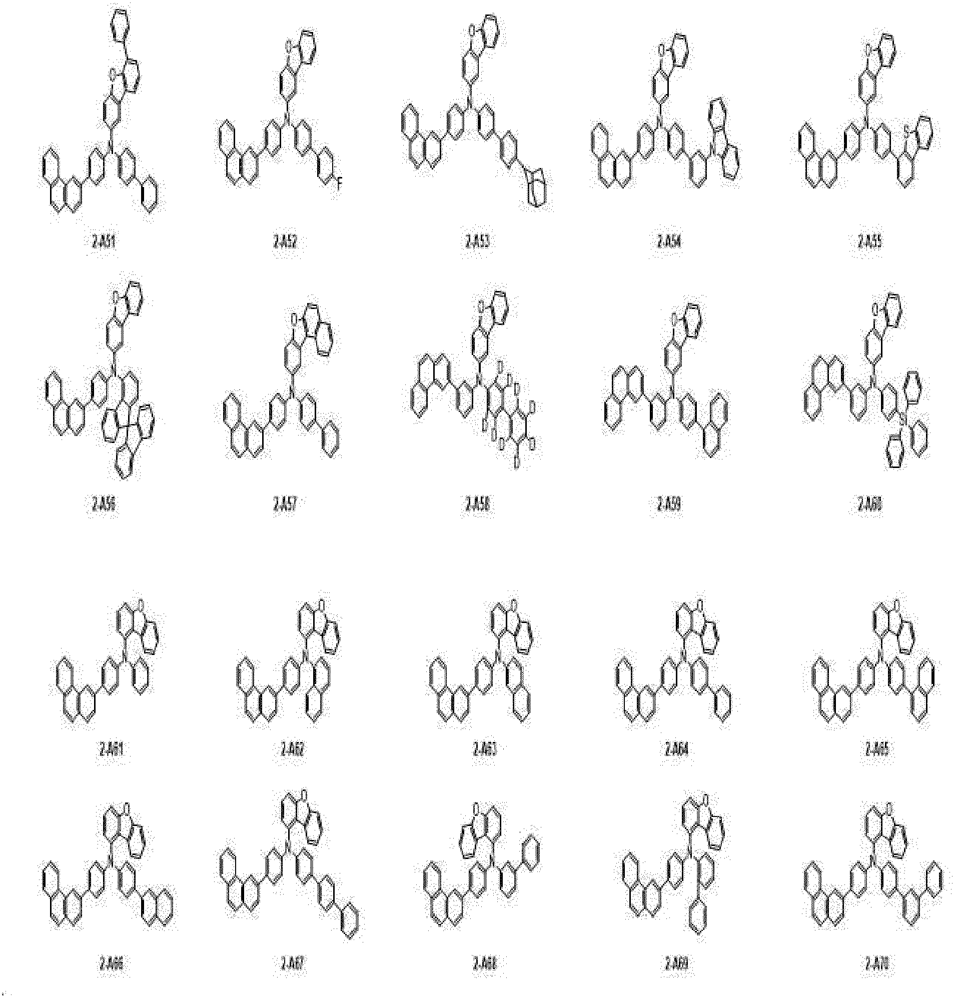

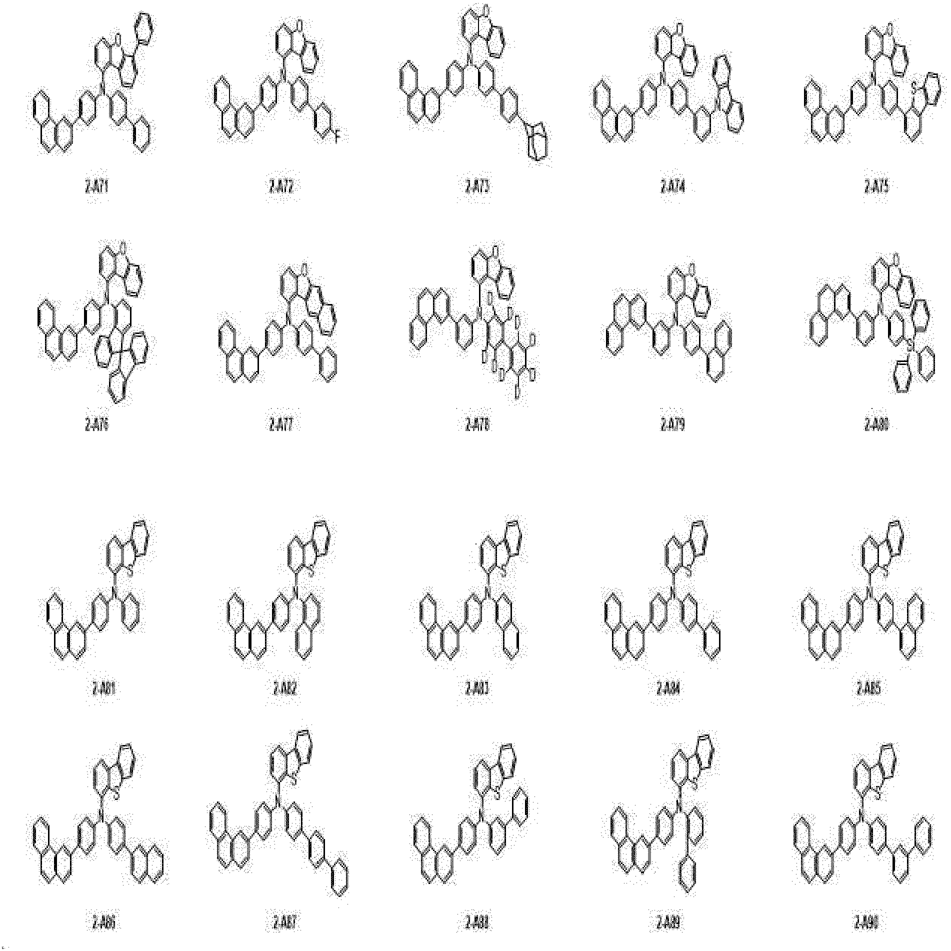

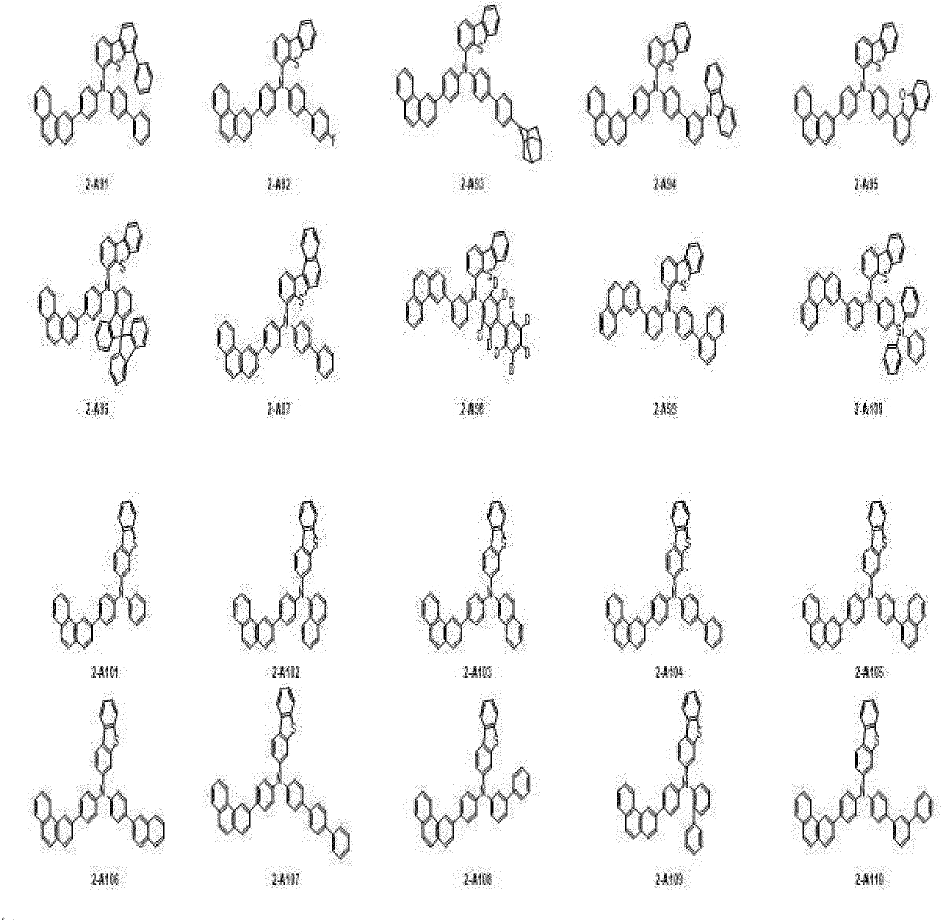

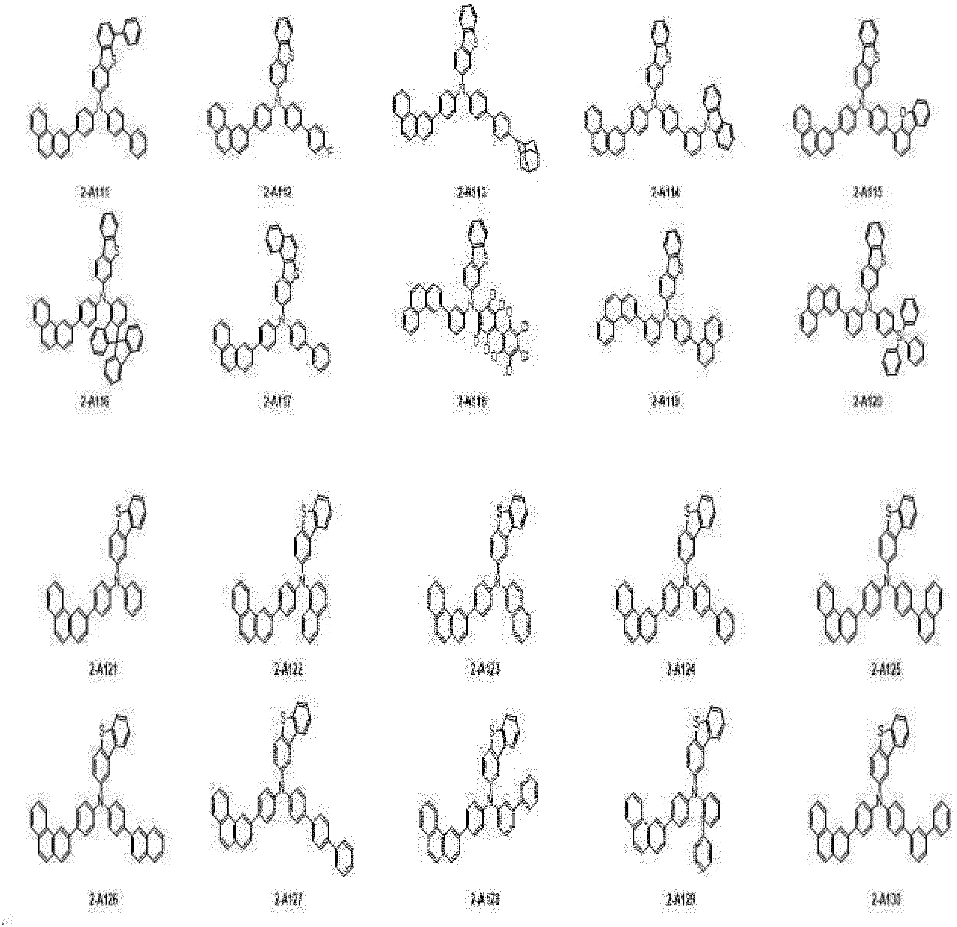

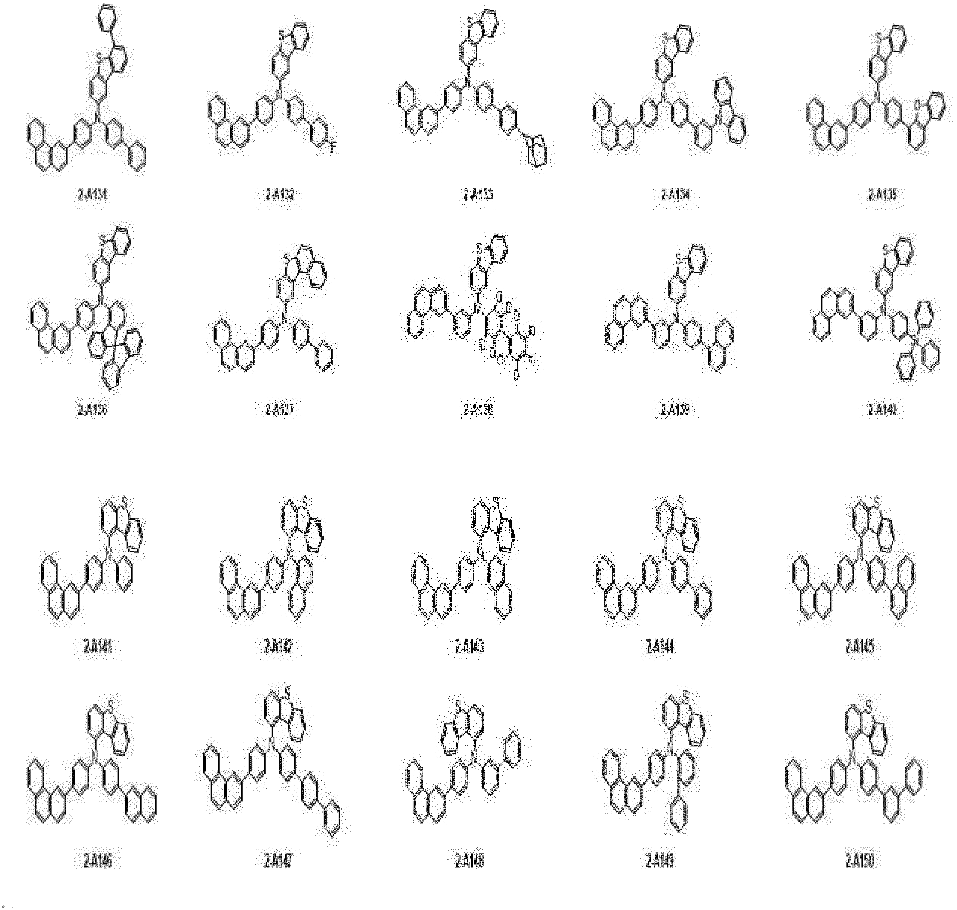

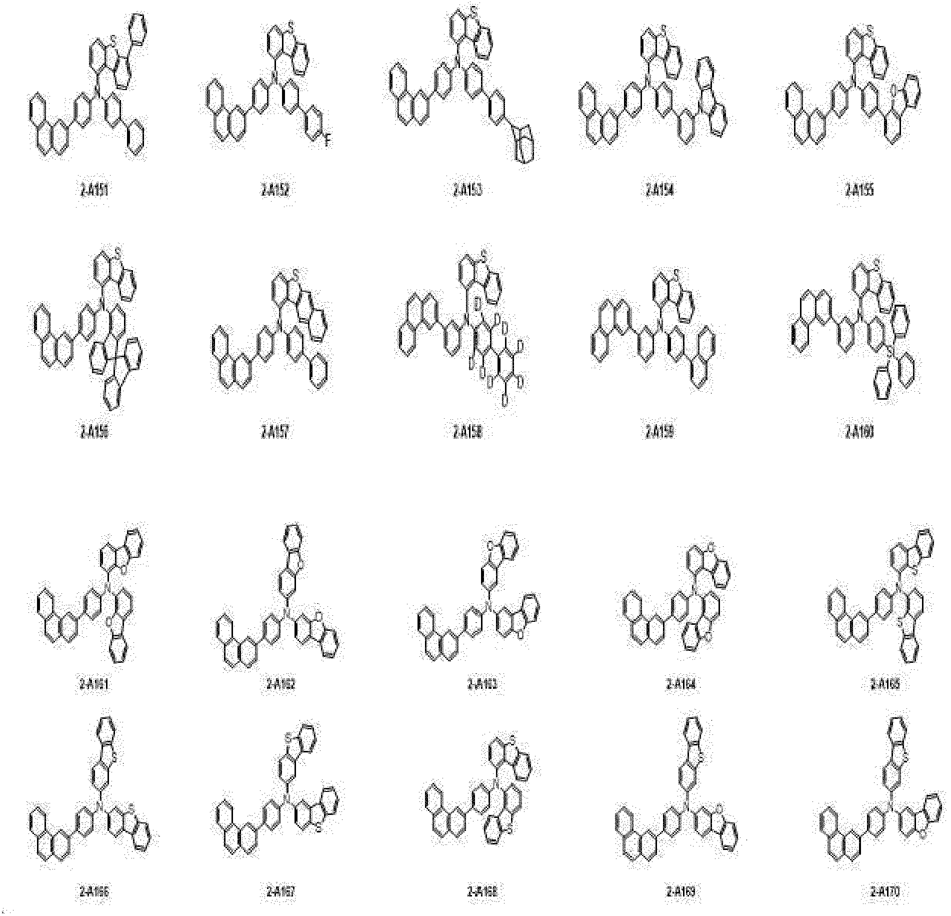

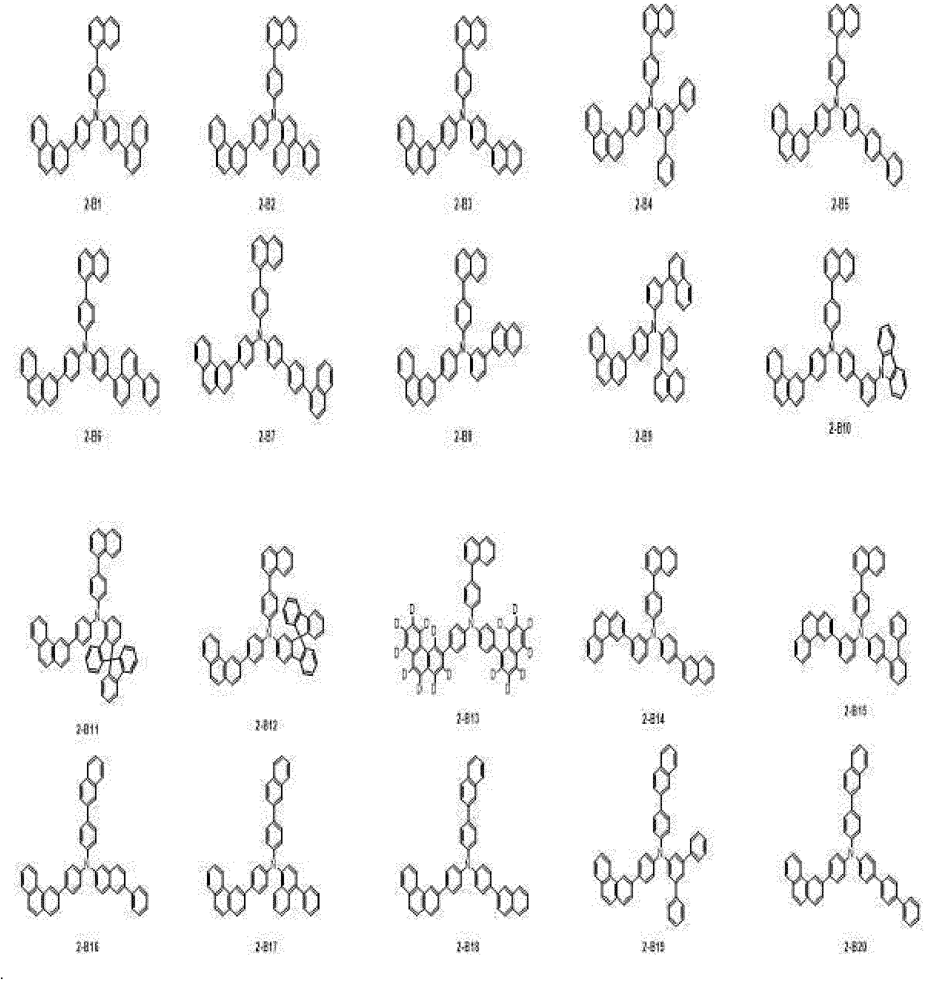

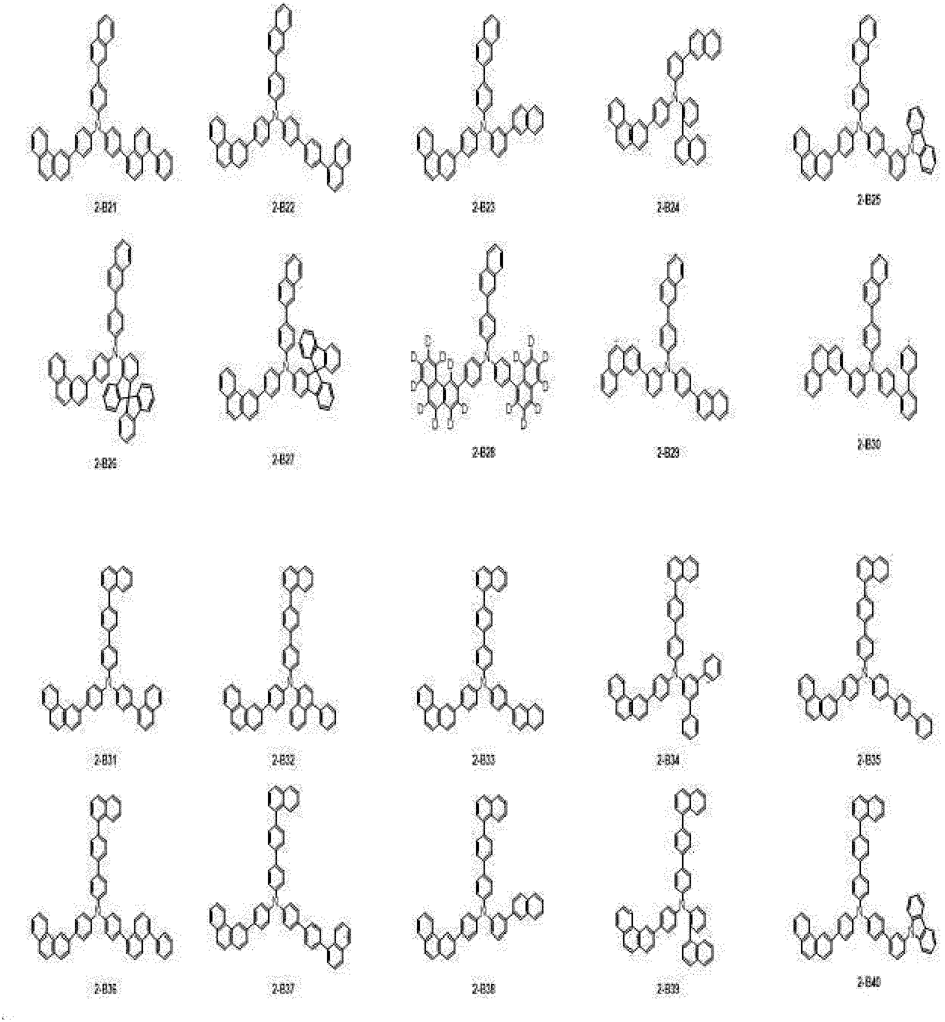

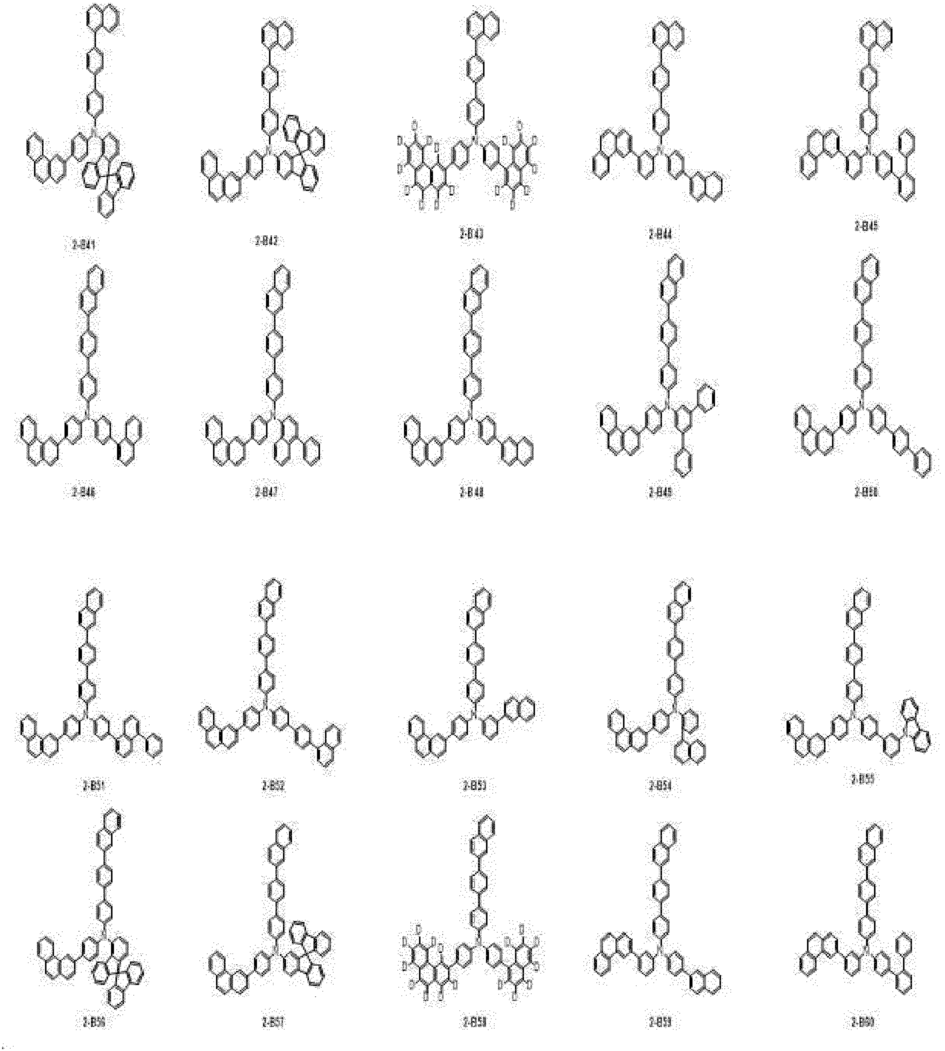

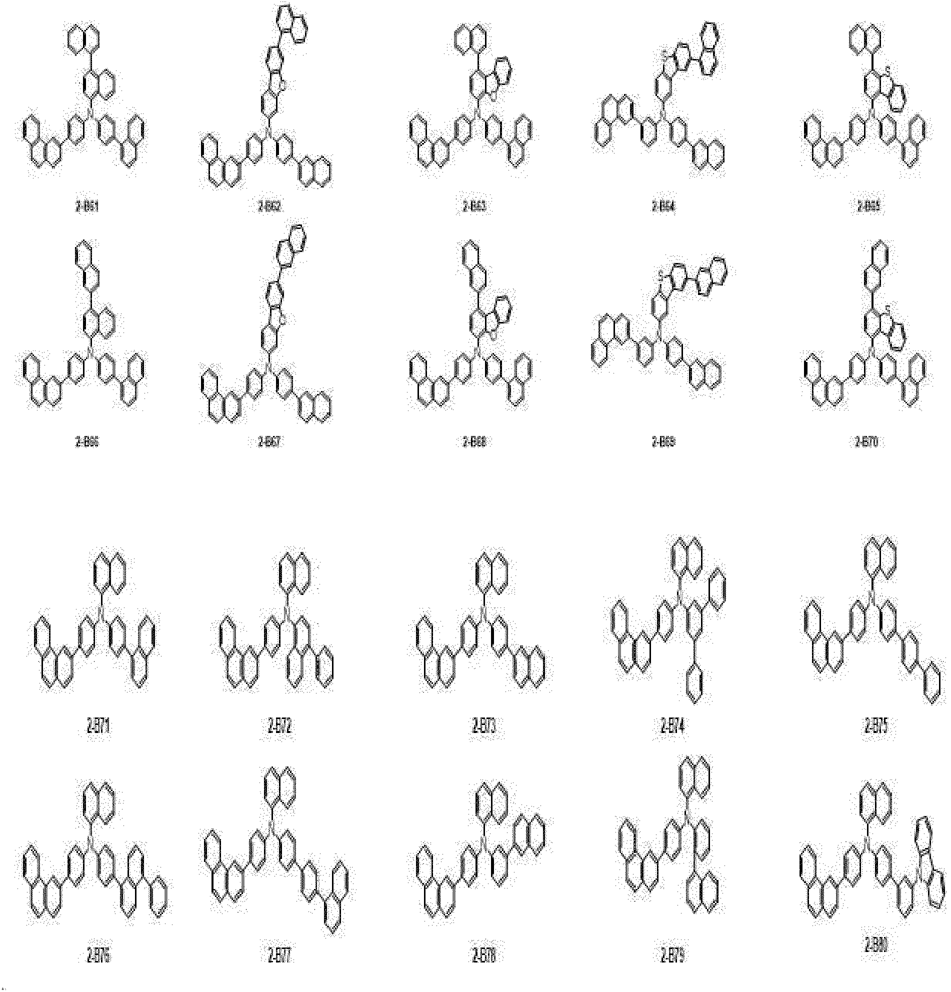

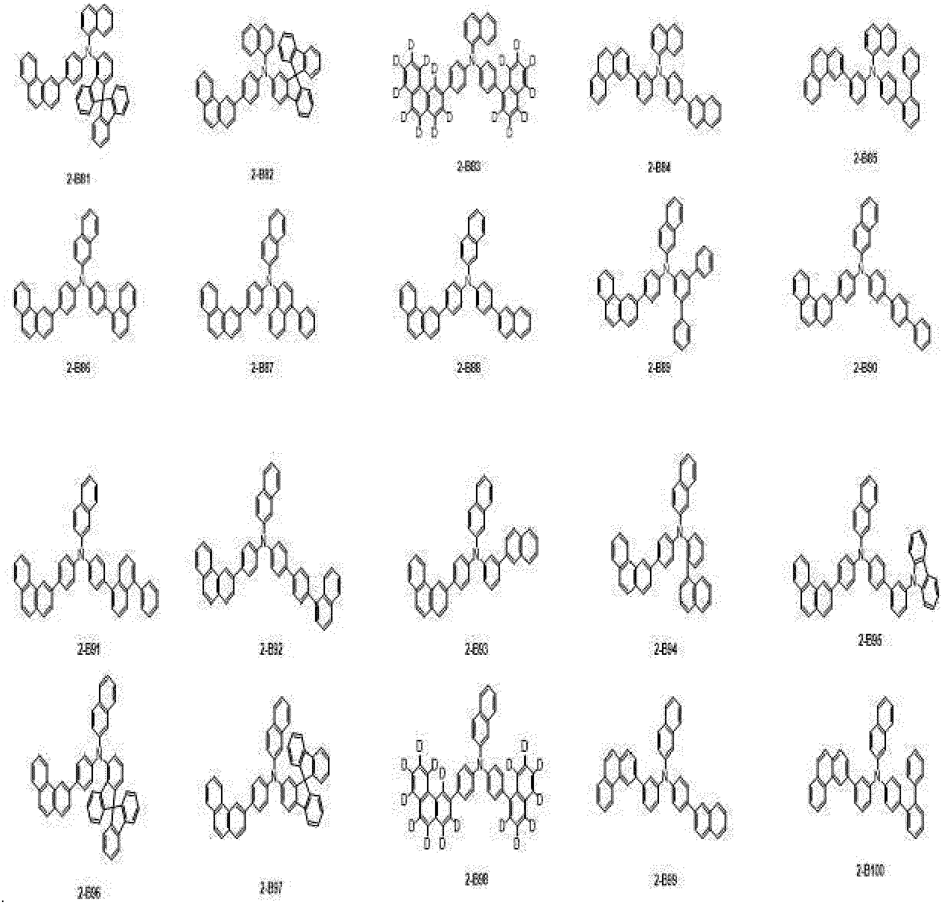

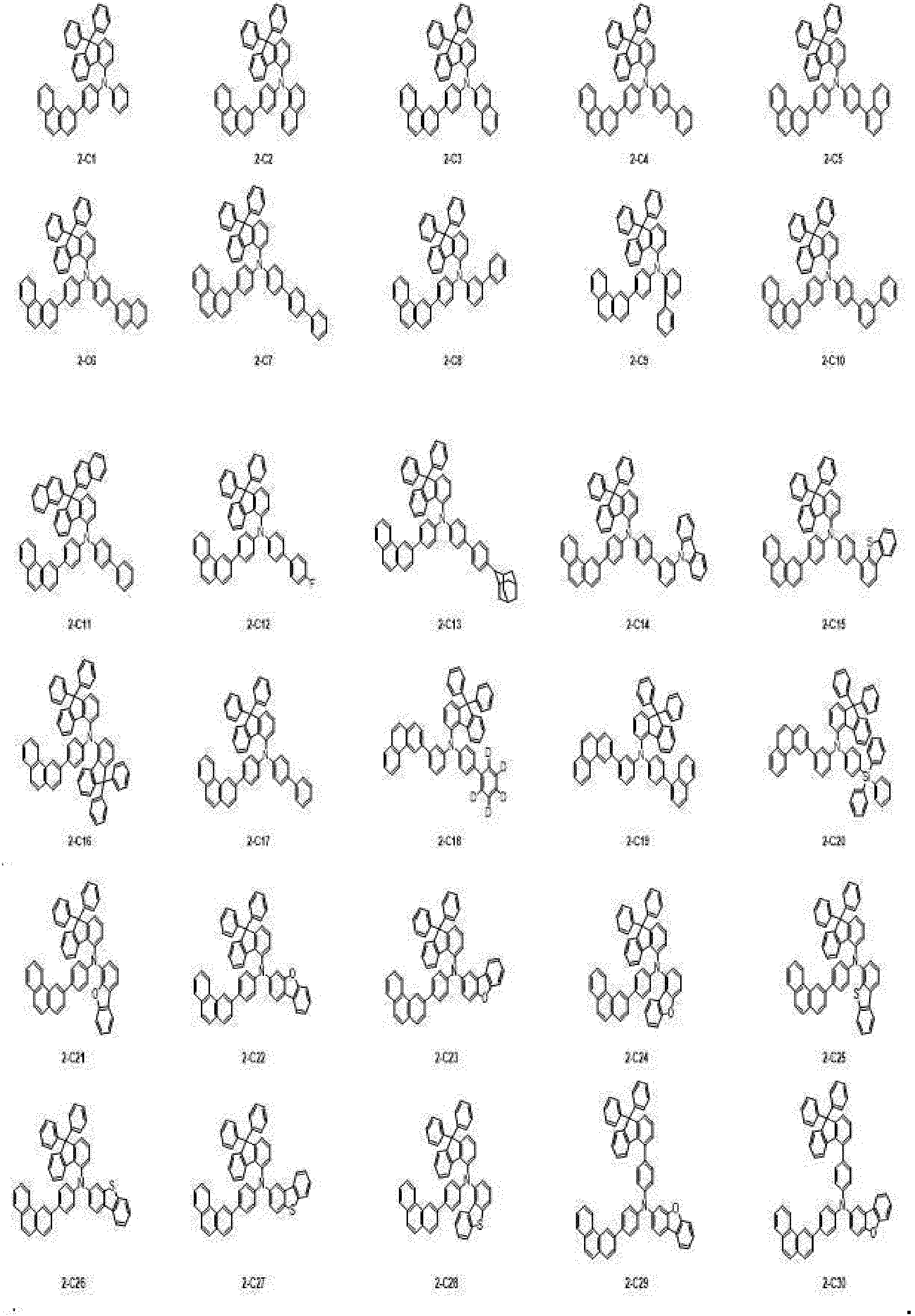

A luminescence device ED according to an embodiment of the present disclosure will be described with reference to FIGS. 3 to 6.

The hole transport region HTR includes an amine compound according to an embodiment of the present disclosure as described above. For example, the hole transport region HTR may include the amine compound represented by Formula 1.

When the hole transport region HTR is a multilayer structure having a plurality of layers, any one layer of the plurality of layers may include the amine compound represented by Formula 1. For example, the hole transport region HTR may include the hole injection layer HIL disposed on the first electrode EL1 and the hole transport layer HTL disposed on the hole injection layer, wherein the hole transport layer HTL may include the amine compound represented by Formula 1. However, embodiments are not limited thereto, for example, the hole injection layer HIL may include the amine compound represented by Formula 1.

The hole transport region HTR may include one or two or more of the amine compounds represented by Formula 1. For example, the hole transport region HTR may include at least one selected from the compounds represented by Compound Group 1 as described above.

The hole transport region HTR may be formed utilizing one or more suitable methods (such as a vacuum deposition method, a spin coating method, a cast method, a Langmuir-Blodgett (LB) method, an inkjet printing method, a laser printing method, and/or a laser induced thermal imaging (LITI) method).

The hole transport region HTR may further include a compound represented by Formula H-1:

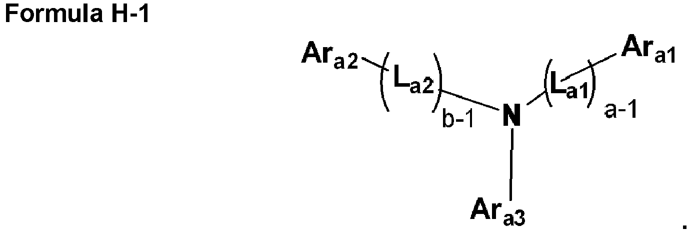

In Formula H-1, Lₐ₁ and Lₐ₂ are each independently a direct linkage, a substituted or unsubstituted arylene group having 6 to 30 ring-forming carbon atoms, or a substituted or unsubstituted heteroarylene group having 2 to 30 ring-forming carbon atoms. For example, Lₐ₁ and Lₐ₂ may each independently be a direct linkage, a substituted or unsubstituted arylene group having 6 to 20 ring-forming carbon atoms, or a substituted or unsubstituted heteroarylene group having 2 to 20 ring-forming carbon atoms. a-1 and b-1 are each independently an integer of 0 to 10. In some embodiments, when a-1 or b-1 is an integer of 2 or more, a plurality of Lₐ₁'s and Lₐ₂'s may each independently be a substituted or unsubstituted arylene group having 6 to 30 ring-forming carbon atoms, or a substituted or unsubstituted heteroarylene group having 2 to 30 ring-forming carbon atoms.

In Formula H-1, Arₐ₁ to Arₐ₃ are each independently a substituted or unsubstituted aryl group having 6 to 30 ring-forming carbon atoms, or a substituted or unsubstituted heteroaryl group having 2 to 30 ring-forming carbon atoms. For example, Arₐ₁ to Arₐ₃ may each independently be a substituted or unsubstituted aryl group having 6 to 20 ring-forming carbon atoms, or a substituted or unsubstituted heteroaryl group having 2 to 20 ring-forming carbon atoms.

The compound represented by Formula H-1 may be a monoamine compound. In some embodiments, the compound represented by Formula H-1 may be a diamine compound in which at least one among Arₐ₁ to Arₐ₃ includes an amine group as a substituent. In some embodiments, the compound represented by Formula H-1 may be a carbazole-based compound including a substituted or unsubstituted carbazole group in at least one of Arₐ₁ or Arₐ₂, or a fluorene-based compound including a substituted or unsubstituted fluorene group in at least one of Arₐ₁ or Arₐ₂.

The compound represented by Formula H-1 may be represented by any one among the compounds of Compound Group H. However, the compounds listed in Compound Group H are examples, and the compounds represented by Formula H-1 are not limited to those represented by Compound Group H:

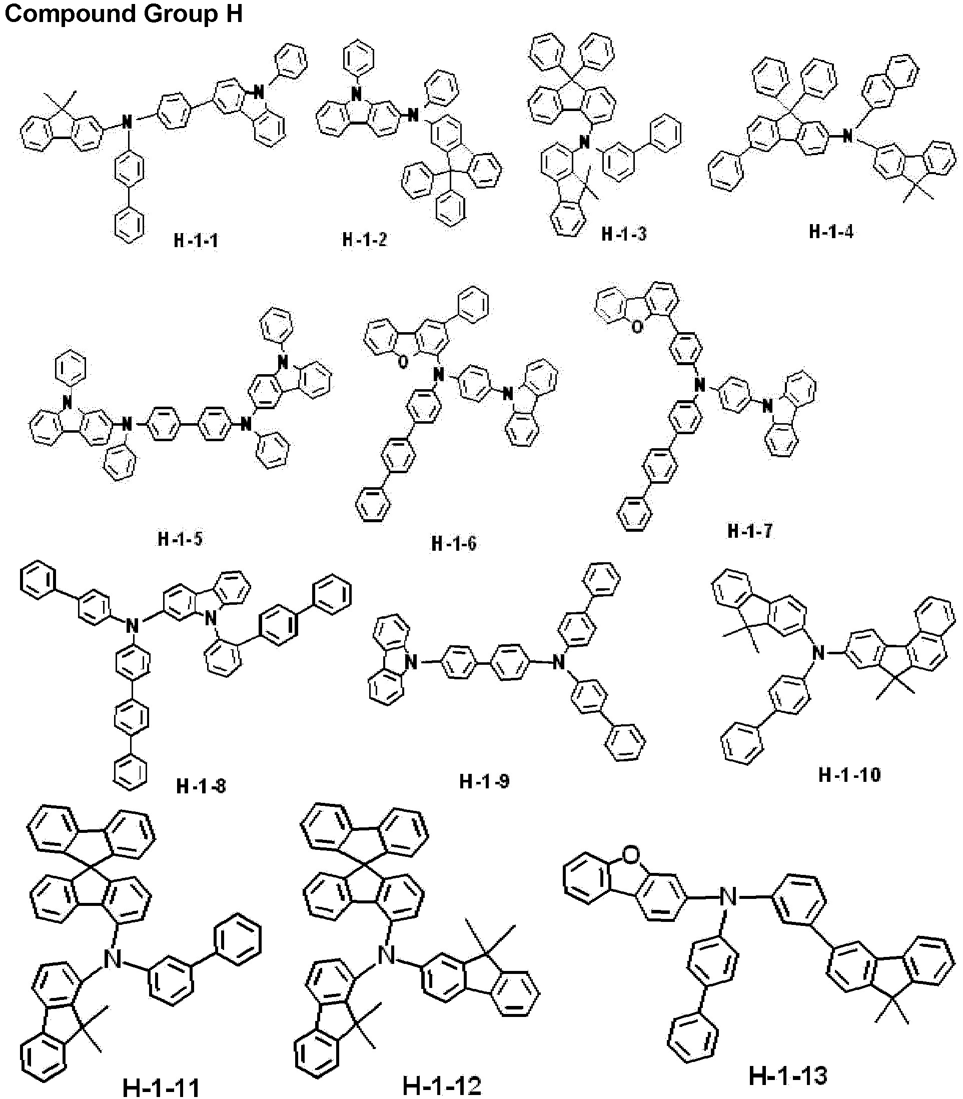

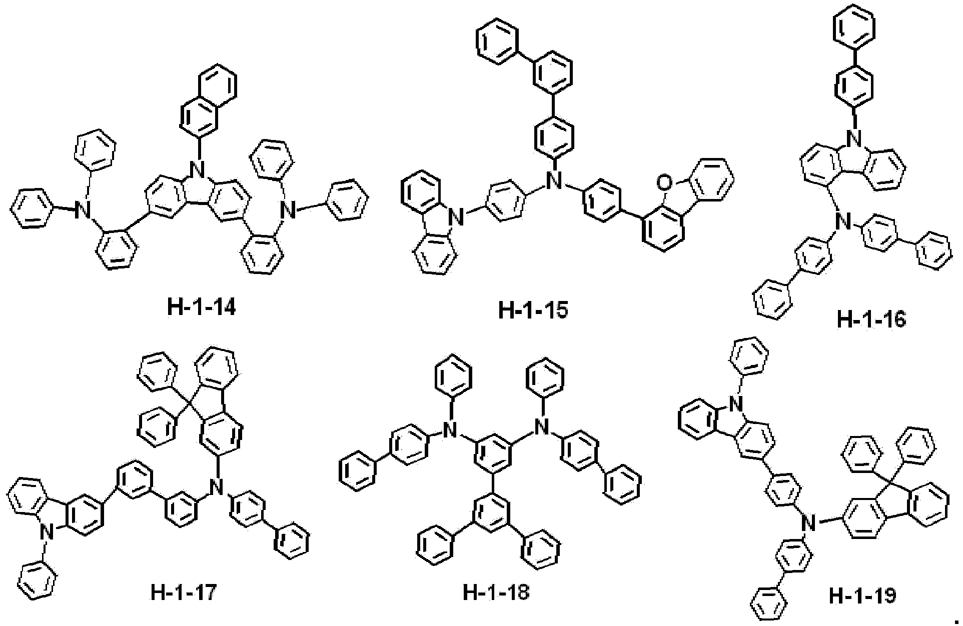

The hole transport region HTR may further include a phthalocyanine compound (such as copper phthalocyanine); N¹,N^{1'}-([1,1'-biphenyl]-4,4'-diyl)bis(N¹-phenyl-N⁴,N⁴-di-m-tolylbenzene-1,4-diamine) (DNTPD), 4,4',4"-[tris(3-methylphenyl)phenylamino] triphenylamine (m-MTDATA), 4,4'4"-tris(N,N-diphenylamino)triphenylamine (TDATA), 4,4',4"-tris[N(1-naphthyl)-N-phenylamino]-triphenylamine (1-TNATA), 4,4',4"-tris[N(2-naphthyl)-N-phenylamino]-triphenylamine (2-TNATA), poly(3,4-ethylene dioxythiophene)/poly(4-styrene sulfonate) (PEDOT/PSS), polyaniline/dodecylbenzene sulfonic acid (PANI/DBSA), polyaniline/camphor sulfonic acid (PANI/CSA), polyaniline/poly(4-styrene sulfonate) (PANI/PSS), N,N'-di(naphthalene-1-yl)-N,N'-diphenyl-benzidine (NPB), triphenylamine-containing polyether ketone (TPAPEK), 4-isopropyl-4'-methyldiphenyliodonium [tetrakis(pentafluorophenyl)borate], dipyrazino[2,3-f: 2',3'-h]quinoxaline-2,3,6,7,10,11-hexacarbonitrile (HATCN), etc.

The hole transport region HTR may further include, for example, carbazole derivatives (such as N-phenyl carbazole and/or polyvinyl carbazole), fluorene derivatives, N,N'-bis(3-methylphenyl)-N,N'-diphenyl-[1,1-biphenyl]-4,4'-diamine (TPD), triphenylamine derivatives (such as 4,4',4"-tris(N-carbazolyl)triphenylamine (TCTA)), N,N'-di(naphthalen-1-yl)-N,N'-diphenyl-benzidine (NPB), 4,4'-cyclohexylidene bis[N,N-bis(4-methylphenyl]benzenamine] (TAPC), 4,4'-bis[N,N'-(3-tolyl)amino]-3,3'-dimethylbiphenyl (HMTPD), 1,3-bis(N-carbazolyl)benzene (mCP), etc.

The hole transport region HTR may further include 9-(4-tert-butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), etc.

The hole transport region HTR may include the above-described compound of the hole transport region in at least one of a hole injection layer HIL, a hole transport layer HTL, or an electron blocking layer EBL.

The thickness of the hole transport region HTR may be about 100 Å to about 10,000 Å, for example, about 100 Å to about 5,000 Å. The thickness of the hole injection layer HIL may be, for example, about 30 Å to about 1,000 Å, and the thickness of the hole transport layer HTL may be about 30 Å to about 1,000 Å. For example, the thickness of the electron blocking layer EBL may be about 10 Å to about 1,000 Å. When the thicknesses of the hole transport region HTR, the hole injection layer HIL, the hole transport layer HTL and the electron blocking layer EBL satisfy the above-described ranges, satisfactory hole transport characteristic may be achieved without a substantial increase in a driving voltage.

The hole transport region HTR may further include a charge generating material to increase conductivity in addition to the above-described materials. The charge generating material may be dispersed substantially uniformly or non-uniformly in the hole transport region HTR. The charge generating material may be, for example, a p-dopant. The p-dopant may be selected from quinone derivatives, metal oxides, or cyano group-containing compounds, but embodiments of the present disclosure are not limited thereto. For example, non-limiting examples of the p-dopant may include quinone derivatives (such as tetracyanoquinodimethane (TCNQ) and/or 2,3,5,6-tetrafluoro-7,7,8,8-tetracyanoquinodimethane (F4-TCNQ)), metal oxides (such as tungsten oxide and/or molybdenum oxide), etc., but embodiments of the present disclosure are not limited thereto.

As described above, the hole transport region HTR may further include at least one of the hole buffer layer or the electron blocking layer EBL in addition to the hole injection layer HIL and the hole transport layer HTL. The hole buffer layer may compensate for a resonance distance according to the wavelength of light emitted from the emission layer EML, and may thus increase light emission efficiency. Materials that may be included in the hole transport region HTR may be included in the hole buffer layer. The electron blocking layer EBL may prevent or reduce electron injection from the electron transport region ETR into the hole transport region HTR.

The emission layer EML is provided on the hole transport region HTR. The emission layer EML may have a thickness of, for example, about 100 Å to about 1,000 Å or about 100 Å to about 300 Å. The emission layer EML may have a single layer formed of a single material, a single layer formed of a plurality of different materials, or a multilayer structure having a plurality of layers formed of a plurality of different materials.

In the luminescence device ED of an embodiment, the emission layer EML may include anthracene derivatives, pyrene derivatives, fluoranthene derivatives, chrysene derivatives, dihydrobenzanthracene derivatives, and/or triphenylene derivatives. For example, the emission layer EML may include anthracene derivatives and/or pyrene derivatives.

In each luminescence device ED of embodiments illustrated in FIGS. 3 to 6, the emission layer EML may include a host and a dopant, and the emission layer EML may include a compound represented by Formula E-1. The compound represented by Formula E-1 may be utilized as a fluorescence host material.

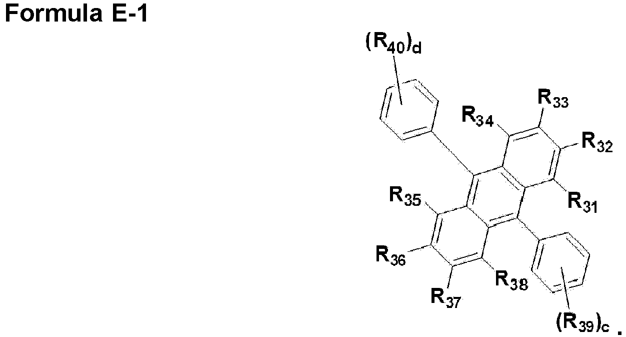

In Formula E-1, R₃₁ to R₄₀ are each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 (*e.g.* 1 to 6) carbon atoms, a substituted or unsubstituted alkenyl group having 2 to 10 (*e.g.* 2 to 6) carbon atoms, a substituted or unsubstituted aryl group having 6 to 30 (e.g. 6 to 20) ring-forming carbon atoms, or a substituted or unsubstituted heteroaryl group having 2 to 30 (*e.g.* 2 to 20) ring-forming carbon atoms, and/or are bonded to an adjacent group to form a ring. In some embodiments, R₃₁ to R₄₀ may be bonded to an adjacent group to form a saturated hydrocarbon ring or an unsaturated hydrocarbon ring, a saturated heterocycle, or an unsaturated heterocycle.

In Formula E-1, c and d are each independently an integer of 0 to 5.

Formula E-1 may be represented by any one among Compound E1 to Compound E19:

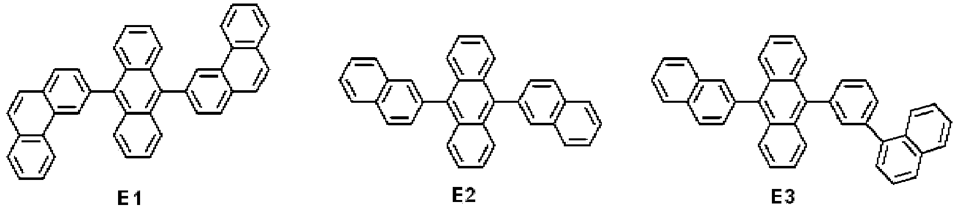

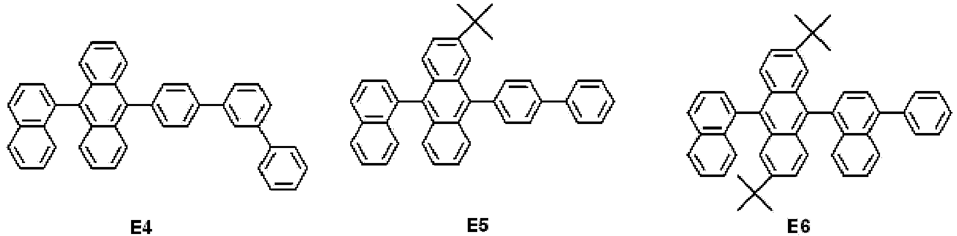

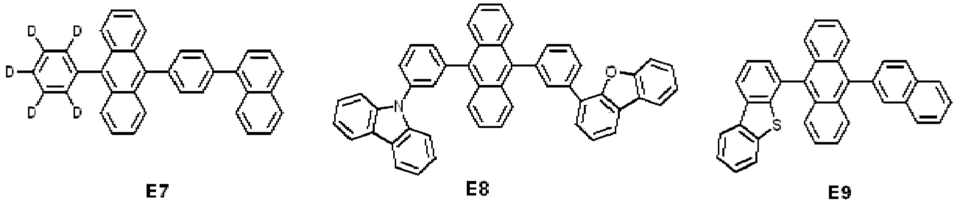

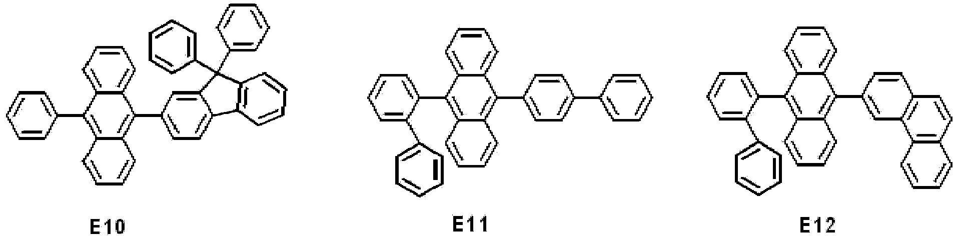

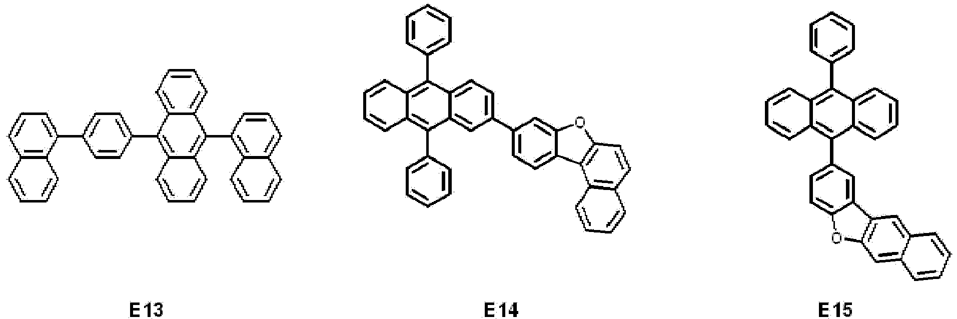

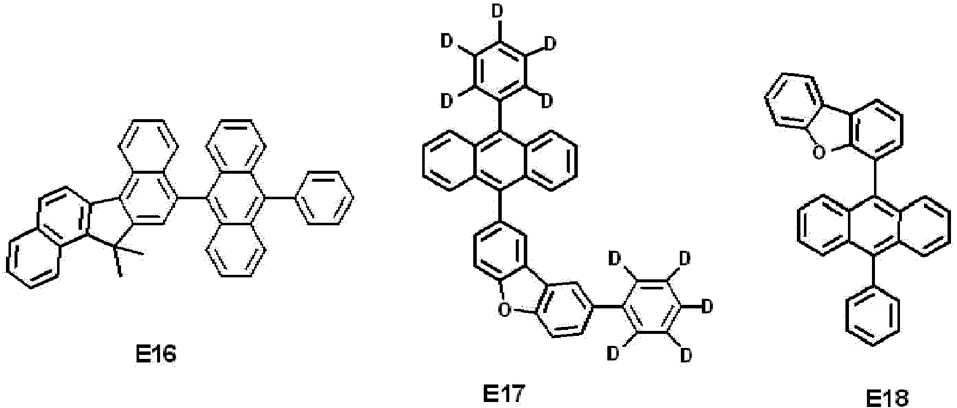

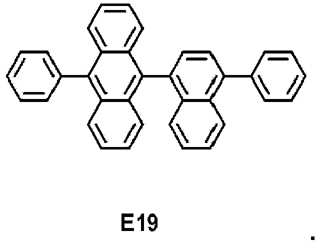

In an embodiment, the emission layer EML may include a compound represented by Formula E-2a or Formula E-2b. The compound represented by Formula E-2a or Formula E-2b may be utilized as a phosphorescence host material.

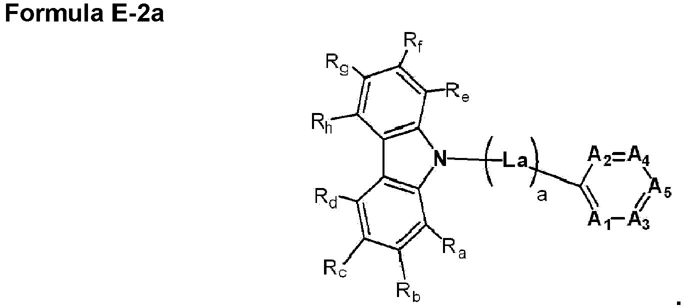

In Formula E-2a, a is an integer of 0 to 10, Lₐ is a direct linkage, a substituted or unsubstituted arylene group having 6 to 30 (e.g. 6 to 20) ring-forming carbon atoms, or a substituted or unsubstituted heteroarylene group having 2 to 30 (*e.g*. 2 to 20) ring-forming carbon atoms. When a is an integer of 2 or more, a plurality of Lₐ's may each independently be a substituted or unsubstituted arylene group having 6 to 30 ring-forming carbon atoms, or a substituted or unsubstituted heteroarylene group having 2 to 30 ring-forming carbon atoms.

In some embodiments, in Formula E-2a, A₁ to A₅ may each independently be N or CRᵢ. Rₐ to Rᵢ are each independently a hydrogen atom, a deuterium atom, a substituted or unsubstituted amine group, a substituted or unsubstituted thio group, a substituted or unsubstituted oxy group, a substituted or unsubstituted alkyl group having 1 to 20 (*e.g.* 1 to 10) carbon atoms, a substituted or unsubstituted alkenyl group having 2 to 20 (*e.g.* 2 to 10) carbon atoms, a substituted or unsubstituted aryl group having 6 to 30 (*e.g.* 6 to 20) ring-forming carbon atoms, or a substituted or unsubstituted heteroaryl group having 2 to 30 (*e.g.* 2 to 20) ring-forming carbon atoms, and/or are bonded to an adjacent group to form a ring. Rₐ to Rᵢ may be bonded to an adjacent group to form a hydrocarbon ring or a heterocycle containing N, O, S, etc. as a ring-forming atom.

In some embodiments, in Formula E-2a, two or three selected from A₁ to A₅ may be N, and the rest may be CRᵢ.

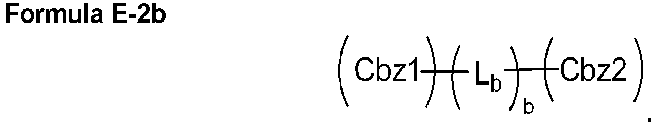

In Formula E-2b, Cbz1 and Cbz2 are each independently an unsubstituted carbazole group, or a carbazole group substituted with an aryl group having 6 to 30 (*e.g.* 6 to 20) ring-forming carbon atoms. L_{b} is a direct linkage, a substituted or unsubstituted arylene group having 6 to 30 (*e.g.* 6 to 20) ring-forming carbon atoms, or a substituted or unsubstituted heteroarylene group having 2 to 30 (*e.g.* 2 to 20) ring-forming carbon atoms. In some embodiments, b may be an integer of 0 to 10, and when b is an integer of 2 or more, a plurality of L_{b}'s may each independently be a substituted or unsubstituted arylene group having 6 to 30 ring-forming carbon atoms, or a substituted or unsubstituted heteroarylene group having 2 to 30 ring-forming carbon atoms.

The compound represented by Formula E-2a or Formula E-2b may be represented by any one among the compounds of Compound Group E-2. However, the compounds listed in Compound Group E-2 are examples, and the compound represented by Formula E-2a or Formula E-2b is not limited to those represented by Compound Group E-2.

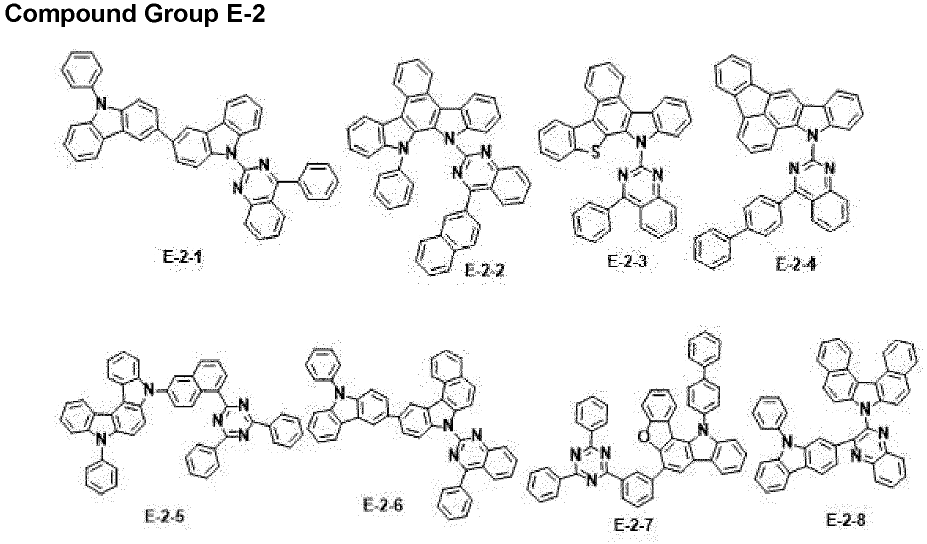

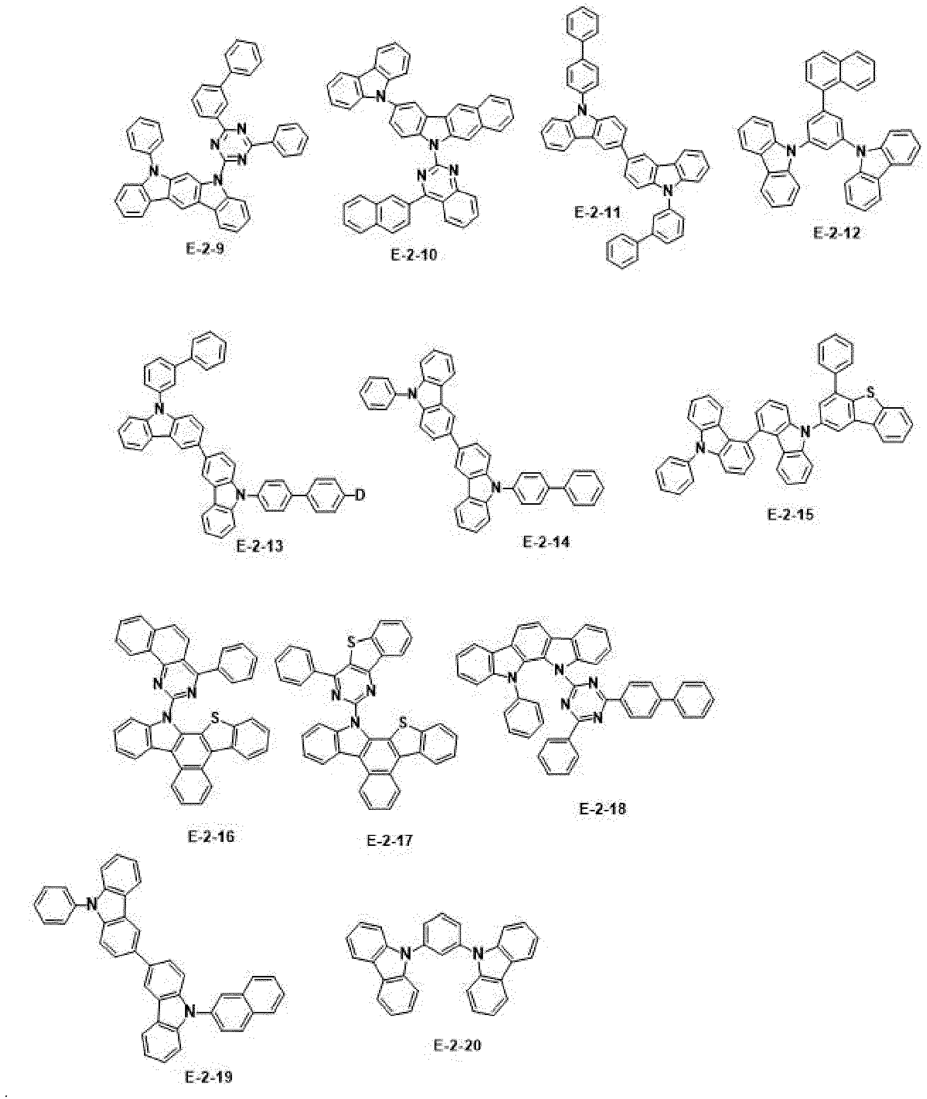

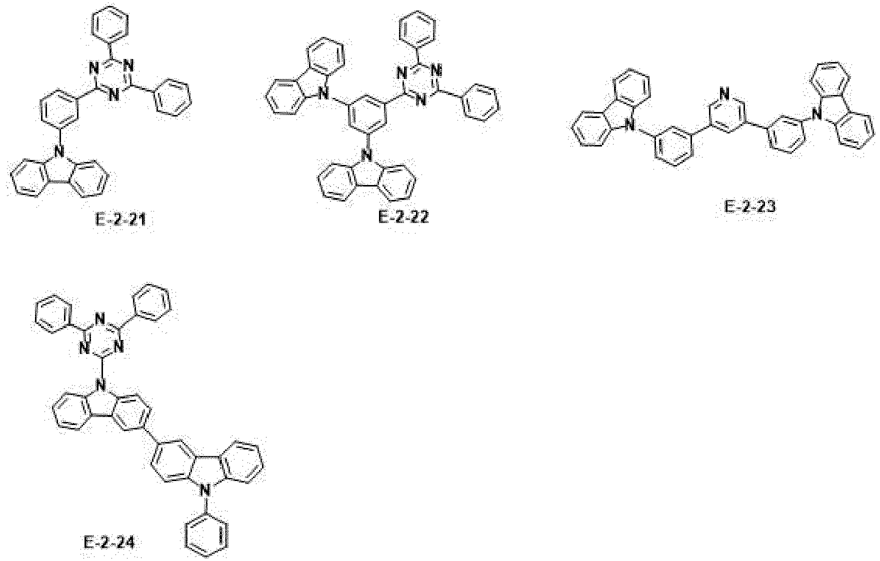

The emission layer EML may further include any suitable material in the art as a host material. For example, the emission layer EML may include, as a host material, at least one of bis[2-(diphenylphosphino)phenyl] ether oxide (DPEPO), 4,4'-bis(N-carbazolyl)-1,1'-biphenyl (CBP), 1,3-bis(carbazol-9-yl)benzene (mCP), 2,8-bis(diphenylphosphoryl)dibenzo[b,d]furan (PPF), 4,4',4"-tris(carbazol-9-yl)-triphenylamine (TCTA), or 1,3,5-tris(1-phenyl-1H-benzo[d]imidazole-2-yl)benzene (TPBi). However, embodiments of the present disclosure are not limited thereto, and for example, tris(8-hydroxyquinolino)aluminium (Alq₃), 9,10-di(naphthalene-2-yl)anthracene (ADN), 2-tert-butyl-9,10-di(naphth-2-yl)anthracene (TBADN), distyrylarylene (DSA), 4,4'-bis(9-carbazolyl)-2,2'-dimethyl-biphenyl (CDBP), 2-methyl-9,10-bis(naphthalen-2-yl)anthracene (MADN), hexaphenyl cyclotriphosphazene (CP1), 1,4-bis(triphenylsilyl)benzene (UGH2), hexaphenylcyclotrisiloxane (DPSiO₃), octaphenylcyclotetra siloxane (DPSiO₄), etc. may be utilized as a host material.

The emission layer EML may include a compound represented by Formula M-a or Formula M-b. The compound represented by Formula M-a or Formula M-b may be utilized as a phosphorescence dopant material.

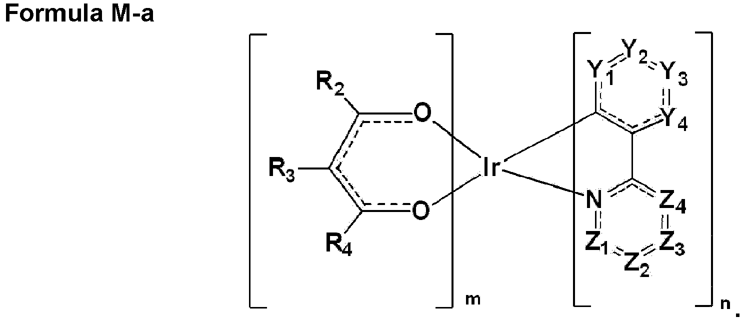

In Formula M-a, Y₁ to Y₄ and Z₁ to Z₄ are each independently CR₁ or N, R₁ to R₄ are each independently a hydrogen atom, a deuterium atom, a substituted or unsubstituted amine group, a substituted or unsubstituted thio group, a substituted or unsubstituted oxy group, a substituted or unsubstituted alkyl group having 1 to 20 (*e.g.* 1 to 10) carbon atoms, a substituted or unsubstituted alkenyl group having 2 to 20 (*e.g.* 2 to 10) carbon atoms, a substituted or unsubstituted aryl group having 6 to 30 (*e.g.* 6 to 20) ring-forming carbon atoms, or a substituted or unsubstituted heteroaryl group having 2 to 30 (*e.g.* 2 to 20) ring-forming carbon atoms, and/or are bonded to an adjacent group to form a ring. In Formula M-a, m is 0 or 1, and n is 2 or 3. In Formula M-a, when m is 0, n is 3, and when m is 1, n is 2.

The compound represented by Formula M-a may be utilized as a red phosphorescence dopant or a green phosphorescence dopant.

The compound represented by Formula M-a may be represented by any one among Compound M-a1 to Compound M-a25. However, Compounds M-a1 to M-a25 are examples, and the compound represented by Formula M-a is not limited to those represented by Compounds M-a1 to M-a25.

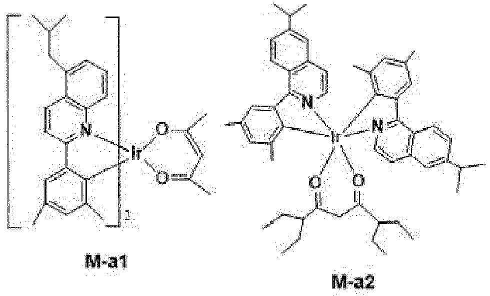

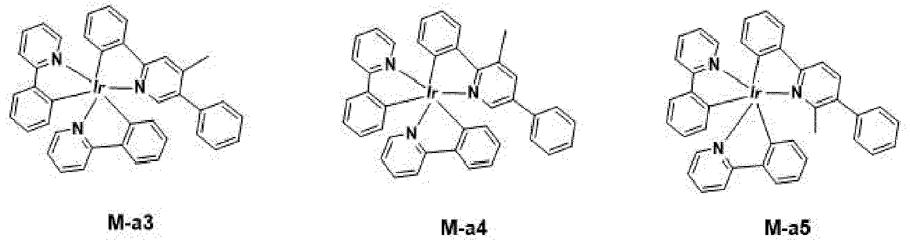

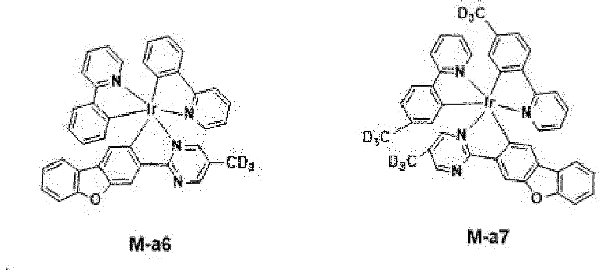

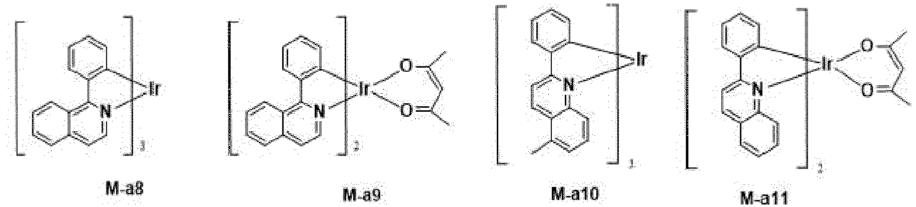

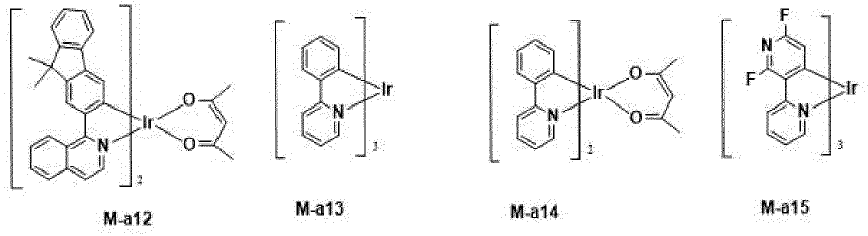

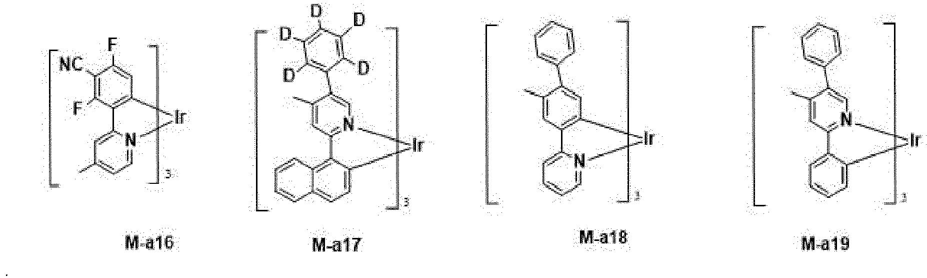

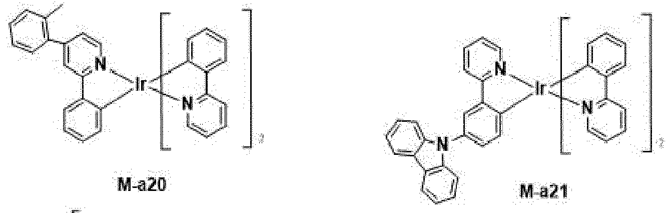

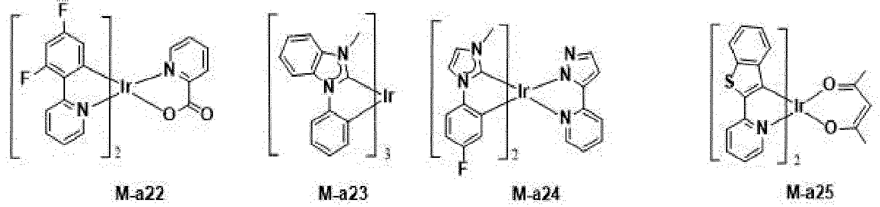

Compound M-a1 and Compound M-a2 may be utilized as a red dopant material, and Compound M-a3 and Compound M-a4 may be utilized as a green dopant material.

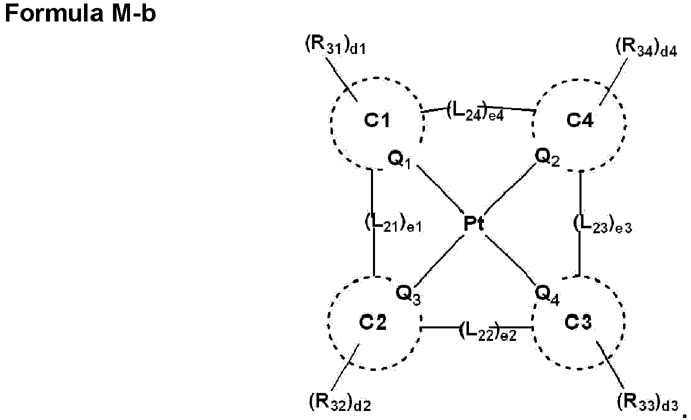

In Formula M-b, Q₁ to Q4 are each independently C or N, and C1 to C4 are each independently a substituted or unsubstituted hydrocarbon ring having 5 to 30 (e.g. 5 to 20) ring-forming carbon atoms, or a substituted or unsubstituted heterocycle having 2 to 30 (e.g. 2 to 20) ring-forming carbon atoms. L₂₁ to L₂₄ are each independently a direct linkage, **∗-O-∗**, **∗-S-∗**, 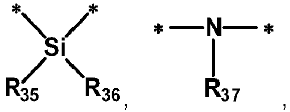 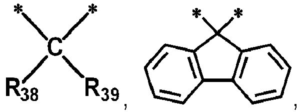 a substituted or unsubstituted divalent alkyl group having 1 to 20 (e.g. 1 to 10) carbon atoms, a substituted or unsubstituted arylene group having 6 to 30 (e.g. 6 to 20) ring-forming carbon atoms, or a substituted or unsubstituted heteroarylene group having 2 to 30 (e.g. 2 to 20) ring-forming carbon atoms, and e1 to e4 are each independently 0 or 1. R₃₁ to R₃₉ are each independently a hydrogen atom, a deuterium atom, a halogen atom, a cyano group, a substituted or unsubstituted amine group, a substituted or unsubstituted alkyl group having 1 to 20 (e.g. 1 to 10) carbon atoms, a substituted or unsubstituted aryl group having 6 to 30 (e.g. 6 to 20) ring-forming carbon atoms, or a substituted or unsubstituted heteroaryl group having 2 to 30 (*e.g*. 2 to 20) ring-forming carbon atoms, or are bonded to an adjacent group to form a ring, and d1 to d4 are each independently an integer of 0 to 4.

The compound represented by Formula M-b may be utilized as a blue phosphorescence dopant or a green phosphorescence dopant.

The compound represented by Formula M-b may be represented by any one among the compounds. However, the compounds are examples, and the compound represented by Formula M-b is not limited to those represented by the compounds.

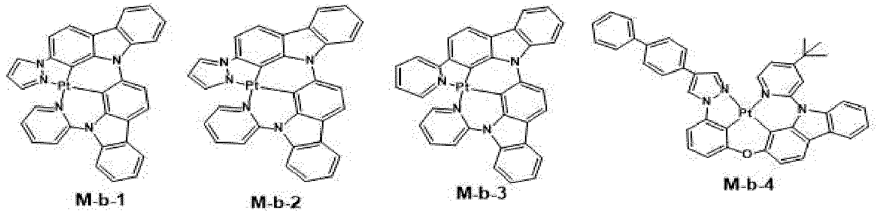

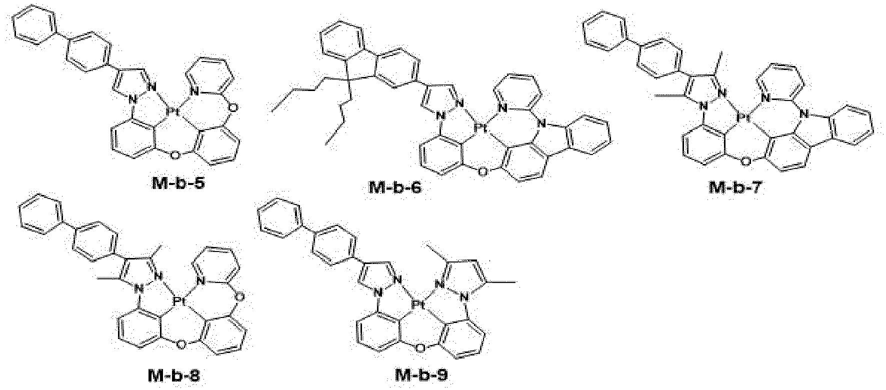

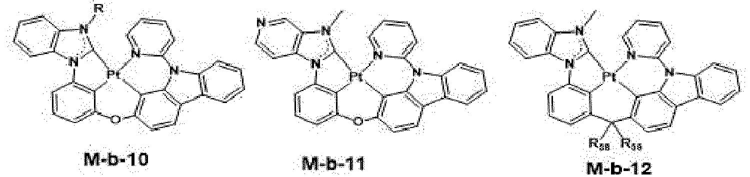

In the compounds, R, R₃₈, and R₃₉ are each independently a hydrogen atom, a deuterium atom, a halogen atom, a cyano group, a substituted or unsubstituted amine group, a substituted or unsubstituted alkyl group having 1 to 20 (e.g. 1 to 10) carbon atoms, a substituted or unsubstituted aryl group having 6 to 30 (e.g. 6 to 20) ring-forming carbon atoms, or a substituted or unsubstituted heteroaryl group having 2 to 30 (*e.g*. 2 to 20) ring-forming carbon atoms.

The emission layer EML may include a compound represented by any one among Formula F-a to Formula F-c. The compound represented by Formula F-a or Formula F-c may be utilized as a fluorescence dopant material.

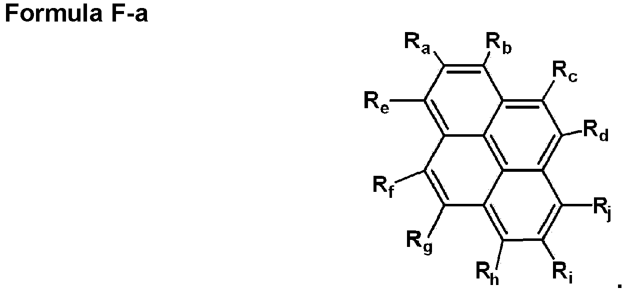

In Formula F-a, two selected from Rₐ to Rⱼ are each independently substituted with **∗**-NAr₁Ar₂. The others among Rₐ to Rⱼ that are not substituted with **∗**-NAr₁Ar₂, are each independently a hydrogen atom, a deuterium atom, a halogen atom, a cyano group, a substituted or unsubstituted amine group, a substituted or unsubstituted alkyl group having 1 to 20 (e.g. 1 to 10) carbon atoms, a substituted or unsubstituted aryl group having 6 to 30 (e.g. 6 to 20) ring-forming carbon atoms, or a substituted or unsubstituted heteroaryl group having 2 to 30 (e.g. 2 to 20) ring-forming carbon atoms. In **∗**-NAr₁Ar₂, Ar₁ and Ar₂ are each independently a substituted or unsubstituted aryl group having 6 to 30 (e.g. 6 to 20) ring-forming carbon atoms, or a substituted or unsubstituted heteroaryl group having 2 to 30 (e.g. 2 to 20) ring-forming carbon atoms. For example, at least one of Ar₁ or Ar₂ may be a heteroaryl group containing O or S as a ring-forming atom.

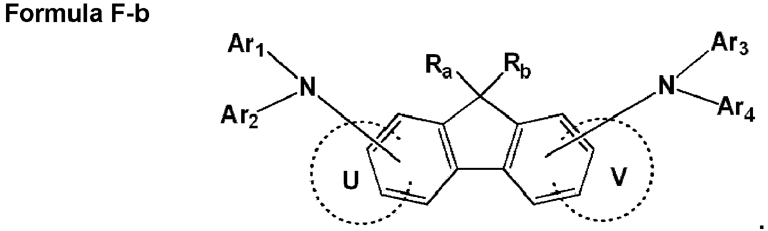

In Formula F-b, Rₐ and R_{b} are each independently a hydrogen atom, a deuterium atom, a substituted or unsubstituted alkyl group having 1 to 20 (e.g. 1 to 10) carbon atoms, a substituted or unsubstituted alkenyl group having 2 to 20 (e.g. 2 to 10) carbon atoms, a substituted or unsubstituted aryl group having 6 to 30 (e.g. 6 to 20) ring-forming carbon atoms, or a substituted or unsubstituted heteroaryl group having 2 to 30 (*e.g*. 2 to 20) ring-forming carbon atoms, or may be bonded to an adjacent group to form a ring. Ar₁ to Ar₄ may each independently be a substituted or unsubstituted aryl group having 6 to 30 ring-forming carbon atoms, or a substituted or unsubstituted heteroaryl group having 2 to 30 ring-forming carbon atoms.

In Formula F-b, U and V are each independently 0 or 1. In Formula F-b, U refers to the number of rings bonded to the position of U, and V refers to the number of rings bonded to the position of V. For example, it refers to that when U or V is 1, a ring described as U or V forms a condensed ring, and when U or V is 0, a ring described as U or V is not present. For example, when U is 0 and V is 1, or when U is 1 and V is 0, the condensed ring having a fluorene core of Formula F-b may be a fourring cyclic compound. In some embodiments, when both (e.g., simultaneously) U and V are 0, the condensed ring of Formula F-b may be a three-ring cyclic compound. Also, when both (e.g., simultaneously) U and V are 1, the condensed ring having a fluorene core of Formula F-b may be a five-ring cyclic compound.

In Formula F-b, when U or V is 1, U and V are each independently a substituted or unsubstituted hydrocarbon ring having 5 to 30 (*e.g*. 5 to 20) ring-forming carbon atoms, or a substituted or unsubstituted heterocycle having 2 to 30 (*e.g.* 2 to 20) ring-forming carbon atoms.

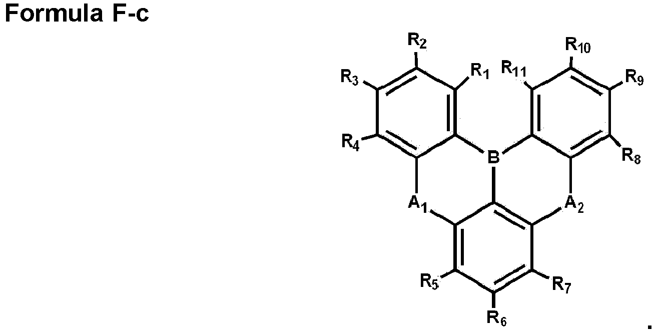

In Formula F-c, A₁ and A₂ are each independently O, S, Se, or NRₘ, and Rₘ is a hydrogen atom, a deuterium atom, a substituted or unsubstituted alkyl group having 1 to 20 (*e.g*. 1 to 10) carbon atoms, a substituted or unsubstituted aryl group having 6 to 30 (*e.g*. 6 to 20) ring-forming carbon atoms, or a substituted or unsubstituted heteroaryl group having 2 to 30 (*e.g*. 2 to 20) ring-forming carbon atoms. R₁ to R₁₁ are each independently a hydrogen atom, a deuterium atom, a halogen atom, a cyano group, a substituted or unsubstituted amine group, a substituted or unsubstituted boryl group, a substituted or unsubstituted oxy group, a substituted or unsubstituted thio group, a substituted or unsubstituted alkyl group having 1 to 20 (e.g. 1 to 10) carbon atoms, a substituted or unsubstituted aryl group having 6 to 30 (e.g. 6 to 20) ring-forming carbon atoms, or a substituted or unsubstituted heteroaryl group having 2 to 30 (*e.g*. 2 to 20) ring-forming carbon atoms, or are bonded to an adjacent group to form a ring.

In Formula F-c, A₁ and A₂ may each independently be bonded to substituents of an adjacent ring to form a condensed ring. For example, when A₁ and A₂ are each NRₘ, A₁ may be bonded to R₄ or R₅ to form a ring. In some embodiments, A₂ may be bonded to R₇ or R₈ to form a ring.

In an embodiment, the emission layer EML may include, as suitable dopant materials, styryl derivatives (e.g., 1,4-bis[2-(3-N-ethylcarbazolyl)vinyl]benzene (BCzVB), 4-(di-p-tolylamino)-4'-[(di-p-tolylamino)styryl]stilbene (DPAVB), and N-(4-((E)-2-(6-((E)-4-(diphenylamino)styryl)naphthalen-2-yl)vinyl)phenyl)-N-phenylbenzenamine (N-BDAVBi), perylene and the derivatives thereof (e.g., 2,5,8,11-tetra-t-butylperylene (TBP)), pyrene and the derivatives thereof (e.g., 1-dipyrene, 1,4-dipyrenylbenzene, 1,4-bis(N,N-diphenylamino)pyrene), etc.

The emission layer EML may include a suitable phosphorescence dopant material. For example, a metal complex including iridium (Ir), platinum (Pt), osmium (Os), gold (Au), titanium (Ti), zirconium (Zr), hafnium (Hf), europium (Eu), terbium (Tb), or thulium (Tm) may be utilized as a phosphorescence dopant. For example, iridium(III) bis(4,6-difluorophenylpyridinato-N,C2')picolinate (Flrpic), bis(2,4-difluorophenylpyridinato)-tetrakis(1-pyrazolyl)borate iridium(III) (Fir6), or platinum octaethyl porphyrin (PtOEP) may be utilized as a phosphorescence dopant. However, the embodiment of the present disclosure is not limited thereto.

The emission layer EML may include a quantum dot material. The core of the quantum dot may be selected from a Group II-VI compound, a Group III-VI compound, a Group I-III-IV compound, a Group III-V compound, a Group III-II-V compound, a Group IV-VI compound, a Group IV element, a Group IV compound, and a combination thereof.

A Group II-VI compound may be selected from the group consisting of a binary compound selected from the group consisting of CdSe, CdTe, CdS, ZnS, ZnSe, ZnTe, ZnO, HgS, HgSe, HgTe, MgSe, MgS, and a mixture thereof, a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and a mixture thereof, and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, HgZnSTe, and a mixture thereof.

The Group III-VI compound may include a binary compound (such as In₂S₃ and/or In₂Se₃), a ternary compound (such as InGaS₃ and/or InGaSe₃), or any combination thereof.

A Group I-III-VI compound may be selected from a ternary compound selected from the group consisting of AglnS, AgInS₂, CulnS, CuInS₂, AgGaS₂, CuGaS₂, CuGaO₂, AgGaO₂, AgAlO₂, and a mixture thereof, and a quaternary compound (such as AgInGaS₂ and/or CuInGaS₂).

The Group III-V compound may be selected from the group consisting of a binary compound selected from the group consisting of GaN, GaP, GaAs, GaSb, AIN, AIP, AlAs, AlSb, InN, InP, InAs, InSb, and a mixture thereof, a ternary compound selected from the group consisting of GaNP, GaNAs, GaNSb, GaPAs, GaPSb, AINP, AINAs, AINSb, AlPAs, AlPSb, InGaP, InAlP, InNP, InNAs, InNSb, InPAs, InPSb, and a mixture thereof, and a quaternary compound selected from the group consisting of GaAlNP, GaAlNAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, and a mixture thereof. In some embodiments, the Group III-V compound may further include a Group II metal. For example, InZnP, etc. may be selected as a Group III-II-V compound.

The Group IV-VI compound may be selected from the group consisting of a binary compound selected from the group consisting of SnS, SnSe, SnTe, PbS, PbSe, PbTe, and a mixture thereof, a ternary compound selected from the group consisting of SnSeS, SnSeTe, SnSTe, PbSeS, PbSeTe, PbSTe, SnPbS, SnPbSe, SnPbTe, and a mixture thereof, and a quaternary compound selected from the group consisting of SnPbSSe, SnPbSeTe, SnPbSTe, and a mixture thereof. The Group IV element may be selected from the group consisting of Si, Ge, and a mixture thereof. The Group IV compound may be a binary compound selected from the group consisting of SiC, SiGe, and a mixture thereof.

In this case, a binary compound, a ternary compound, or a quaternary compound may be present in particles in a substantially uniform concentration distribution, or may be present in substantially the same particle in a partially different concentration distribution. In some embodiments, the quantum dot may have a core/shell structure in which one quantum dot surrounds another quantum dot. In a core/shell structure, the interface of the shell may have a concentration gradient in which the concentration of an element present in the shell becomes lower towards the core.

In some embodiments, a quantum dot may have the above-described coreshell structure including a core containing nanocrystals and a shell surrounding the core. The shell of the quantum dot may serve as a protection layer to prevent or reduce the chemical deformation of the core so as to maintain semiconductor properties, and/or a charging layer to impart electrophoresis properties to the quantum dot. The shell may be a single layer or a multilayer. An example of the shell of the quantum dot may include a metal or non-metal oxide, a semiconductor compound, or a combination thereof.

For example, the metal or non-metal oxide may be a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, and NiO, or a ternary compound such as MgAl₂O₄, CoFe₂O₄, NiFe₂O₄, and CoMn₂O₄, but the embodiment of the present disclosure is not limited thereto.

Also, the semiconductor compound may be, for example, CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AIP, AlSb, etc., but the embodiment of the present disclosure is not limited thereto.

The quantum dot may have a full width of half maximum (FWHM) of a light emission wavelength spectrum of about 45 nm or less, about 40 nm or less, and more about 30 nm or less, and color purity or color reproducibility may be improved in the above range. In some embodiments, light emitted through such a quantum dot is emitted in all directions, and thus a wide viewing angle may be improved.

In some embodiments, although the form of a quantum dot is not particularly limited as long as it is a form commonly utilized in the art, more specifically, a quantum dot in the form of spherical, pyramidal, multi-arm, or cubic nanoparticles, nanotubes, nanowires, nanofibers, nanoparticles, etc. may be utilized.

The quantum dot may control the color of emitted light according to the particle size thereof. Accordingly, the quantum dot may have one or more suitable light emission colors such as blue, red, and green.

In each luminescence device ED of embodiments illustrated in FIGS. 3 to 6, the electron transport region ETR is provided on the emission layer EML. The electron transport region ETR may include at least one of the hole blocking layer HBL, the electron transport layer ETL, or the electron injection layer EIL, but the embodiment of the present disclosure is not limited thereto.

The electron transport region ETR may have a single layer formed of a single material, a single layer formed of a plurality of different materials, or a multilayer structure including a plurality of layers formed of a plurality of different materials.

For example, the electron transport region ETR may have a single layer structure of the electron injection layer EIL or the electron transport layer ETL, and may have a single layer structure formed of an electron injection material and an electron transport material. In some embodiments, the electron transport region ETR may have a single layer structure formed of a plurality of different materials, or may have a structure in which an electron transport layer ETL/electron injection layer EIL, a hole blocking layer HBL/electron transport layer ETL/electron injection layer EIL are stacked in order from the emission layer EML, but the embodiment of the present disclosure is not limited thereto. The electron transport region ETR may have a thickness, for example, from about 1,000 Å to about 1,500 Å.

The electron transport region ETR may be formed by utilizing one or more suitable methods such as a vacuum deposition method, a spin coating method, a cast method, a Langmuir-Blodgett (LB) method, an inkjet printing method, a laser printing method, a laser induced thermal imaging (LITI) method, etc.

The electron transport region ETR may include a compound represented by Formula ET-1:

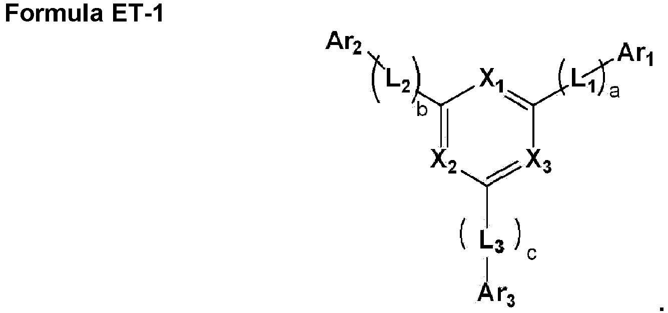

In Formula ET-1, at least one among X₁ to X₃ is N, and the rest are CRₐ. Rₐ is a hydrogen atom, a deuterium atom, a substituted or unsubstituted alkyl group having 1 to 20 (*e.g*. 1 to 10) carbon atoms, a substituted or unsubstituted aryl group having 6 to 30 (*e.g*. 6 to 20) ring-forming carbon atoms, or a substituted or unsubstituted heteroaryl group having 2 to 30 (*e.g.* 2 to 20) ring-forming carbon atoms. Ar₁ to Ar₃ are each independently a hydrogen atom, a deuterium atom, a substituted or unsubstituted alkyl group having 1 to 20 (*e.g.* 1 to 10) carbon atoms, a substituted or unsubstituted aryl group having 6 to 30 (*e.g*. 6 to 20) ring-forming carbon atoms, or a substituted or unsubstituted heteroaryl group having 2 to 30 (*e.g.* 2 to 20) ring-forming carbon atoms.

In Formula ET-1, a to c are each independently an integer of 0 to 10. In Formula ET-1, L₁ to L₃ are each independently a direct linkage, a substituted or unsubstituted arylene group having 6 to 30 (*e.g.* 6 to 20) ring-forming carbon atoms, or a substituted or unsubstituted heteroarylene group having 2 to 30 (*e.g*. 2 to 20) ring-forming carbon atoms. In some embodiments, when a to c are an integer of 2 or more, L₁ to L₃ may each independently be a substituted or unsubstituted arylene group having 6 to 30 ring-forming carbon atoms, or a substituted or unsubstituted heteroarylene group having 2 to 30 ring-forming carbon atoms.

When the electron transport region ETR includes the electron transport layer ETL, the electron transport region ETR may include an anthracene-based compound. However, the embodiment of the present disclosure is not limited thereto, and the electron transport region may include, for example, tris(8-hydroxyquinolinato)aluminium (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazol-1-yl)phenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-tert-butylphenyl)-1,3,4-oxadiazole (tBu-PBD), bis(2-methyl-8-quinolinolato-N1,O8)-(1,1'-biphenyl-4-olato)aluminium (BAlq), beryllium bis(benzoquinolin-10-olate (Bebq₂), 9,10-di(naphthalene-2-yl)anthracene (ADN), 1,3-bis[3,5-di(pyridin-3-yl)phenyl]benzene (BmPyPhB), or a mixture thereof.

The electron transport region ETR may include at least one among Compound ET1 to Compound ET36:

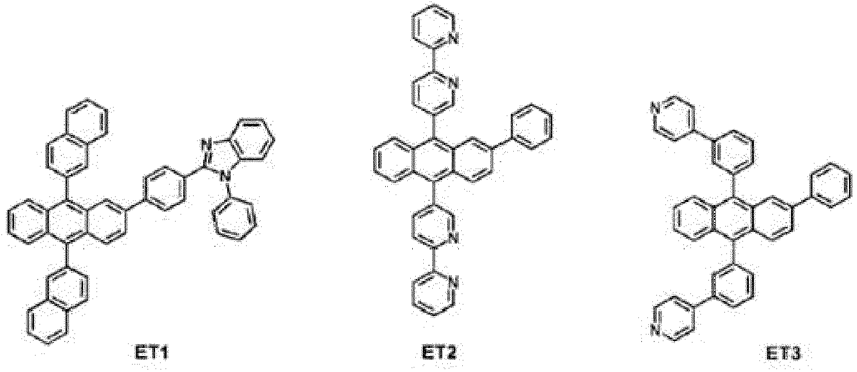

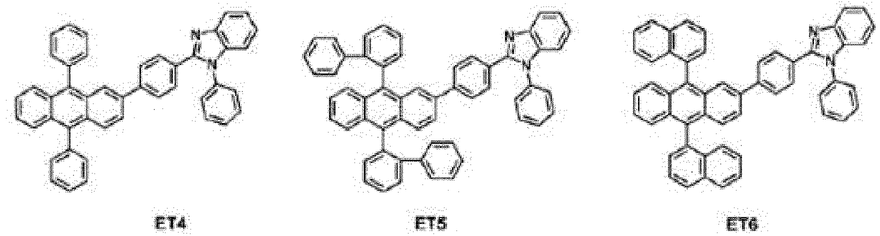

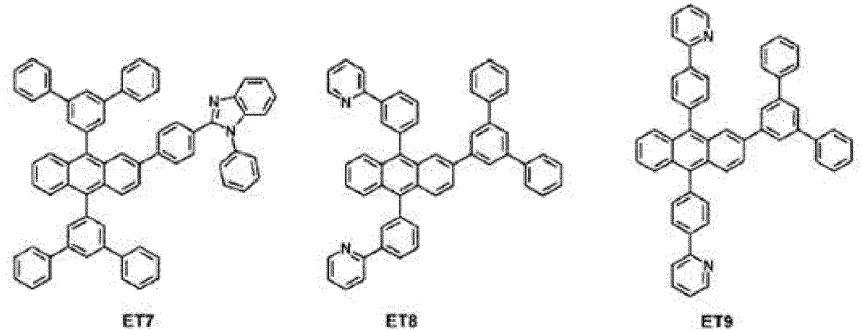

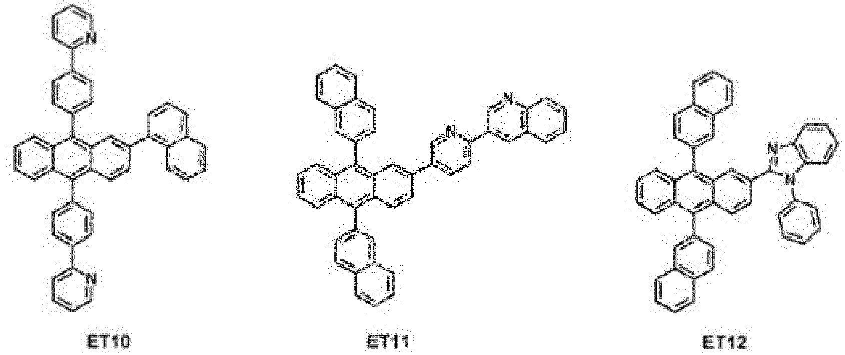

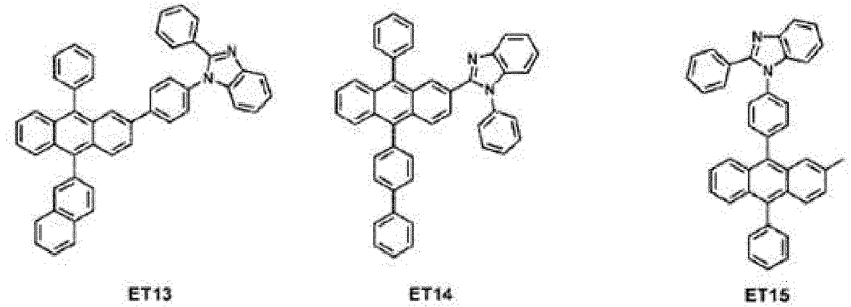

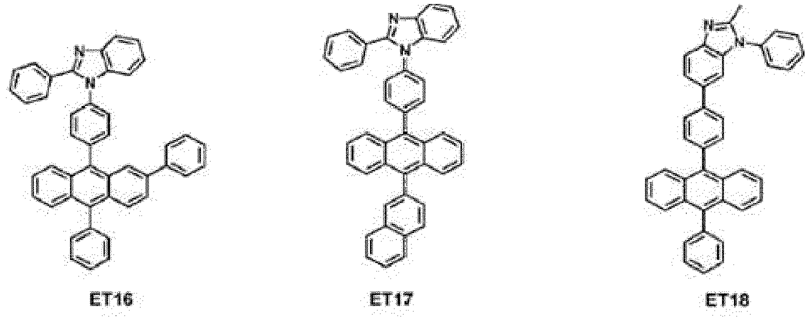

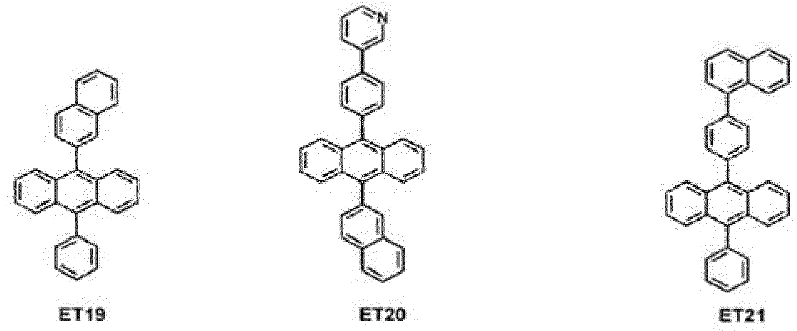

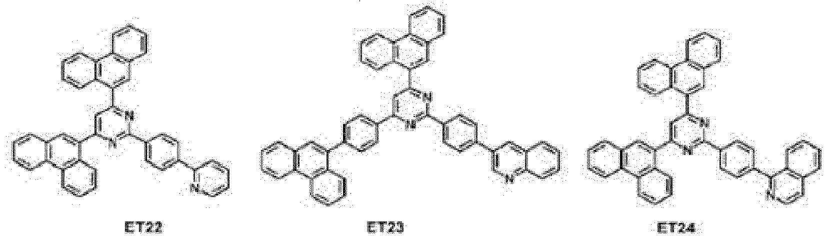

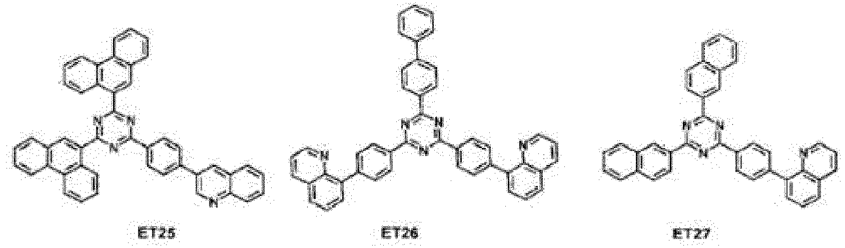

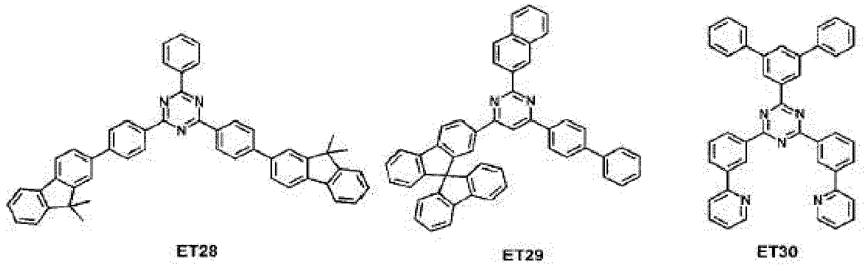

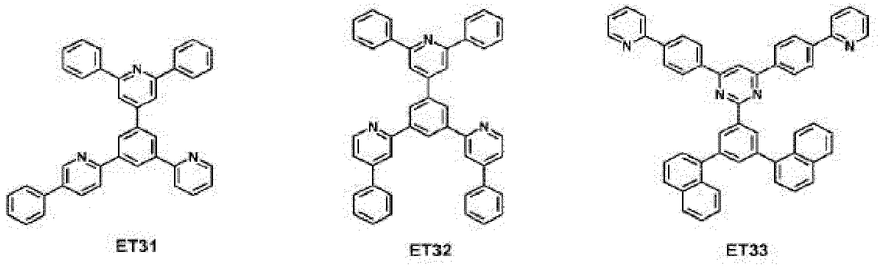

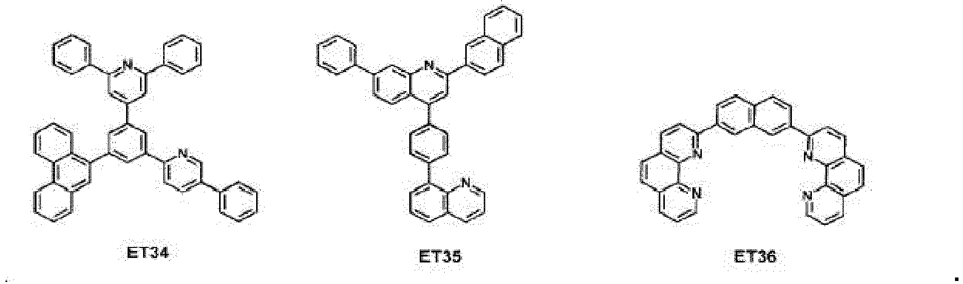

The electron transport regions ETR may include a metal halide (such as LiF, NaCl, CsF, RbCI, RbI, Cul, and/or KI), a lanthanide metal (such as Yb), and a co-deposited material of the metal halide and the lanthanide metal. For example, the electron transport region ETR may include KI:Yb, RbI:Yb, etc. as a co-deposited material. In some embodiments, the electron transport region ETR may be formed utilizing a metal oxide (such as Li₂O and/or BaO), or 8-hydroxyl-lithium quinolate (LiQ), etc., but embodiments of the present disclosure are not limited thereto. The electron transport region ETR may also be formed of a mixture of an electron transport material and an insulating organometallic salt. The organometallic salt may be a material having an energy band gap of about 4 eV or more. For example, the organometallic salt may include, for example, metal acetates, metal benzoates, metal acetoacetates, metal acetylacetonates, and/or metal stearates, but embodiments of the present disclosure are not limited thereto.

The electron transport region ETR may include the above-described compounds of the hole transport region in at least one of the electron injection layer EIL, the electron transport layer ETL, or the hole blocking layer HBL.

When the electron transport region ETR includes the electron transport layer ETL, the electron transport layer ETL may have a thickness of about 100 Å to about 1,000 Å, for example, about 150 Å to about 500 Å. When the thickness of the electron transport layer ETL satisfies the aforementioned range, satisfactory electron transport characteristics may be obtained without a substantial increase in driving voltage. When the electron transport region ETR includes the electron injection layer EIL, the electron injection layer EIL may have a thickness of about 1 Å to about 100 Å, for example, about 3 Å to about 90 Å. When the thickness of the electron injection layer EIL satisfies the above-described range, satisfactory electron injection characteristics may be obtained without a substantial increase in driving voltage.

The second electrode EL2 is provided on the electron transport region ETR. The second electrode EL2 may be a common electrode. The second electrode EL2 may be a cathode or an anode, but embodiments of the present disclosure are not limited thereto. For example, when the first electrode EL1 is an anode, the second electrode EL2 may be a cathode, and when the first electrode EL1 is a cathode, the second electrode EL2 may be an anode.

The second electrode EL2 may be a transmissive electrode, a transflective electrode, or a reflective electrode. When the second electrode EL2 is a transmissive electrode, the second electrode EL2 may be formed of a transparent metal oxide, for example, indium tin oxide (ITO), indium zinc oxide (IZO), zinc oxide (ZnO), indium tin zinc oxide (ITZO), etc.

When the second electrode EL2 is a transflective electrode or a reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, or a compound or mixture thereof (e.g., AgMg, AgYb, or MgAg). In some embodiments, the second electrode EL2 may have a multilayer structure including a reflective film or a transflective film formed of the above-described materials, and a transparent conductive film formed of ITO, IZO, ZnO, ITZO, etc. For example, the second electrode EL2 may include the above-described metal materials, combinations of at least two metal materials of the above-described metal materials, oxides of the above-described metal materials, and/or the like.

In some embodiments, the second electrode EL2 may be connected with an auxiliary electrode. When the second electrode EL2 is connected with the auxiliary electrode, the resistance of the second electrode EL2 may decrease.

In some embodiments, a capping layer CPL may further be disposed on the second electrode EL2 of the luminescence device ED of an embodiment. The capping layer CPL may include a multilayer or a single layer.

In an embodiment, the capping layer CPL may be an organic layer or an inorganic layer. For example, when the capping layer CPL includes an inorganic material, the inorganic material may include an alkaline metal compound (such as LiF), an alkaline earth metal compound (such as MgF₂, SiON, SiNₓ, SiO_{y}), etc.

For example, when the capping layer CPL includes an organic material, the organic material may include α-NPD, NPB, TPD, m-MTDATA, Alq₃, CuPc, N4,N4,N4',N4'-tetra(biphenyl-4-yl)biphenyl-4,4'-diamine (TPD15), 4,4',4"-tris(carbazol-9-yl)triphenylamine (TCTA), etc., an epoxy resin, or an acrylate (such as methacrylate). However, embodiments of the present disclosure are not limited thereto, and the organic material may also include one or more of Compounds P1 to P5:

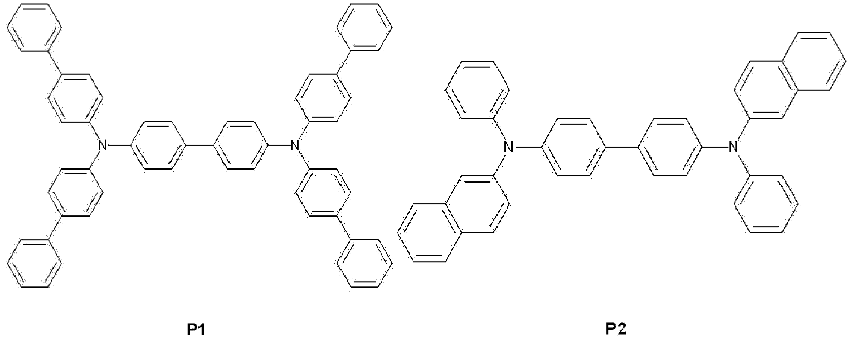

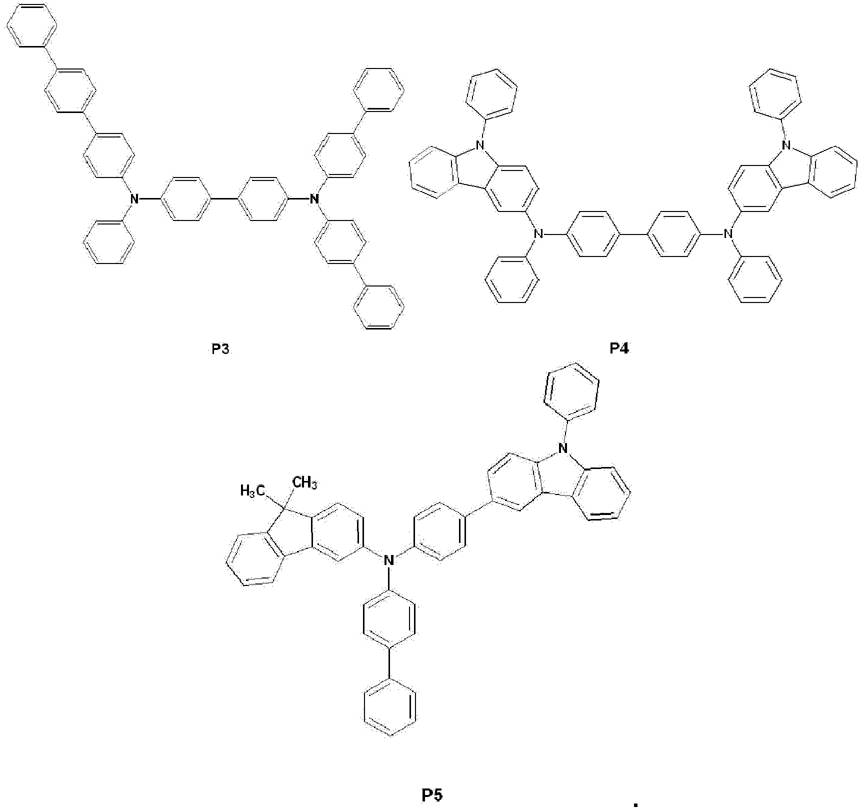

In some embodiments, the refractive index of the capping layer CPL may be about 1.6 or more. For example, the refractive index of the capping layer CPL may be about 1.6 or more with respect to light in a wavelength range of about 550 nm to about 660 nm.

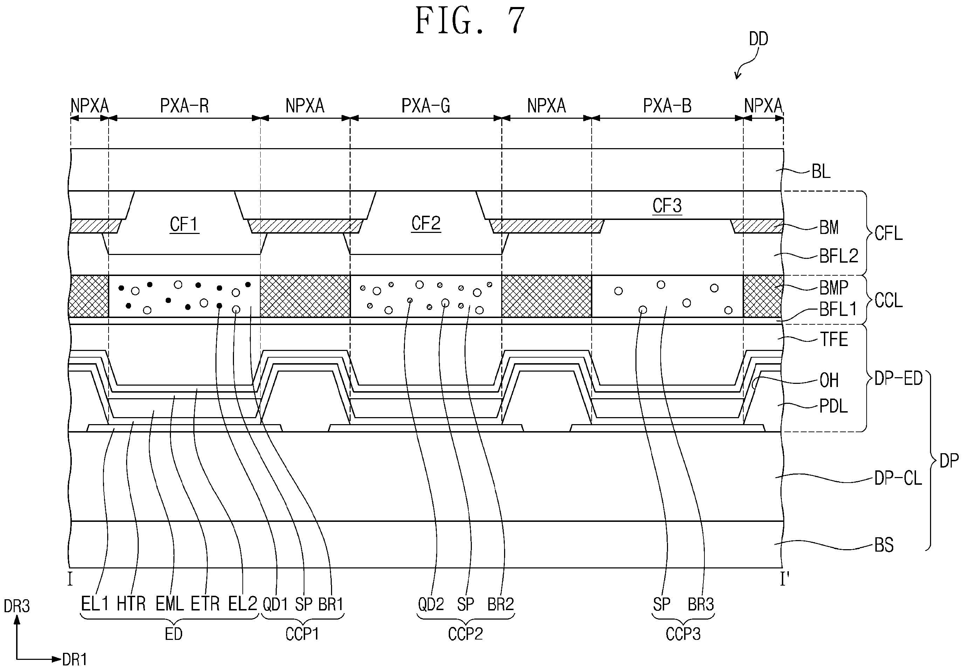

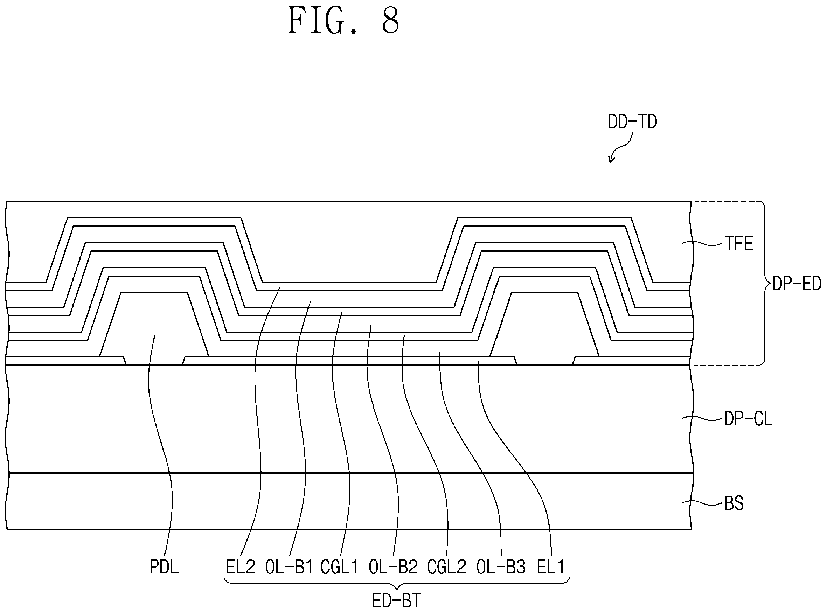

FIGS. 7 and 8 are each a cross-sectional view of a display apparatus according to an embodiment. Hereinafter, in describing the display apparatus of an embodiment with reference to FIGS. 7 and 8, the duplicated features that have been described in FIGS. 1 to 6 are not described again, but their differences will be mainly described.

Referring to FIG. 7, the display apparatus DD according to an embodiment may include a display panel DP including a display device layer DP-ED, a light control layer CCL disposed on the display panel DP, and a color filter layer CFL.

In an embodiment illustrated in FIG. 7, the display panel DP may include a base layer BS, a circuit layer DP-CL provided on the base layer BS, and the display device layer DP-ED, and the display device layer DP-ED may include an organic electroluminescence device ED.

The organic electroluminescence device ED may include a first electrode EL1, a hole transport region HTR disposed on the first electrode EL1, an emission layer EML disposed on the hole transport region HTR, an electron transport region ETR disposed on the emission layer EML, and a second electrode EL2 disposed on the electron transport region ETR. In some embodiments, the structures of the organic electroluminescence devices of FIGS. 4 to 6 as described above may be equally applied to the structure of the organic electroluminescence device ED shown in FIG. 7.

Referring to FIG. 7, the emission layer EML may be disposed in an opening OH defined in a pixel defining film PDL. For example, the emission layer EML, which is divided by the pixel defining film PDL and provided to correspond to each of light emitting regions PXA-R, PXA-G, and PXA-B may be to emit light in substantially the same wavelength range. In the display apparatus DD of an embodiment, the emission layer EML may be to emit blue light. In some embodiments, unlike shown, in an embodiment, the emission layer EML may be provided as a common layer in the entire light emitting regions PXA-R, PXA-G, and PXA-B.

The light control layer CCL may be disposed on the display panel DP. The light control layer CCL may include a light conversion body. The light conversion body may be a quantum dot, a phosphor, and/or the like. The light conversion body may be to emit provided light by converting the wavelength thereof. For example, the light control layer CCL may be a layer containing the quantum dot or a layer containing the phosphor.

The light control layer CCL may include a plurality of light control units CCP1, CCP2 and CCP3. The light control units CCP1, CCP2, and CCP3 may be spaced apart from one another.

Referring to FIG. 7, divided patterns BMP may be disposed between the light control units CCP1, CCP2 and CCP3, which are spaced apart from each other, but embodiments of the present disclosure are not limited thereto. FIG. 7 illustrates that the divided patterns BMP do not overlap the light control units CCP1, CCP2 and CCP3, but at least a portion of the edges of the light control units CCP1, CCP2 and CCP3 may overlap the divided patterns BMP.

The light control layer CCL may include a first light control unit CCP1 containing (including) a first quantum dot QD1, which converts first color light provided from the organic electroluminescence device ED into second color light, a second light control unit CCP2 containing a second quantum dot QD2, which converts the first color light into third color light, and a third light control unit CCP3, which transmits the first color light.

In an embodiment, the first light control unit CCP1 may provide red light that is the second color light, and the second light control unit CCP2 may provide green light that is the third color light. The third light control unit CCP3 may provide blue light by transmitting the blue light that is the first color light provided in the organic electroluminescence device ED. For example, the first quantum dot QD1 may be a red quantum dot, and the second quantum dot QD2 may be a green quantum dot. The same as described above may be applied with respect to the quantum dots QD1 and QD2.

In some embodiments, the light control layer CCL may further include a scatterer SP. The first light control unit CCP1 may include the first quantum dot QD1 and the scatterer SP, the second light control unit CCP2 may include the second quantum dot QD2 and the scatterer SP, and the third light control unit CCP3 may not include any quantum dot but include the scatterer SP.

The scatterer SP may be or include inorganic particles. For example, the scatterer SP may include at least one of TiO₂, ZnO, Al₂O₃, SiO₂, or hollow silica. The scatterer SP may include any one of TiO₂, ZnO, Al₂O₃, SiO₂, or hollow silica, or may be a mixture of at least two materials selected from TiO₂, ZnO, Al₂O₃, SiO₂, and hollow silica.

The light control layer CCL may include a barrier layer BFL1. The barrier layer BFL1 may serve to prevent or reduce the penetration of moisture and/or oxygen (hereinafter, referred to as 'moisture/oxygen'). The barrier layer BFL1 may be disposed on the light control units CCP1, CCP2, and CCP3 to block or reduce the light control units CCP1, CCP2 and CCP3 from being exposed to moisture/oxygen. In some embodiments, the barrier layer BFL1 may cover the light control units CCP1, CCP2, and CCP3. In some embodiments, a barrier layer BFL2 may be provided between the light control units CCP1, CCP2, and CCP3 and the color filter layer CFL.

The barrier layers BFL1 and BFL2 may include at least one inorganic layer. For example, the barrier layers BFL1 and BFL2 may include an inorganic material. For example, the barrier layers BFL1 and BFL2 may include a silicon nitride, an aluminium nitride, a zirconium nitride, a titanium nitride, a hafnium nitride, a tantalum nitride, a silicon oxide, an aluminium oxide, a titanium oxide, a tin oxide, a cerium oxide, a silicon oxynitride, a metal thin film that secures a transmittance, etc. In some embodiments, the barrier layers BFL1 and BFL2 may further include an organic film.

The barrier layers BFL1 and BFL2 may be formed of a single layer or a plurality of layers.

In the display apparatus DD of an embodiment, the color filter layer CFL may be disposed on the light control layer CCL. For example, the color filter layer CFL may be directly disposed on the light control layer CCL. In this case, the barrier layer BFL2 may be omitted.

The color filter layer CFL may include a light shielding unit BM and filters CF-3, CF-2, and CF-1. The color filter layer CFL may include a first filter CF1 configured to transmit the second color light, a second filter CF2 configured to transmit the third color light, and a third filter CF3 configured to transmit the first color light. For example, the first filter CF1 may be a red filter, the second filter CF2 may be a green filter, and the third filter CF3 may be a blue filter. The filters CF1, CF2, and CF3 may each include a polymeric photosensitive resin and a pigment or dye. The first filter CF1 may include a red pigment or dye, the second filter CF2 may include a green pigment or dye, and the third filter CF3 may include a blue pigment or dye. However, embodiments of the present disclosure are not limited thereto, and the third filter CF3 may not include a pigment or dye. The third filter CF3 may include a polymeric photosensitive resin and may not include a pigment or dye. The third filter CF3 may be transparent. The third filter CF3 may be formed of a transparent photosensitive resin.

Furthermore, in an embodiment, the first filter CF1 and the second filter CF2 may be a yellow filter. The first filter CF1 and the second filter CF2 may not be separated but be provided as one filter.

The light shielding unit BM may be a black matrix. The light shielding unit BM may include an organic light shielding material or an inorganic light shielding material containing a black pigment or dye. The light shielding unit BM may prevent or reduce light leakage, and may separate boundaries between the adjacent filters CF1, CF2, and CF3. In an embodiment, the light shielding unit BM may be formed of a blue filter.

The first to third filters CF1, CF2, and CF3 may be disposed corresponding to the red light emitting region PXA-R, the green light emitting region PXA-G, and the blue light emitting region PXA-B, respectively.

A base substrate BL may be disposed on the color filter layer CFL. The base substrate BL may provide a base surface in which the color filter layer CFL, the light control layer CCL, and/or the like are disposed. The base substrate BL may be a glass substrate, a metal substrate, a plastic substrate, etc. However, embodiments of the present disclosure are not limited thereto, and the base substrate BL may be an inorganic layer, an organic layer, or a composite material layer. In an embodiment, the base substrate BL may be omitted.

FIG. 8 is a cross-sectional view illustrating a part of a display apparatus according to an embodiment. FIG. 8 illustrates a cross-sectional view of a part corresponding to the display panel DP of FIG. 7. In the display apparatus DD-TD of an embodiment, the organic electroluminescence device ED-BT may include a plurality of light emitting structures OL-B1, OL-B2, and OL-B3. The organic electroluminescence device ED-BT may include a first electrode EL1 and a second electrode EL2 facing each other, and the plurality of light emitting structures OL-B1, OL-B2, and OL-B3 sequentially stacked in the thickness direction between the first electrode EL1 and the second electrode EL2. The light emitting structures OL-B1, OL-B2, and OL-B3 may each include an emission layer EML (FIG. 7), and a hole transport region HTR and an electron transport region ETR disposed with the emission layer EML (FIG. 7) therebetween.

For example, the organic electroluminescence device ED-BT included in the display apparatus DD-TD of an embodiment may be an organic electroluminescence device having a tandem structure and including a plurality of emission layers.

In an embodiment illustrated in FIG. 8, all light beams respectively emitted from the light emitting structures OL-B1, OL-B2, and OL-B3 may be blue light. However, embodiments of the present disclosure are not limited thereto, and the light beams respectively emitted from the light emitting structures OL-B1, OL-B2, and OL-B3 may have wavelength ranges different from each other. For example, the organic electroluminescence device ED-BT including the plurality of light emitting structures OL-B1, OL-B2, and OL-B3 which emit light beams having wavelength ranges different from each other may be to emit white light.

Charge generation layers CGL1 and CGL2 may be disposed between the neighboring light emitting structures OL-B1, OL-B2, and OL-B3. The charge generation layers CGL1 and CGL2 may include a p-type charge generation layer or an n-type charge generation layer.

Hereinafter, the present disclosure will be described in more detail through selected Examples and Comparative Examples. The Examples are only illustrations for assisting the understanding of the present disclosure, and the scope of the present disclosure is not limited thereto.

### Synthetic Examples

An amine compound according to an embodiment of the present disclosure may be synthesized, for example, as follows. However, synthetic methods of the amine compound according to an embodiment of the present disclosure are not limited thereto.

### 1. Synthesis of Compound A4

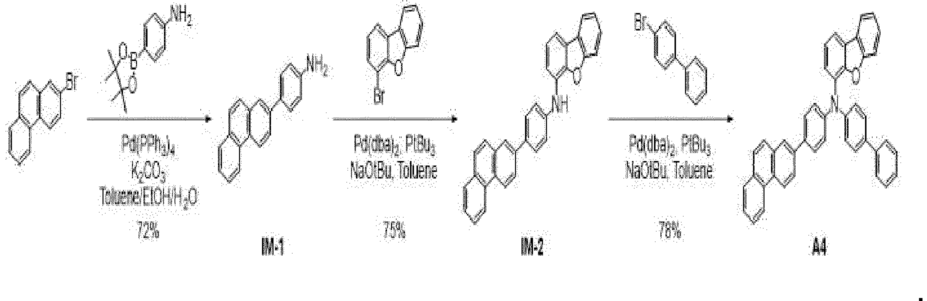

### Synthesis of Intermediate IM-1

In an Ar atmosphere, in a 1000 mL three-neck flask, 2-bromophenanthrene (20.00 g, 77.78 mmol), 4-(4,4,5,5-tetramethyl-1,3,2-dioxaborolan-2-yl)aniline (18.75 g, 1.1 equiv, 85.6 mmol), K₂CO₃ (32.25 g, 3.0 equiv, 233.3 mmol), Pd(PPh₃)₄ (4.49 g, 0.05 eq, 3.9 mmol), and a mixed solution of toluene/EtOH/H₂O (4/2/1) (544 mL) were sequentially added, and heated and stirred at about 80 °C. After air cooling to room temperature, the reaction solution was extracted with toluene. A water layer was removed, the organic layers were washed with saturated saline, and then dried over MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-1 (15.08 g, yield 72%).

By measuring FAB-MS, a mass number of m/z = 269 was observed by molecular ion peak, thereby identifying Intermediate IM-1.

### Synthesis of Intermediate IM-2

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 4-bromodibenzofuran (10.09 g, 1.1 equiv, 40.9 mmol), and P(tBu)₃ (alternatively referred to as PtBu₃, where "tBu" indicates a tertiary butyl group) (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-2 (12.13 g, yield 75%).

By measuring FAB-MS, a mass number of m/z = 435 was observed by molecular ion peak, thereby identifying Compound IM-2.

### Synthesis of Compound A4

In an Ar atmosphere, in a 300 mL three-neck flask, Intermediate IM-2 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 4-bromobiphenyl (5.89 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound A4 (10.53 g, yield 78%).

By measuring FAB-MS, a mass number of m/z = 587 was observed by molecular ion peak, thereby identifying Compound A4.

### 2. Synthesis of Compound A24

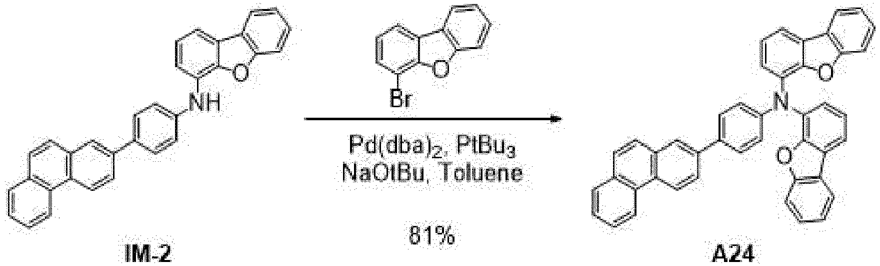

### Synthesis of Compound A24

In an Ar atmosphere, in a 300 mL three-neck flask, Intermediate IM-2 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 4-bromodibenzofuran (6.24 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound A24 (11.19 g, yield 81%).

By measuring FAB-MS, a mass number of m/z = 601 was observed by molecular ion peak, thereby identifying Compound A24.

### 3. Synthesis of Compound D8

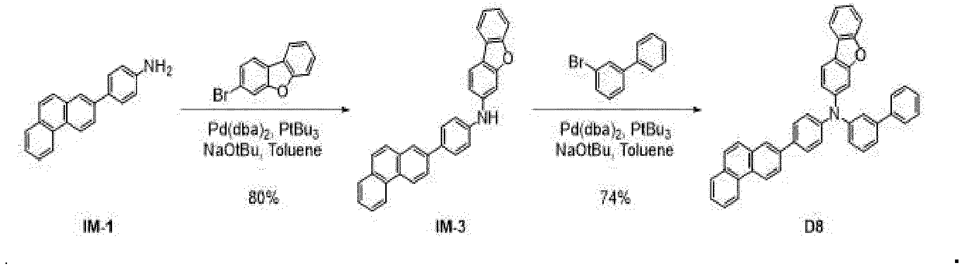

### Synthesis of Intermediate IM-3

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 3-bromodibenzofuran (10.09 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-3 (12.94 g, yield 80%).

By measuring FAB-MS, a mass number of m/z = 435 was observed by molecular ion peak, thereby identifying Compound IM-3.

### Synthesis of Compound D8

In an Ar atmosphere, in a 300 mL three-neck flask, Intermediate IM-3 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 3-bromo-1,1'-biphenyl (5.89 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound D8 (9.99 g, yield 74%).

By measuring FAB-MS, a mass number of m/z = 587 was observed by molecular ion peak, thereby identifying Compound D8.

### 4. Synthesis of Compound E6

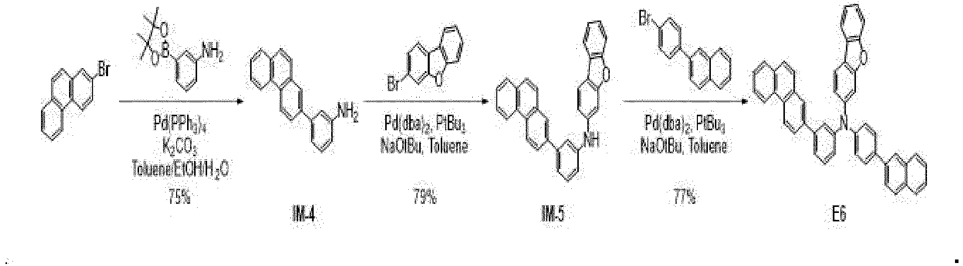

### Synthesis of Intermediate IM-4

In an Ar atmosphere, in a 1000 mL three-neck flask, 2-bromophenanthrene (20.00 g, 77.78 mmol), 3-(4,4,5,5-tetramethyl-1,3,2-dioxaborolan-2-yl)aniline (18.75 g, 1.1 equiv, 85.6 mmol), K₂CO₃ (32.25 g, 3.0 equiv, 233.3 mmol), Pd(PPh₃)₄ (4.49 g, 0.05 eq, 3.9 mmol), and a mixed solution of toluene/EtOH/H₂O (4/2/1) (544 mL) were sequentially added, and heated and stirred at about 80 °C. After air cooling to room temperature, the reaction solution was extracted with toluene. A water layer was removed, the organic layers were washed with saturated saline, and then dried over MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-4 (15.71 g, yield 75%).

By measuring FAB-MS, a mass number of m/z = 269 was observed by molecular ion peak, thereby identifying Intermediate IM-4.

### Synthesis of Intermediate IM-5

In an Ar atmosphere, in a 300 mL three-neck flask, Intermediate IM-4 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 3-bromodibenzofuran (10.09 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-5 (12.77 g, yield 79%).

By measuring FAB-MS, a mass number of m/z = 435 was observed by molecular ion peak, thereby identifying Compound IM-5.

### Synthesis of Compound E6

In an Ar atmosphere, in a 300 mL three-neck flask, Intermediate IM-5 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 2-(4-bromophenyl)naphthalene (7.15 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound E6 (11.28 g, yield 77%).

By measuring FAB-MS, a mass number of m/z = 637 was observed by molecular ion peak, thereby identifying Compound E6.

### 5. Synthesis of Compound G26

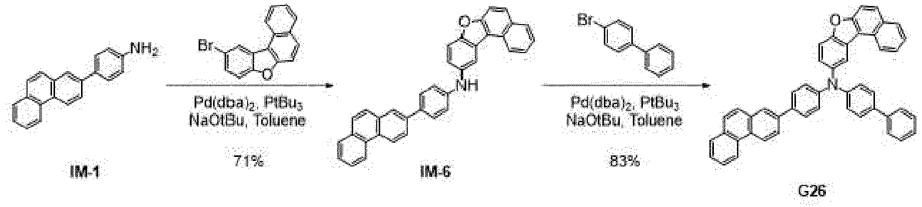

### Synthesis of Intermediate IM-6

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 10-bromonaphtho[2,1-b]benzofuran (12.14 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-6 (12.80 g, yield 71%).

By measuring FAB-MS, a mass number of m/z = 485 was observed by molecular ion peak, thereby identifying Compound IM-6.

### Synthesis of Compound G26

In an Ar atmosphere, in a 300 mL three-neck flask, Intermediate IM-6 (10.00 g, 20.6 mmol), Pd(dba)₂ (0.36 g, 0.03 equiv, 0.6 mmol), NaOtBu (3.96 g, 2.0 equiv, 41.2 mmol), toluene (103 mL), 4-bromobiphenyl (5.28 g, 1.1 equiv, 22.7 mmol), and P(tBu)₃ (0.42 g, 0.1 equiv, 2.1 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound G26 (10.90 g, yield 83%).

By measuring FAB-MS, a mass number of m/z = 637 was observed by molecular ion peak, thereby identifying Compound G26.

### 6. Synthesis of Compound I3

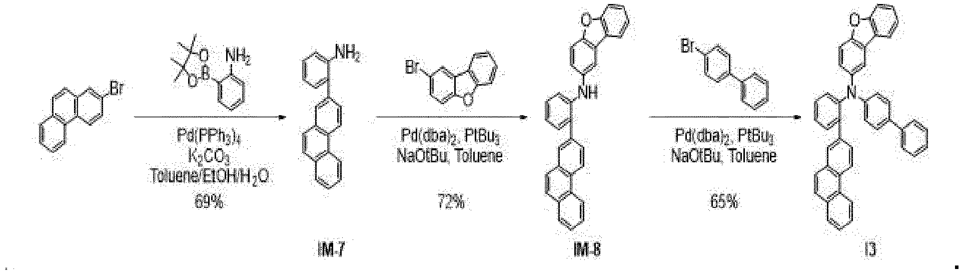

### Synthesis of Intermediate IM-7

In an Ar atmosphere, in a 1000 mL three-neck flask, 2-bromophenanthrene (20.00 g, 77.78 mmol), 2-(4,4,5,5-tetramethyl-1,3,2-dioxaborolan-2-yl)aniline (18.75 g, 1.1 equiv, 85.6 mmol), K₂CO₃ (32.25 g, 3.0 equiv, 233.3 mmol), Pd(PPh₃)₄ (4.49 g, 0.05 eq, 3.9 mmol), and a mixed solution of toluene/EtOH/H₂O (4/2/1) (544 mL) were sequentially added, and heated and stirred at about 80 °C. After air cooling to room temperature, the reaction solution was extracted with toluene. A water layer was removed, the organic layers were washed with saturated saline, and then dried over MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-7 (14.46 g, yield 69%).

By measuring FAB-MS, a mass number of m/z = 269 was observed by molecular ion peak, thereby identifying Intermediate IM-7.

### Synthesis of Intermediate IM-8

In an Ar atmosphere, in a 300 mL three-neck flask, Intermediate IM-7 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 2-bromodibenzofuran (10.09 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-8 (11.64 g, yield 72%).

By measuring FAB-MS, a mass number of m/z = 435 was observed by molecular ion peak, thereby identifying Compound IM-8.

### Synthesis of Compound I3

In an Ar atmosphere, in a 300 mL three-neck flask, Intermediate IM-8 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 4-bromobiphenyl (5.89 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound I3(8.77 g, yield 65%).

By measuring FAB-MS, a mass number of m/z = 587 was observed by molecular ion peak, thereby identifying Compound 13.

### 7. Synthesis of Compound J6

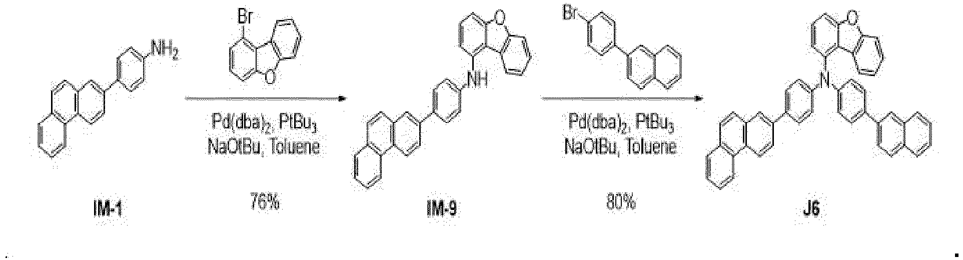

### Synthesis of Intermediate IM-9

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 1-bromodibenzofuran (10.09 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-9 (12.29 g, yield 76%).

By measuring FAB-MS, a mass number of m/z = 435 was observed by molecular ion peak, thereby identifying Compound IM-9.

### Synthesis of Compound J6

In an Ar atmosphere, in a 300 mL three-neck flask, Intermediate IM-9 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 2-(4-bromophenyl)naphthalene (7.15 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound J6 (11.72 g, yield 80%).

By measuring FAB-MS, a mass number of m/z = 637 was observed by molecular ion peak, thereby identifying Compound J6.

### 8. Synthesis of Compound J25

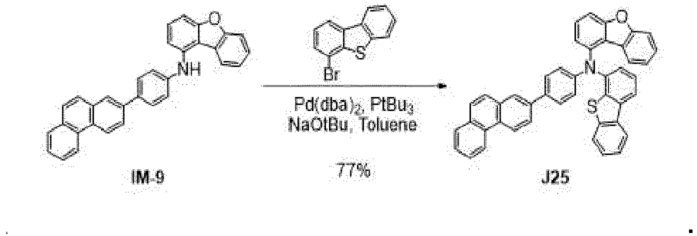

### Synthesis of Compound J25

In an Ar atmosphere, in a 300 mL three-neck flask, Intermediate IM-9 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 4-bromodibenzothiophene (6.65 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound J25 (10.92 g, yield 77%).

By measuring FAB-MS, a mass number of m/z = 617 was observed by molecular ion peak, thereby identifying Compound J25.

### 9. Synthesis of Compound M2

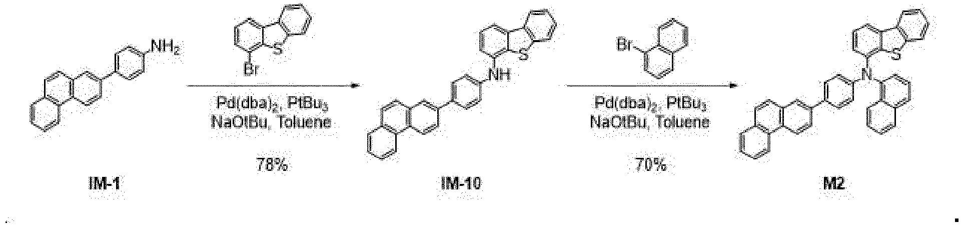

### Synthesis of Intermediate IM-10

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 4-bromodibenzothiophene (10.75 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-10 (13.08 g, yield 78%).

By measuring FAB-MS, a mass number of m/z = 451 was observed by molecular ion peak, thereby identifying Compound IM-10.

### Synthesis of Compound M2

In an Ar atmosphere, in a 300 mL three-neck flask, Intermediate IM-10 (10.00 g, 22.1 mmol), Pd(dba)₂ (0.38 g, 0.03 equiv, 0.7 mmol), NaOtBu (4.26 g, 2.0 equiv, 44.3 mmol), toluene (111 mL), 1-bromonaphthalene (5.04 g, 1.1 equiv, 24.4 mmol), and P(tBu)₃ (0.45 g, 0.1 equiv, 2.2 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound M2 (8.96 g, yield 70%).

By measuring FAB-MS, a mass number of m/z = 577 was observed by molecular ion peak, thereby identifying Compound M2.

### 10. Synthesis of Compound M15

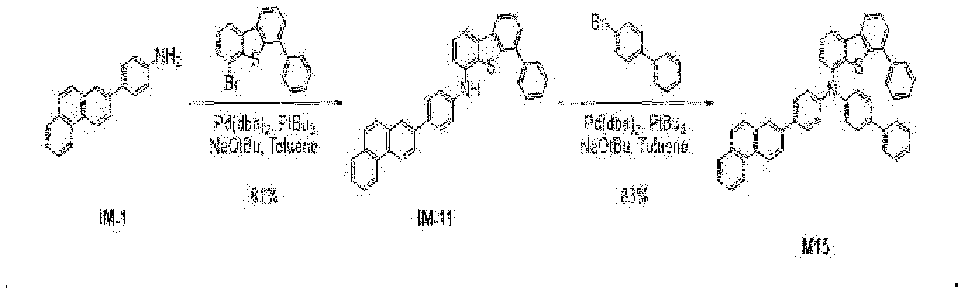

### Synthesis of Intermediate IM-11

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 4-bromo-6-phenyl-dibenzothiophene (13.85 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-11 (15.87 g, yield 81%).

By measuring FAB-MS, a mass number of m/z = 527 was observed by molecular ion peak, thereby identifying Compound IM-11.

### Synthesis of Compound M15

In an Ar atmosphere, in a 300 mL three-neck flask, Intermediate IM-11 (10.00 g, 19.0 mmol), Pd(dba)₂ (0.33 g, 0.03 equiv, 0.6 mmol), NaOtBu (3.64 g, 2.0 equiv, 37.9 mmol), toluene (95 mL), 4-bromobiphenyl (4.86 g, 1.1 equiv, 20.8 mmol), and P(tBu)₃ (0.38 g, 0.1 equiv, 1.9 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound M15 (10.69 g, yield 83%).

By measuring FAB-MS, a mass number of m/z = 679 was observed by molecular ion peak, thereby identifying Compound M15.

### 11. Synthesis of Compound P7

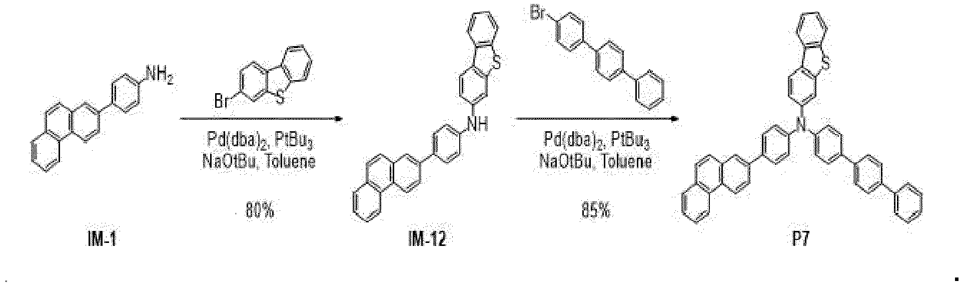

### Synthesis of Intermediate IM-12

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 3-bromodibenzothiophene (10.75 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-12 (13.41 g, yield 80%).

By measuring FAB-MS, a mass number of m/z = 451 was observed by molecular ion peak, thereby identifying Compound IM-12.

### Synthesis of Compound P7

In an Ar atmosphere, in a 300 mL three-neck flask, Intermediate IM-12 (10.00 g, 22.1 mmol), Pd(dba)₂ (0.38 g, 0.03 equiv, 0.7 mmol), NaOtBu (4.26 g, 2.0 equiv, 44.3 mmol), toluene (111 mL), 4-bromo-1,1':4',1"-terphenyl (7.53 g, 1.1 equiv, 24.4 mmol), and P(tBu)₃ (0.45 g, 0.1 equiv, 2.2 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound P7 (12.80 g, yield 85%).

By measuring FAB-MS, a mass number of m/z = 679 was observed by molecular ion peak, thereby identifying Compound P7.

### 12. Synthesis of Compound T10

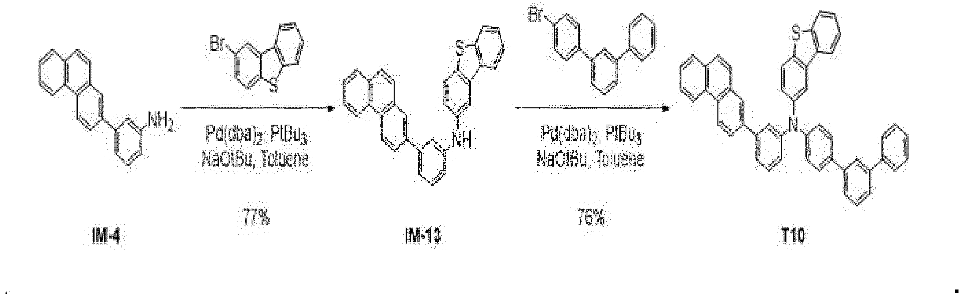

### Synthesis of Intermediate IM-13

In an Ar atmosphere, in a 300 mL three-neck flask, Intermediate IM-4 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 2-bromodibenzothiophene (10.75 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-13 (12.91 g, yield 77%).

By measuring FAB-MS, a mass number of m/z = 451 was observed by molecular ion peak, thereby identifying Compound IM-13.

### Synthesis of Compound T10

In an Ar atmosphere, in a 300 mL three-neck flask, Intermediate IM-13 (10.00 g, 22.1 mmol), Pd(dba)₂ (0.38 g, 0.03 equiv, 0.7 mmol), NaOtBu (4.26 g, 2.0 equiv, 44.3 mmol), toluene (111 mL), 4-bromo-1,1':3',1"-terphenyl (7.53 g, 1.1 equiv, 24.4 mmol), and P(tBu)₃ (0.45 g, 0.1 equiv, 2.2 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound T10 (11.44 g, yield 76%).

By measuring FAB-MS, a mass number of m/z = 679 was observed by molecular ion peak, thereby identifying Compound T10.

### 13. Synthesis of Compound V5

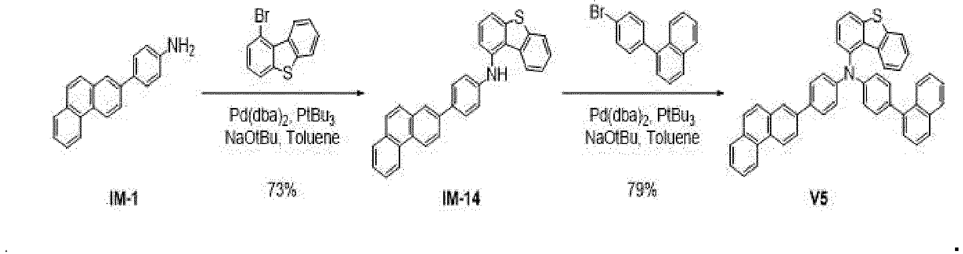

### Synthesis of Intermediate IM-14

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 1-bromodibenzothiophene (10.75 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-14 (12.24 g, yield 73%).

By measuring FAB-MS, a mass number of m/z = 451 was observed by molecular ion peak, thereby identifying Compound IM-14.

### Synthesis of Compound V5

In an Ar atmosphere, in a 300 mL three-neck flask, Intermediate IM-14 (10.00 g, 22.1 mmol), Pd(dba)₂ (0.38 g, 0.03 equiv, 0.7 mmol), NaOtBu (4.26 g, 2.0 equiv, 44.3 mmol), toluene (111 mL), 1-(4-bromophenyl)naphthalene (6.90 g, 1.1 equiv, 24.4 mmol), and P(tBu)₃ (0.45 g, 0.1 equiv, 2.2 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound V5 (11.44 g, yield 79%).

By measuring FAB-MS, a mass number of m/z = 653 was observed by molecular ion peak, thereby identifying Compound V5.

### 14. Synthesis of Compound AA1

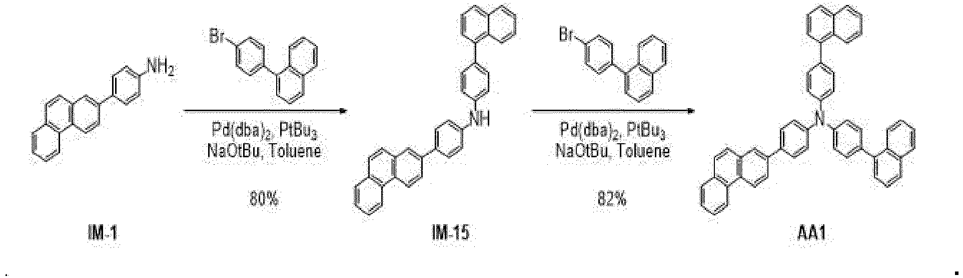

### Synthesis of Intermediate IM-15

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 1-(4-bromophenyl)naphthalene (11.56 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-15 (14.01 g, yield 80%).

By measuring FAB-MS, a mass number of m/z = 471 was observed by molecular ion peak, thereby identifying Compound IM-15.

### Synthesis of Compound AA1

In an Ar atmosphere, in a 300 mL three-neck flask, Intermediate IM-15 (10.00 g, 21.2 mmol), Pd(dba)₂ (0.37 g, 0.03 equiv, 0.6 mmol), NaOtBu (4.08 g, 2.0 equiv, 42.4 mmol), toluene (106 mL), 1-(4-bromophenyl)naphthalene (6.60 g, 1.1 equiv, 23.3 mmol), and P(tBu)₃ (0.43 g, 0.1 equiv, 2.1 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound AA1 (11.72 g, yield 82%).

By measuring FAB-MS, a mass number of m/z = 673 was observed by molecular ion peak, thereby identifying Compound AA1.

### 15. Synthesis of Compound AB4

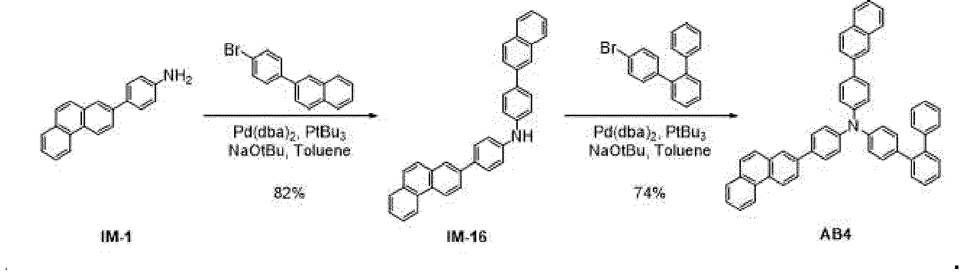

### Synthesis of Intermediate IM-16

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 2-(4-bromophenyl)naphthalene (11.56 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-16 (14.36 g, yield 82%).

By measuring FAB-MS, a mass number of m/z = 471 was observed by molecular ion peak, thereby identifying Compound IM-16.

### Synthesis of Compound AB4

In an Ar atmosphere, in a 300 mL three-neck flask, Intermediate IM-16 (10.00 g, 21.2 mmol), Pd(dba)₂ (0.37 g, 0.03 equiv, 0.6 mmol), NaOtBu (4.08 g, 2.0 equiv, 42.4 mmol), toluene (106 mL), 4-bromo-1,1':2',1"-terphenyl (7.21 g, 1.1 equiv, 23.3 mmol), and P(tBu)₃ (0.43 g, 0.1 equiv, 2.1 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound AB4 (10.98 g, yield 74%).

By measuring FAB-MS, a mass number of m/z = 699 was observed by molecular ion peak, thereby identifying Compound AB4.

### 16. Synthesis of Compound AE1

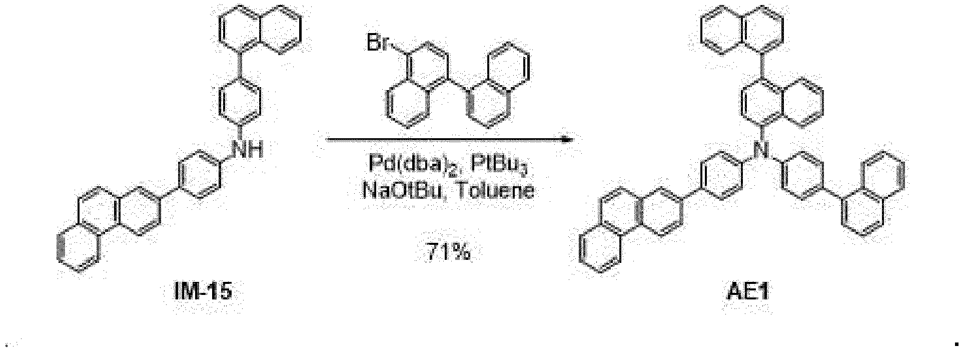

In an Ar atmosphere, in a 300 mL three-neck flask, Intermediate IM-15 (10.00 g, 21.2 mmol), Pd(dba)₂ (0.37 g, 0.03 equiv, 0.6 mmol), NaOtBu (4.08 g, 2.0 equiv, 42.4 mmol), toluene (106 mL), 4-bromo-1,1'-binaphthalene (7.77 g, 1.1 equiv, 23.3 mmol), and P(tBu)₃ (0.43 g, 0.1 equiv, 2.1 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound AE1 (10.90 g, yield 71%).

By measuring FAB-MS, a mass number of m/z = 723 was observed by molecular ion peak, thereby identifying Compound AE1.

### 17. Synthesis of Compound AE7

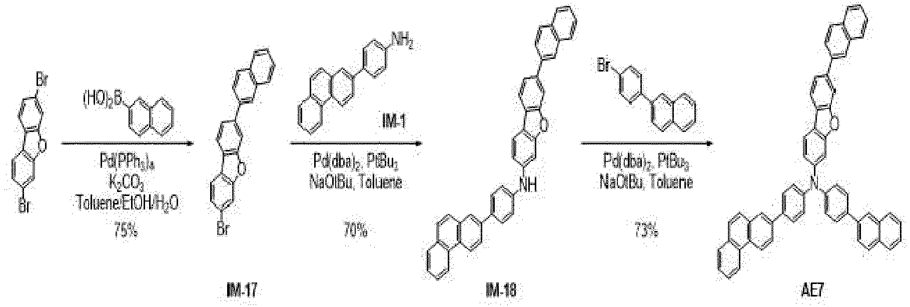

### Synthesis of Intermediate IM-17

In an Ar atmosphere, in a 1000 mL three-neck flask, 3,7-dibromodibenzofuran (15.00 g, 46.0 mmol), naphthalen-2-ylboronic acid (8.71 g, 1.1 equiv, 50.1 mmol), K₂CO₃ (19.08 g, 3.0 equiv, 138.0 mmol), Pd(PPh₃)₄ (2.66 g, 0.05 eq, 2.3 mmol), and a mixed solution of toluene/EtOH/H₂O (4/2/1) (322 mL) were sequentially added and heated and stirred at about 80 °C. After air cooling to room temperature, the reaction solution was extracted with toluene. A water layer was removed, the organic layers were washed with saturated saline, and then dried over MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-17 (12.88 g, yield 75%).

By measuring FAB-MS, a mass number of m/z = 373 was observed by molecular ion peak, thereby identifying Intermediate IM-17.

### Synthesis of Intermediate IM-18

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), Intermediate IM-17 (15.24 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-18 (14.60 g, yield 70%).

By measuring FAB-MS, a mass number of m/z = 561 was observed by molecular ion peak, thereby identifying Compound IM-18.

### Synthesis of Compound AE7

In an Ar atmosphere, in a 300 mL three-neck flask, Intermediate IM-18 (10.00 g, 17.8 mmol), Pd(dba)₂ (0.31 g, 0.03 equiv, 0.5 mmol), NaOtBu (3.42 g, 2.0 equiv, 35.6 mmol), toluene (89 mL), 2-(4-bromophenyl)naphthalene (5.55 g, 1.1 equiv, 19.6 mmol), and P(tBu)₃ (0.36 g, 0.1 equiv, 1.8 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound AE7 (9.93 g, yield 73%).

By measuring FAB-MS, a mass number of m/z = 763 was observed by molecular ion peak, thereby identifying Compound AE7.

### 18. Synthesis of Compound AF1

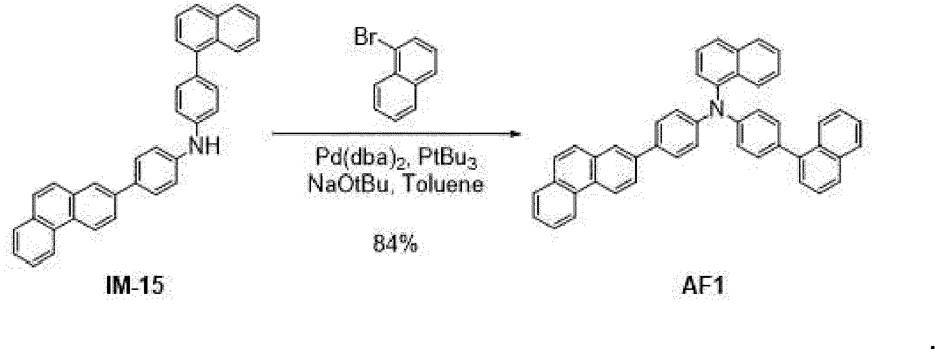

In an Ar atmosphere, in a 300 mL three-neck flask, Intermediate IM-15 (10.00 g, 21.2 mmol), Pd(dba)₂ (0.37 g, 0.03 equiv, 0.6 mmol), NaOtBu (4.08 g, 2.0 equiv, 42.4 mmol), toluene (106 mL), 1-bromonaphthalene (4.83 g, 1.1 equiv, 23.3 mmol), and P(tBu)₃ (0.43 g, 0.1 equiv, 2.1 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound AF1 (10.65 g, yield 84%).

By measuring FAB-MS, a mass number of m/z = 597 was observed by molecular ion peak, thereby identifying Compound AF1.

### 19. Synthesis of Compound BA1

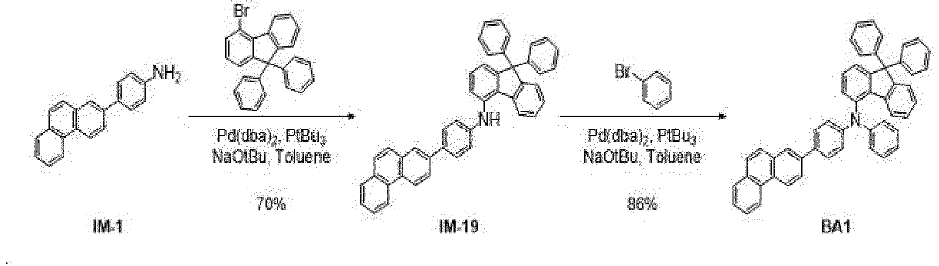

### Synthesis of Intermediate IM-19

In an Ar atmosphere, in a 300 mL three-neck flask, Intermediate IM-1 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 4-bromo-9,9-diphenyl-9H-fluorene (16.23 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-19 (15.22 g, yield 70%).

By measuring FAB-MS, a mass number of m/z = 585 was observed by molecular ion peak, thereby identifying Compound IM-19.

### Synthesis of Compound BA1

In an Ar atmosphere, in a 300 mL three-neck flask, Intermediate IM-19 (10.00 g, 17.1 mmol), Pd(dba)₂ (0.29 g, 0.03 equiv, 0.5 mmol), NaOtBu (3.28 g, 2.0 equiv, 34.1 mmol), toluene (85 mL), bromobenzene (2.95 g, 1.1 equiv, 18.8 mmol), and P(tBu)₃ (0.35 g, 0.1 equiv, 1.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound BA1 (9.72 g, yield 86%).

By measuring FAB-MS, a mass number of m/z = 661 was observed by molecular ion peak, thereby identifying Compound BA1.

### 20. Synthesis of Compound BA9

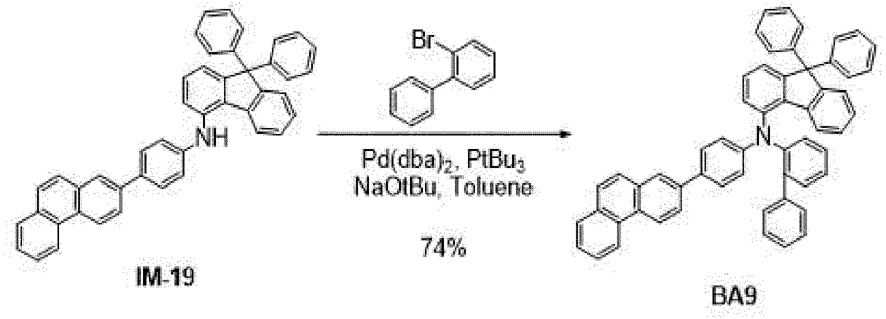

In an Ar atmosphere, in a 300 mL three-neck flask, Intermediate IM-19 (10.00 g, 17.1 mmol), Pd(dba)₂ (0.29 g, 0.03 equiv, 0.5 mmol), NaOtBu (3.28 g, 2.0 equiv, 34.1 mmol), toluene (85 mL), 2-bromo-1,1'-biphenyl (4.38 g, 1.1 equiv, 18.8 mmol), and P(tBu)₃ (0.35 g, 0.1 equiv, 1.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound BA9 (9.32 g, yield 74%).

By measuring FAB-MS, a mass number of m/z = 737 was observed by molecular ion peak, thereby identifying Compound BA9.

### 21. Synthesis of Compound 2-A4

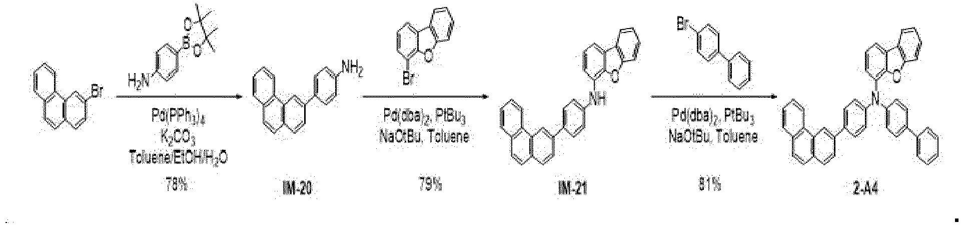

### Synthesis of Intermediate IM-20

In an Ar atmosphere, in a 2000 mL three-neck flask, 3-bromophenanthrene (50.00 g, 194.5 mmol), 4-(4,4,5,5-tetramethyl-1,3,2-dioxaborolan-2-yl)aniline (46.86 g, 1.1 equiv, 213.9 mmol), K₂CO₃ (80.63 g, 3.0 equiv, 583.4 mmol), Pd(PPh₃)₄ (11.24 g, 0.05 eq, 9.7 mmol), and a mixed solution of toluene/EtOH/H₂O (4/2/1) (1362 mL) were sequentially added and heated and stirred at about 80 °C. After air cooling to room temperature, the reaction solution was extracted with toluene. A water layer was removed, the organic layers were washed with saturated saline, and then dried over MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-20 (40.85 g, yield 78%).

By measuring FAB-MS, a mass number of m/z = 269 was observed by molecular ion peak, thereby identifying Intermediate IM-20.

### Synthesis of Intermediate IM-21

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 4-bromodibenzofuran (10.09 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-21 (12.77 g, yield 79%).

By measuring FAB-MS, a mass number of m/z = 435 was observed by molecular ion peak, thereby identifying Compound IM-21.

### Synthesis of Compound 2-A4

In an Ar atmosphere, in a 300 mL three-neck flask, Intermediate IM-21 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 4-bromobiphenyl (5.89 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A4 (10.93 g, yield 81%).

By measuring FAB-MS, a mass number of m/z = 587 was observed by molecular ion peak, thereby identifying Compound 2-A4.

### 22. Synthesis of Compound 2-A28

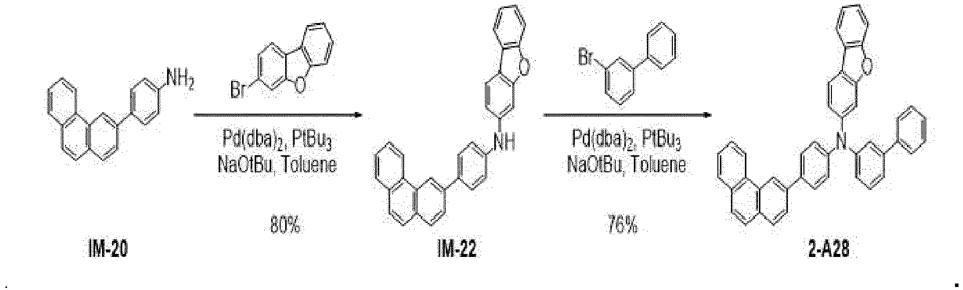

### Synthesis of Intermediate IM-22

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 3-bromodibenzofuran (10.09 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-22 (12.94 g, yield 80%).

By measuring FAB-MS, a mass number of m/z = 435 was observed by molecular ion peak, thereby identifying Compound IM-22.

### Synthesis of Compound 2-A28

In an Ar atmosphere, in a 300 mL three-neck flask, Intermediate IM-22 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 3-bromobiphenyl (5.89 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A28 (10.26 g, yield 76%).

By measuring FAB-MS, a mass number of m/z = 587 was observed by molecular ion peak, thereby identifying Compound 2-A28.

### 23. Synthesis of Compound 2-A57

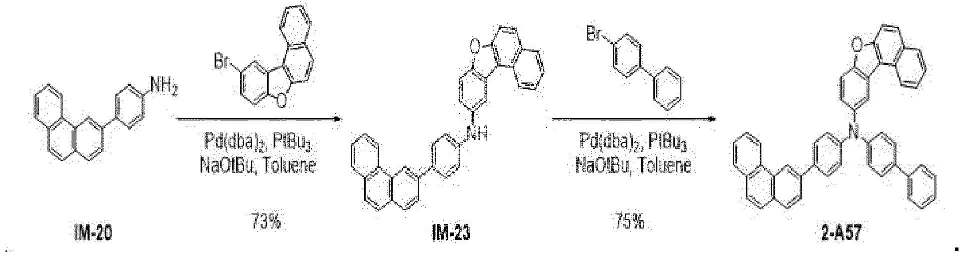

### Synthesis of Intermediate IM-23

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 10-bromonaphtho[2,1-b]benzofuran (12.14 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-23 (13.16 g, yield 73%).

By measuring FAB-MS, a mass number of m/z = 485 was observed by molecular ion peak, thereby identifying Compound IM-23.

### Synthesis of Compound 2-A57

In an Ar atmosphere, in a 300 mL three-neck flask, Intermediate IM-23 (10.00 g, 20.6 mmol), Pd(dba)₂ (0.36 g, 0.03 equiv, 0.7 mmol), NaOtBu (3.96 g, 2.0 equiv, 41.2 mmol), toluene (103 mL), 4-bromobiphenyl (5.28 g, 1.1 equiv, 22.7 mmol), and P(tBu)₃ (0.42 g, 0.1 equiv, 2.1 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A57 (9.85 g, yield 75%).

By measuring FAB-MS, a mass number of m/z = 637 was observed by molecular ion peak, thereby identifying Compound 2-A57.

### 24. Synthesis of Compound 2-A66

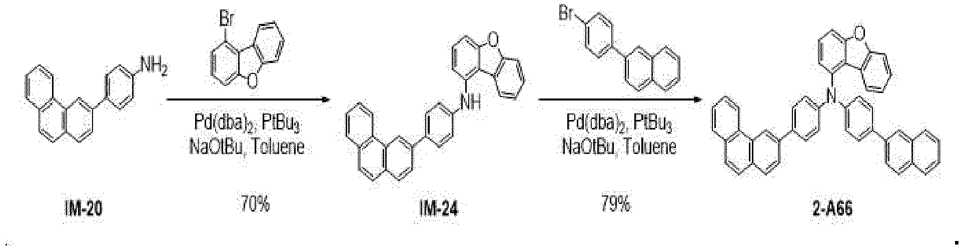

### Synthesis of Intermediate IM-24

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 1-bromodibenzofuran (10.09 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-24 (11.32 g, yield 70%).

By measuring FAB-MS, a mass number of m/z = 435 was observed by molecular ion peak, thereby identifying Compound IM-24.

### Synthesis of Compound 2-A66

In an Ar atmosphere, in a 300 mL three-neck flask, Intermediate IM-24 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 2-(4-bromophenyl)naphthalene (7.15 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A66 (11.57 g, yield 79%).

By measuring FAB-MS, a mass number of m/z = 637 was observed by molecular ion peak, thereby identifying Compound 2-A66.

### 25. Synthesis of Compound 2-A85

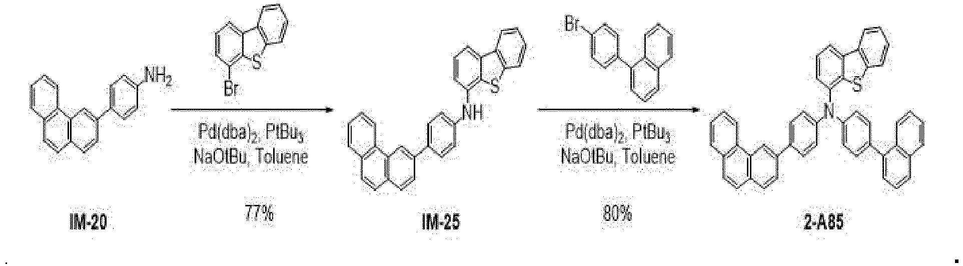

### Synthesis of Intermediate IM-25

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 4-bromodibenzothiophene (10.75 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-25 (12.91 g, yield 77%).

By measuring FAB-MS, a mass number of m/z = 451 was observed by molecular ion peak, thereby identifying Compound IM-25.

### Synthesis of Compound 2-A85

In an Ar atmosphere, in a 300 mL three-neck flask, Intermediate IM-25 (10.00 g, 22.1 mmol), Pd(dba)₂ (0.38 g, 0.03 equiv, 0.7 mmol), NaOtBu (4.26 g, 2.0 equiv, 44.3 mmol), toluene (110 mL), 1-(4-bromophenyl)naphthalene (6.90 g, 1.1 equiv, 24.4 mmol), and P(tBu)₃ (0.45 g, 0.1 equiv, 2.2 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A85 (11.58 g, yield 80%).

By measuring FAB-MS, a mass number of m/z = 653 was observed by molecular ion peak, thereby identifying Compound 2-A85.

### 26. Synthesis of Compound 2-A91

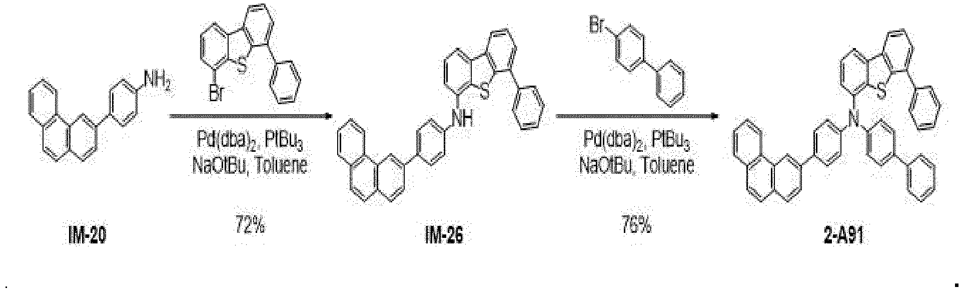

### Synthesis of Intermediate IM-26

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 4-bromo-6-phenyldibenzothiophene (13.85 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-26 (14.11 g, yield 72%).

By measuring FAB-MS, a mass number of m/z = 527 was observed by molecular ion peak, thereby identifying Compound IM-26.

### Synthesis of Compound 2-A91

In an Ar atmosphere, in a 300 mL three-neck flask, Intermediate IM-26 (10.00 g, 19.0 mmol), Pd(dba)₂ (0.33 g, 0.03 equiv, 0.6 mmol), NaOtBu (3.64 g, 2.0 equiv, 37.9 mmol), toluene (95 mL), 4-bromobiphenyl (4.86 g, 1.1 equiv, 20.8 mmol), and P(tBu)₃ (0.38 g, 0.1 equiv, 1.9 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A91 (9.79 g, yield 76%).

By measuring FAB-MS, a mass number of m/z = 679 was observed by molecular ion peak, thereby identifying Compound 2-A91.

### 27. Synthesis of Compound 2-A103

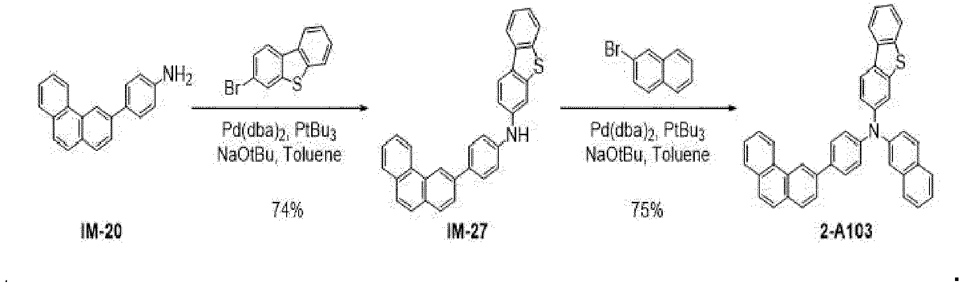

### Synthesis of Intermediate IM-27

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 3-bromodibenzothiophene (10.75 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-27 (12.41 g, yield 74%).

By measuring FAB-MS, a mass number of m/z = 451 was observed by molecular ion peak, thereby identifying Compound IM-27.

### Synthesis of Compound 2-A103

In an Ar atmosphere, in a 300 mL three-neck flask, Intermediate IM-27 (10.00 g, 22.1 mmol), Pd(dba)₂ (0.38 g, 0.03 equiv, 0.7 mmol), NaOtBu (4.26 g, 2.0 equiv, 44.3 mmol), toluene (110 mL), 2-bromonaphthalene (5.04 g, 1.1 equiv, 24.4 mmol), and P(tBu)₃ (0.45 g, 0.1 equiv, 2.2 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A103 (9.60 g, yield 75%).

By measuring FAB-MS, a mass number of m/z = 577 was observed by molecular ion peak, thereby identifying Compound 2-A103.

### 28. Synthesis of Compound 2-A162

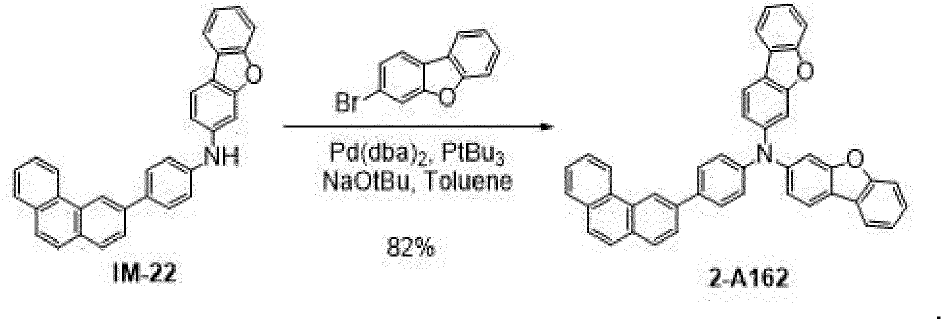

In an Ar atmosphere, in a 300 mL three-neck flask, Intermediate IM-22 (10.00 g, 23.0 mmol), Pd(dba)₂ (0.40 g, 0.03 equiv, 0.7 mmol), NaOtBu (4.41 g, 2.0 equiv, 45.9 mmol), toluene (115 mL), 3-bromodibenzofuran (6.24 g, 1.1 equiv, 25.3 mmol), and P(tBu)₃ (0.46 g, 0.1 equiv, 2.3 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A162 (11.33 g, yield 82%).

By measuring FAB-MS, a mass number of m/z = 601 was observed by molecular ion peak, thereby identifying Compound 2-A162.

### 29. Synthesis of Compound 2-A165

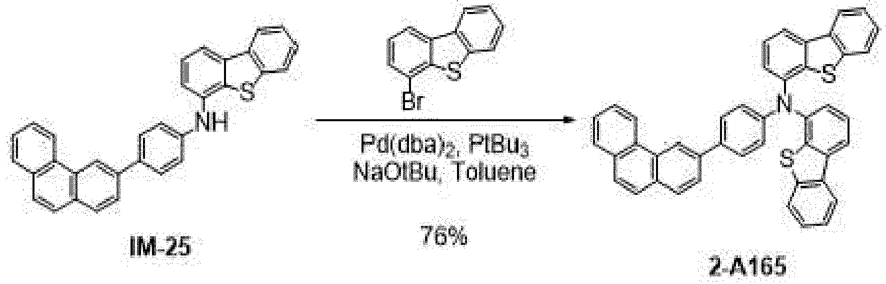

In an Ar atmosphere, in a 300 mL three-neck flask, Intermediate IM-25 (10.00 g, 22.1 mmol), Pd(dba)₂ (0.38 g, 0.03 equiv, 0.7 mmol), NaOtBu (4.26 g, 2.0 equiv, 44.3 mmol), toluene (110 mL), 4-bromodibenzothiophene (6.40 g, 1.1 equiv, 24.4 mmol), and P(tBu)₃ (0.45 g, 0.1 equiv, 2.2 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A165 (10.67 g, yield 76%).

By measuring FAB-MS, a mass number of m/z = 633 was observed by molecular ion peak, thereby identifying Compound 2-A165.

### 30. Synthesis of Compound 2-A169

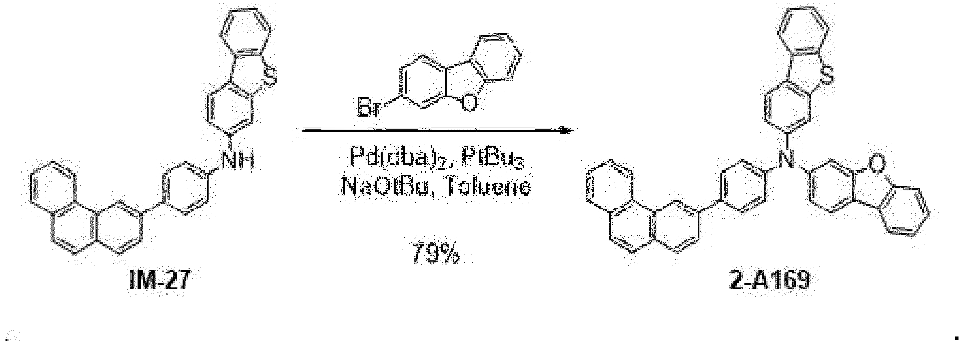

### Synthesis of Compound 2-A169

In an Ar atmosphere, in a 300 mL three-neck flask, Intermediate IM-27 (10.00 g, 22.1 mmol), Pd(dba)₂ (0.38 g, 0.03 equiv, 0.7 mmol), NaOtBu (4.26 g, 2.0 equiv, 44.3 mmol), toluene (110 mL), 3-bromodibenzofuran (6.02 g, 1.1 equiv, 24.4 mmol), and P(tBu)₃ (0.45 g, 0.1 equiv, 2.2 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-A169 (10.81 g, yield 79%).

By measuring FAB-MS, a mass number of m/z = 617 was observed by molecular ion peak, thereby identifying Compound 2-A169.

### 31. Synthesis of Compound 2-B1

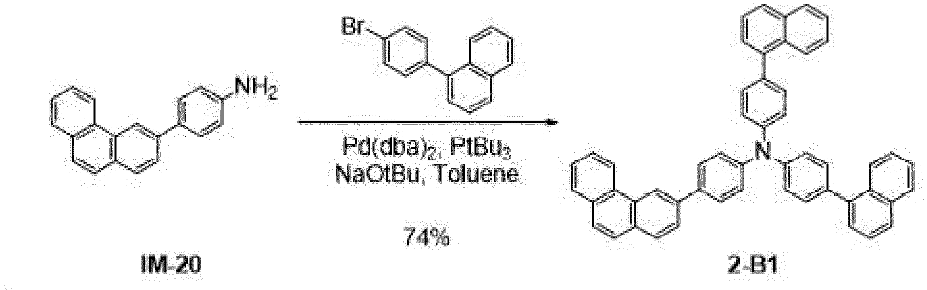

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (10.10 g, 3.0 equiv, 111.4 mmol), toluene (185 mL), 1-(4-bromophenyl)naphthalene (23.13 g, 2.2 equiv, 81.7 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-B1 (18.51 g, yield 74%).

By measuring FAB-MS, a mass number of m/z = 673 was observed by molecular ion peak, thereby identifying Compound 2-B1.

### 32. Synthesis of Compound 2-B26

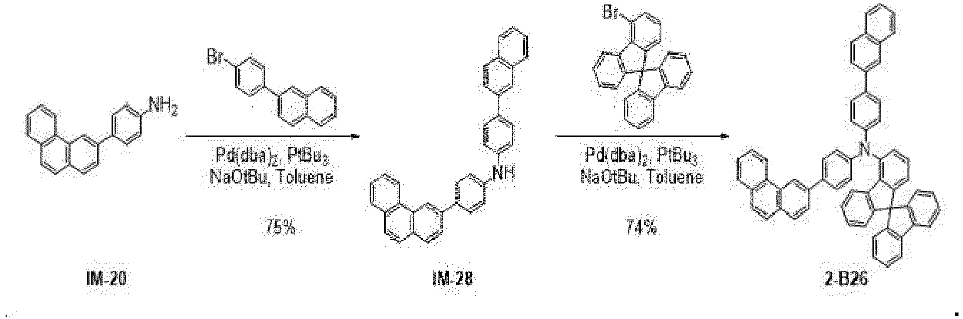

### Synthesis of Intermediate IM-28

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 2-(4-bromophenyl)naphthalene (11.56 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-28 (13.13 g, yield 74%).

By measuring FAB-MS, a mass number of m/z = 471 was observed by molecular ion peak, thereby identifying Compound IM-28.

### Synthesis of Compound 2-B26

In an Ar atmosphere, in a 300 mL three-neck flask, Intermediate IM-28 (10.00 g, 21.2 mmol), Pd(dba)₂ (0.37 g, 0.03 equiv, 0.6 mmol), NaOtBu (4.08 g, 2.0 equiv, 42.4 mmol), toluene (106 mL), 4-bromo-9,9'-spirobi[fluorene] (9.22 g, 1.1 equiv, 23.3 mmol), and P(tBu)₃ (0.43 g, 0.1 equiv, 2.1 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-B26 (12.33 g, yield 74%).

By measuring FAB-MS, a mass number of m/z = 785 was observed by molecular ion peak, thereby identifying Compound 2-B26.

### 33. Synthesis of Compound 2-B30

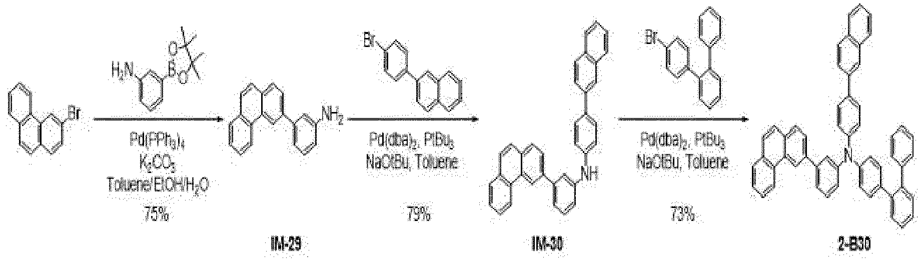

### Synthesis of Intermediate IM-29

In an Ar atmosphere, in a 2000 mL three-neck flask, 3-bromophenanthrene (50.00 g, 194.5 mmol), 3-(4,4,5,5-tetramethyl-1,3,2-dioxaborolan-2-yl)aniline (46.86 g, 1.1 equiv, 213.9 mmol), K₂CO₃ (80.63 g, 3.0 equiv, 583.4 mmol), Pd(PPh₃)₄ (11.24 g, 0.05 eq, 9.7 mmol), and a mixed solution of toluene/EtOH/H₂O (4/2/1) (1362 mL) were sequentially added and heated and stirred at about 80 °C. After air cooling to room temperature, the reaction solution was extracted with toluene. A water layer was removed, the organic layers were washed with saturated saline, and then dried over MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-29 (39.28 g, yield 75%).

By measuring FAB-MS, a mass number of m/z = 269 was observed by molecular ion peak, thereby identifying Intermediate IM-29.

### Synthesis of Intermediate IM-30

In an Ar atmosphere, in a 300 mL three-neck flask, Intermediate IM-29 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 2-(4-bromophenyl)naphthalene (11.56 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-30 (13.83 g, yield 79%).

By measuring FAB-MS, a mass number of m/z = 471 was observed by molecular ion peak, thereby identifying Compound IM-30.

### Synthesis of Compound 2-B30

In an Ar atmosphere, in a 300 mL three-neck flask, Intermediate IM-30 (10.00 g, 21.2 mmol), Pd(dba)₂ (0.37 g, 0.03 equiv, 0.6 mmol), NaOtBu (4.07 g, 2.0 equiv, 42.4 mmol), toluene (106 mL), 4-bromo-1,1':2',1"-terphenyl (7.21 g, 1.1 equiv, 23.3 mmol), and P(tBu)₃ (0.43 g, 0.1 equiv, 2.1 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-B30 (10.83 g, yield 73%).

By measuring FAB-MS, a mass number of m/z = 699 was observed by molecular ion peak, thereby identifying Compound 2-B30.

### 34. Synthesis of Compound 2-B61

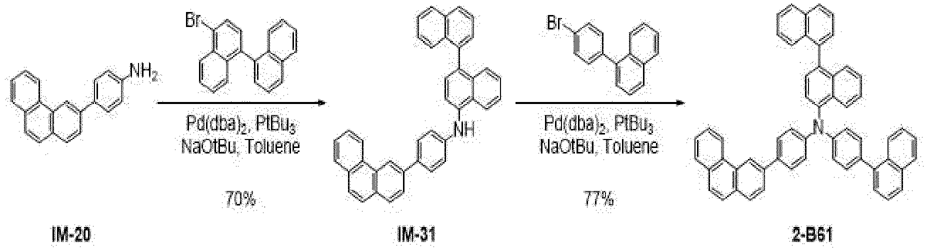

### Synthesis of Intermediate IM-31

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 4-bromo-1,1'-binaphthalene (13.61 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-31 (13.55 g, yield 70%).

By measuring FAB-MS, a mass number of m/z = 521 was observed by molecular ion peak, thereby identifying Compound IM-31.

### Synthesis of Compound 2-B61

In an Ar atmosphere, in a 300 mL three-neck flask, Intermediate IM-31 (10.00 g, 19.2 mmol), Pd(dba)₂ (0.33 g, 0.03 equiv, 0.6 mmol), NaOtBu (3.68 g, 2.0 equiv, 38.3 mmol), toluene (96 mL), 1-(4-bromophenyl)naphthalene (5.97 g, 1.1 equiv, 21.1 mmol), and P(tBu)₃ (0.39 g, 0.1 equiv, 1.9 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-B61 (10.69 g, yield 77%).

By measuring FAB-MS, a mass number of m/z = 723 was observed by molecular ion peak, thereby identifying Compound 2-B61.

### 35. Synthesis of Compound 2-B67

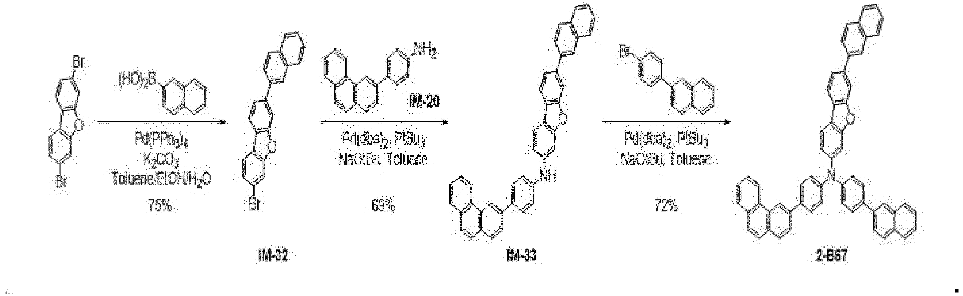

### Synthesis of Intermediate IM-32

In an Ar atmosphere, in a 1000 mL three-neck flask, 3,7-dibromodibenzofuran (15.00 g, 46.0 mmol), naphthalen-2-ylboronic acid (8.71 g, 1.1 equiv, 50.1 mmol), K₂CO₃ (19.08 g, 3.0 equiv, 138.0 mmol), Pd(PPh₃)₄ (2.66 g, 0.05 eq, 2.3 mmol), and a mixed solution of toluene/EtOH/H₂O (4/2/1) (322 mL) were sequentially added and heated and stirred at about 80 °C. After air cooling to room temperature, the reaction solution was extracted with toluene. A water layer was removed, the organic layers were washed with saturated saline, and then dried over MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-32 (12.88 g, yield 75%).

By measuring FAB-MS, a mass number of m/z = 373 was observed by molecular ion peak, thereby identifying Intermediate IM-32.

### Synthesis of Intermediate IM-33

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), Intermediate IM-32 (15.24 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-33 (14.39 g, yield 69%).

By measuring FAB-MS, a mass number of m/z = 561 was observed by molecular ion peak, thereby identifying Compound IM-33.

### Synthesis of Compound 2-B67

In an Ar atmosphere, in a 300 mL three-neck flask, Intermediate IM-33 (10.00 g, 17.8 mmol), Pd(dba)₂ (0.31 g, 0.03 equiv, 0.5 mmol), NaOtBu (3.42 g, 2.0 equiv, 35.6 mmol), toluene (89 mL), 1-(4-bromophenyl)naphthalene (5.55 g, 1.1 equiv, 19.6 mmol), and P(tBu)₃ (0.36 g, 0.1 equiv, 1.8 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-B67 (9.79 g, yield 72%).

By measuring FAB-MS, a mass number of m/z = 763 was observed by molecular ion peak, thereby identifying Compound 2-B67.

### 36. Synthesis of Compound 2-B71

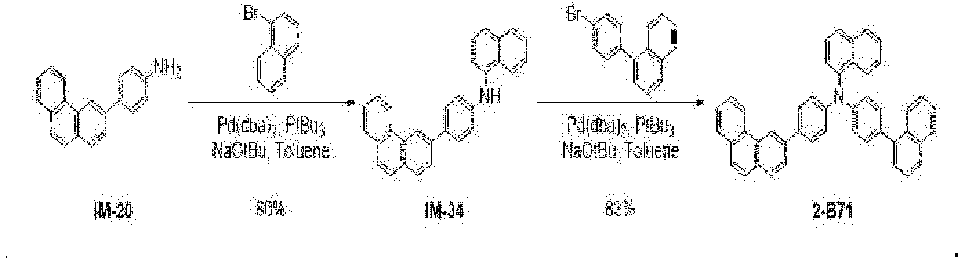

### Synthesis of Intermediate IM-34

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 1-bromonaphthalene (8.46 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-34 (11.75 g, yield 80%).

By measuring FAB-MS, a mass number of m/z = 395 was observed by molecular ion peak, thereby identifying Compound IM-34.

### Synthesis of Compound 2-B71

In an Ar atmosphere, in a 300 mL three-neck flask, Intermediate IM-34 (10.00 g, 25.3 mmol), Pd(dba)₂ (0.44 g, 0.03 equiv, 0.8 mmol), NaOtBu (4.86 g, 2.0 equiv, 50.6 mmol), toluene (126 mL), 1-(4-bromophenyl)naphthalene (7.88 g, 1.1 equiv, 27.8 mmol), and P(tBu)₃ (0.51 g, 0.1 equiv, 2.5 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-B71 (12.54 g, yield 83%).

By measuring FAB-MS, a mass number of m/z = 597 was observed by molecular ion peak, thereby identifying Compound 2-B71.

### 37. Synthesis of Compound 2-B97

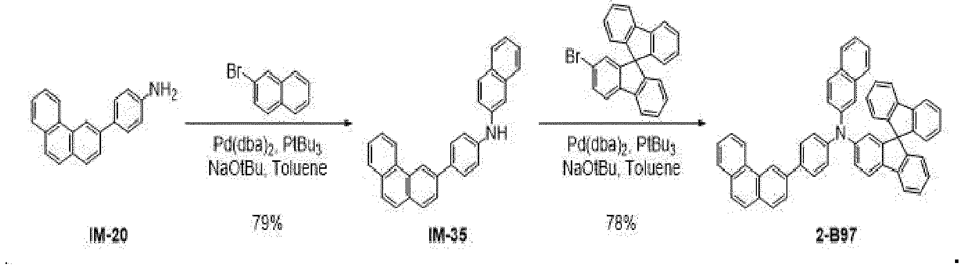

### Synthesis of Intermediate IM-35

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 2-bromonaphthalene (8.46 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-35 (11.60 g, yield 79%).

By measuring FAB-MS, a mass number of m/z = 395 was observed by molecular ion peak, thereby identifying Compound IM-35.

### Synthesis of Compound 2-B97

In an Ar atmosphere, in a 300 mL three-neck flask, Intermediate IM-35 (10.00 g, 25.3 mmol), Pd(dba)₂ (0.44 g, 0.03 equiv, 0.8 mmol), NaOtBu (4.86 g, 2.0 equiv, 50.6 mmol), toluene (126 mL), 2-bromo-9,9'-spirobi[fluorene] (10.99 g, 1.1 equiv, 27.8 mmol), and P(tBu)₃ (0.51 g, 0.1 equiv, 2.5 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-B97 (14.00 g, yield 78%).

By measuring FAB-MS, a mass number of m/z = 709 was observed by molecular ion peak, thereby identifying Compound 2-B97.

### 38. Synthesis of Compound 2-C1

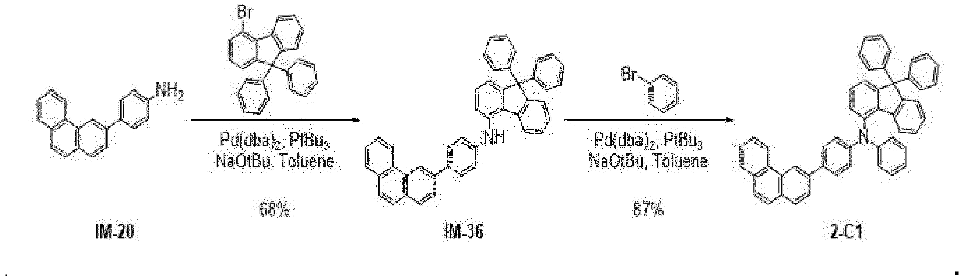

### Synthesis of Intermediate IM-36

In an Ar atmosphere, in a 300 mL three-neck flask, Intermediate IM-20 (10.00 g, 37.1 mmol), Pd(dba)₂ (0.64 g, 0.03 equiv, 1.1 mmol), NaOtBu (3.57 g, 1.0 equiv, 37.1 mmol), toluene (186 mL), 4-bromo-9,9-diphenyl-9H-fluorene (16.23 g, 1.1 equiv, 40.9 mmol), and P(tBu)₃ (0.75 g, 0.1 equiv, 3.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Intermediate IM-36 (14.79 g, yield 68%).

By measuring FAB-MS, a mass number of m/z = 585 was observed by molecular ion peak, thereby identifying Compound IM-36.

### Synthesis of Compound 2-C1

In an Ar atmosphere, in a 300 mL three-neck flask, Intermediate IM-36 (10.00 g, 17.1 mmol), Pd(dba)₂ (0.29 g, 0.03 equiv, 0.5 mmol), NaOtBu (3.28 g, 2.0 equiv, 34.1 mmol), toluene (85 mL), bromobenzene (2.93 g, 1.1 equiv, 18.8 mmol), and P(tBu)₃ (0.35 g, 0.1 equiv, 1.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-C1 (9.83 g, yield 87%).

By measuring FAB-MS, a mass number of m/z = 661 was observed by molecular ion peak, thereby identifying Compound 2-C1.

### 39. Synthesis of Compound 2-C22

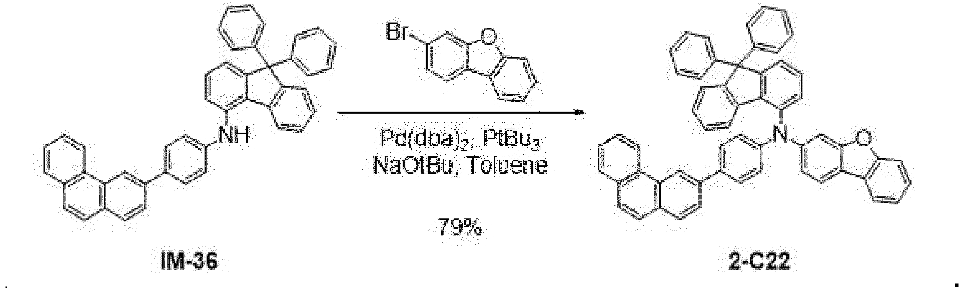

### Synthesis of Compound 2-C-22

In an Ar atmosphere, in a 300 mL three-neck flask, Intermediate IM-36 (10.00 g, 17.1 mmol), Pd(dba)₂ (0.29 g, 0.03 equiv, 0.5 mmol), NaOtBu (3.28 g, 2.0 equiv, 34.1 mmol), toluene (85 mL), 3-bromodibenzofuran (4.64 g, 1.1 equiv, 18.8 mmol), and P(tBu)₃ (0.35 g, 0.1 equiv, 1.7 mmol) were sequentially added and heated and stirred under reflux. After air cooling to room temperature, the organic layer was fractionated by adding water to the reaction solvent. The organic layer was further extracted by adding toluene to a water layer, and then the combined organic layers were washed with saline and dried with MgSO₄. MgSO₄ was filtered off and the organic layers were concentrated, and then the resulting crude product was purified by silica gel column chromatography (utilizing a mixed solvent of hexane and toluene as an eluent) to obtain Compound 2-C22 (10.14 g, yield 87%).

By measuring FAB-MS, a mass number of m/z = 751 was observed by molecular ion peak, thereby identifying Compound 2-C22.

### Device manufacturing Example 1

Organic electroluminescence devices were manufactured utilizing Example Compounds and Comparative Example Compounds as a hole transport layer material:

### Example Compounds

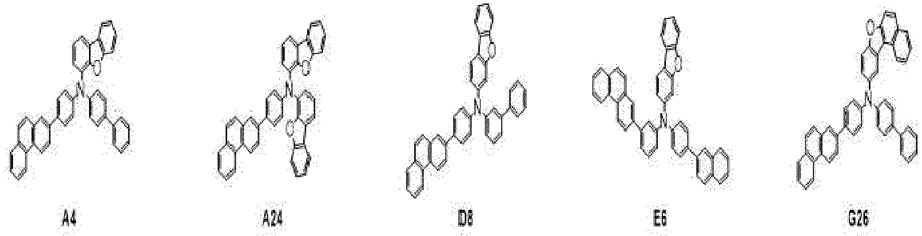

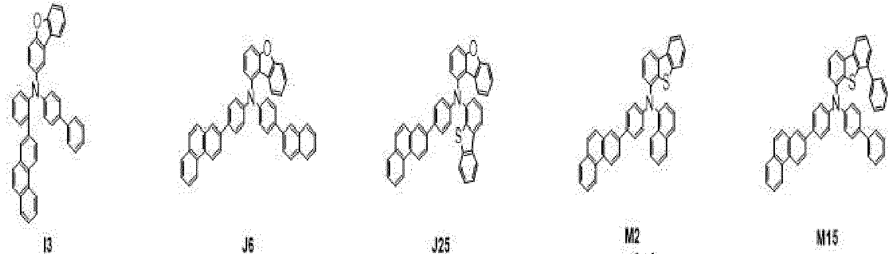

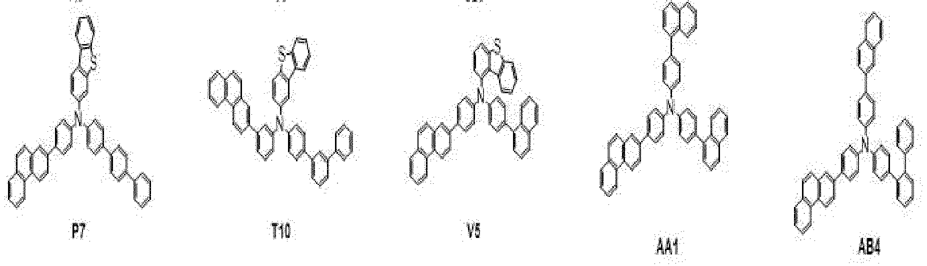

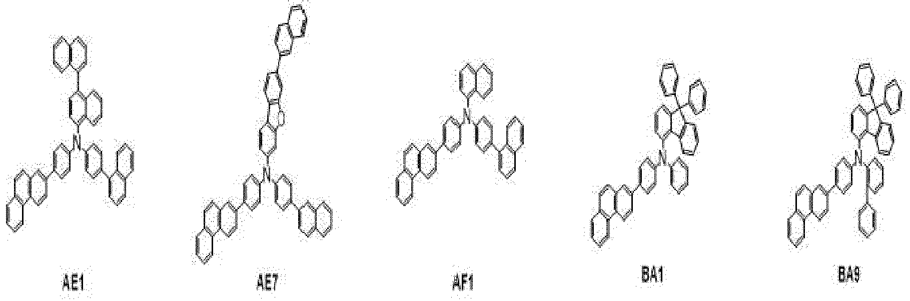

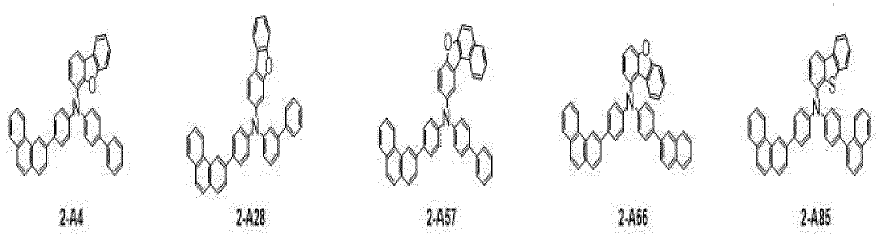

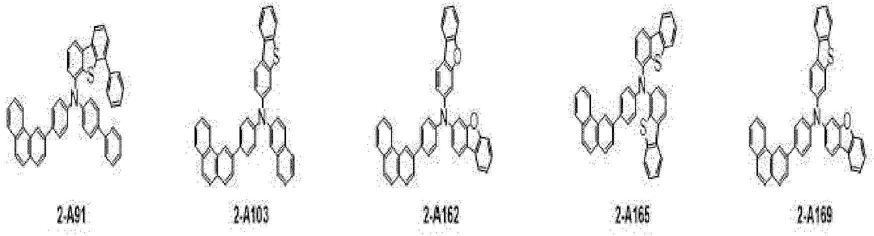

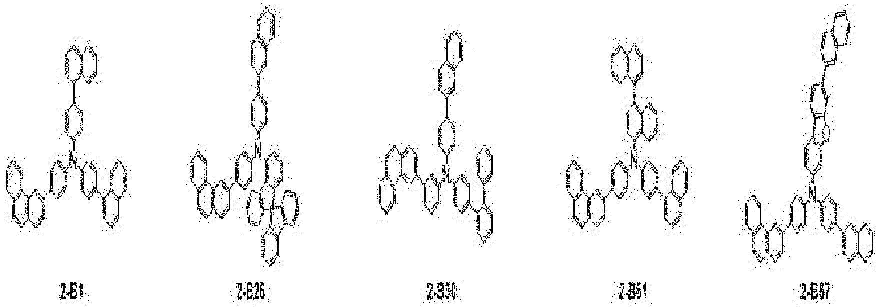

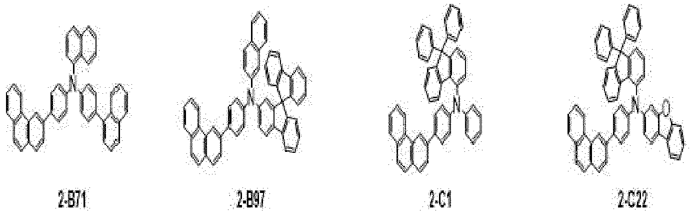

### Comparative Example Compounds

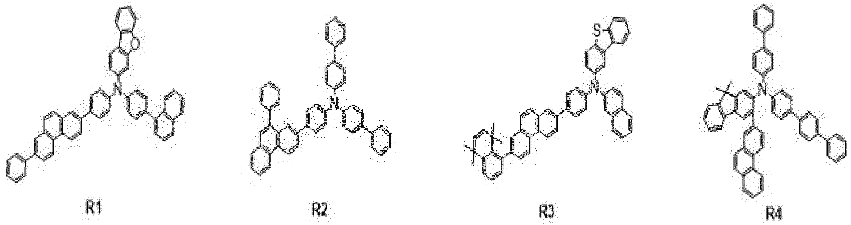

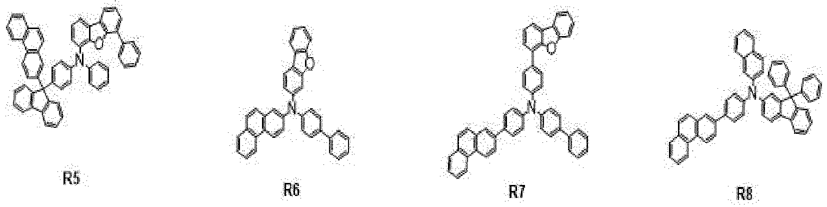

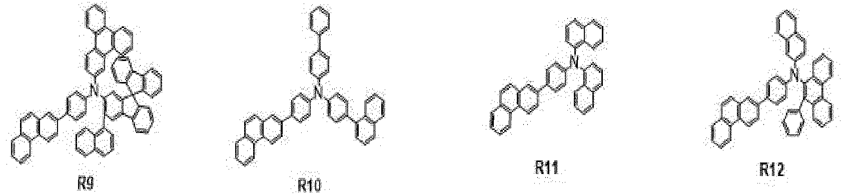

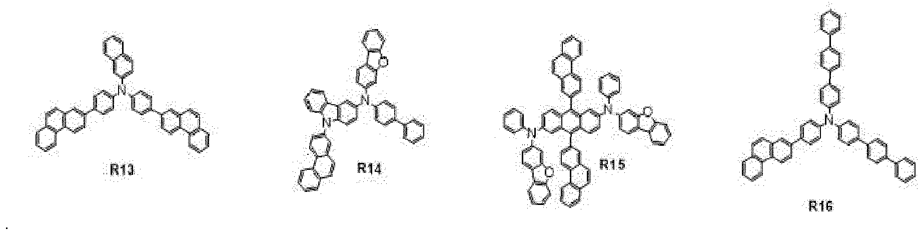

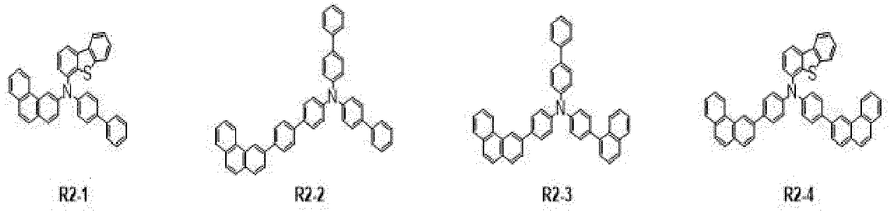

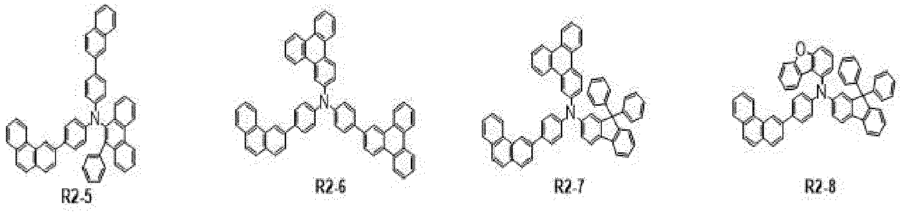

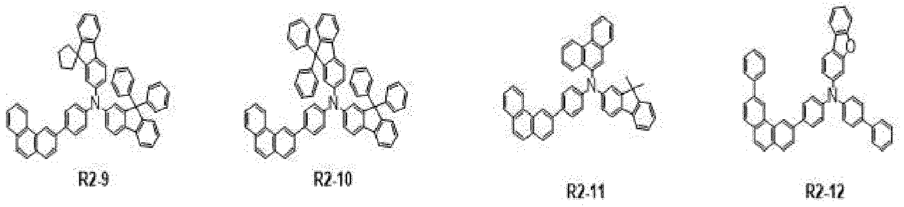

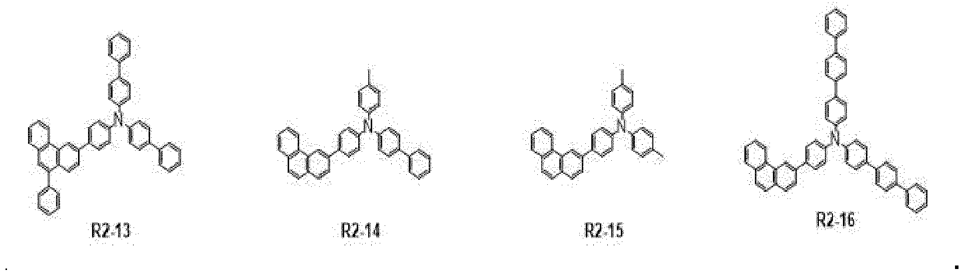

The organic electroluminescence devices of Examples and Comparative Examples were manufactured by the following method. A 150 nm-thick ITO layer was patterned on a glass substrate, and then the glass substrate was washed with ultrapure water and treated with UV and ozone to form a first electrode. Then, 2-TNATA was deposited thereon to a thickness of about 60 nm, and Example Compounds or Comparative Example Compounds were utilized to form a 30 nm-thick hole transport layer. Then, TBP was doped into ADN by 3 wt% to form a 25 nm-thick emission layer, a 25 nm-thick layer was formed with Alq₃ on the emission layer, and a 1 nm-thick layer was formed with LiF to form an electron transport region. Then, a 100 nm-thick second electrode was formed with aluminium (Al). Each layer was formed by a vacuum deposition method. The voltage, luminous efficiency, and service life of each organic electroluminescence device were measured and the results are shown in Table 1 and Table 2. The current efficiency was set to a value at 10 mA/cm², while the half service life is a result at 1.0 mA/cm².

**Table 1**

| | Hole transport layer | Voltage (V) | Luminous efficiency (cd/A) | Service life LT50(h) |
|---|---|---|---|---|
| Example 1 | Example Compound A4 | 5.5 | 7.6 | 1850 |
| Example 2 | Example Compound A24 | 5.5 | 7.8 | 1900 |
| Example 3 | Example Compound D8 | 5.6 | 7.6 | 1800 |
| Example 4 | Example Compound E6 | 5.7 | 7.9 | 1800 |
| Example 5 | Example Compound G26 | 5.6 | 7.8 | 1850 |
| Example 6 | Example Compound I3 | 5.5 | 7.7 | 1800 |
| Example 7 | Example Compound J6 | 5.5 | 7.5 | 1900 |
| Example 8 | Example Compound J25 | 5.6 | 7.9 | 1900 |
| Example 9 | Example Compound M2 | 5.7 | 7.7 | 1950 |
| Example 10 | Example Compound M15 | 5.6 | 7.5 | 1850 |
| Example 11 | Example Compound P7 | 5.7 | 7.6 | 1800 |
| Example 12 | Example Compound T10 | 5.6 | 7.6 | 1800 |
| Example 13 | Example Compound V5 | 5.4 | 7.6 | 1900 |
| Example 14 | Example Compound BA1 | 5.5 | 7.0 | 2100 |
| Example 15 | Example Compound BA9 | 5.6 | 7.1 | 2150 |
| Example 16 | Example Compound AA1 | 5.7 | 7.2 | 2150 |
| Example 17 | Example Compound AB4 | 5.6 | 7.0 | 1950 |
| Example 18 | Example Compound AE1 | 5.6 | 7.3 | 2200 |
| Example 19 | Example Compound AE7 | 5.6 | 7.5 | 2100 |
| Example 20 | Example Compound AF1 | 5.6 | 7.4 | 2100 |
| Comparative Example 1 | Comparative Example Compound R1 | 6.1 | 6.3 | 1650 |
| Comparative Example 2 | Comparative Example Compound R2 | 6.2 | 6.2 | 1600 |
| Comparative Example 3 | Comparative Example Compound R3 | 6.4 | 5.0 | 1450 |
| Comparative Example 4 | Comparative Example Compound R4 | 6.3 | 5.9 | 1650 |
| Comparative Example 5 | Comparative Example Compound R5 | 6.4 | 5.6 | 1450 |
| Comparative Example 6 | Comparative Example Compound R6 | 6.1 | 6.1 | 1550 |
| Comparative Example 7 | Comparative Example Compound R7 | 6.0 | 6.7 | 1550 |
| Comparative Example 8 | Comparative Example Compound R8 | 6.2 | 5.4 | 1650 |
| Comparative Example 9 | Comparative Example Compound R9 | 6.4 | 5.5 | 1600 |
| Comparative Example 10 | Comparative Example Compound R10 | 6.0 | 5.8 | 1650 |
| Comparative Example 11 | Comparative Example Compound R11 | 5.9 | 6.0 | 1600 |
| Comparative Example 12 | Comparative Example Compound R12 | 6.2 | 5.9 | 1550 |
| Comparative Example 13 | Comparative Example Compound R13 | 6.1 | 5.8 | 1600 |
| Comparative Example 14 | Comparative Example Compound R14 | 6.4 | 5.6 | 1400 |
| Comparative Example 15 | Comparative Example Compound R15 | 6.0 | 5.0 | 1600 |
| Comparative Example 16 | Comparative Example Compound R16 | 5.9 | 5.5 | 1550 |

**Table 2**

| | Hole transport layer | Voltage (V) | Luminous efficiency (cd/A) | Service life LT50(h) |
|---|---|---|---|---|
| Example 21 | Example Compound 2-A4 | 5.5 | 7.5 | 1850 |
| Example 22 | Example Compound 2-A28 | 5.5 | 7.6 | 1850 |
| Example 23 | Example Compound 2-A57 | 5.6 | 7.7 | 1850 |
| Example 24 | Example Compound 2-A66 | 5.7 | 7.7 | 1900 |
| Example 25 | Example Compound 2-A85 | 5.6 | 7.6 | 1900 |
| Example 26 | Example Compound 2-A91 | 5.5 | 7.7 | 1800 |
| Example 27 | Example Compound 2-A103 | 5.5 | 7.5 | 1850 |
| Example 28 | Example Compound 2-A162 | 5.6 | 7.9 | 1950 |
| Example 29 | Example Compound 2-A165 | 5.7 | 7.8 | 1850 |
| Example 30 | Example Compound 2-A169 | 5.6 | 7.7 | 1950 |
| Example 31 | Example Compound 2-B1 | 5.7 | 7.3 | 2100 |
| Example 32 | Example Compound 2-B26 | 5.6 | 7.2 | 2100 |
| Example 33 | Example Compound 2-B30 | 5.4 | 7.0 | 2000 |
| Example 34 | Example Compound 2-B61 | 5.5 | 7.4 | 2100 |
| Example 35 | Example Compound 2-B67 | 5.6 | 7.4 | 2150 |
| Example 36 | Example Compound 2-B71 | 5.7 | 7.4 | 2100 |
| Example 37 | Example Compound 2-B97 | 5.6 | 7.2 | 2150 |
| Example 38 | Example Compound 2-C1 | 5.7 | 7.1 | 2050 |
| Example 39 | Example Compound 2-C22 | 5.7 | 7.4 | 2200 |
| Comparative Example 17 | Comparative Example Compound R2-1 | 6.1 | 5.2 | 1550 |
| Comparative Example 18 | Comparative Example Compound R2-2 | 6.4 | 5.6 | 1600 |
| Comparative Example 19 | Comparative Example Compound R2-3 | 5.9 | 6.0 | 1600 |
| Comparative Example 20 | Comparative Example Compound R2-4 | 6.3 | 5.9 | 1650 |
| Comparative Example 21 | Comparative Example Compound R2-5 | 6.4 | 5.6 | 1450 |
| Comparative Example 22 | Comparative Example Compound R2-6 | 6.6 | 5.7 | 1500 |
| Comparative Example 23 | Comparative Example Compound R2-7 | 6.3 | 5.3 | 1500 |
| Comparative Example 24 | Comparative Example Compound R2-8 | 6.0 | 5.4 | 1550 |
| Comparative Example 25 | Comparative Example Compound R2-9 | 6.4 | 5.0 | 1450 |
| Comparative Example 26 | Comparative Example Compound R2-10 | 5.9 | 5.4 | 1650 |
| Comparative Example 27 | Comparative Example Compound R2-11 | 6.3 | 5.2 | 1450 |
| Comparative Example 28 | Comparative Example Compound R2-12 | 6.2 | 5.9 | 1600 |
| Comparative Example 29 | Comparative Example Compound R2-13 | 6.3 | 5.5 | 1600 |
| Comparative Example 30 | Comparative Example Compound R2-14 | 6.0 | 4.9 | 1400 |
| Comparative Example 31 | Comparative Example Compound R2-15 | 6.1 | 4.8 | 1350 |
| Comparative Example 32 | Comparative Example Compound R2-16 | 5.8 | 5.3 | 1500 |

Table 1 above shows the results of Examples 1 to 20 and Comparative Examples 1 to 16. Table 2 above shows the results of Examples 21 to 39 and Comparative Examples 17 to 32. Referring to Table 1 and 2, it may be confirmed that Examples 1 to 39 each achieved a low voltage, high efficiency, and a long service life, concurrently (e.g., simultaneously), compared to each of Comparative Examples 1 to 32.

The amine compound according to an embodiment of the present disclosure includes a phenanthrene skeleton (e.g., group), and thereby achieves a low voltage, a long service life, and high efficiency. The long service life was achieved by introducing the phenanthrene skeleton having excellent or suitable heat resistance and charge resistance to the amine compound that is a hole transport material and has a long service life. In some embodiments, it is believed that the phenanthrene skeleton included in the molecule causes an affinity interaction (e.g., may participate in a stabilizing intermolecular interaction, such as a pi stacking or Van der Waals interaction) with a polycyclic aromatic skeleton included in the host material of the emission layer adjacent to the phenanthrene skeleton, and thus the interfacial stability between the hole transport region and the emission layer may be improved, and the hole movement from the hole transport region to the emission layer accelerates, thereby improving luminous efficiency.

Examples 1 to 13, 19, 21 to 30, 35, and 39 are amine compounds containing a dibenzoheterole group in the molecule, and particularly show improved luminous efficiencies. It is believed that heteroatoms included in the dibenzoheterole group improve the hole transport ability of the entire molecule, and thus the recombination probability of holes and electrons in the emission layer is improved, thereby improving luminous efficiency.

Examples 14, 15, 32, 37, 38 and 39 are amine compounds containing a 4-fluorenyl group in the molecule, and particularly show improved device service lives. It is believed that because the volume of the 4-fluorenyl group is large, the distance between the molecules is appropriately formed, thereby suppressing the deterioration of materials by the reaction between the molecules when radical cations are generated during the driving by electrification.

Examples 4, 7, 9, 13, 16 to 20, 24, 25, 27, and 31 to 37 are amine compounds containing a naphthyl group in the molecule, and particularly show improved device service lives. It is believed that this is because by introducing a naphthalene skeleton having excellent or suitable charge resistance due to the conjugation by the polycyclic rings, the stability in the radical state of materials is improved, and thus the deterioration of materials during the driving by electrification may be suppressed or reduced.

In contrast, Comparative Examples 1 to 3, 28, and 29 show reduced efficiencies and service lives (e.g., simultaneously) compared to the Examples because the material is decomposed due to an increase in the deposition temperature of the material and the layer-forming property thereof is reduced as an aryl group is substituted at a phenanthrene ring and thus the planarity of the entire molecule increases, thereby enhancing the stacking between molecules.

Comparative Examples 4 and 5 are materials in which a linking group between a phenanthrene ring and a nitrogen atom is a fluorene group, and show results of reducing both efficiencies and service lives (e.g., simultaneously) of the devices compared to Examples. Further, Comparative Examples 8 and 9 are materials having a 2-fluorenyl group at the position other than the group containing a phenanthrene ring, and show results of reducing both (e.g., simultaneously) efficiencies and service lives of the devices compared to Examples. It is assumed that the device performance is deteriorated because in the fluorene ring, the bonding around a quaternary carbon located at the position of benzyl group is prone to be cleaved.

In some embodiments, like Examples 14 and 15, in the case where the fluorene group is bonded to the nitrogen atom at the fourth position, excellent or suitable device characteristics are exhibited. For the 4-fluorenyl group, as the volume of the entire molecule is increased, the interaction between molecules gets weak, and thus the deposition temperature is reduced to thereby suppress or reduce the thermal decomposition of materials, and thereby excellent or suitable device characteristics are exhibited.

Comparative Examples 6 and 17 are materials in which a phenanthrene ring and a nitrogen atom are directly bonded, and both (e.g., simultaneously) efficiencies and service lives of the devices are deteriorated compared to Examples. The phenanthrene is suitable that an electrophilic substitution reaction by the ninth and tenth position easily occurs, and it is believed that the amine is directly bonded, and thus the reactivity is more improved, thereby deteriorating materials during the driving by electrification.

Comparative Example 7 is a material in which a phenylene group is interposed between the dibenzofuranyl group and the amine nitrogen atom, and shows a reduced a service life of the device compared to Examples. It is believed that when a dibenzoheterocycle is closer to the nitrogen atom of an amine compound, effects of improving hole transport ability of the entire molecule, improving luminous efficiency, and stabilizing active species may be exhibited. Such effects would be decreased in the molecule which has a linking group between the nitrogen atom and the dibenzoheterocycle. Examples 1 to 13 and 19 may exhibit excellent or suitable device characteristics because the dibenzoheterocycle and the nitrogen atom are directly bonded, thereby improving the stability of the amine compound.

Similar to the compounds on the present disclosure, Comparative Examples 10, 11 and 19 are amine compounds containing a 2-phenanthryl group or a 3-phenanthryl group, and a naphthyl group, but show reduced service lives of the devices compared to Examples. Even though the 2-phenanthryl group or the 3-phenanthryl group, and the naphthyl group having excellent or suitable charge resistance are included, when the number of ring-forming carbon atoms that constitute the rest of the aryl groups is less than 16, the compound is not sufficiently stabilized by the number of carbon atoms in aromatic rings, which form active species (such as cations, anions, radical cations, and/or radical anions), and thus deterioration of materials occurs during electrical driving, thereby reducing device characteristics. Examples 16 to 20 and 31 to 37 include a 2-phenanthryl group or a 3-phenanthryl group, and a naphthyl group, and concurrently (e.g., simultaneously) have 16 or more ring-forming carbon atoms that constitute the rest of the aryl groups, and thus exhibit excellent or suitable device characteristics. In particular, in the materials including only aryl groups without including a dibenzoheterole group and a 4-fluorenyl group in the molecule, the number of ring-forming carbon atoms in each substituent may be an important factor for the stability. It is believed that when a dibenzoheterole group is contained in the molecule, it is unnecessary to control the number of ring-forming carbon atoms that are bonded to the amine so that the heteroatom of the dibenzoheterole group stabilizes any one of cations, anions, radical cations, or radical anions. When a 4-fluorenyl group is included, because the volume of the molecule is large, the distance between the molecules becomes longer, and thus reactivity between molecules may be reduced. Therefore, it is believed that it is unnecessary to control the number of ring-forming carbon atoms of the rest one which is bonded to the amine.

Comparative Examples 12, 13, 20, and 21 are materials that have two phenanthrene groups in the molecule, such that stacking properties (e.g., stacking affinity) between the phenanthrene groups are high, and the deposition temperature of materials significantly increases, thereby causing the material decomposition during the deposition. Comparative Examples 12, 13, 20, and 21 show results of reducing both (e.g., simultaneously) efficiencies and service lives of the devices compared to the Examples.

Comparative Example 14 contains a carbazole as a linking group between an amine nitrogen and a phenanthrene group, and shows a result of reducing both (e.g., simultaneously) efficiency and service life of the device compared to Examples. It is believed that the carbazole increases a hole injecting property higher than necessary to thus deteriorate charge balance, thereby reducing the efficiency and service life of the device.

Comparative Example 15 contains an anthracene as a linking group between an amine nitrogen and a phenanthrene group, and shows a result of particularly reducing efficiency of the device compared to Examples. It is believed that the anthracene group absorbs the energy of an emission layer, thereby reducing the efficiency.

Comparative Examples 16, 18, and 32 are amine compounds including a 2-phenanthryl group or a 3-phenanthryl group, but do not have a dibenzoheterole group, a naphthyl group, or a 4-fluorenyl group in the molecule, and is constituted of only aryl groups. When these substituents (e.g., a dibenzoheterole group, a naphthyl group, or a 4-fluorenyl group) are not included, active species (such as cations, anions, radical cations, and/or radical anions) are not sufficiently stabilized, and thus results of reducing service lives of all the devices compared to Examples are shown.

Comparative Example 22 is an amine compound in which two triphenylene groups are included in the molecule, and shows a result of reducing both (e.g., simultaneously) efficiency and service life of the device compared to Examples because the deposition temperature of the material increases and the layer-forming property thereof is reduced as the planarity of the entire molecule increases, thereby enhancing the stacking between molecules.

Comparative Examples 23 to 27 are amine compounds including a fluorene group in the molecule and show results of reducing both (e.g., simultaneously) efficiencies and service lives of the devices compared to Examples. It is assumed that the device characteristics are deteriorated because in the fluorene ring, and that bonding around a quaternary carbon located at the position of benzyl group is prone to be cleaved. As shown in Examples 38 and 39, even though these materials have the fluorene group in the molecule, when the fluorenyl group is bonded to the nitrogen atom at the fourth position, excellent or suitable device characteristics are exhibited. For the 4-fluorenyl group, as the volume of the entire molecule is increased, the interaction between molecules gets weak, and thus the deposition temperature can be reduced to thereby suppress or reduce the thermal decomposition of materials, and thereby excellent or suitable device characteristics are exhibited. In some embodiments, like the amine compounds of Examples 32 and 37, for the spirobi(fluorene) group, the stability under high-temperature conditions is improved by the rigid ring structure, and thereby excellent or suitable device characteristics may be exhibited.

Comparative Examples 30 and 31 contain a phenyl group for which an alkyl group is substituted, and thus the hydrogen atom located at the benzyl position is unstable because the hydrogen atom is in the state of a radical or a radical cation. Therefore, it is believed that the material is decomposed during the driving by electrification and deteriorated.

### Device manufacturing example 2

Organic electroluminescence devices were manufactured utilizing Example Compounds and Comparative Example Compounds as an electron blocking material.

The organic electroluminescence devices of Examples and Comparative Examples were manufactured by the following method. A 150 nm-thick ITO was patterned on a glass substrate, and then the glass substrate was washed with ultrapure water and treated with UV and ozone to form a first electrode. Thereafter, 2-NATA was deposited to a thickness of about 60 nm, HTL1 was deposited to a thickness of about 20 nm, and a 10 nm-thick electron blocking layer was formed of Example Compound or Comparative Example Compound. Then, TBP was doped to ADN by 3 wt% to form an emission layer having a thickness of about 25 nm, a layer having a thickness of about 25 nm was formed with Alq₃ on the emission layer, and a layer having a thickness of about 1 nm was formed with LiF to form an electron transport region. Then, a 100 nm-thick second electrode was formed with aluminium

(Al). Each layer was formed by a vacuum deposition method. Comparative Example 2-33 was manufactured in substantially the same manner as above, except that HTL1 was deposited to a thickness of about 20 nm and a 10 nm-thick electron blocking layer was formed of HTL1.

The voltage, luminous efficiency, and service life of each organic electroluminescence device were measured and the results are shown in Table 3 and

Table 4. In some embodiments, the current efficiency shows a value at 10 mA/cm², and the half service life is a result at 1.0 mA/cm².

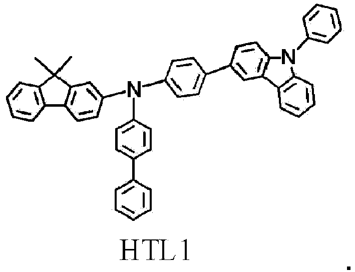

**Table 3**

| | Electron blocking layer | Voltage (V) | Luminous efficiency (cd/A) | Service life LT50(h) |
|---|---|---|---|---|
| Example 2-1 | Example Compound A4 | 5.3 | 8.2 | 2000 |
| Example 2-2 | Example Compound A24 | 5.5 | 8.4 | 2100 |
| Example 2-3 | Example Compound D8 | 5.4 | 8.4 | 1950 |
| Example 2-4 | Example Compound E6 | 5.5 | 8.3 | 2100 |
| Example 2-5 | Example Compound G26 | 5.5 | 8.6 | 2050 |
| Example 2-6 | Example Compound I3 | 5.5 | 8.4 | 1900 |
| Example 2-7 | Example Compound J6 | 5.3 | 8.2 | 2050 |
| Example 2-8 | Example Compound J25 | 5.5 | 8.7 | 2150 |
| Example 2-9 | Example Compound M2 | 5.6 | 8.3 | 2150 |
| Example 2-10 | Example Compound M15 | 5.6 | 8.1 | 1950 |
| Example 2-11 | Example Compound P7 | 5.5 | 8.3 | 2000 |
| Example 2-12 | Example Compound T10 | 5.4 | 8.2 | 1950 |
| Example 2-13 | Example Compound V5 | 5.3 | 8.4 | 2150 |
| Example 2-14 | Example Compound BA1 | 5.5 | 7.9 | 2300 |
| Example 2-15 | Example Compound BA9 | 5.5 | 7.7 | 2250 |
| Example 2-16 | Example Compound AA1 | 5.7 | 7.7 | 2300 |
| Example 2-17 | Example Compound AB4 | 5.5 | 7.5 | 2150 |
| Example 2-18 | Example Compound AE1 | 5.4 | 7.9 | 2300 |
| Example 2-19 | Example Compound AE7 | 5.5 | 8.0 | 2350 |
| Example 2-20 | Example Compound AF1 | 5.6 | 8.0 | 2300 |
| Comparative Example 2-1 | Comparative Example Compound R1 | 6.1 | 6.5 | 1550 |
| Comparative Example 2-2 | Comparative Example Compound R2 | 6.3 | 6.3 | 1500 |
| Comparative Example 2-3 | Comparative Example Compound R3 | 6.3 | 5.5 | 1550 |
| Comparative Example 2-4 | Comparative Example Compound R4 | 6.2 | 5.8 | 1650 |
| Comparative Example 2-5 | Comparative Example Compound R5 | 6.3 | 5.6 | 1500 |
| Comparative Example 2-6 | Comparative Example Compound R6 | 6.1 | 6.0 | 1550 |
| Comparative Example 2-7 | Comparative Example Compound R7 | 5.9 | 6.6 | 1600 |
| Comparative Example 2-8 | Comparative Example Compound R8 | 6.3 | 5.5 | 1600 |
| Comparative Example 2-9 | Comparative Example Compound R9 | 6.6 | 5.5 | 1600 |
| Comparative Example 2-10 | Comparative Example Compound R10 | 6.2 | 5.9 | 1650 |
| Comparative Example 2-11 | Comparative Example Compound R11 | 6.0 | 6.0 | 1650 |
| Comparative Example 2-12 | Comparative Example Compound R12 | 6.2 | 5.7 | 1500 |
| Comparative Example 2-13 | Comparative Example Compound R13 | 6.2 | 5.9 | 1650 |
| Comparative Example 2-14 | Comparative Example Compound R14 | 6.2 | 5.6 | 1500 |
| Comparative Example 2-15 | Comparative Example Compound R15 | 6.0 | 5.1 | 1600 |
| Comparative Example 2-16 | Comparative Example Compound R16 | 6.0 | 5.6 | 1550 |

**Table 4**

| | Electron blocking layer | Voltage (V) | Luminous efficiency (cd/A) | Service life LT50(h) |
|---|---|---|---|---|
| Example 2-21 | Example Compound 2-A4 | 5.4 | 8.0 | 200 |
| Example 2-22 | Example Compound 2-A28 | 5.5 | 8.3 | 1950 |
| Example 2-23 | Example Compound 2-A57 | 5.6 | 8.4 | 1950 |
| Example 2-24 | Example Compound 2-A66 | 5.5 | 8.6 | 2100 |
| Example 2-25 | Example Compound 2-A85 | 5.6 | 8.4 | 1950 |
| Example 2-26 | Example Compound 2-A91 | 5.5 | 8.4 | 1950 |
| Example 2-27 | Example Compound 2-A103 | 5.6 | 8.3 | 1950 |
| Example 2-28 | Example Compound 2-A162 | 5.3 | 8.6 | 2050 |
| Example 2-29 | Example Compound 2-A165 | 5.6 | 8.5 | 2000 |
| Example 2-30 | Example Compound 2-A169 | 5.6 | 8.4 | 2000 |
| Example 2-31 | Example Compound 2-B1 | 5.5 | 7.8 | 2200 |
| Example 2-32 | Example Compound 2-B26 | 5.6 | 7.9 | 2250 |
| Example 2-33 | Example Compound 2-B30 | 5.2 | 7.6 | 2200 |
| Example 2-34 | Example Compound 2-B61 | 5.5 | 7.9 | 2250 |
| Example 2-35 | Example Compound 2-B67 | 5.4 | 8.0 | 2250 |
| Example 2-36 | Example Compound 2-B71 | 5.7 | 8.1 | 2200 |
| Example 2-37 | Example Compound 2-B97 | 5.5 | 7.9 | 2200 |
| Example 2-38 | Example Compound 2-C1 | 5.7 | 7.7 | 2100 |
| Example 2-39 | Example Compound 2-C22 | 5.6 | 8.1 | 2300 |
| Comparative Example 2-17 | Comparative Example Compound R2-1 | 6.1 | 5.3 | 1550 |
| Comparative Example 2-18 | Comparative Example Compound R2-2 | 6.5 | 5.7 | 1600 |
| Comparative Example 2-19 | Comparative Example Compound R2-3 | 5.8 | 6.1 | 1550 |
| Comparative Example 2-20 | Comparative Example Compound R2-4 | 6.3 | 5.7 | 1600 |
| Comparative Example 2-21 | Comparative Example Compound R2-5 | 6.4 | 5.3 | 1400 |
| Comparative Example 2-22 | Comparative Example Compound R2-6 | 6.8 | 5.5 | 1500 |
| Comparative Example 2-23 | Comparative Example Compound R2-7 | 6.2 | 5.5 | 1600 |
| Comparative Example 2-24 | Comparative Example Compound R2-8 | 6.1 | 5.5 | 1550 |
| Comparative Example 2-25 | Comparative Example Compound R2-9 | 6.6 | 5.2 | 1450 |
| Comparative Example 2-26 | Comparative Example Compound R2-10 | 5.7 | 5.7 | 1450 |
| Comparative Example 2-27 | Comparative Example Compound R2-11 | 6.2 | 5.4 | 1550 |
| Comparative Example 2-28 | Comparative Example Compound R2-12 | 6.4 | 5.5 | 1500 |
| Comparative Example 2-29 | Comparative Example Compound R2-13 | 6.3 | 5.4 | 1500 |
| Comparative Example 2-30 | Comparative Example Compound R2-14 | 6.2 | 4.8 | 1350 |
| Comparative Example 2-31 | Comparative Example Compound R2-15 | 6.2 | 4.7 | 1300 |
| Comparative Example 2-32 | Comparative Example Compound R2-16 | 5.9 | 5.5 | 1550 |
| Comparative Example 2-33 | Comparative Example Compound HTL1 | 6.1 | 4.9 | 1300 |

Table 3 above shows the results of Examples 2-1 to 2-20 and Comparative Examples 2-1 to 2-16. Table 4 above shows the results of Examples 2-21 to 2-39 and Comparative Examples 2-17 to 2-33. It may be confirmed that even when the compound of the present disclosure is utilized in the electron blocking layer, Examples 2-1 to 2-39 each achieved a low voltage, high efficiency, and a long service life, concurrently (e.g., simultaneously), compared to each of Comparative Examples 2-1 to 2-33.

Comparative Example 2-33 is an example in which HTL1 is utilized in the hole transport layer and the electron blocking layer, but shows a result of reducing both (e.g., simultaneously) efficiency and service life of the device compared to Examples. It is believed that because a phenanthrene which is a substituent having excellent or suitable charge resistance is not present, the materials are deteriorated at the interface between the electron blocking layer and the emission layer during the driving of the device.

It is believed that because the device deterioration by electrons injected in the emission layer, to which a phenanthrene skeleton having excellent or suitable charge resistance due to the conjugation by the polycyclic rings is adjacent, is prevented or reduced, the device performance is improved.

The amine compounds according to examples of the present disclosure are used in the electron blocking layer of the hole transport region to make a contribution to a low driving voltage, high efficiency, and a long service life of organic electroluminescence devices.

The amine compounds according to examples of the present disclosure are utilized in the hole transport region to make a contribution to a low driving voltage, high efficiency, and a long service life of organic electroluminescence devices.

The luminescence device according to an embodiment of the present disclosure has excellent or suitable efficiency.

The amine compound according to an embodiment of the present disclosure may be utilized as a material of the hole transport region of the luminescence device, and thereby the luminescence device may have improved efficiency.

As used herein, the terms "substantially," "about," and similar terms are used as terms of approximation and not as terms of degree, and are intended to account for the inherent deviations in measured or calculated values that would be recognized by those of ordinary skill in the art. "About" or "approximately," as used herein, is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" may mean within one or more standard deviations, or within ± 30%, 20%, 10%, 5% of the stated value.

Any numerical range recited herein is intended to include all sub-ranges of the same numerical precision subsumed within the recited range. For example, a range of "1.0 to 10.0" is intended to include all subranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited herein is intended to include all lower numerical limitations subsumed therein and any minimum numerical limitation recited in this specification is intended to include all higher numerical limitations subsumed therein.

Although the embodiments of the present disclosure are described, those with ordinary skill in the technical field to which the present disclosure pertains will understood that the present disclosure may be carried out in other forms without changing the technical idea, essential features, or the scope of the present disclosure as set forth in the following claims and their equivalents. Therefore, the above-described embodiments are to be understood in all aspects as illustrative and not restrictive.

## Claims

1. A luminescence device comprising:
a first electrode;
a hole transport region on the first electrode;
an emission layer on the hole transport region;
an electron transport region on the emission layer; and
a second electrode on the electron transport region,
wherein the hole transport region comprises at least one layer selected from a hole injection layer, a hole transport layer, a buffer layer, and an electron blocking layer, and the at least one layer comprises an amine compound represented by Formula 1: 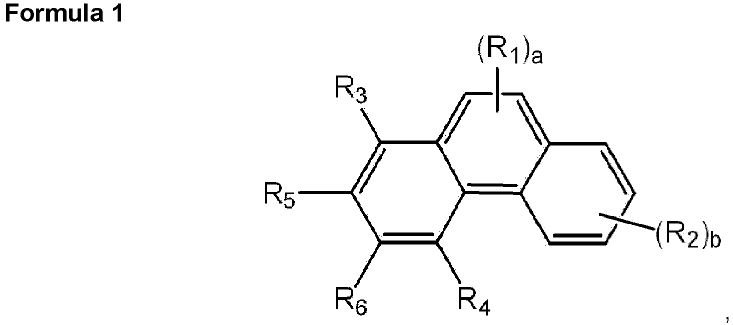 wherein, in Formula 1,
R₁ to R₄ are each independently a hydrogen atom, a deuterium atom, a halogen atom, or a substituted or unsubstituted alkyl group having 1 to 20 carbon atoms,
a is an integer of 0 to 2,
b is an integer of 0 to 4, and
one of R₅ or R₆ is represented by Formula 2, and the other is a hydrogen atom, a deuterium atom, a halogen atom, or a substituted or unsubstituted alkyl group having 1 to 20 carbon atoms: 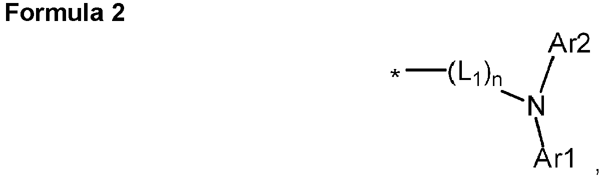 wherein, in Formula 2,
L₁ is a substituted or unsubstituted arylene group having 6 to 30 ring-forming carbon atoms, or a substituted or unsubstituted heteroarylene group having 2 to 30 ring-forming carbon atoms, and does not comprise a fluorenylene group, a carbazolene group, or an anthracenylene group,
n is 1 or 2,
Ar1 is represented by any one among Formula 3-1 to Formula 3-3,
Ar2 is a substituted or unsubstituted aryl group having 6 to 30 ring-forming carbon atoms, or a substituted or unsubstituted heteroaryl group having 2 to 30 ring-forming carbon atoms, and does not comprise a phenanthrene group, a triphenylene group, or a 2-fluorenyl group,
where, when Ar1 is represented by Formula 3-3, Ar2 is a substituted or unsubstituted aryl group having 16 to 30 ring-forming carbon atoms, or a substituted or unsubstituted heteroaryl group having 2 to 30 ring-forming carbon atoms: 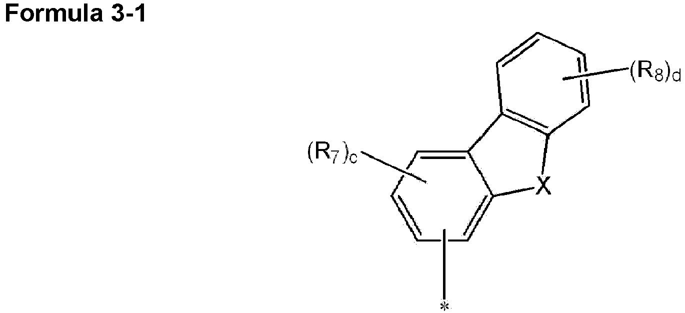 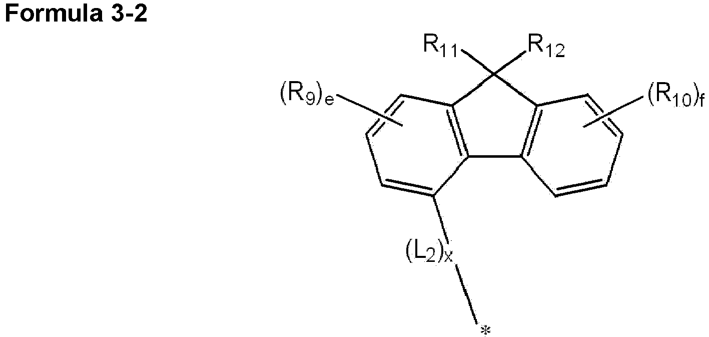 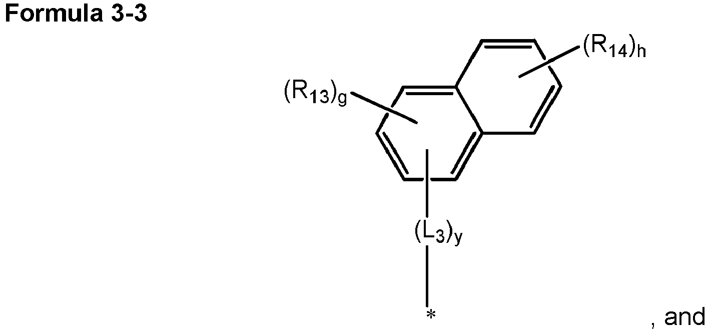 wherein, in Formula 3-1 to Formula 3-3,
X is O or S,
R₇ to R₁₂ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, or are bonded to an adjacent group to form a ring, and do not comprise an amine group,
R₁₃ and R₁₄ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and do not comprise an amine group,
L₂ and L₃ are each independently a substituted or unsubstituted arylene group having 6 to 30 ring-forming carbon atoms, or a substituted or unsubstituted heteroarylene group having 2 to 30 ring-forming carbon atoms,
c, e, and g are each independently an integer of 0 to 3,
d, f, and h are each independently an integer of 0 to 4, and
x and y are each independently an integer of 0 to 3.

2. The luminescence device of claim 1, wherein the hole transport region comprises the hole injection layer and the hole transport layer, and
wherein the hole transport layer is on the hole injection layer, and
at least one of the hole transport layer or the hole transport layer comprises the amine compound represented by Formula 1.

3. The luminescence device of claim 1, wherein the hole transport region comprises the hole transport layer and the electron blocking layer, and
wherein the electron blocking layer is on the hole transport layer, and
the electron blocking layer comprises the amine compound represented by Formula 1.

4. The luminescence device of any one of claims 1 to 3, wherein the amine compound represented by Formula 1 is represented by Formula 4 or Formula 5: 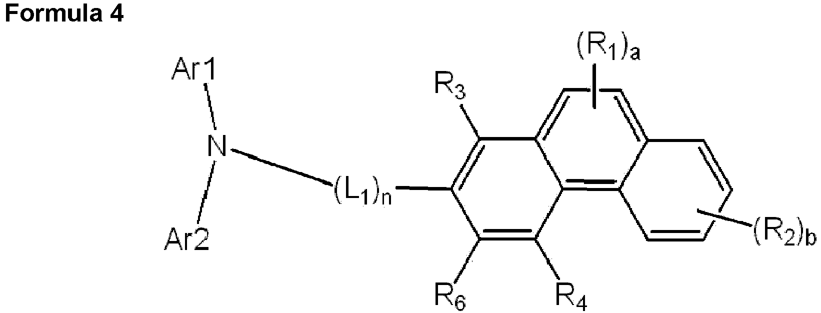 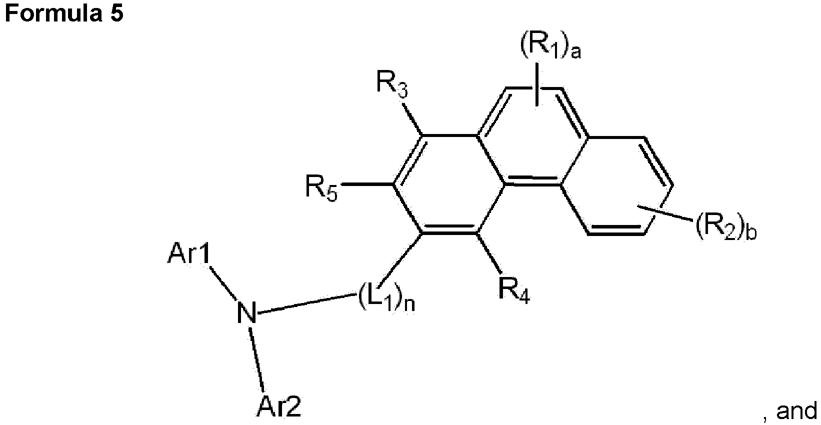 wherein, in Formula 4 and Formula 5,
R₁ to R₆, Ar1, Ar2, L₁, n, a, and b are each independently the same as defined in Formula 1 and Formula 2.

5. The luminescence device of any one of claims 1 to 3, wherein the amine compound represented by Formula 1 is represented by Formula 6-1: 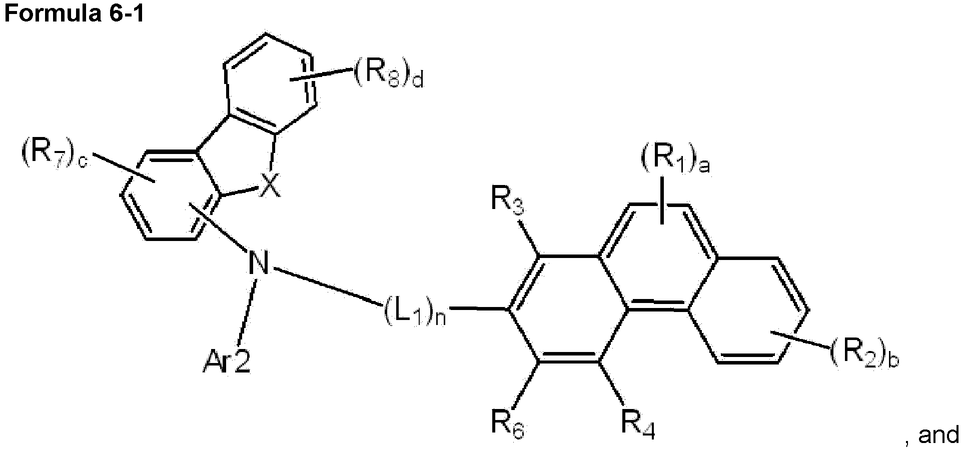 wherein, in Formula 6-1,
Ar2, R₁ to R₄, R₆ to R₈, L₁, n, X, and a to d are each independently the same as defined in Formula 1 to Formula 3-1.

6. The luminescence device of any one of claims 1 to 3, wherein the amine compound represented by Formula 1 is represented by Formula 6-2: 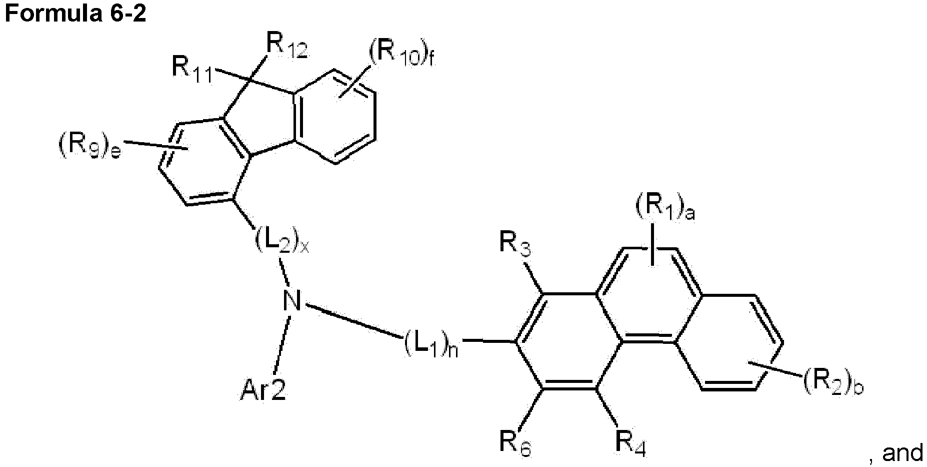 wherein, in Formula 6-2,
Ar2, R₁ to R₄, R₆, R₉ to R₁₂, L₁, L₂, n, x, a, b, e, and f are each independently the same as defined in Formula 1, Formula 2, and Formula 3-2.

7. The luminescence device of any one of claims 1 to 3, wherein the amine compound represented by Formula 1 is represented by Formula 6-3: 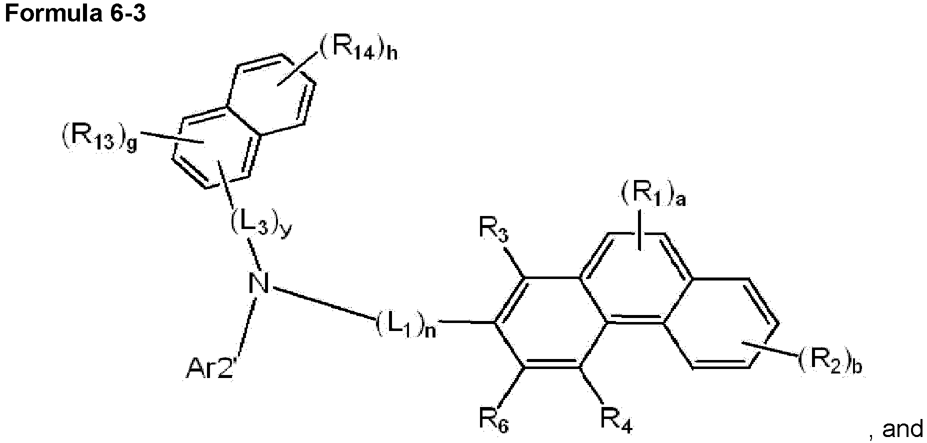 wherein, in Formula 6-3,
Ar2' is a substituted or unsubstituted aryl group having 16 to 30 ring-forming carbon atoms, or a substituted or unsubstituted heteroaryl group having 2 to 30 ring-forming carbon atoms, and does not comprise a phenanthrene group, a triphenylene group, or a 2-fluorenyl group, and
R₁ to R₄, R₆, R₁₃, R₁₄, L₁, L₃, a, b, n, y, g and h are each independently the same as defined in Formula 1, Formula 2, and Formula 3-3.

8. The luminescence device of any one of claims 1 to 3, wherein the amine compound represented by Formula 1 is represented by Formula 7-1: 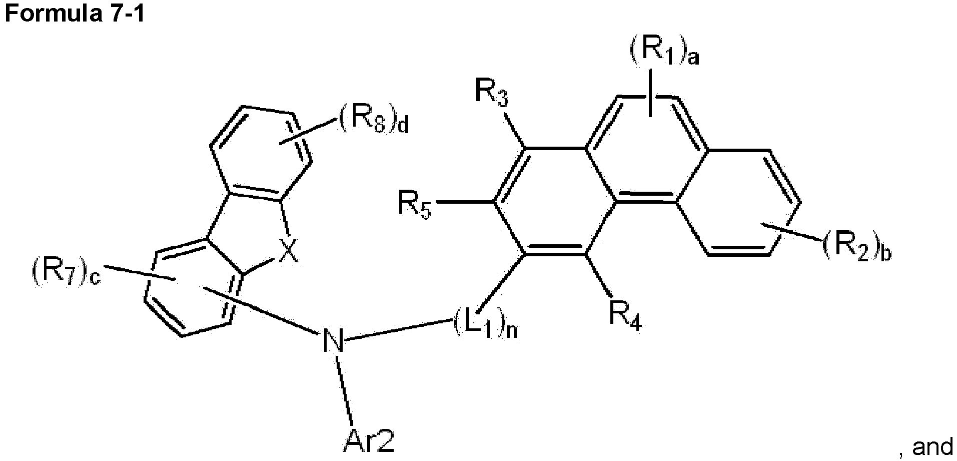 wherein, in Formula 7-1,
Ar2, R₁ to R₅, R₇, R₈, L₁, n, X, and a to d are each independently the same as defined in Formula 1 to Formula 3-1.

9. The luminescence device of any one of claims 1 to 3, wherein the amine compound represented by Formula 1 is represented by Formula 7-2: 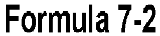 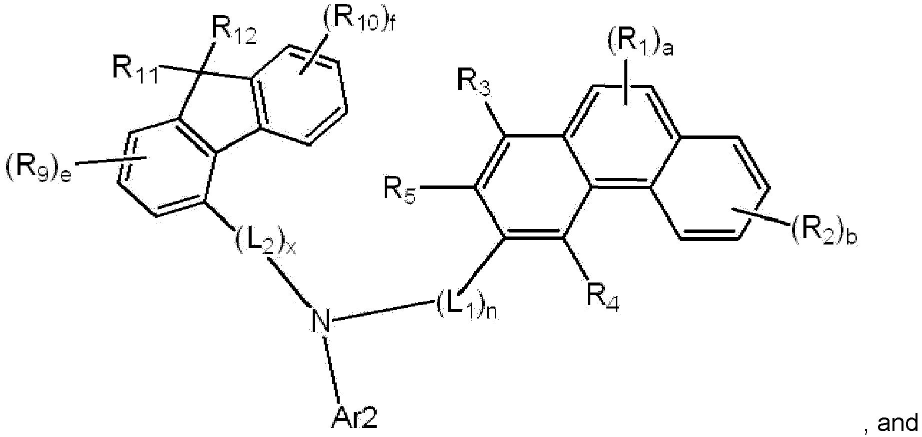 wherein, in Formula 7-2,
Ar2, R₁ to R₅, R₉ to R₁₂, L₁, L₂, n, x, a, b, e, and f are each independently the same as defined in Formula 1, Formula 2, and Formula 3-2.

10. The luminescence device of any one of claims 1 to 3, wherein the amine compound represented by Formula 1 is represented by Formula 7-3: 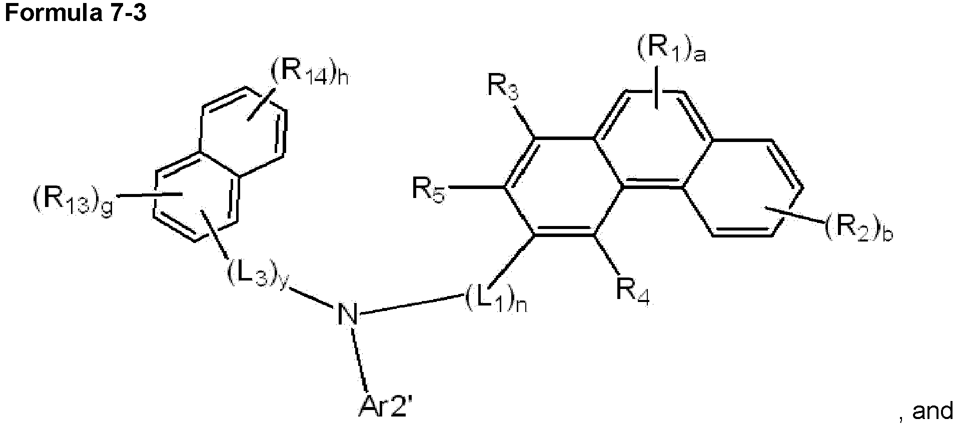 wherein, in Formula 7-3,
Ar2' is a substituted or unsubstituted aryl group having 16 to 30 ring-forming carbon atoms, or a substituted or unsubstituted heteroaryl group having 2 to 30 ring-forming carbon atoms, and does not comprise a phenanthrene group, a triphenylene group, or a 2-fluorenyl group, and
R₁ to R₅, R₁₃, R₁₄, L₁, L₃, a, b, n, y, g and h are each independently the same as defined in Formula 1, Formula 2, and Formula 3-3.

11. The luminescence device of any one of claims 3 to 10, wherein the hole transport layer comprises a compound represented by Formula H-1: 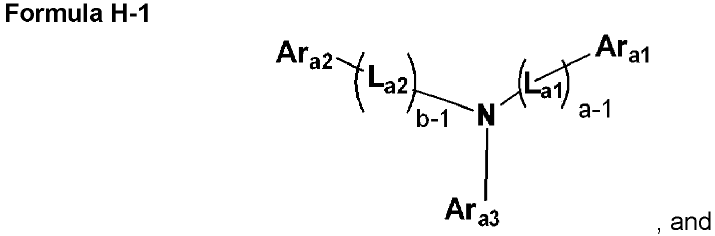 wherein, in Formula H-1,
Lₐ₁ and Lₐ₂ are each independently a direct linkage, a substituted or unsubstituted arylene group having 6 to 30 ring-forming carbon atoms, or a substituted or unsubstituted heteroarylene group having 2 to 30 ring-forming carbon atoms,
a-1 and b-1 are each independently an integer of 0 to 10, and
Arₐ₁ to Arₐ₃ are each independently a substituted or unsubstituted aryl group having 6 to 30 ring-forming carbon atoms, or a substituted or unsubstituted heteroaryl group having 2 to 30 ring-forming carbon atoms.

12. The luminescence device of any one of claims 1 to 11, wherein the emission layer comprises a polycyclic compound represented by Formula E-1: 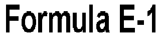 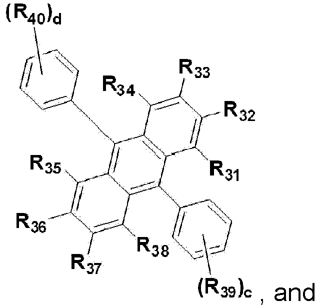 wherein, in Formula E-1,
R₃₁ to R₄₀ are each independently a hydrogen atom, a deuterium atom, a halogen atom, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, and/or are bonded to an adjacent group to form a ring, and
c and d are each independently an integer of 0 to 5.

13. The luminescence device of claim 1, wherein the amine compound represented by Formula 1 is at least one selected from compounds represented by Compound Group 1:

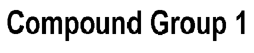

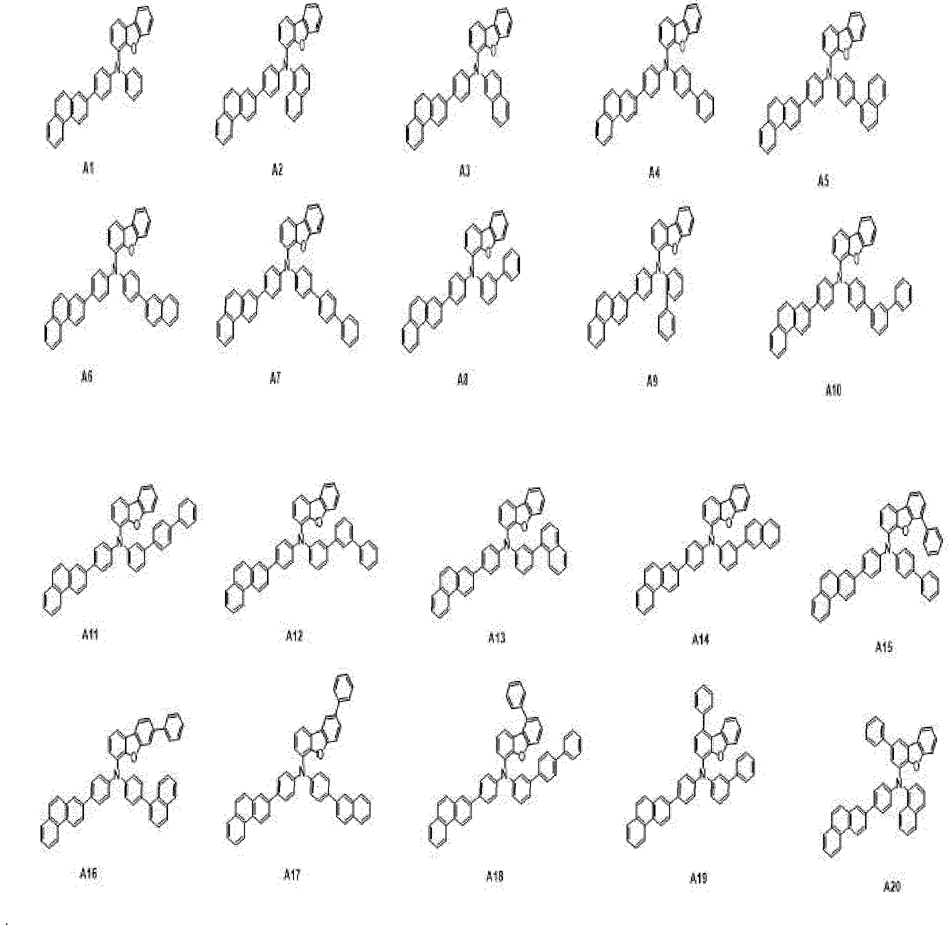

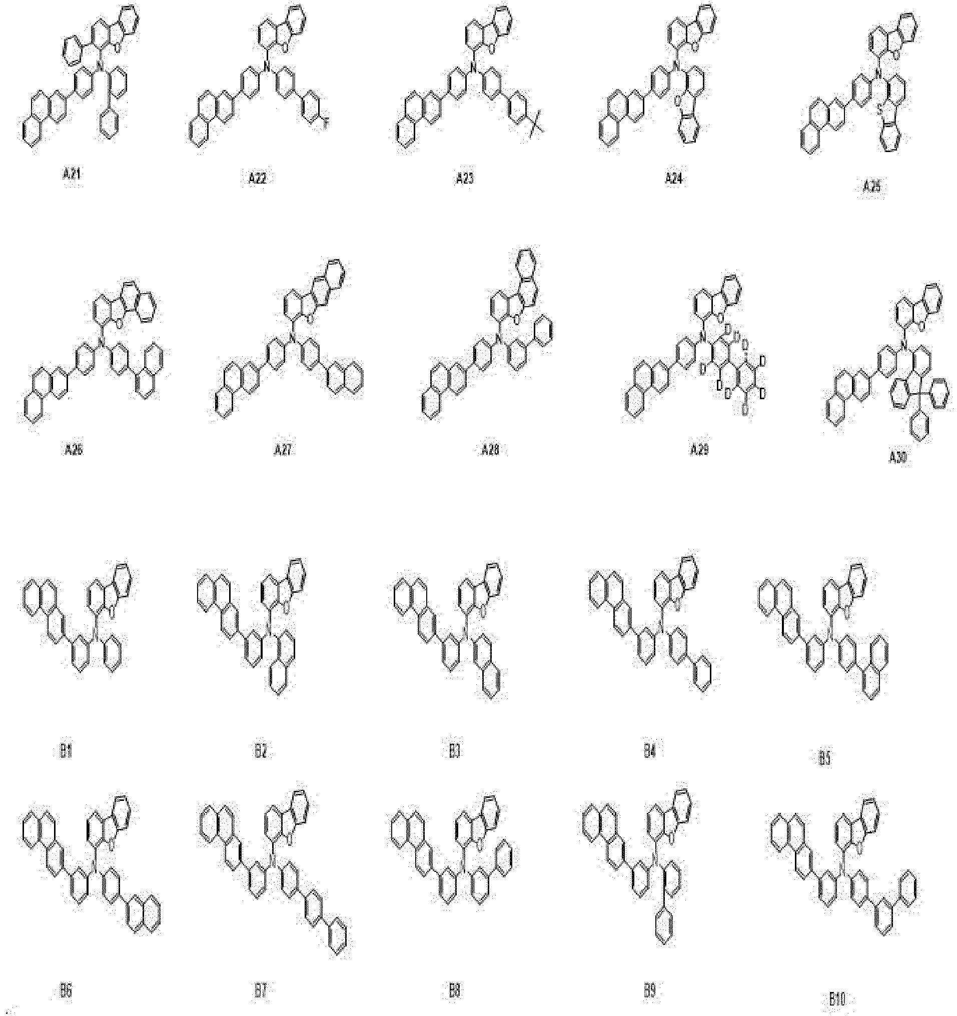

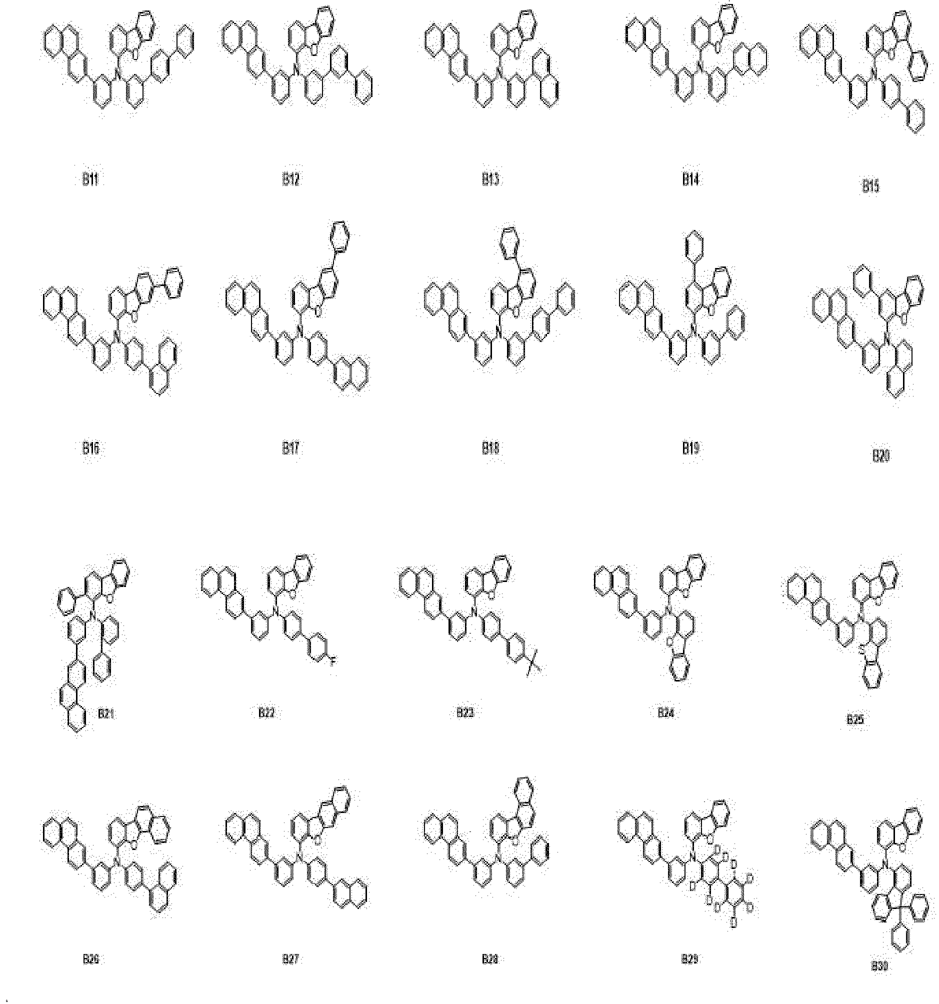

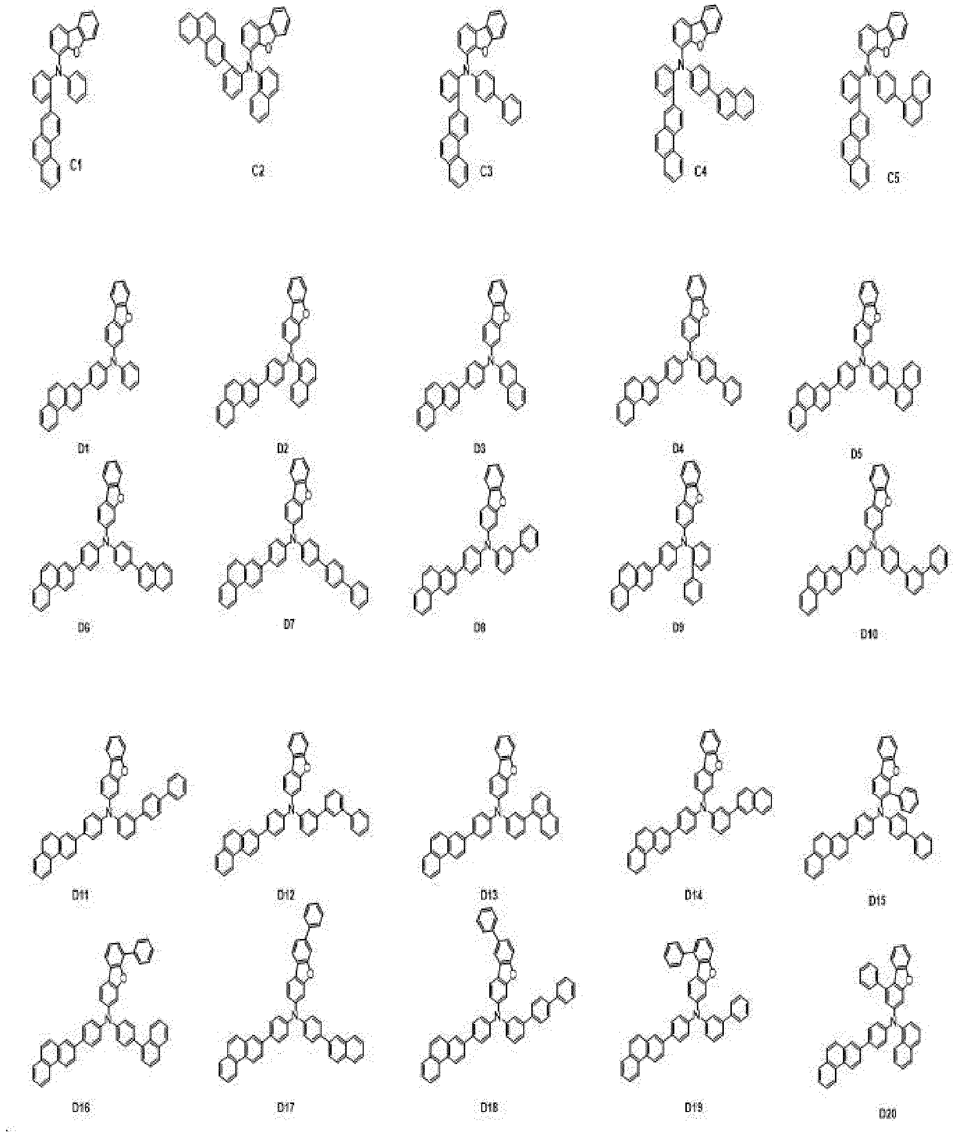

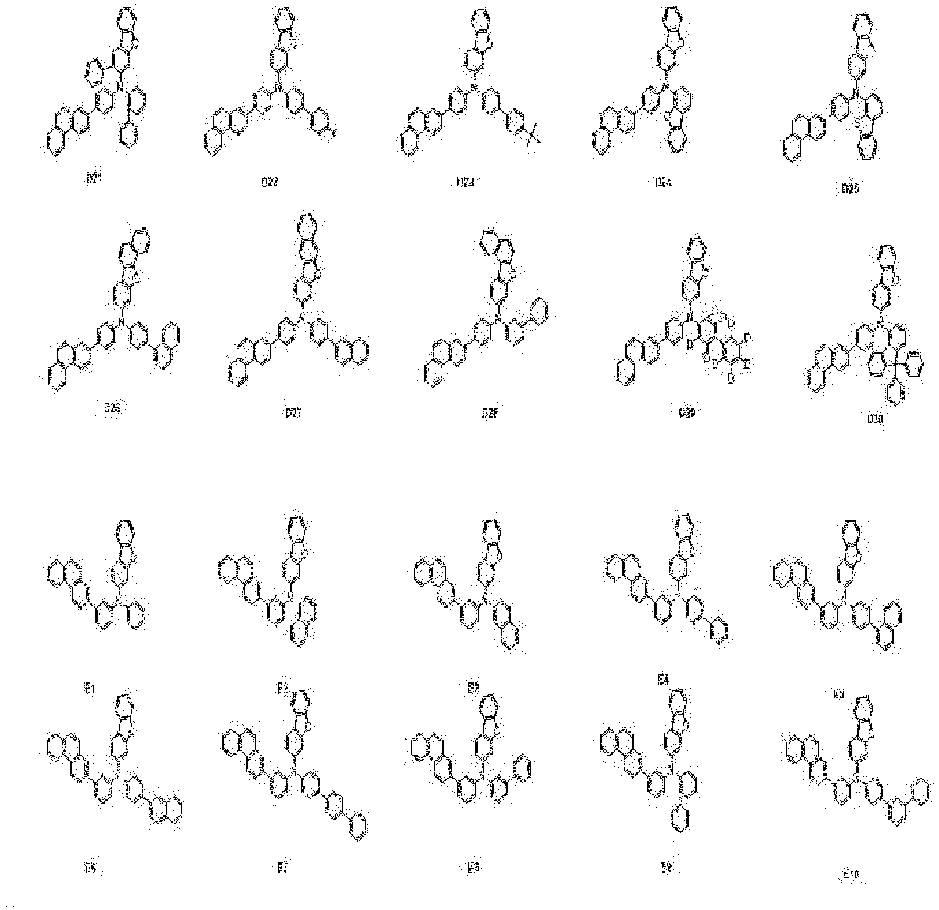

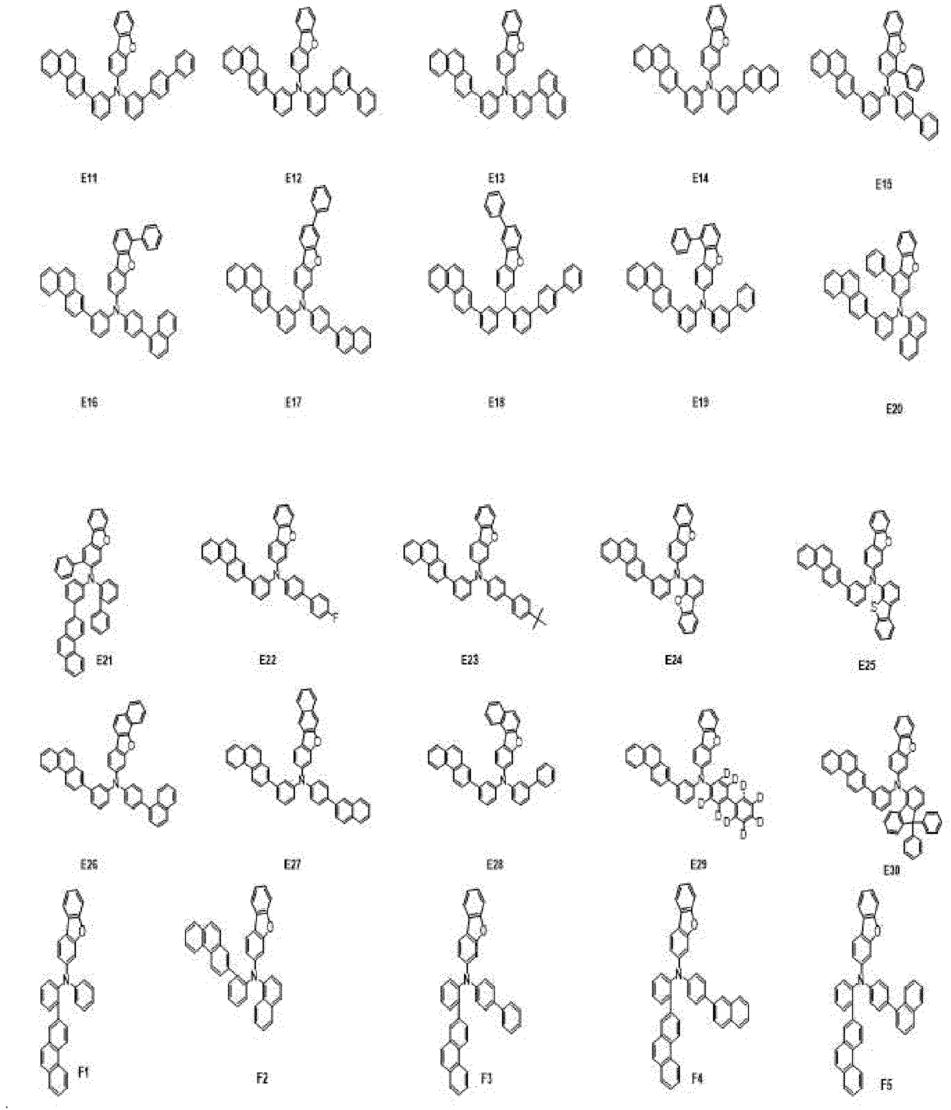

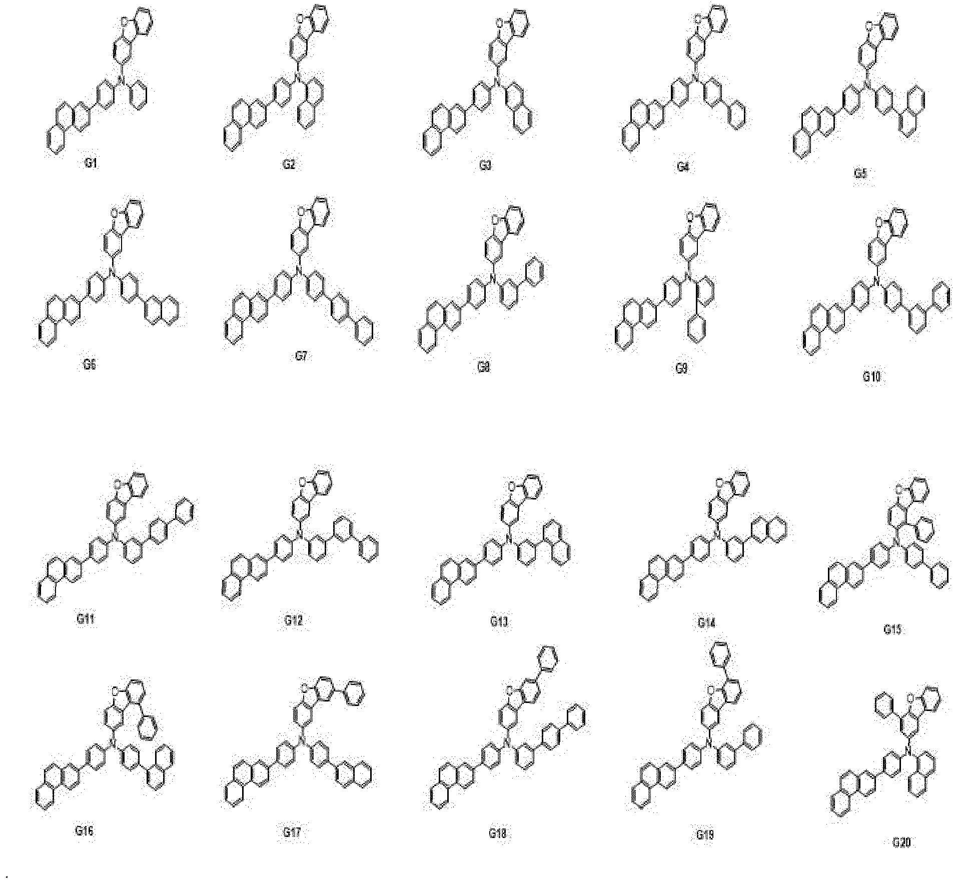

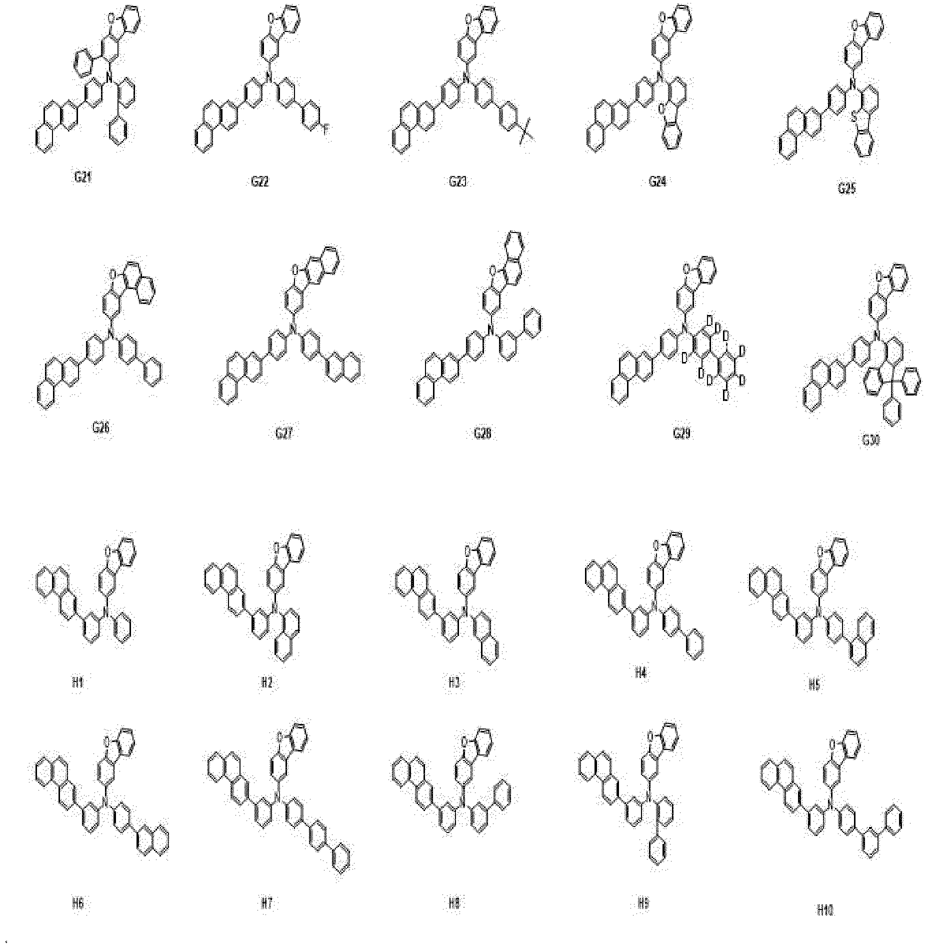

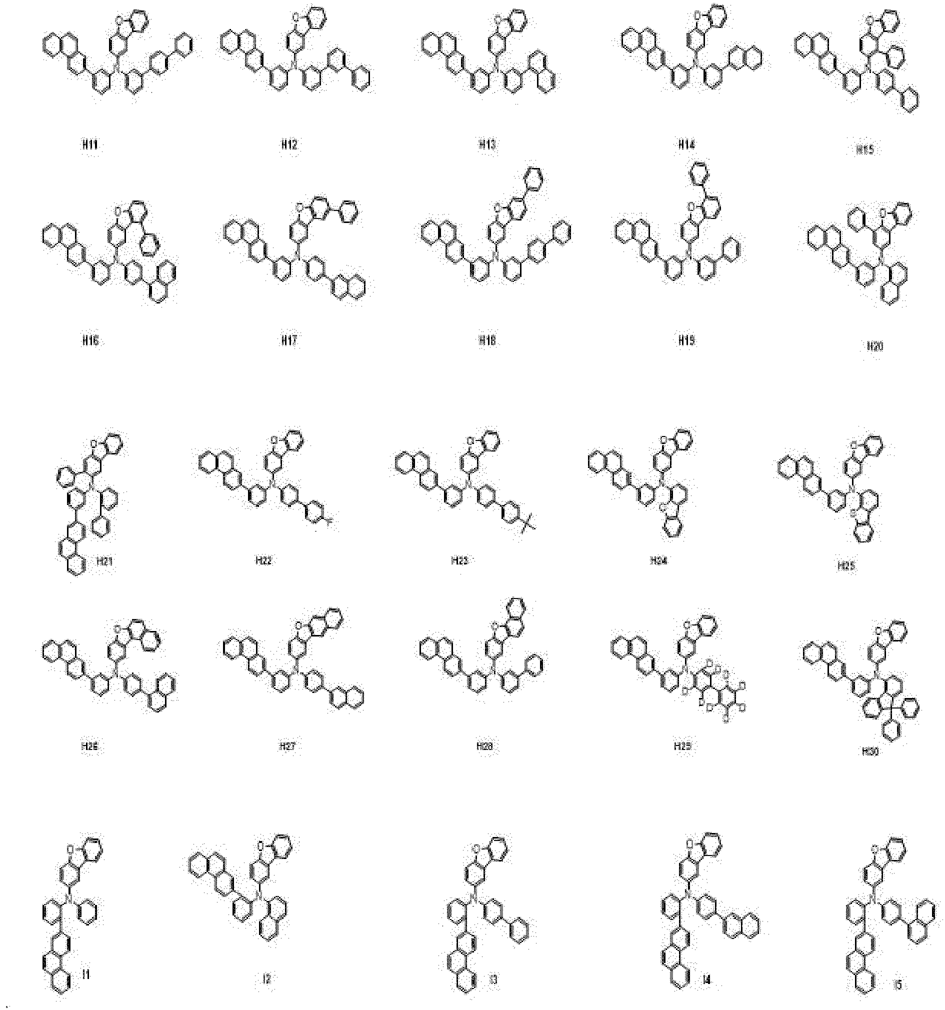

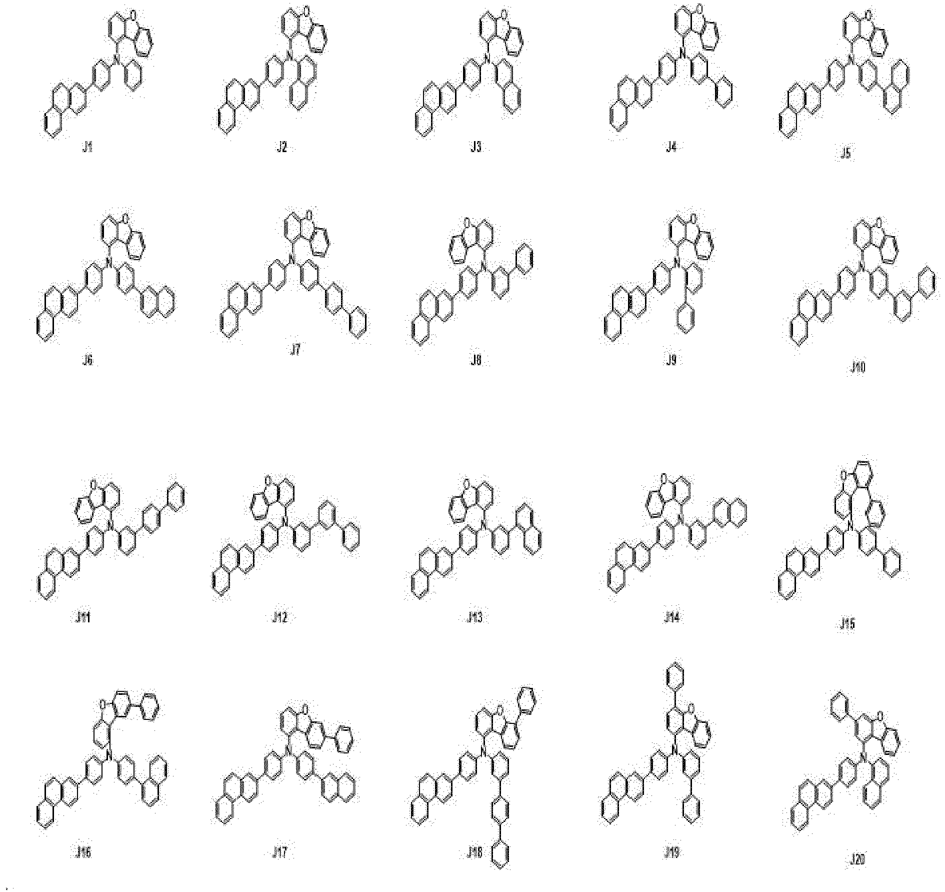

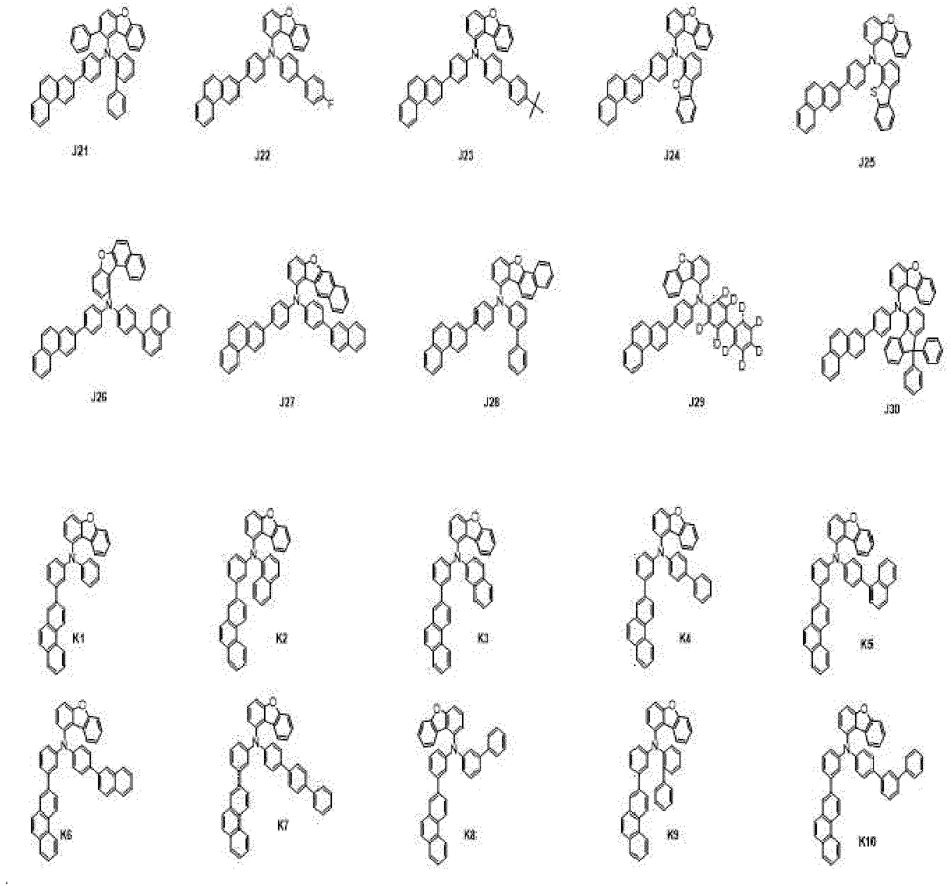

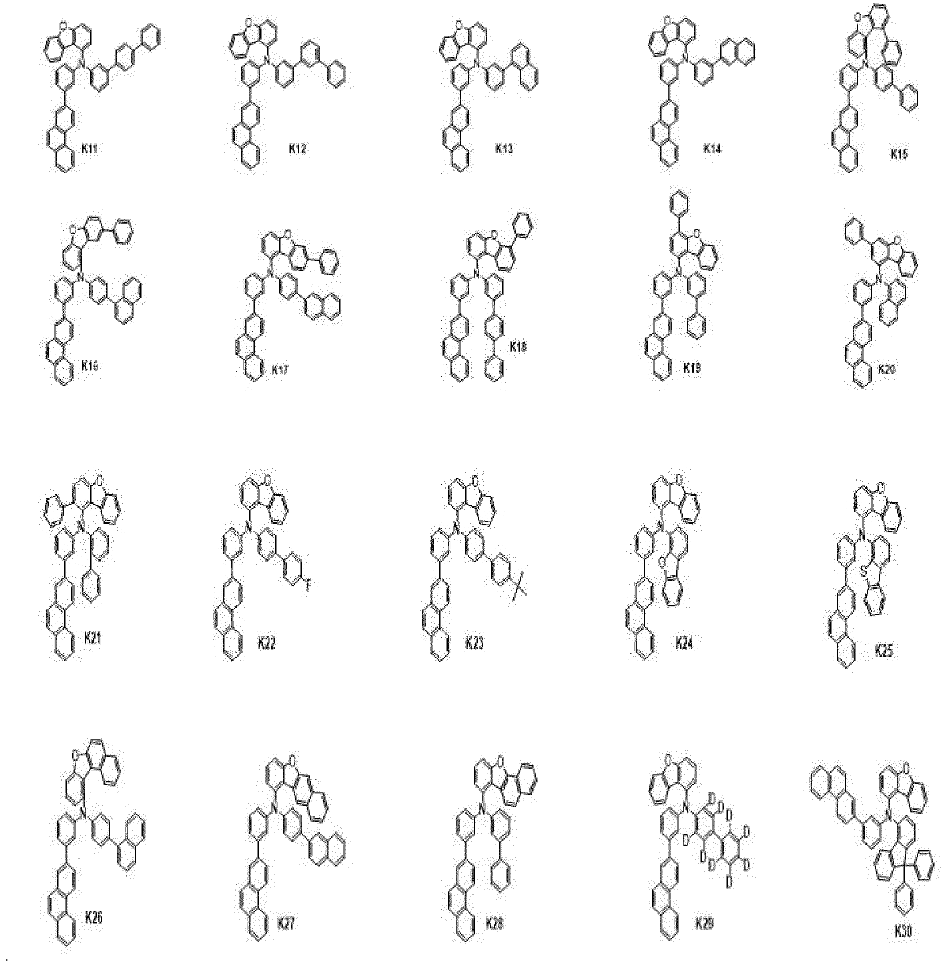

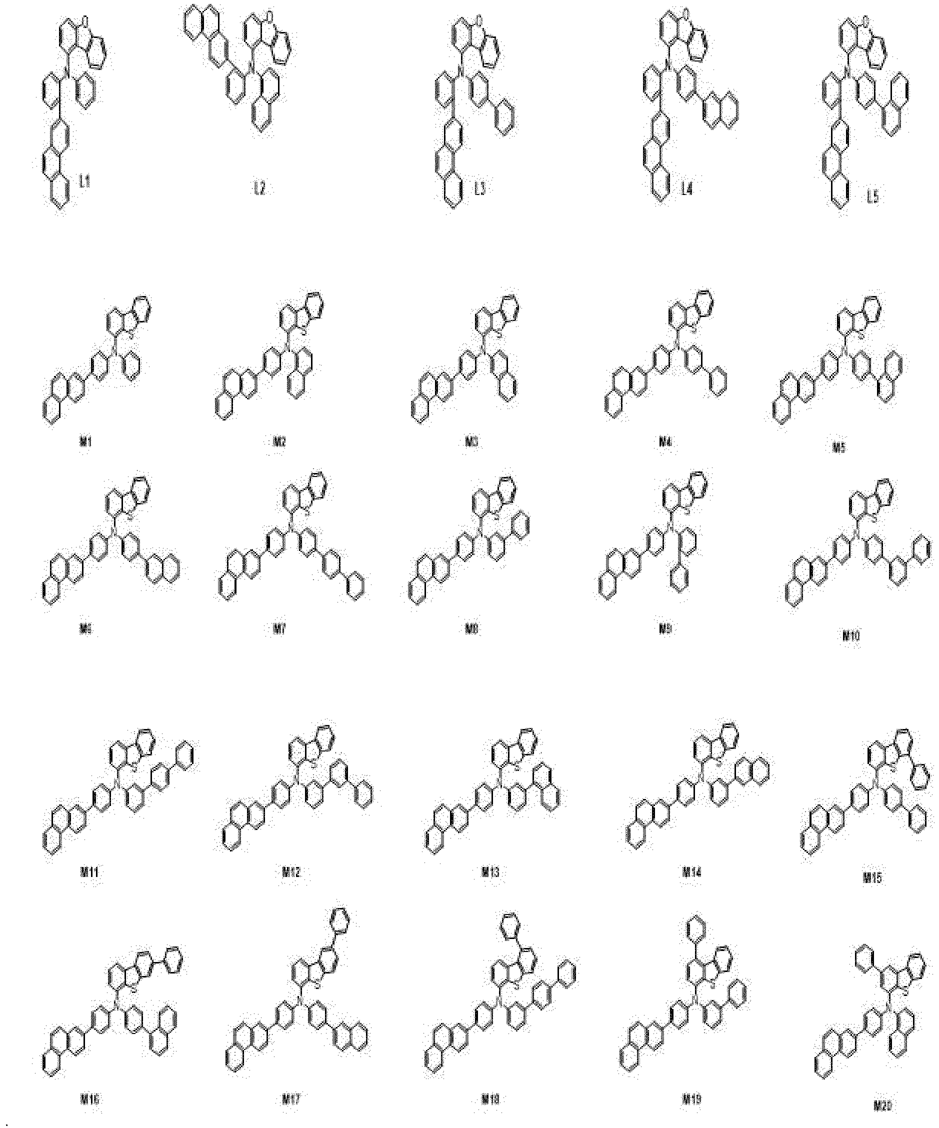

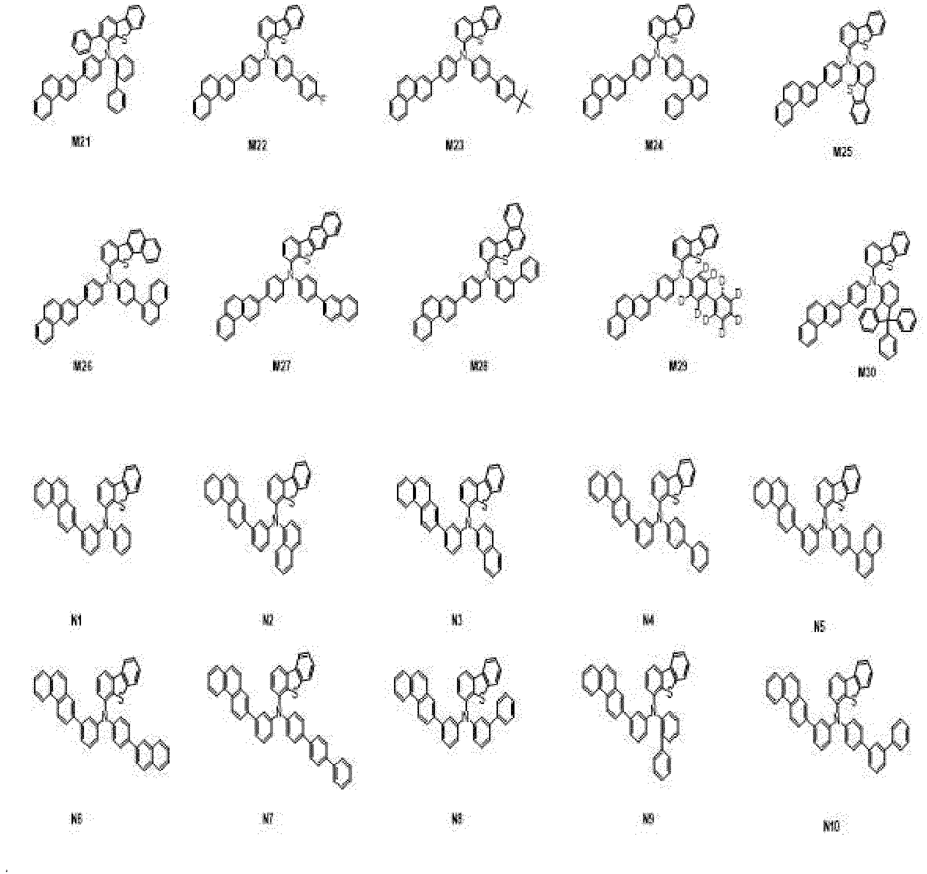

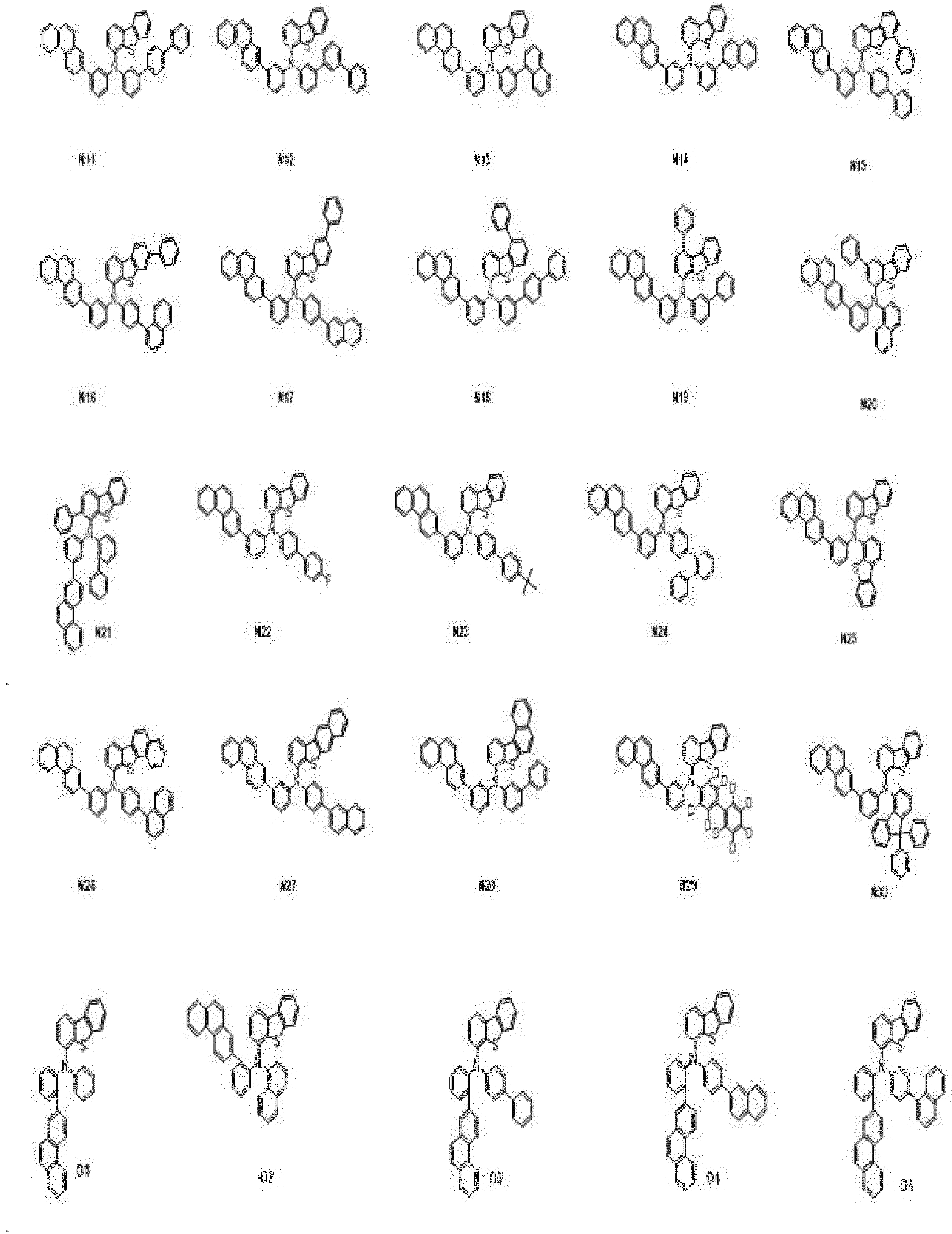

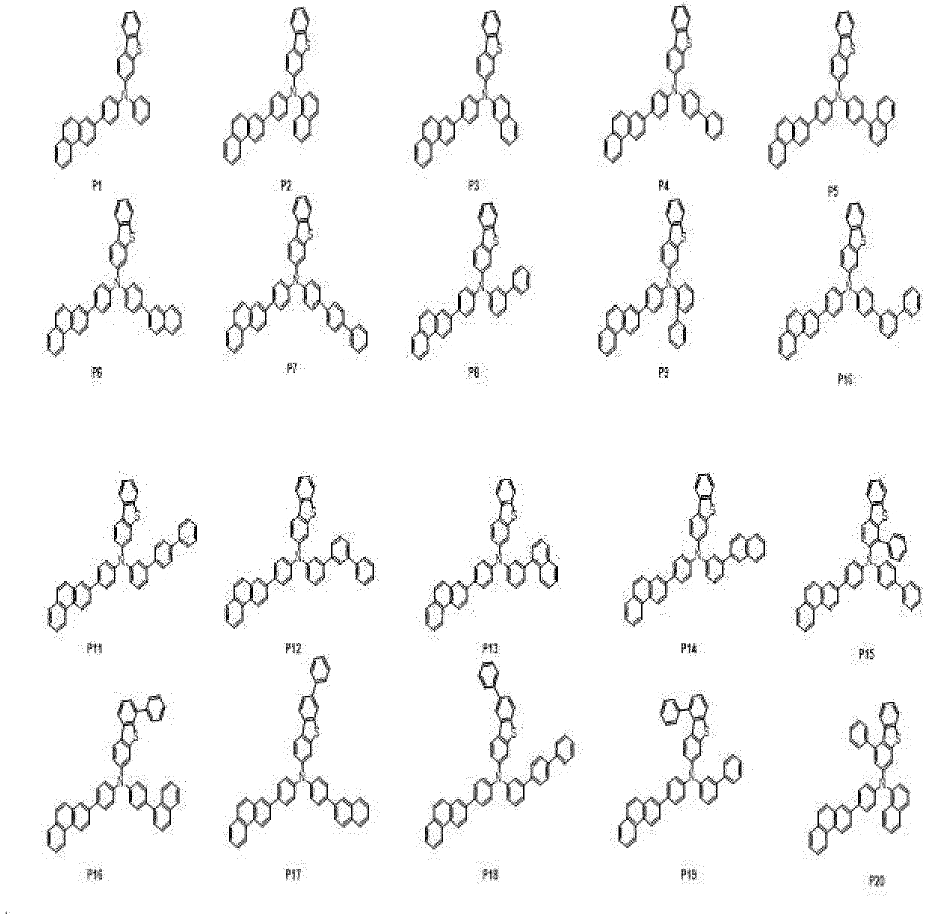

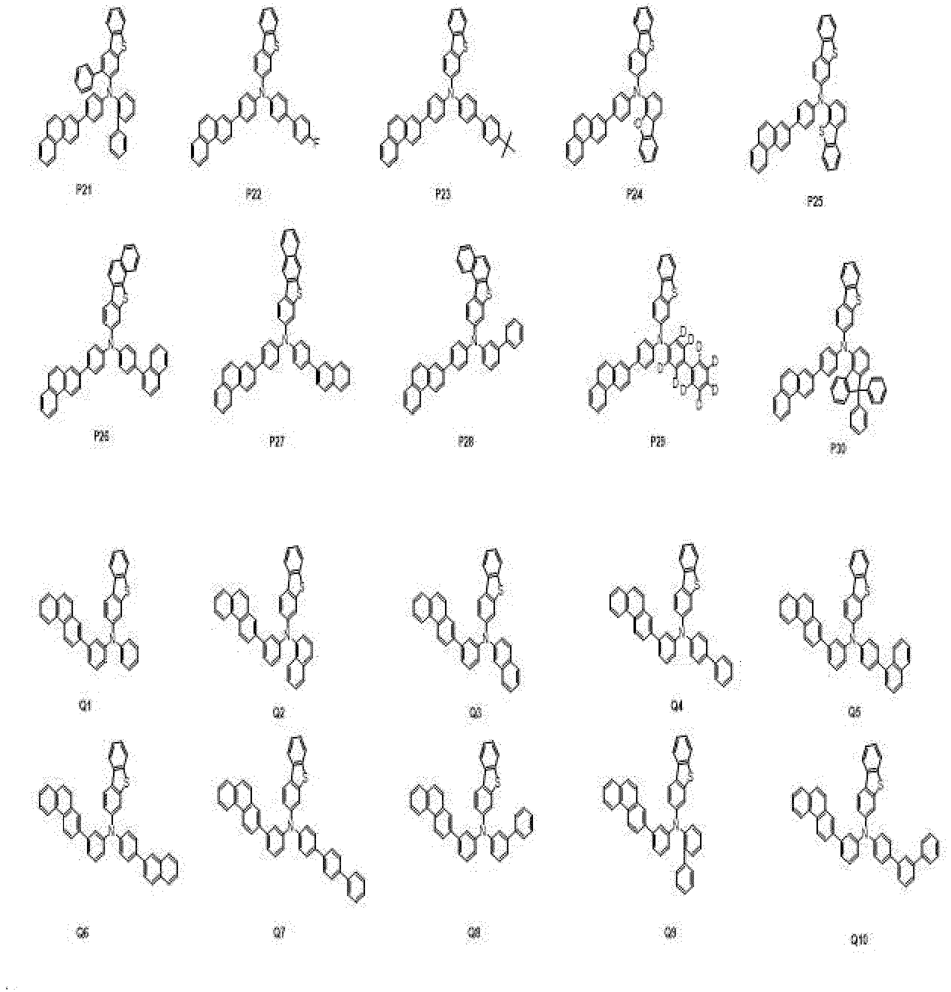

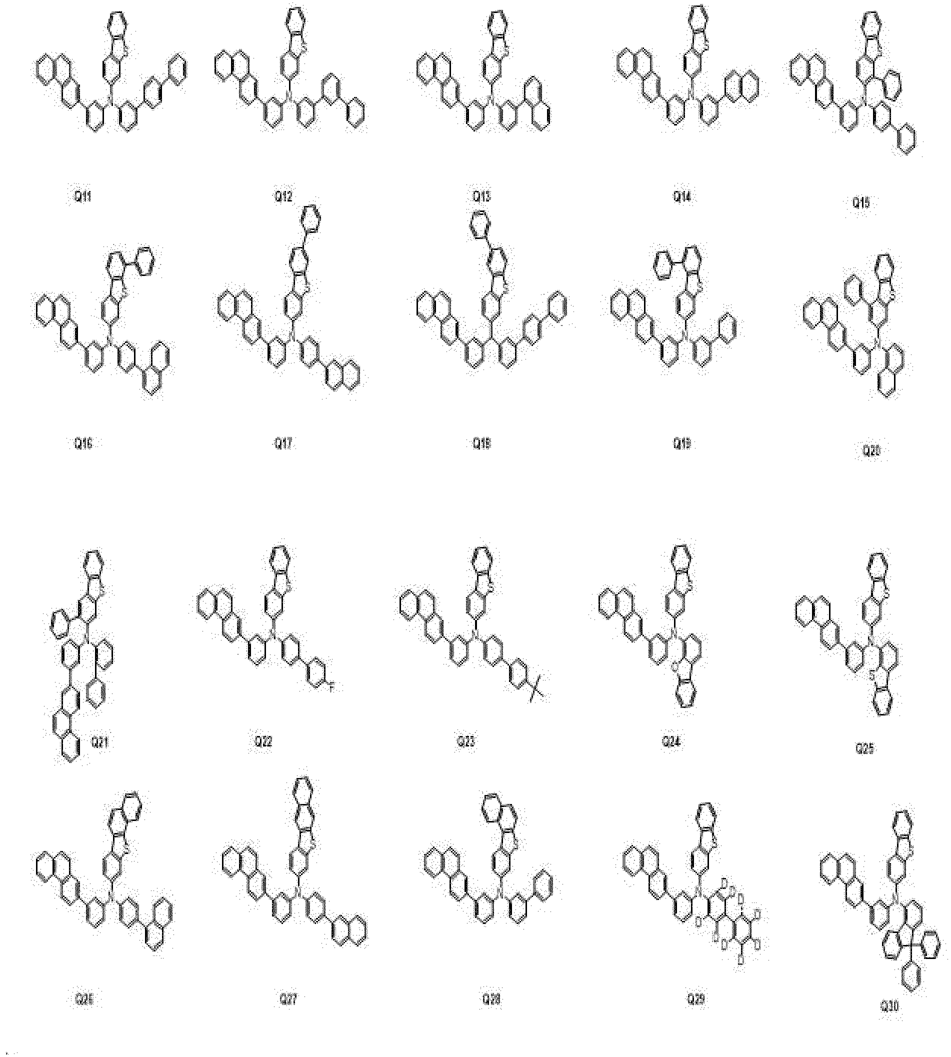

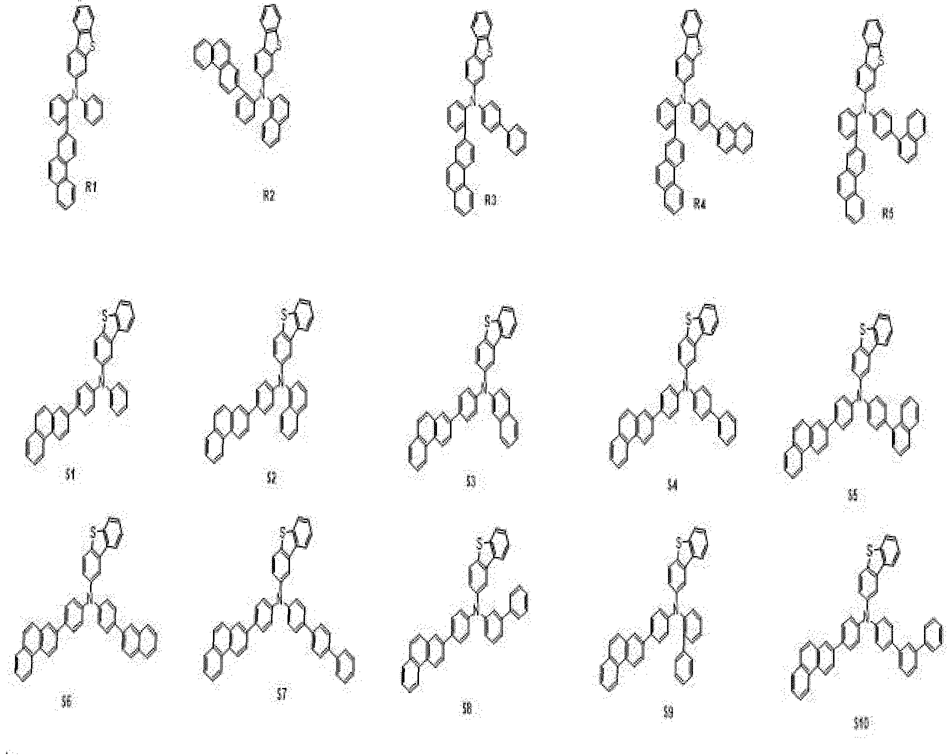

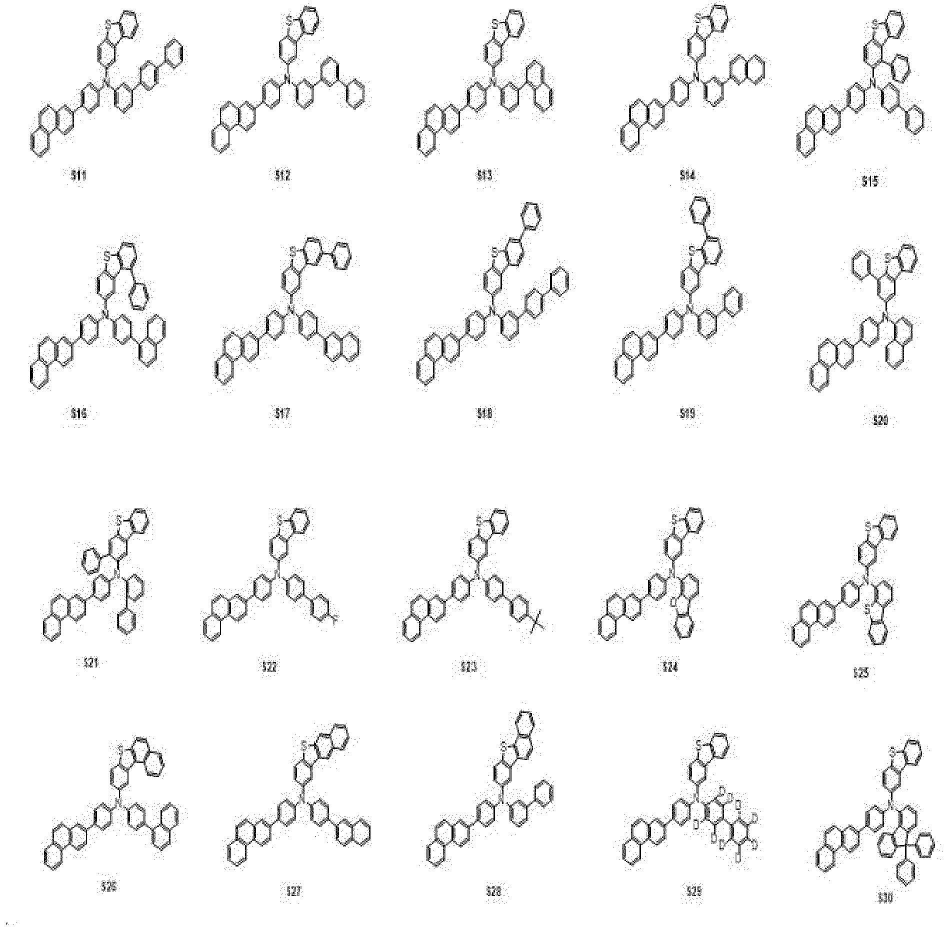

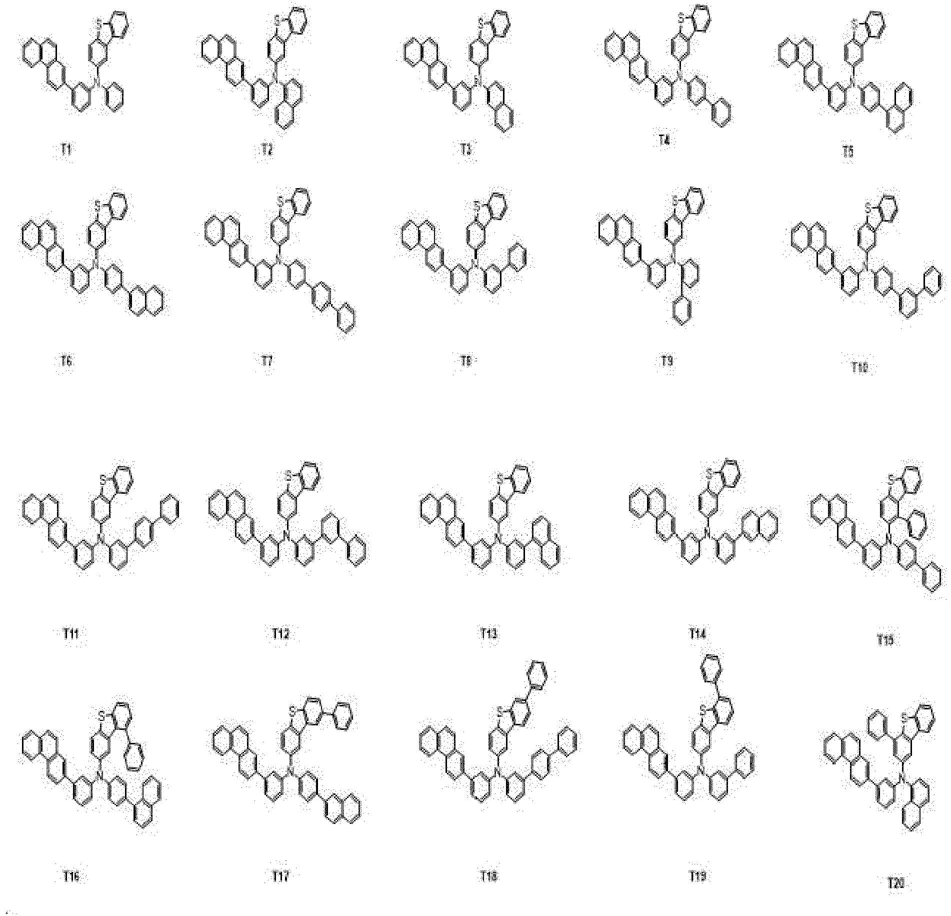

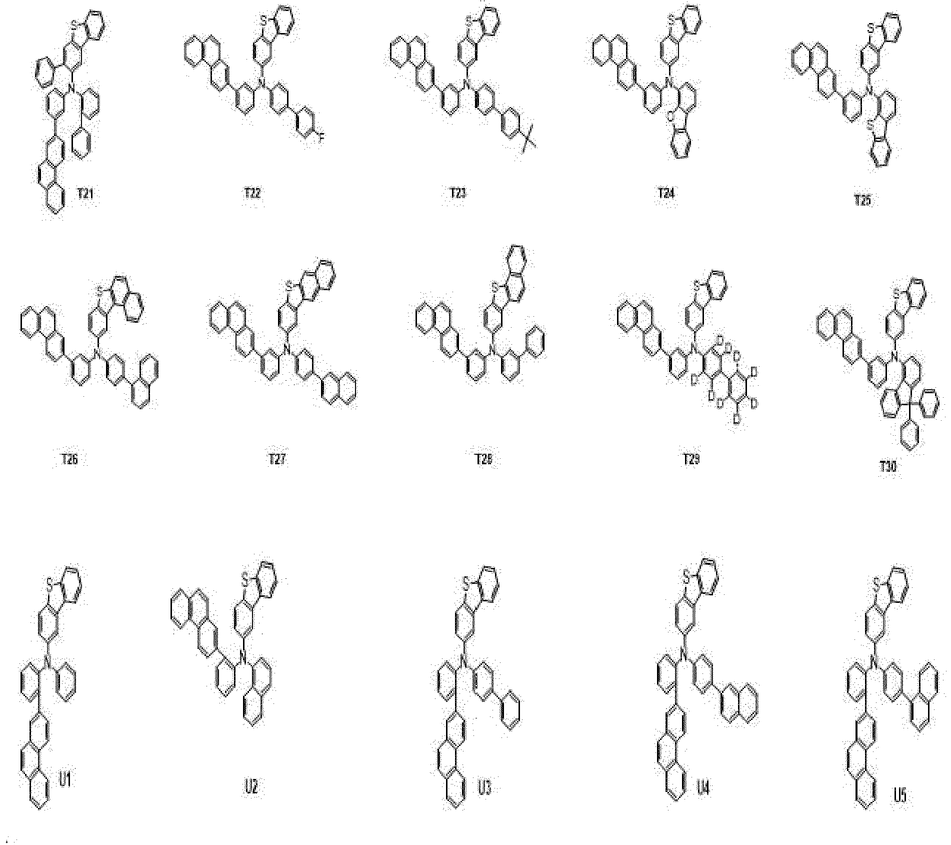

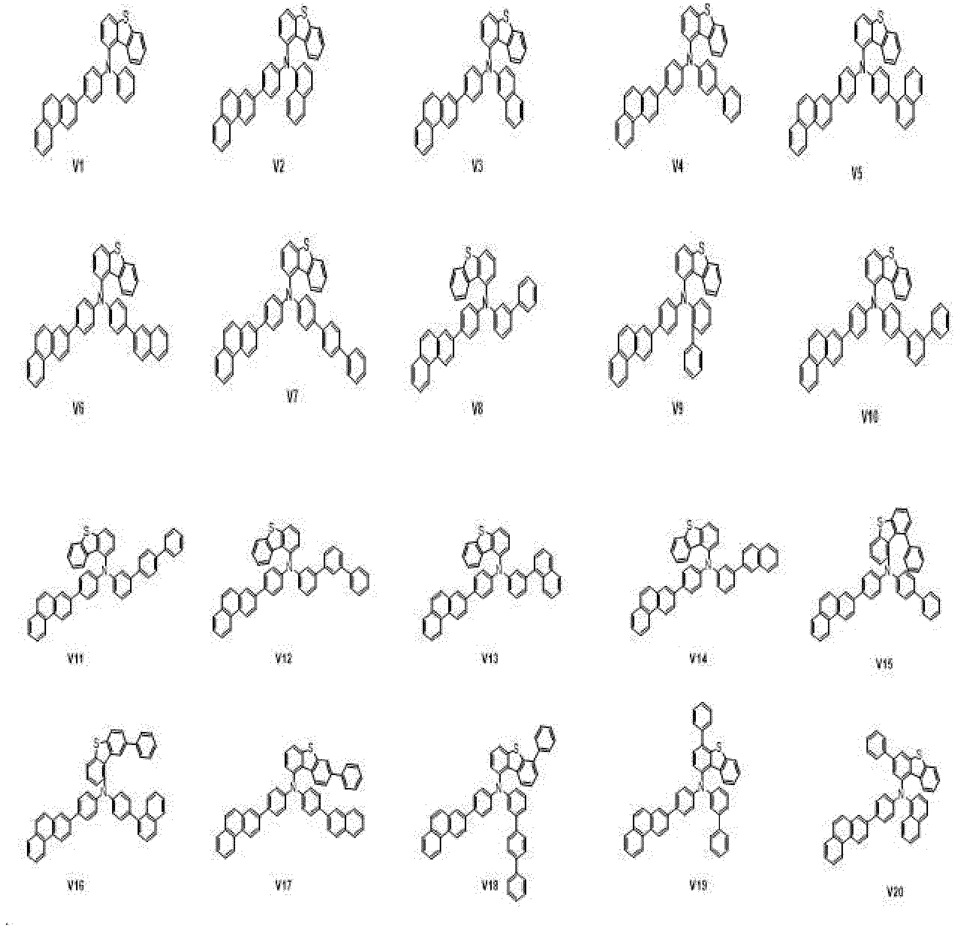

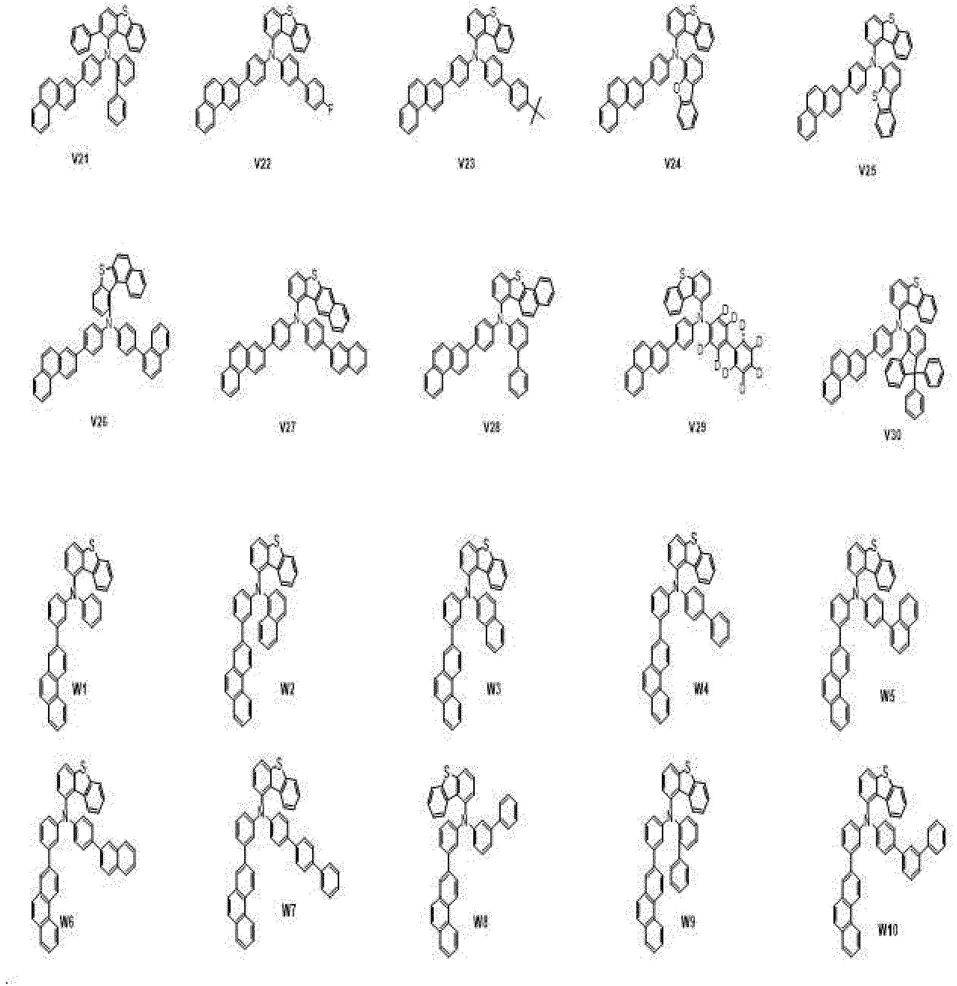

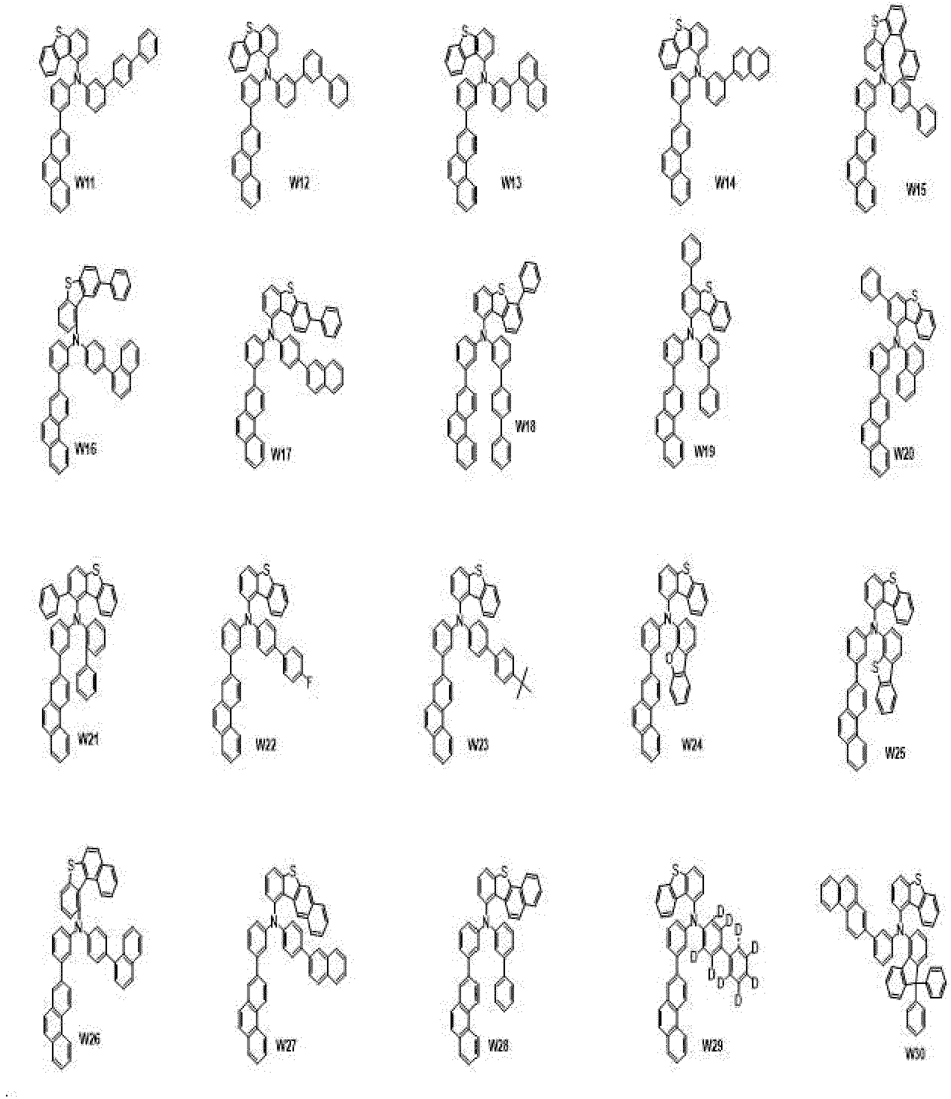

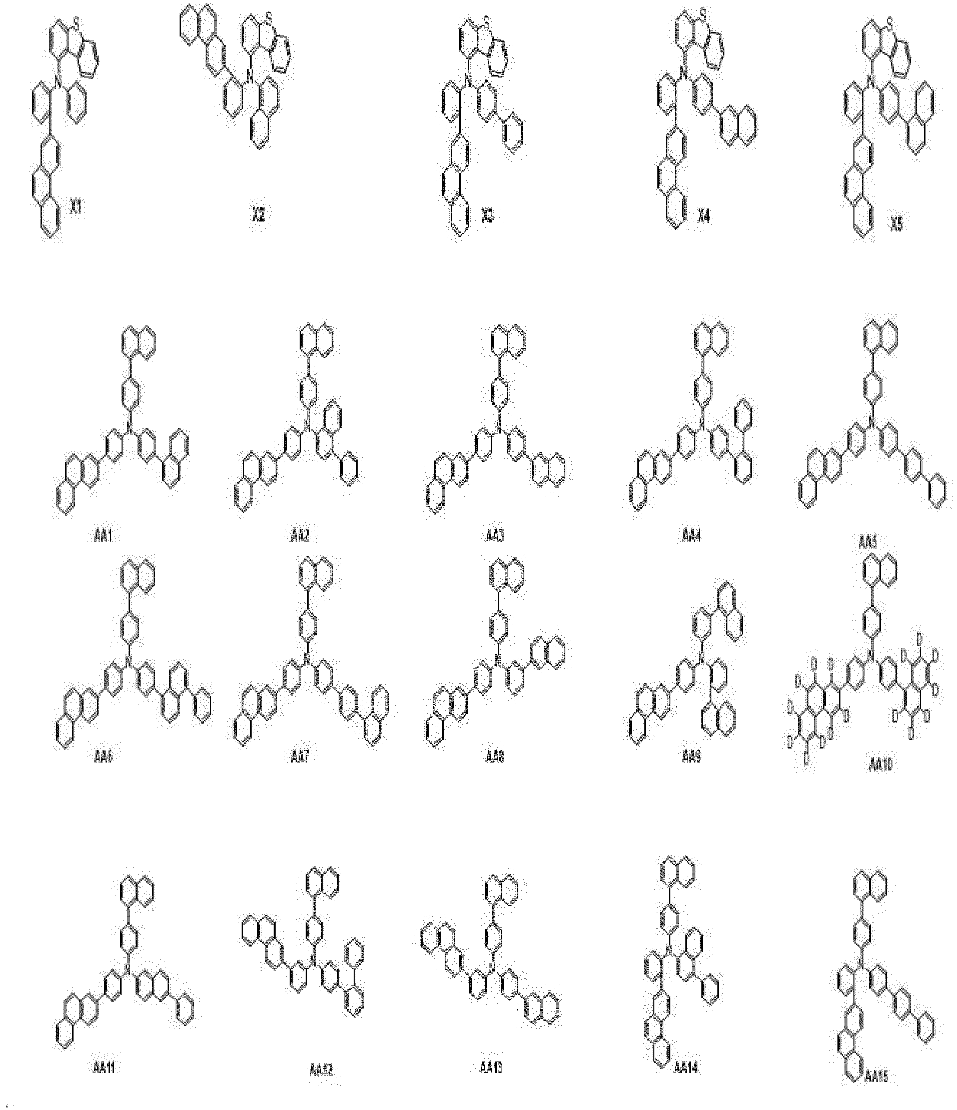

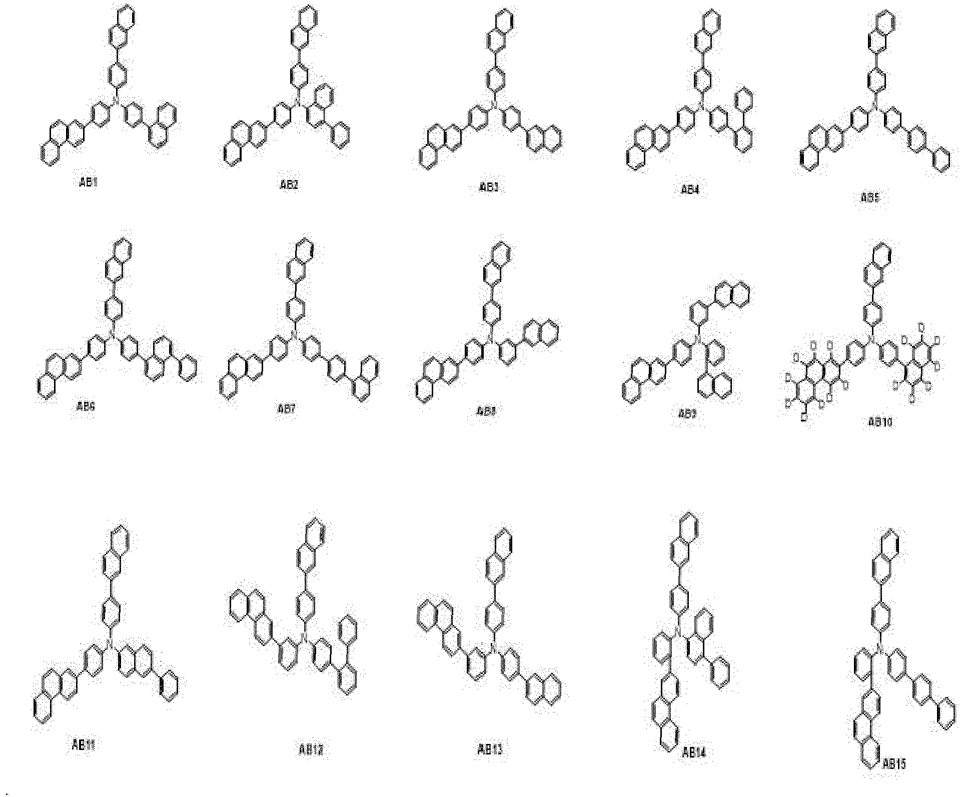

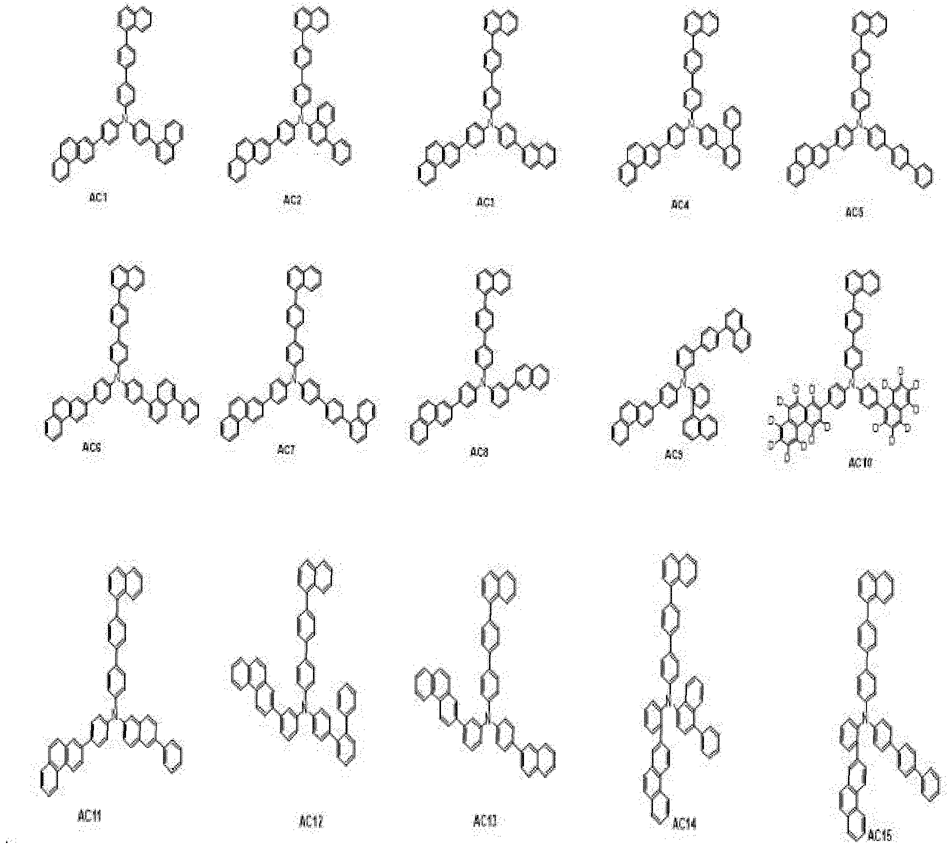

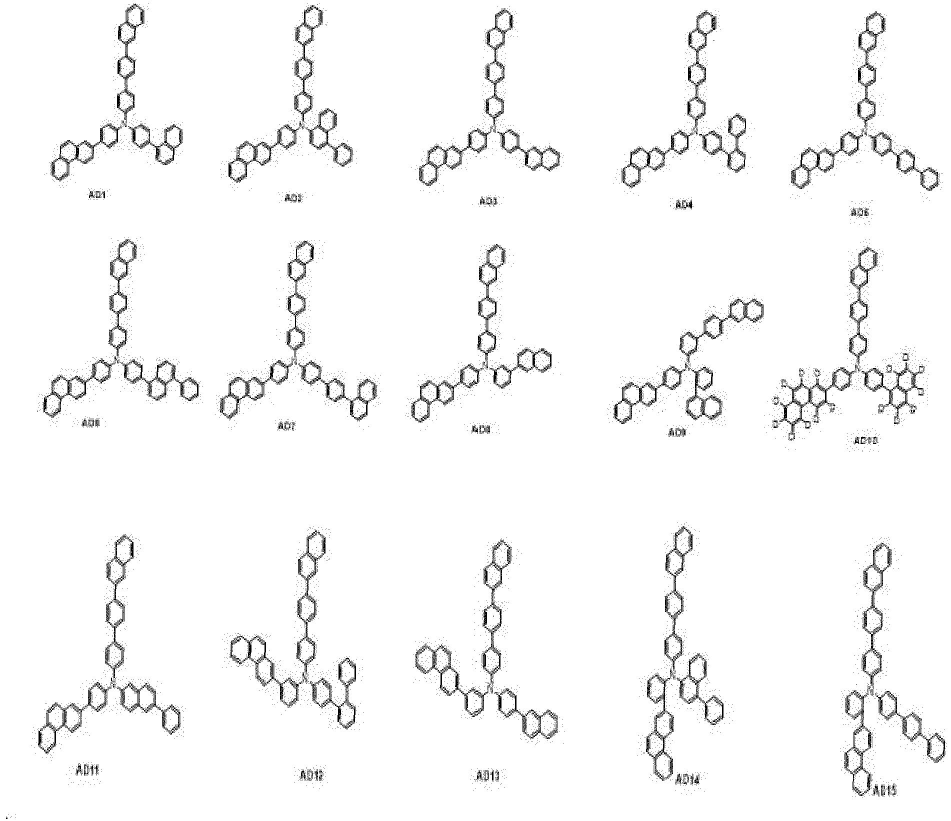

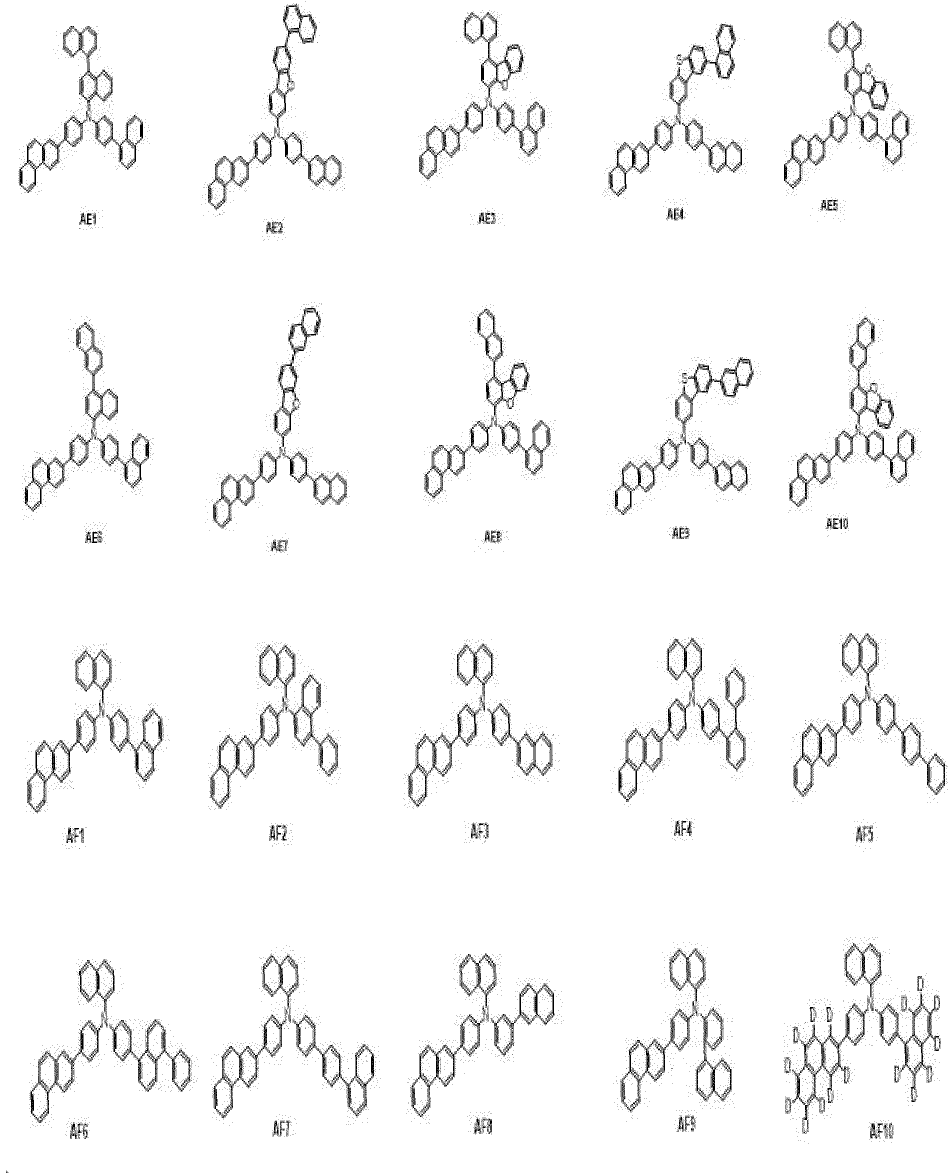

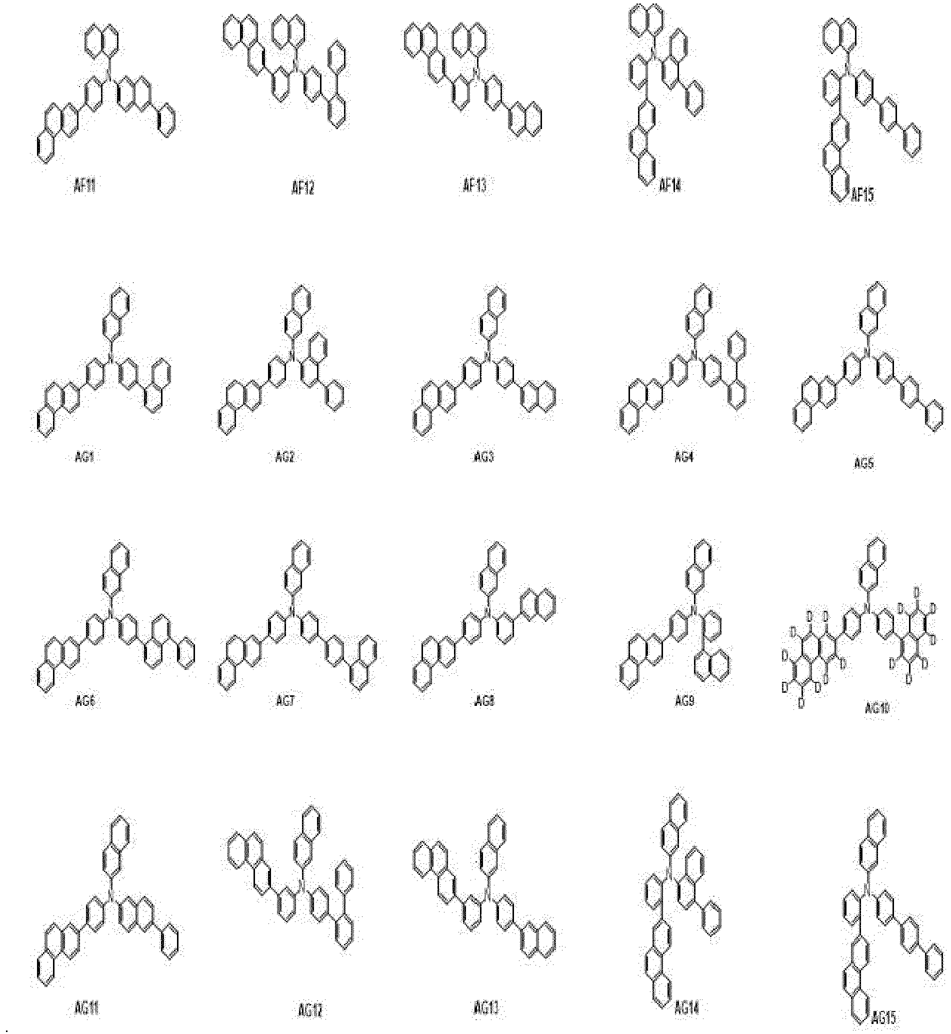

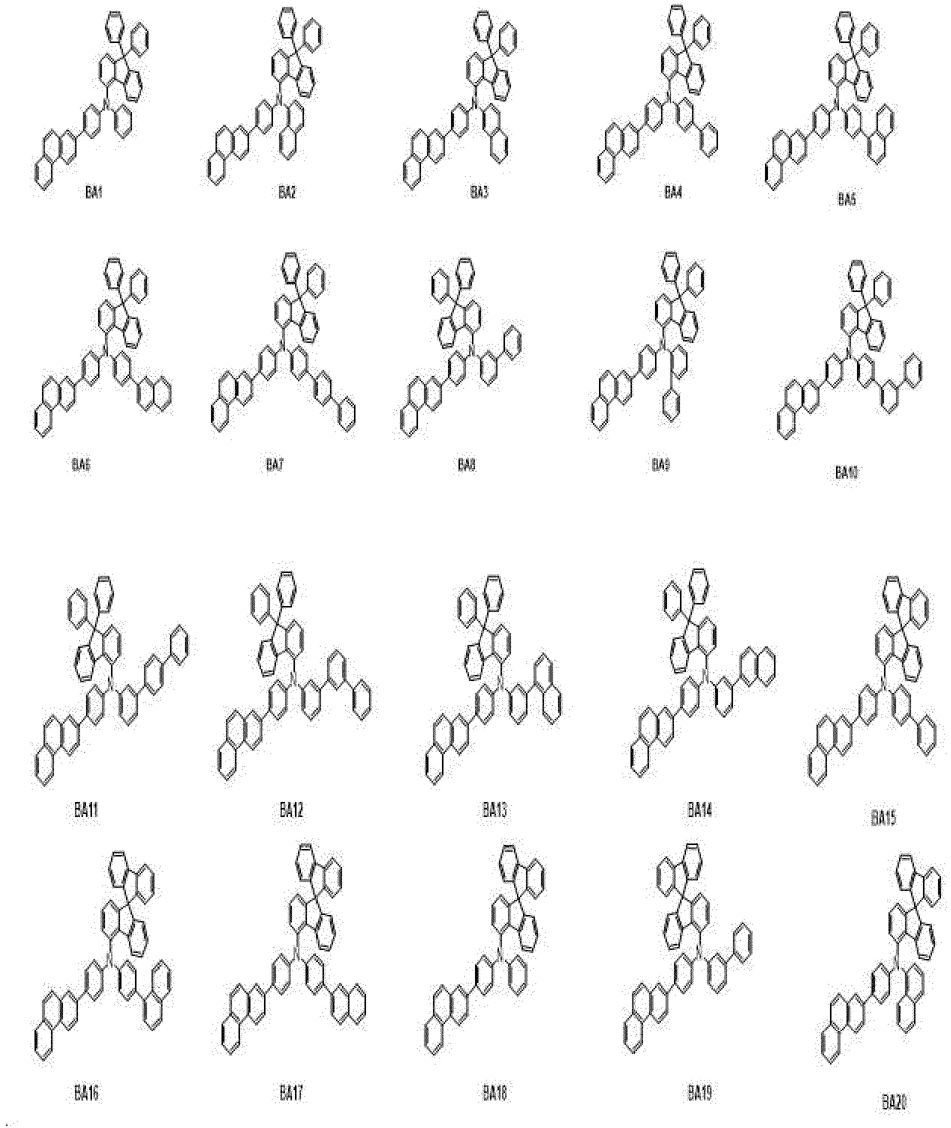

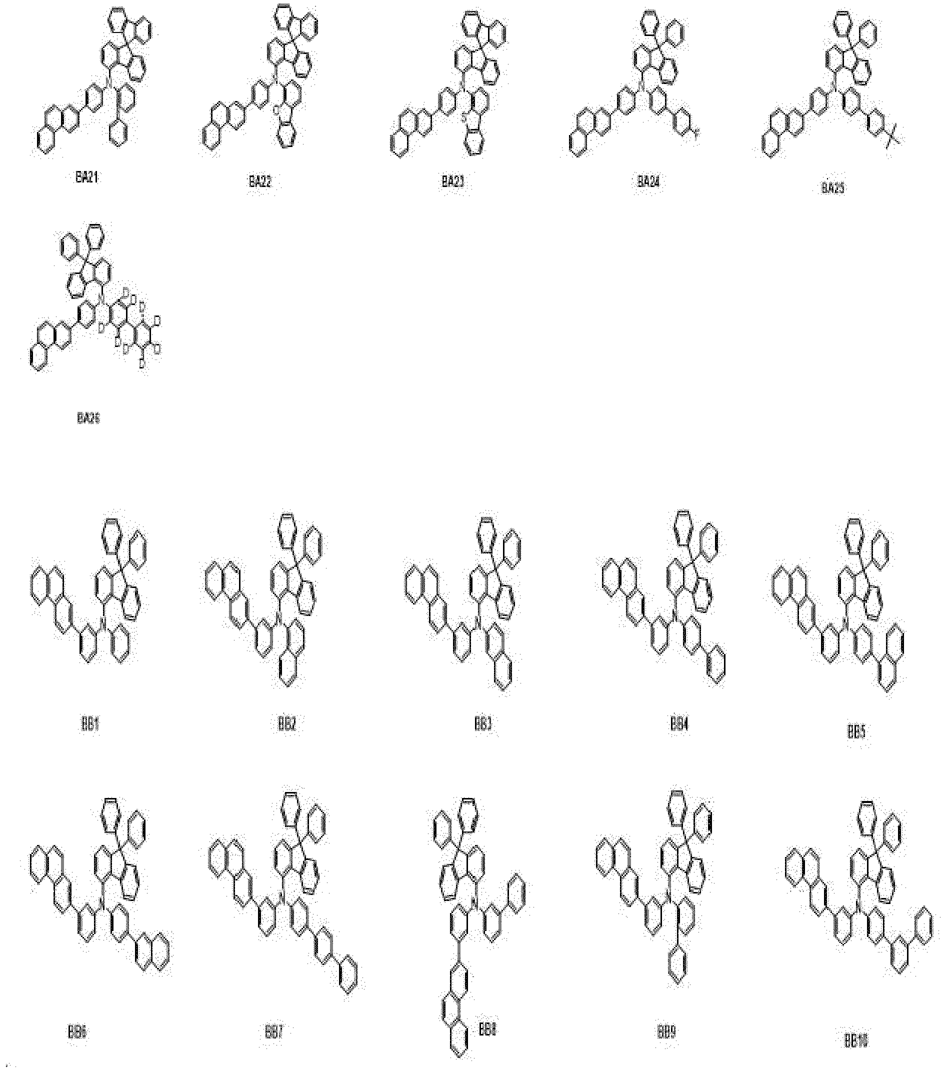

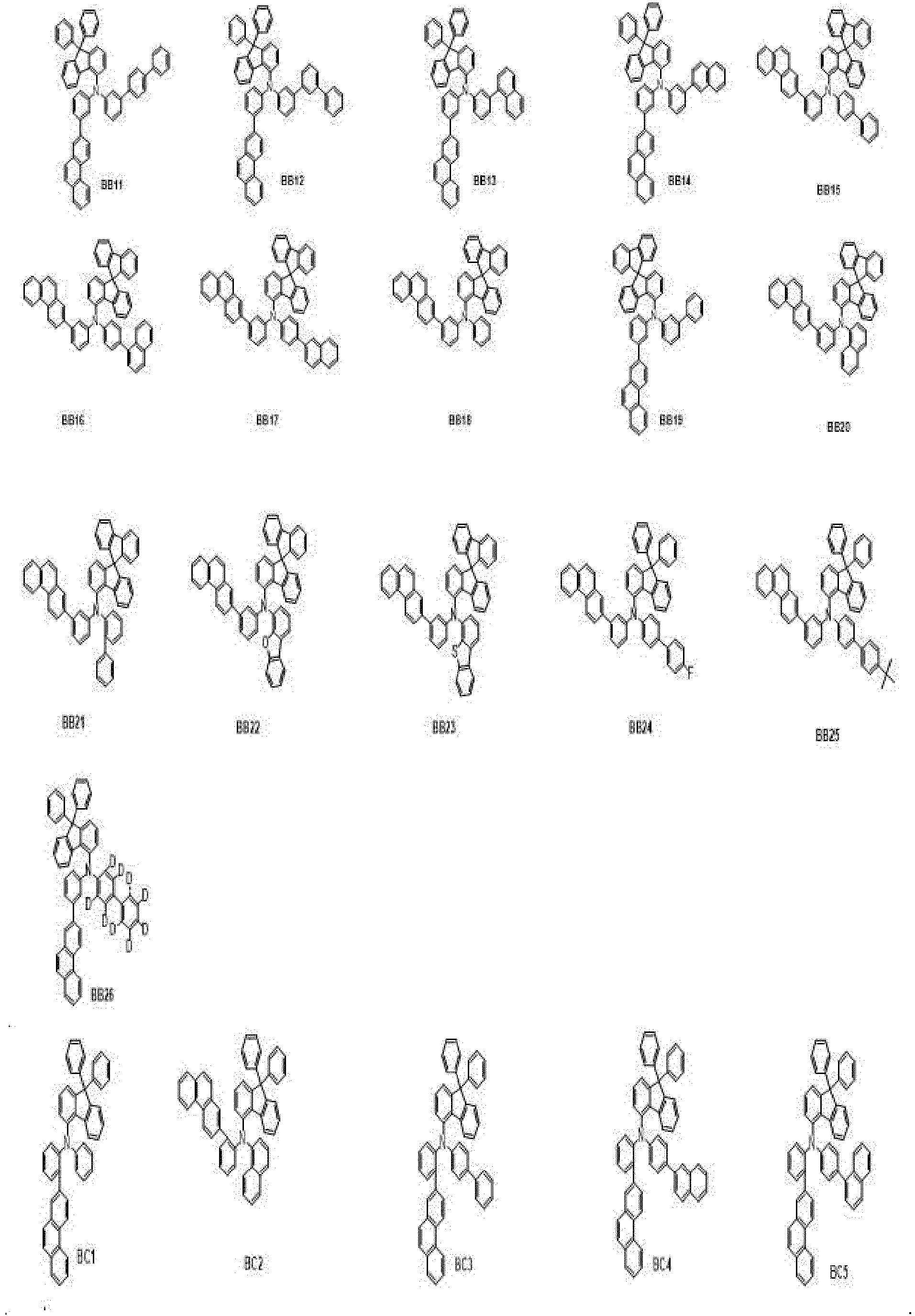

14. The luminescence device of claim 1, wherein the amine compound represented by Formula 1 is at least one selected from compounds represented by Compound Group 2:

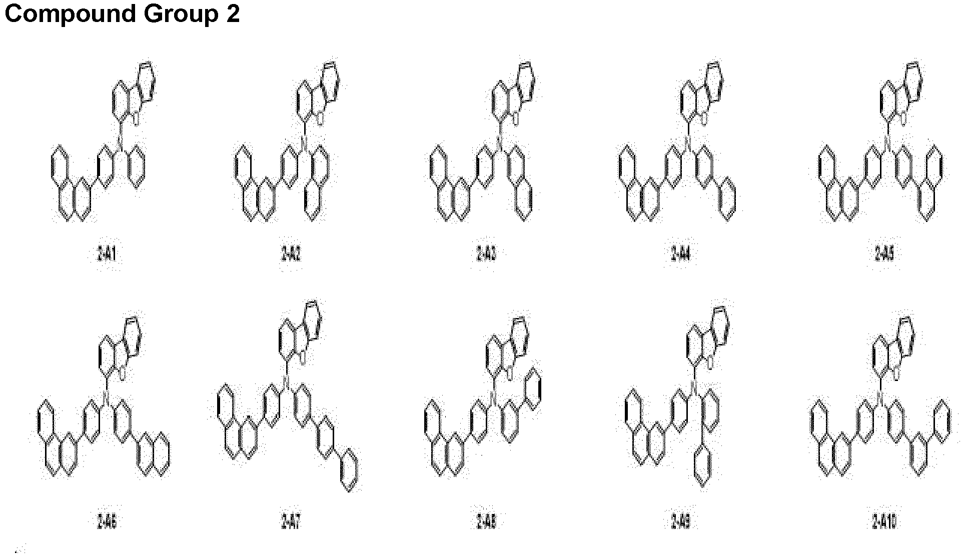

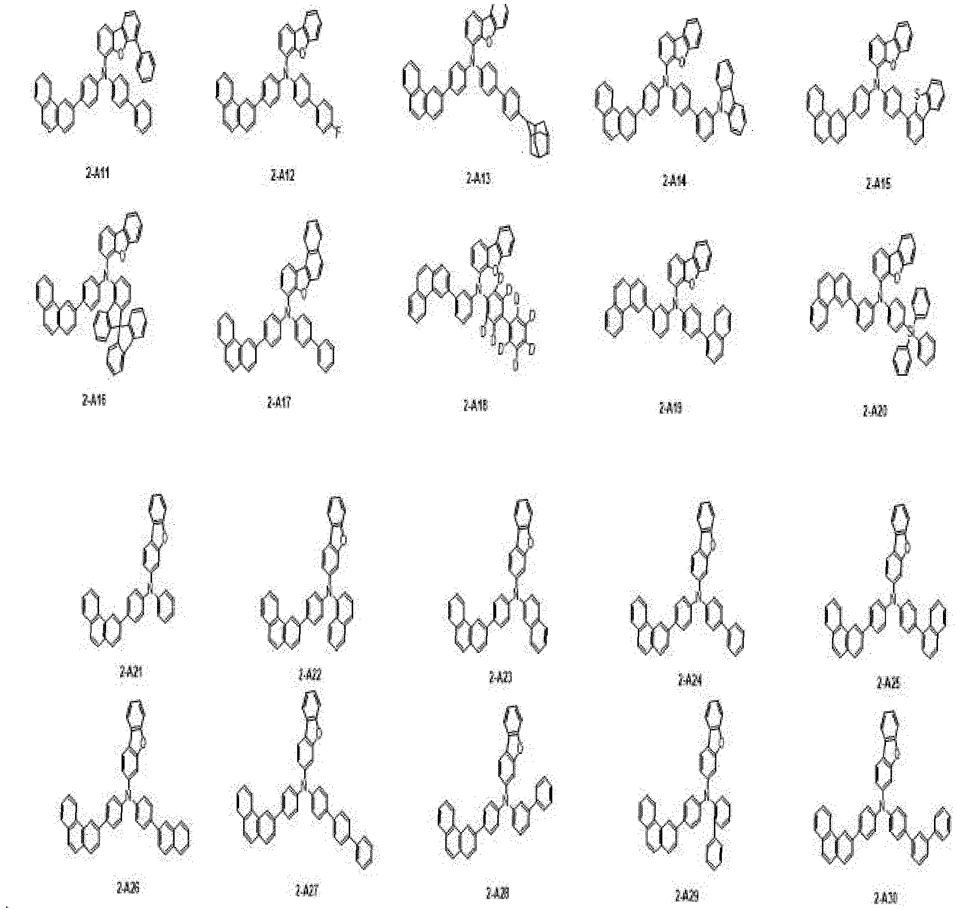

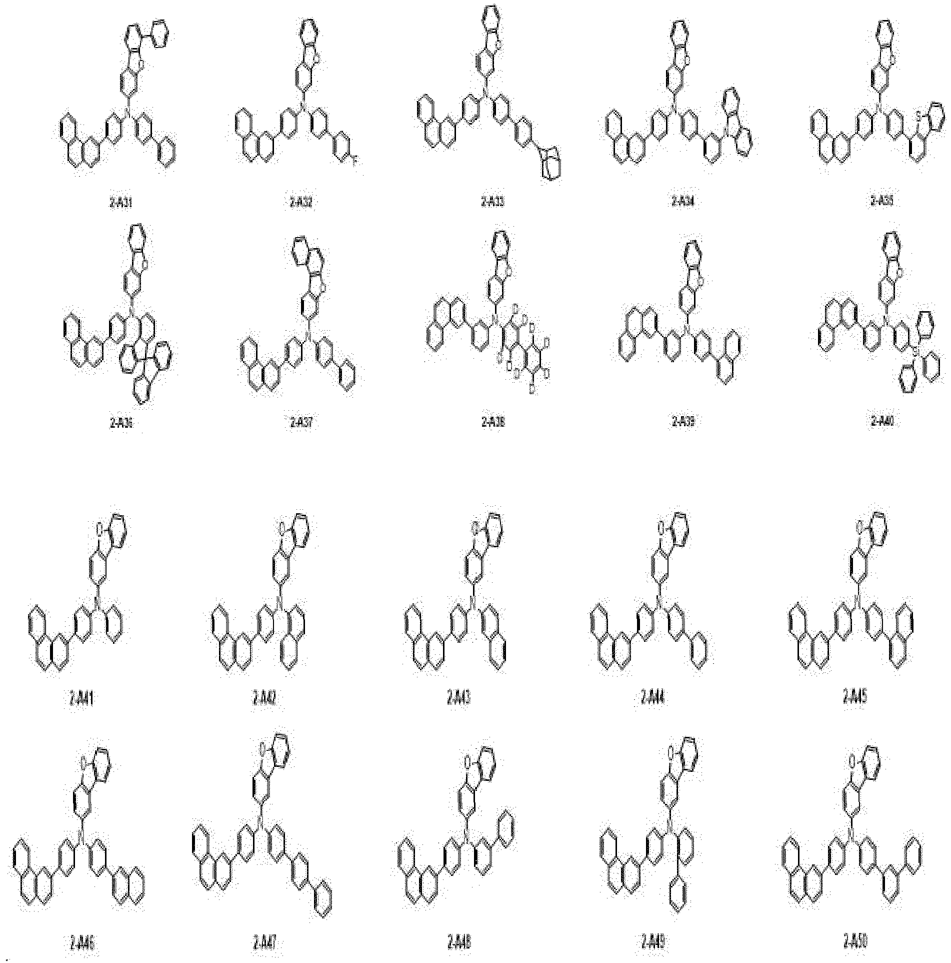

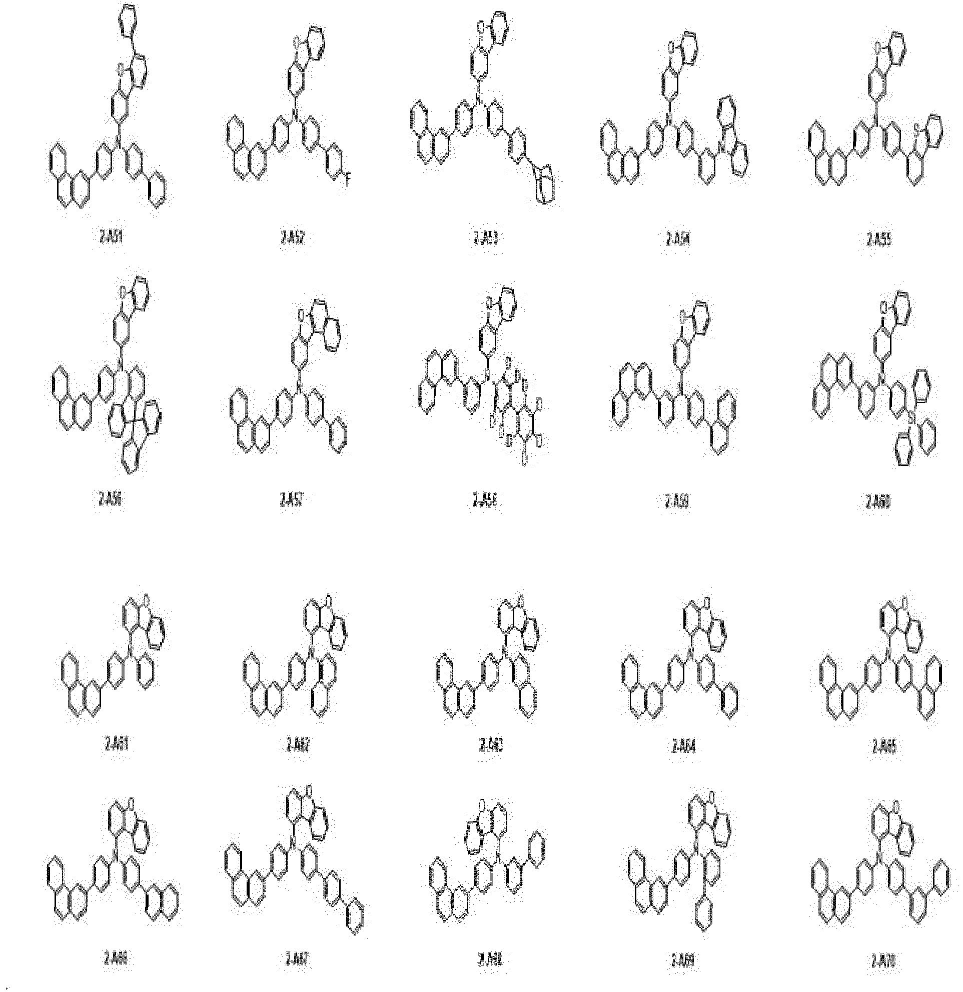

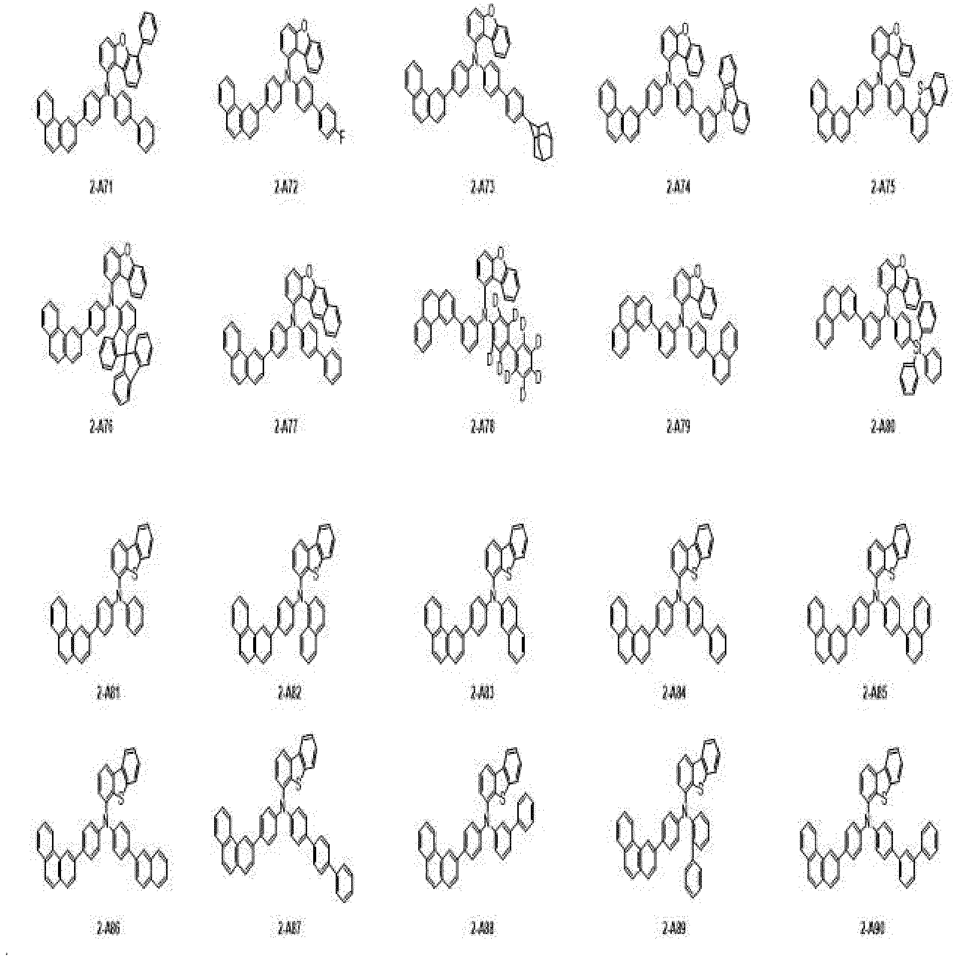

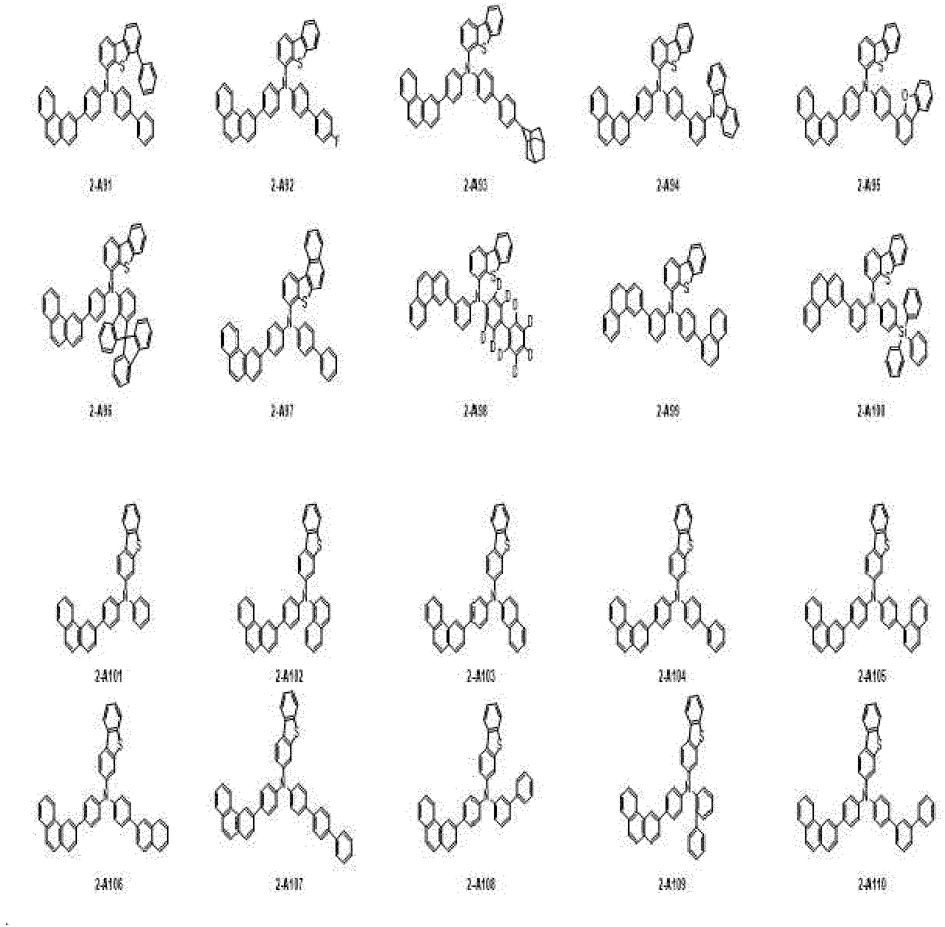

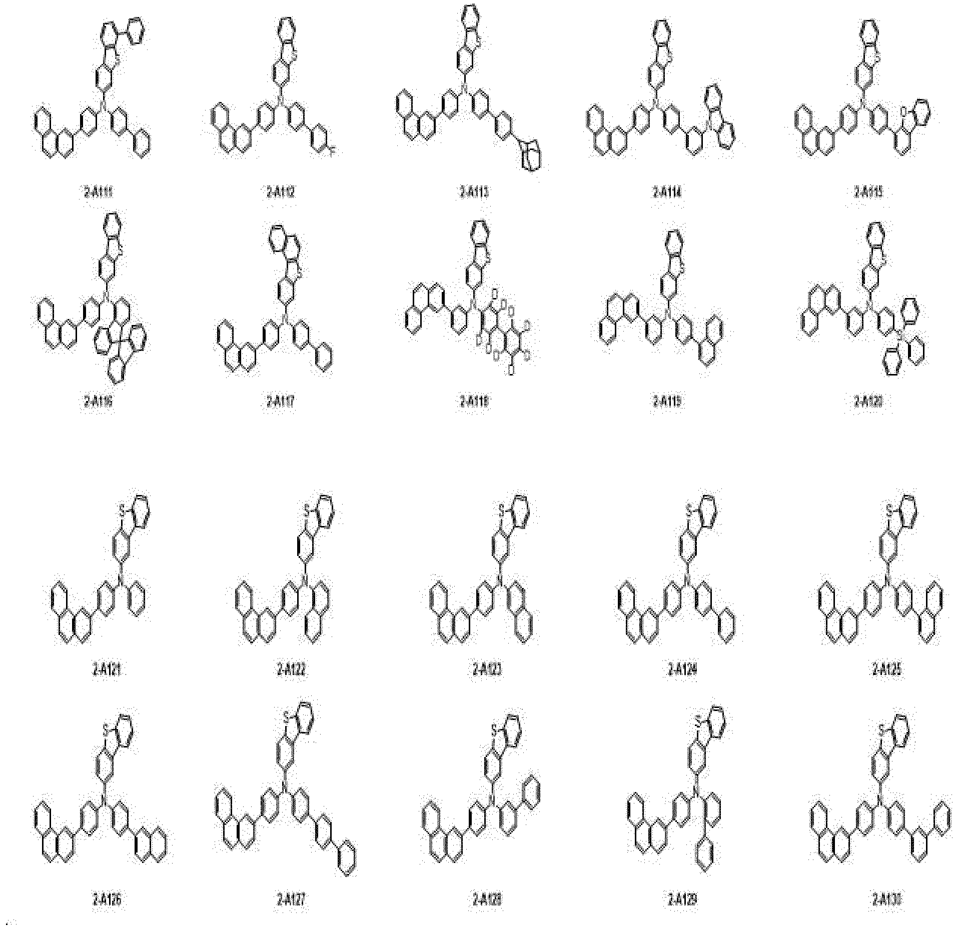

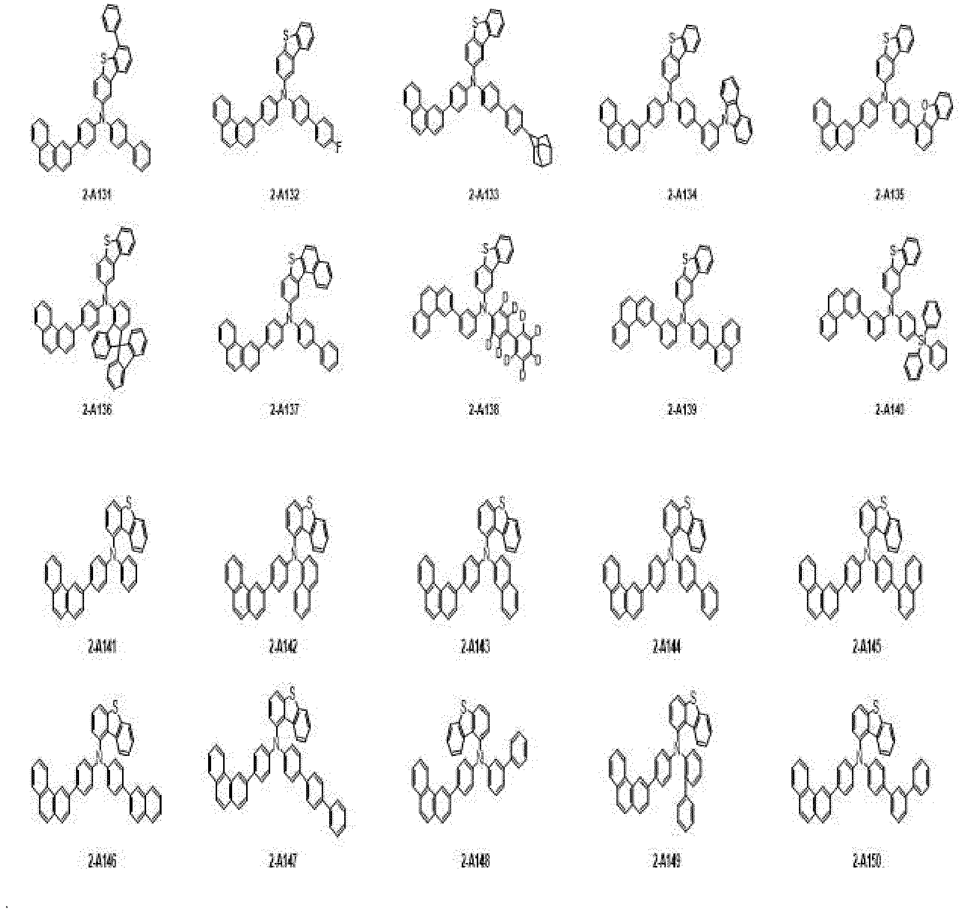

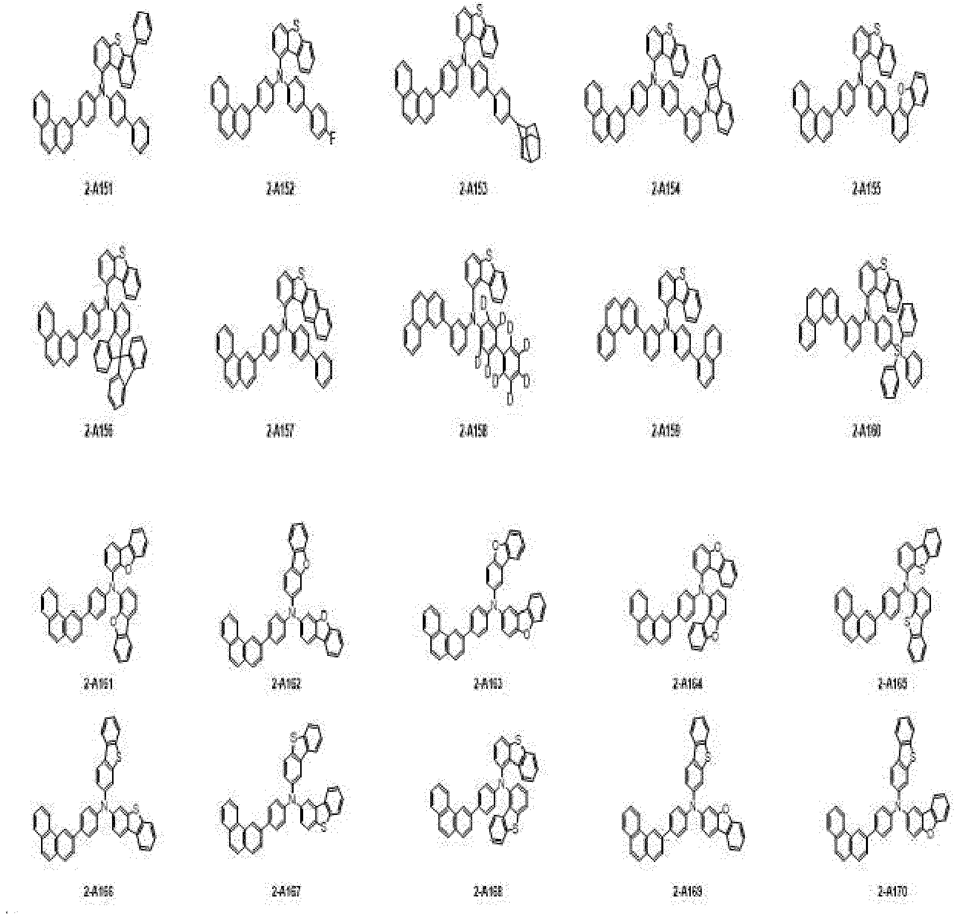

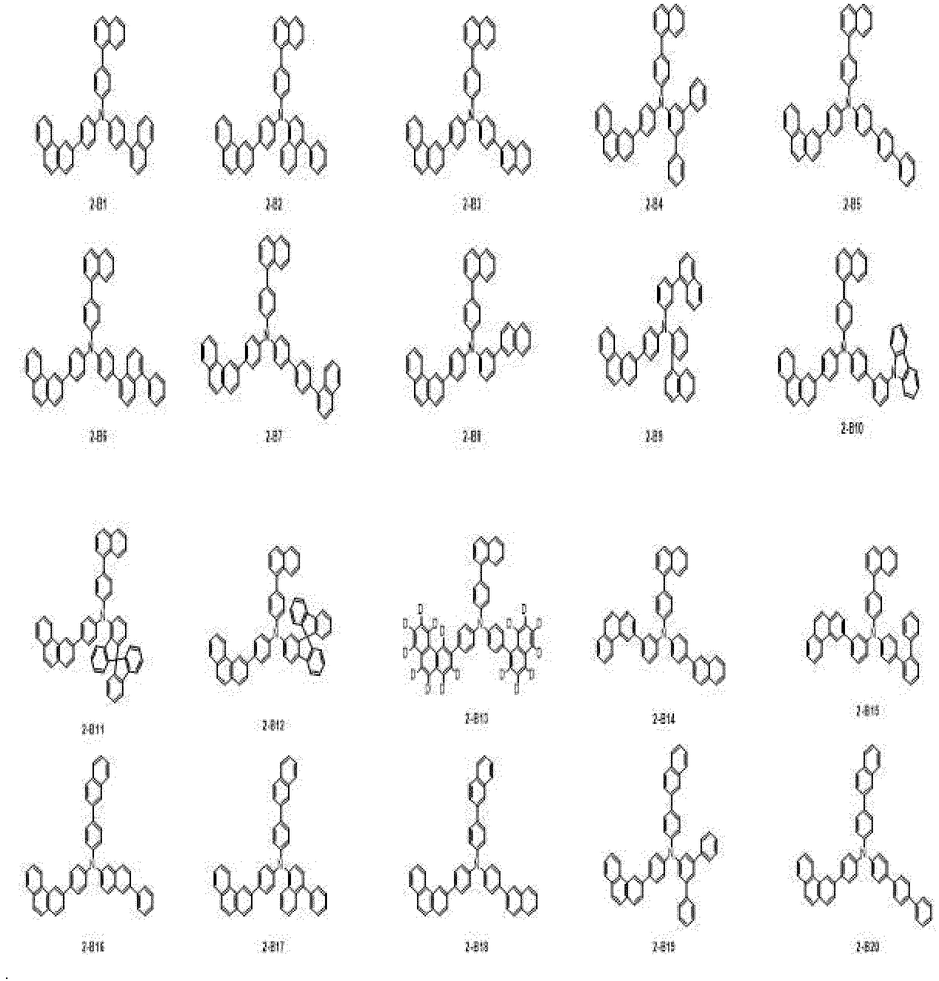

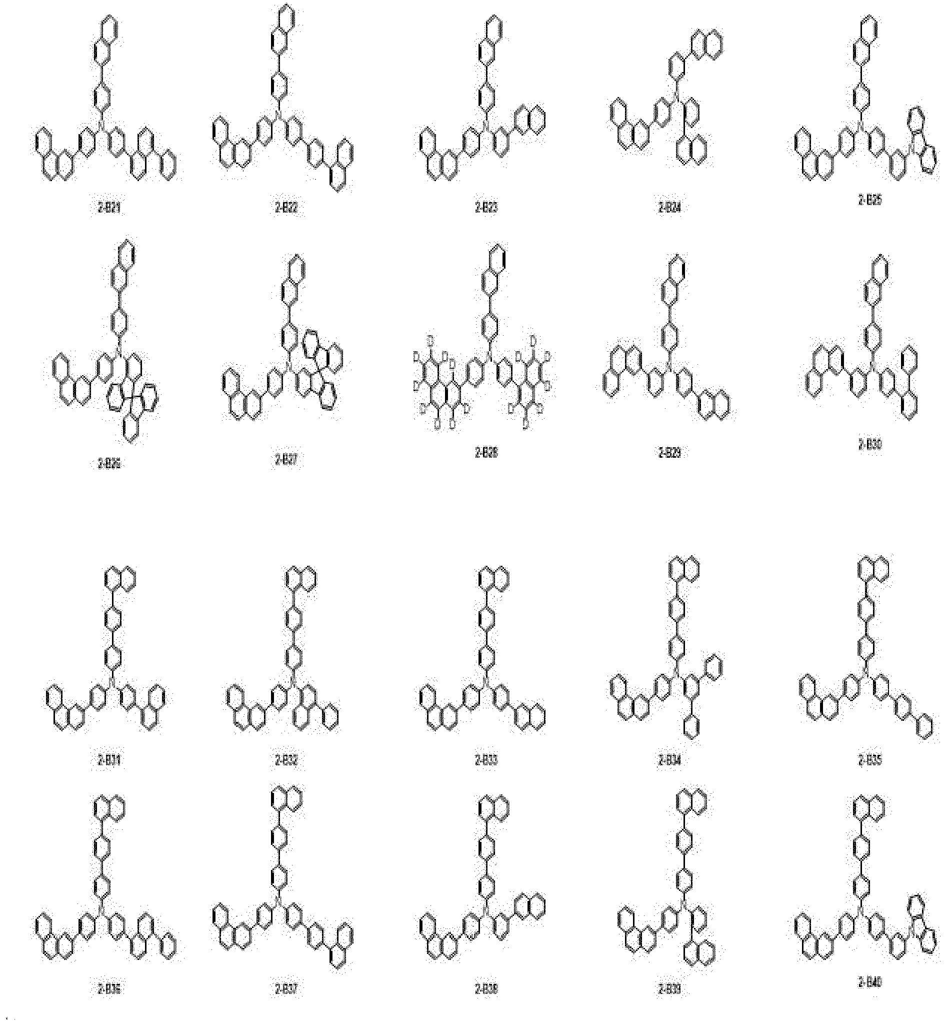

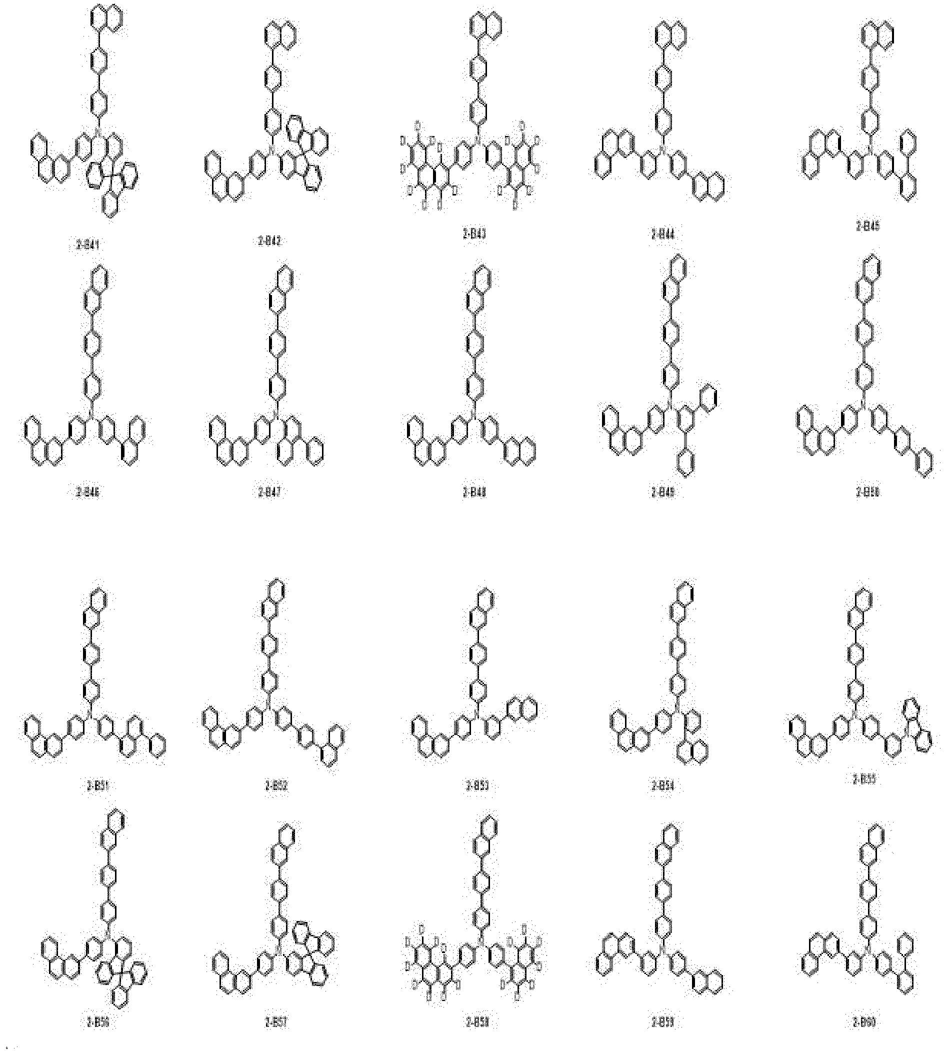

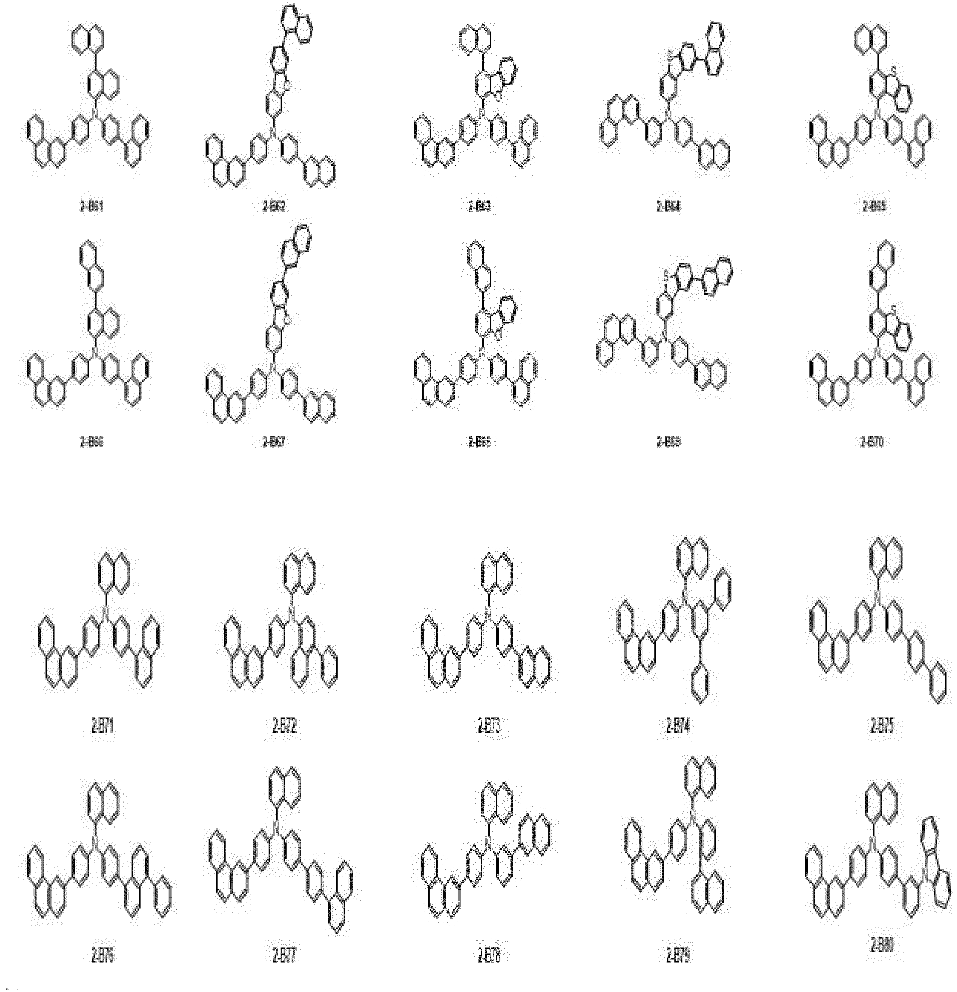

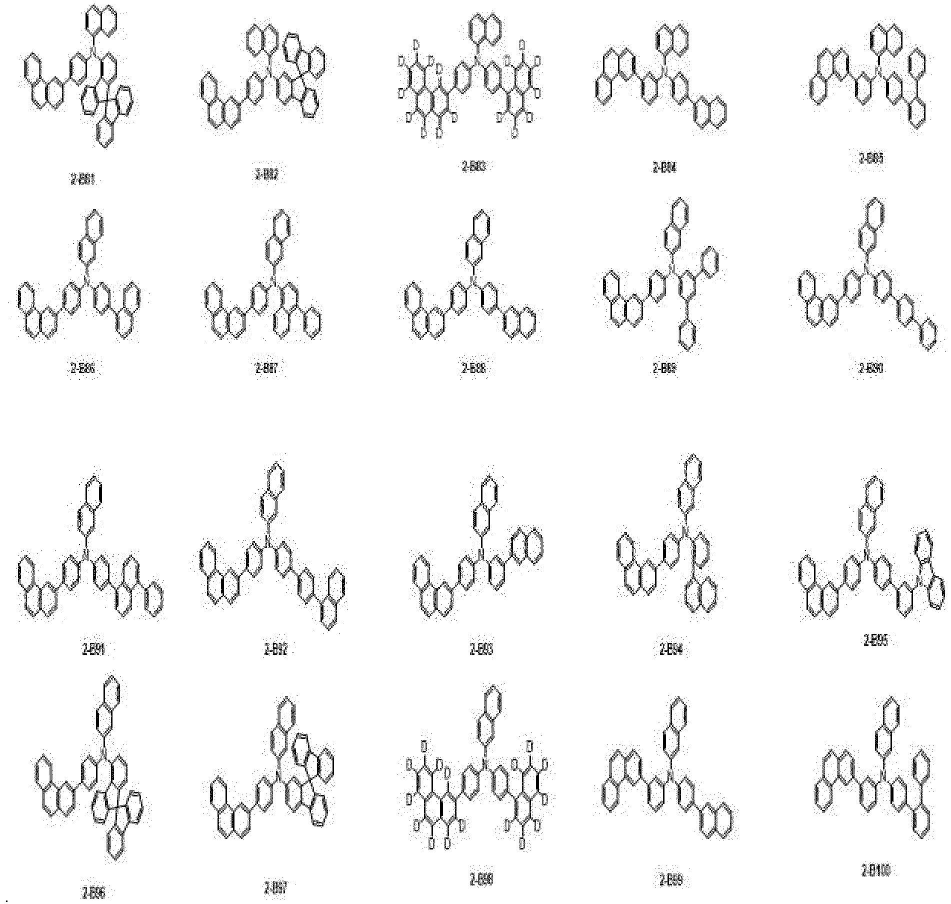

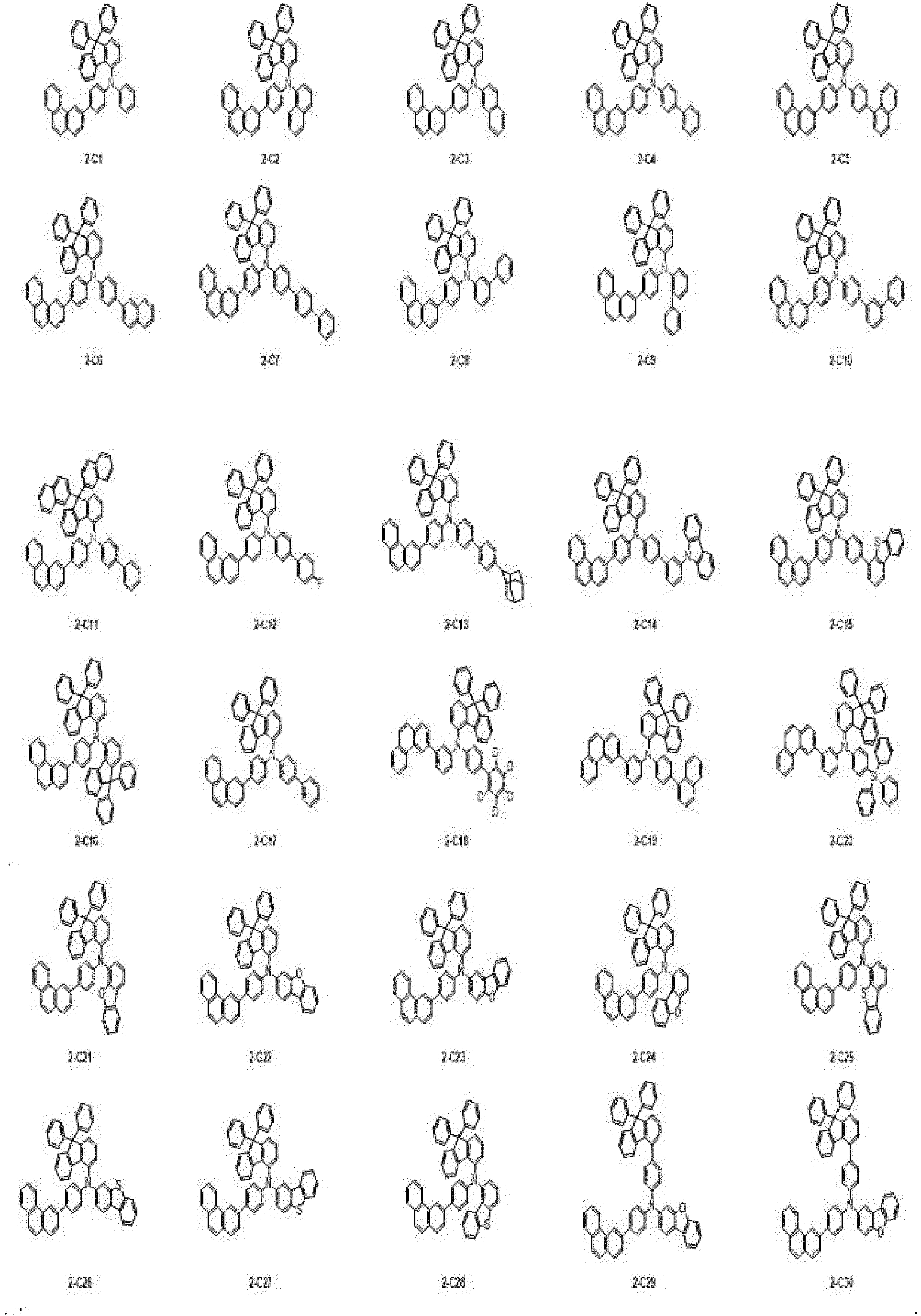

15. An amine compound represented by Formula 1: 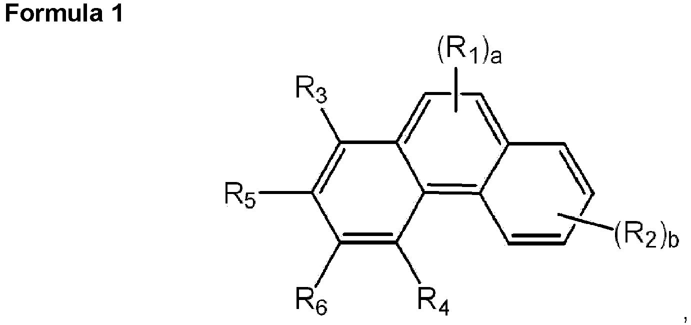 wherein, in Formula 1,
R₁ to R₄ are each independently a hydrogen atom, a deuterium atom, a halogen atom, or a substituted or unsubstituted alkyl group having 1 to 20 carbon atoms,
a is an integer of 0 to 2,
b is an integer of 0 to 4, and
one of R₅ or R₆ is represented by Formula 2, and the other is a hydrogen atom, a deuterium atom, a halogen atom, or a substituted or unsubstituted alkyl group having 1 to 20 carbon atoms: 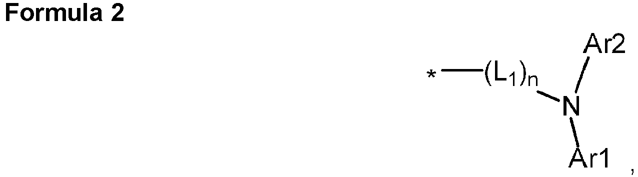 wherein, in Formula 2,
L₁ is a substituted or unsubstituted arylene group having 6 to 30 ring-forming carbon atoms, or a substituted or unsubstituted heteroarylene group having 2 to 30 ring-forming carbon atoms, and does not comprise a fluorenylene group, a carbazolene group, or an anthracenylene group,
n is 1 or 2,
Ar1 is represented by any one among Formula 3-1 to Formula 3-3,
Ar2 is a substituted or unsubstituted aryl group having 6 to 30 ring-forming carbon atoms, or a substituted or unsubstituted heteroaryl group having 2 to 30 ring-forming carbon atoms, and does not comprise a phenanthrene group, a triphenylene group, or a 2-fluorenyl group,
where, when Ar1 is represented by Formula 3-3, Ar2 is a substituted or unsubstituted aryl group having 16 to 30 ring-forming carbon atoms, or a substituted or unsubstituted heteroaryl group having 2 to 30 ring-forming carbon atoms: 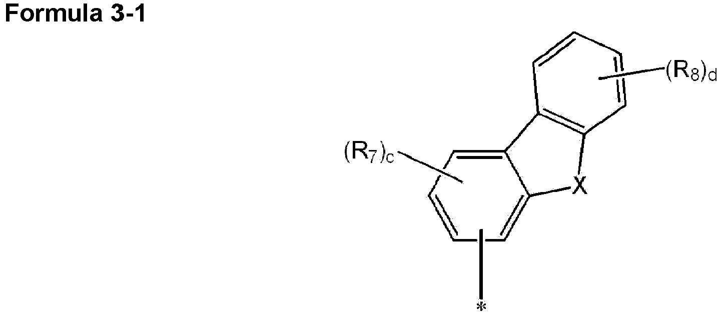 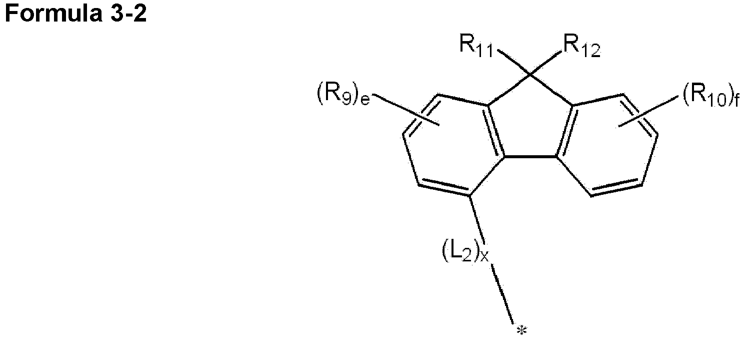 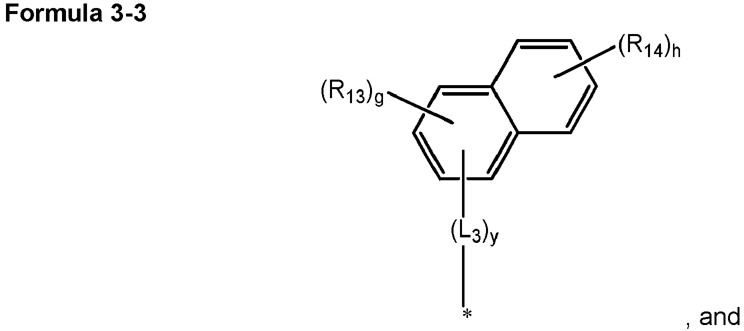 wherein, in Formula 3-1 to Formula 3-3,
X is O or S,
R₇ to R₁₂ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, or are bonded to an adjacent group to form a ring, and do not comprise an amine group,
R₁₃ and R₁₄ are each independently a hydrogen atom, a deuterium atom, a halogen atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, and do not comprise an amine group,
L₂ and L₃ are each independently a substituted or unsubstituted arylene group having 6 to 30 ring-forming carbon atoms, or a substituted or unsubstituted heteroarylene group having 2 to 30 ring-forming carbon atoms,
c, e, and g are each independently an integer of 0 to 3,
d, f, and h are each independently an integer of 0 to 4, and
x and y are each independently an integer of 0 to 3.
